(19) Europäisches Patentamt / European Patent Office / Office européen des brevets

(11) **EP 2 205 632 B1**

(12) **EUROPEAN PATENT SPECIFICATION**

(45) Date of publication and mention of the grant of the patent:
**16.11.2016 Bulletin 2016/46**

(21) Application number: **08838455.7**

(22) Date of filing: **03.10.2008**

(51) Int Cl.:
*C07K 16/18* (2006.01)     *A61K 39/395* (2006.01)

(86) International application number:
**PCT/US2008/011492**

(87) International publication number:
**WO 2009/048538 (16.04.2009 Gazette 2009/16)**

(54) **USE OF ANTI-AMYLOID BETA ANTIBODY IN OCULAR DISEASES**

VERWENDUNG EINES ANTI-AMYLOID-BETA-ANTIKÖRPERS BEI AUGENERKRANKUNGEN

UTILISATION D'ANTICORPS ANTI-BETA AMYLOIDE AUX MALADIES OCULAIRES

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **05.10.2007  US 960617 P**

(43) Date of publication of application:
**14.07.2010  Bulletin 2010/28**

(83) **Declaration under Rule 32(1) EPC (expert solution)**

(60) Divisional application:
**12189919.9 / 2 650 308**

(73) Proprietors:
• **Genentech, Inc.**
  **South San Francisco, CA 94080 (US)**
• **AC Immune S.A.**
  **1015 Lausanne (CH)**

(72) Inventors:
• **PFEIFER, Andrea**
  **CH-1806 St.-légier (CH)**
• **MUHS, Andreas**
  **CH-1053 Cugy (CH)**
• **WATTS, Ryan**
  **San Mateo**
  **CA 94402 (US)**
• **PIHLGREN, Maria**
  **1052 Mont-sur-Lausanne (CH)**

(74) Representative: **Vossius & Partner Patentanwälte Rechtsanwälte mbB Siebertstrasse 3 81675 München (DE)**

(56) References cited:
WO-A-2006/039327      WO-A-2006/083533
WO-A-2007/068412      WO-A-2008/011348
WO-A-2008/060364      WO-A-2008/061795
WO-A-2008/110885      WO-A-2008/156622
US-A1- 2003 190 689      US-B1- 6 737 056

• DING ET AL: "Targeting age-related macular degeneration with Alzheimer's disease based immunotherapies: Anti-amyloid-beta antibody attenuates pathologies in an age-related macular degeneration mouse model" VISION RESEARCH, PERGAMON PRESS, OXFORD, GB, vol. 48, no. 3, 20 September 2007 (2007-09-20), pages 339-345, XP022452227 ISSN: 0042-6989

• GUO LI ET AL: "Targeting amyloid-beta in glaucoma treatment" PROCEEDINGS OF THE NATIONAL ACADEMY OF SCIENCES OF USA, NATIONAL ACADEMY OF SCIENCE, WASHINGTON, DC.; US, vol. 104, no. 33, 1 August 2007 (2007-08-01), pages 13444-13449, XP002471873 ISSN: 0027-8424

• MCKINNON S J ET AL: "Caspase activation and amyloid precursor protein cleavage in rat ocular hypertension" INVESTIGATIVE OPHTHALMOLOGY AND VISUAL SCIENCE 2002 US, vol. 43, no. 4, 2002, pages 1077-1087, XP009120016 ISSN: 0146-0404

- Gregory A Nelson ET AL: "Ocular amyloidosis and secondary glaucoma", Ophthalmology, vol. 106, no. 7, 1 July 1999 (1999-07-01), pages 1363-1366, XP055143833, ISSN: 0161-6420, DOI: 10.1016/S0161-6420(99)00726-5
- JUAN MESA-GUTIERREZ ET AL: 'Primary localized conjunctival amyloidosis: A case report with a ten-year follow-up period' CLINICAL OPHTHALMOLOGY vol. 2, no. 3, 01 September 2008, pages 685 - 687, XP055143811 DOI: 10.2147/OPTH.S2884 ISSN: 1177-5467
- SHIELDS M BRUCE: "Normal-tension glaucoma: is it different from primary open-angle glaucoma?", CURRENT OPINION IN OPHTHALMOLOGY MAR 2008, vol. 19, no. 2, March 2008 (2008-03), pages 85-88, ISSN: 1040-8738
- NICOLL JAMES A R ET AL: "Neuropathology of human Alzheimer disease after immunization with amyloid-beta peptide: a case report.", NATURE MEDICINE APR 2003, vol. 9, no. 4, April 2003 (2003-04), pages 448-452, ISSN: 1078-8956
- NUCCI CARLO ET AL: "Glaucoma progression associated with altered cerebral spinal fluid levels of amyloid beta and tau proteins.", CLINICAL & EXPERIMENTAL OPHTHALMOLOGY APR 2011, vol. 39, no. 3, April 2011 (2011-04), pages 279-281, ISSN: 1442-9071

Remarks:

The file contains technical information submitted after the application was filed and not included in this specification

**SEQUENCE LISTING:**

<110> AC Immune

<120> Humanized Antibody

<130> 089667-0156

<141> 2008-10-03

<150> US 60/960,617
<151> 2007-10-05

<160> 32

<170> Patent In version 3.3

<210> 1
<211> 10
<212> PRT
<213> Mus musculus

<220>
<223> C2 HuVH AF 4 humanised heavy chain variable region (CDR1)

<400> 1

Gly Phe Thr Phe Ser Ser Tyr Gly Met Ser
1               5                       10

<210> 2
<211> 17
<212> PRT
<213> Mus musculus

<220>

<223>  C2 HuVH AF 4 humanised heavy chain variable region  (CDR2)

<400>  2

Ser Ile Asn Ser Asn Gly Gly Ser Thr Tyr Tyr Pro Asp Ser Val Lys
1               5                       10
15

Gly

<210> 3
<211> 3
<212> PRT
<213> Mus musculus

<220>
<223> C2 HuVH AF 4 humanised heavy chain variable region (CDR3)

<400> 3

Gly Asp Tyr
1

<210> 4
<211> 16
<212> PRT
<213> Mus musculus

<220>
<223> C2 HuVK 1 humanised light chain variable region (CDR1)

<400> 4

Arg Ser Ser Gln Ser Leu Val Tyr Ser Asn Gly Asp Thr Tyr Leu His
1               5                       10
15

<210> 5
<211> 7
<212> PRT
<213> Mus musculus

<220>
<223> C2 HuVK 1 humanised light chain variable region (CDR2)

<400> 5

Lys Val Ser Asn Arg Phe Ser
1                   5

<210> 6
<211> 9
<212> PRT
<213> Mus musculus

<220>

<223> C2 HuVK 1 humanised light chain variable region (CDR3)

<400> 6

Ser Gln Ser Thr His Val Pro Trp Thr
1                   5

<210> 7

<211> 6

<212> PRT

<213> Homo sapiens

<220>

<223> Aβ epitope, region 2

<400> 7

Val Phe Phe Ala Glu Asp
1                   5

<210> 8

<211> 5

<212> PRT

<213> Homo sapiens

<220>

<223> Aβ epitope, region 1

<400> 8

His Gln Lys Leu Val
1                   5

<210> 9

<211> 6

<212> PRT

<213> Homo sapiens

<220>

<221> misc_feature

<222> (1)..(1)

<223> Xaa can be Ala, Val, Leu, norleucine, Met, Phe, or Ile

<220>

<221> misc_feature

<222> (4)..(4)

<223> Xaa can be Ala, Val, Leu, Ser or Ile

<220>

<221> misc_feature

<222> (5)..(5)

<223> Xaa can be Glu or Asp

<220>

<221> misc_feature
<222> (6)..(6)
<223> Xaa can be Glu or Asp

<400> 9

```
Xaa Phe Phe Xaa Xaa Xaa
1                       5
```

<210> 10
<211> 5
<212> PRT
<213> Homo sapiens

<220>
<221> misc_feature
<222> (1)..(1)
<223> Xaa can be His, Asn, Gln Lys, or Arg

<220>
<221> misc_feature
<222> (2)..(2)
<223> Xaa can be Asn or Gln

<220>
<221> misc_feature
<222> (5)..(5)
<223> Xaa can be Ala, Val, Leu, norleucine, Met, Phe, or Ile

<400> 10

```
Xaa Xaa Lys Leu Xaa
1                   5
```

<210> 11
<211> 10
<212> PRT
<213> Homo sapiens

<220>
<221> misc_feature
<222> (1)..(1)
<223> Xaa can be His, Asn, Gln Lys, or Arg

<220>
<221> misc_feature
<222> (2)..(2)
<223> Xaa can be Asn or Gln

<220>
<221> misc_feature
<222> (5)..(5)
<223> Xaa can be Ala, Val, Leu, norleucine, Met, Phe, or Ile

<220>
<221> misc_feature
<222> (8)..(8)

<223> Xaa can be Ala, Val, Leu, Ser or Ile

<220>
<221> misc_feature
<222> (9)..(9)
<223> Xaa can be Glu or Asp

<220>
<221> misc_feature
<222> (10)..(10)
<223> Xaa can be Glu or Asp

<400> 11

```
        Xaa Xaa Lys Leu Xaa Phe Phe Xaa Xaa Xaa
        1               5                   10
```

<210> 12
<211> 112
<212> PRT
<213> Artificial sequence

<220>
<223> artificial humanized C2 HuVK 1 variable light chain

<400> 12

```
    Asp Ile Val Met Thr Gln Ser Pro Leu Ser Leu Pro Val Thr Pro Gly
    1               5                   10                  15

    Glu Pro Ala Ser Ile Ser Cys Arg Ser Ser Gln Ser Leu Val Tyr Ser
                    20                  25                      30

    Asn Gly Asp Thr Tyr Leu His Trp Tyr Leu Gln Lys Pro Gly Gln Ser
                35                  40                      45

    Pro Gln Leu Leu Ile Tyr Lys Val Ser Asn Arg Phe Ser Gly Val Pro
            50                  55                  60

    Asp Arg Phe Ser Gly Ser Gly Ser Gly Thr Asp Phe Thr Leu Lys Ile
    65                  70                  75                  80

    Ser Arg Val Glu Ala Glu Asp Val Gly Val Tyr Tyr Cys Ser Gln Ser
                    85                  90                  95

    Thr His Val Pro Trp Thr Phe Gly Gln Gly Thr Lys Val Glu Ile Lys
                    100                 105                     110
```

<210> 13

<211> 219
<212> PRT
<213> artificial sequence

<220>
<223> artificial humanized C2 light chain

<400> 13

```
Asp Ile Val Met Thr Gln Ser Pro Leu Ser Leu Pro Val Thr Pro Gly
1               5                   10
15

Glu Pro Ala Ser Ile Ser Cys Arg Ser Ser Gln Ser Leu Val Tyr Ser
                20                  25                          30

Asn Gly Asp Thr Tyr Leu His Trp Tyr Leu Gln Lys Pro Gly Gln Ser
            35                  40                          45

Pro Gln Leu Leu Ile Tyr Lys Val Ser Asn Arg Phe Ser Gly Val Pro
        50                  55                  60

Asp Arg Phe Ser Gly Ser Gly Ser Gly Thr Asp Phe Thr Leu Lys Ile
65                  70                  75
80

Ser Arg Val Glu Ala Glu Asp Val Gly Val Tyr Tyr Cys Ser Gln Ser
                85                      90
95

Thr His Val Pro Trp Thr Phe Gly Gln Gly Thr Lys Val Glu Ile Lys
            100                 105                         110

Arg Thr Val Ala Ala Pro Ser Val Phe Ile Phe Pro Pro Ser Asp Glu
        115                 120                         125

Gln Leu Lys Ser Gly Thr Ala Ser Val Val Cys Leu Leu Asn Asn Phe
        130                 135                         140

Tyr Pro Arg Glu Ala Lys Val Gln Trp Lys Val Asp Asn Ala Leu Gln
145                 150                         155
160

Ser Gly Asn Ser Gln Glu Ser Val Thr Glu Gln Asp Ser Lys Asp Ser
                165                         170
175
```

Thr Tyr Ser Leu Ser Ser Thr Leu Thr Leu Ser Lys Ala Asp Tyr Glu
                180                     185                     190

Lys His Lys Val Tyr Ala Cys Glu Val Thr His Gln Gly Leu Ser Ser
            195                     200                     205

Pro Val Thr Lys Ser Phe Asn Arg Gly Glu Cys
            210                 215

<210> 14
<211> 107
<212> PRT
<213> artificial sequence

<220>
<223> artificial humanized C2 light chain constant region

<400> 14

Arg Thr Val Ala Ala Pro Ser Val Phe Ile Phe Pro Pro Ser Asp Glu
1                   5                   10
15

Gln Leu Lys Ser Gly Thr Ala Ser Val Val Cys Leu Leu Asn Asn Phe
                20                      25                      30

Tyr Pro Arg Glu Ala Lys Val Gln Trp Lys Val Asp Asn Ala Leu Gln
            35                      40                      45

Ser Gly Asn Ser Gln Glu Ser Val Thr Glu Gln Asp Ser Lys Asp Ser
            50                      55                      60

Thr Tyr Ser Leu Ser Ser Thr Leu Thr Leu Ser Lys Ala Asp Tyr Glu
65                      70                      75
80

Lys His Lys Val Tyr Ala Cys Glu Val Thr His Gln Gly Leu Ser Ser

                                        85                              90

                95

                Pro Val Thr Lys Ser Phe Asn Arg Gly Glu Cys
                                    100                    105

<210> 15
<211> 112
<212> PRT
<213> artificial sequence

<220>
<223> artificial humanized C2 HuVH AF 4 variable heavy chain

<400> 15

    Glu Val Gln Leu Val Glu Ser Gly Gly Gly Leu Val Gln Pro Gly Gly
    1               5                   10

    15

    Ser Leu Arg Leu Ser Cys Ala Ala Ser Gly Phe Thr Phe Ser Ser Tyr
                    20                  25                      30

    Gly Met Ser Trp Val Arg Gln Ala Pro Gly Lys Gly Leu Glu Leu Val
                35                  40                      45

    Ala Ser Ile Asn Ser Asn Gly Gly Ser Thr Tyr Tyr Pro Asp Ser Val
                50                  55                  60

    Lys Gly Arg Phe Thr Ile Ser Arg Asp Asn Ala Lys Asn Ser Leu Tyr

65       70       75

80

Leu Gln Met Asn Ser Leu Arg Ala Glu Asp Thr Ala Val Tyr Tyr Cys
       85       90

95

Ala Ser Gly Asp Tyr Trp Gly Gln Gly Thr Thr Val Thr Val Ser Ser
       100       105       110

<210> 16
<211> 439
<212> PRT
<213> artificial sequence

<220>
<223> artificial humanized C2 heavy chain

<400> 16

Glu Val Gln Leu Val Glu Ser Gly Gly Gly Leu Val Gln Pro Gly Gly
1       5       10

15

Ser Leu Arg Leu Ser Cys Ala Ala Ser Gly Phe Thr Phe Ser Ser Tyr
       20       25       30

Gly Met Ser Trp Val Arg Gln Ala Pro Gly Lys Gly Leu Glu Leu Val
       35       40       45

Ala Ser Ile Asn Ser Asn Gly Gly Ser Thr Tyr Tyr Pro Asp Ser Val
            50                      55                      60

Lys Gly Arg Phe Thr Ile Ser Arg Asp Asn Ala Lys Asn Ser Leu Tyr
65                      70                      75
80

Leu Gln Met Asn Ser Leu Arg Ala Glu Asp Thr Ala Val Tyr Tyr Cys
                        85                      90
95

Ala Ser Gly Asp Tyr Trp Gly Gln Gly Thr Thr Val Thr Val Ser Ser
                100                     105                         110

Ala Ser Thr Lys Gly Pro Ser Val Phe Pro Leu Ala Pro Cys Ser Arg
            115                     120                     125

Ser Thr Ser Glu Ser Thr Ala Ala Leu Gly Cys Leu Val Lys Asp Tyr
            130                     135                     140

Phe Pro Glu Pro Val Thr Val Ser Trp Asn Ser Gly Ala Leu Thr Ser
145                     150                     155
160

Gly Val His Thr Phe Pro Ala Val Leu Gln Ser Ser Gly Leu Tyr Ser
                        165                     170
175

Leu Ser Ser Val Val Thr Val Pro Ser Ser Ser Leu Gly Thr Lys Thr
                180                     185                      190

Tyr Thr Cys Asn Val Asp His Lys Pro Ser Asn Thr Lys Val Asp Lys
            195                     200                     205

Arg Val Glu Ser Lys Tyr Gly Pro Pro Cys Pro Pro Cys Pro Ala Pro
        210                     215                     220

Glu Phe Leu Gly Gly Pro Ser Val Phe Leu Phe Pro Pro Lys Pro Lys
225                     230                     235
240

Asp Thr Leu Met Ile Ser Arg Thr Pro Glu Val Thr Cys Val Val Val
                245                     250
255

Asp Val Ser Gln Glu Asp Pro Glu Val Gln Phe Asn Trp Tyr Val Asp
                260                     265                         270

Gly Val Glu Val His Asn Ala Lys Thr Lys Pro Arg Glu Glu Gln Phe
            275                     280                     285

Asn Ser Thr Tyr Arg Val Val Ser Val Leu Thr Val Leu His Gln Asp
            290                     295                     300

Trp Leu Asn Gly Lys Glu Tyr Lys Cys Lys Val Ser Asn Lys Gly Leu

```
                  305                     310                          315
                  320
```

```
Pro Ser Ser Ile Glu Lys Thr Ile Ser Lys Ala Lys Gly Gln Pro Arg
                          325                     330
335
```

```
Glu Pro Gln Val Tyr Thr Leu Pro Pro Ser Gln Glu Glu Met Thr Lys
                  340                     345                         350
```

```
Asn Gln Val Ser Leu Thr Cys Leu Val Lys Gly Phe Tyr Pro Ser Asp
                      355                     360                     365
```

```
Ile Ala Val Glu Trp Glu Ser Asn Gly Gln Pro Glu Asn Asn Tyr Lys
              370                     375                     380
```

```
Thr Thr Pro Pro Val Leu Asp Ser Asp Gly Ser Phe Phe Leu Tyr Ser
385                     390                         395
400
```

```
Arg Leu Thr Val Asp Lys Ser Arg Trp Gln Glu Gly Asn Val Phe Ser
                          405                     410
415
```

```
Cys Ser Val Met His Glu Ala Leu His Asn His Tyr Thr Gln Lys Ser
                      420                     425                     430
```

```
Leu Ser Leu Ser Leu Gly Lys
```

435

<210> 17
<211> 326
<212> PRT
<213> Homo sapiens

<220>
<223> IG GAMMA-4 CHAIN C REGION - modified

<400> 17

Ala Ser Thr Lys Gly Pro Ser Val Phe Pro Leu Ala Pro Cys Ser Arg
1               5                   10
15

Ser Thr Ser Glu Ser Thr Ala Ala Leu Gly Cys Leu Val Lys Asp Tyr
            20                  25                              30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
        50                  55                      60

Leu Ser Ser Val Val Thr Val Pro Ser Ser Ser Leu Gly Thr Lys Thr
65                      70                  75
80

Tyr Thr Cys Asn Val Asp His Lys Pro Ser Asn Thr Lys Val Asp Lys
                        85                  90
95

Arg Val Glu Ser Lys Tyr Gly Pro Pro Cys Pro Pro Cys Pro Ala Pro

100       105       110

Glu Phe Leu Gly Gly Pro Ser Val Phe Leu Phe Pro Pro Lys Pro Lys
     115         120         125

Asp Thr Leu Met Ile Ser Arg Thr Pro Glu Val Thr Cys Val Val Val
    130          135         140

Asp Val Ser Gln Glu Asp Pro Glu Val Gln Phe Asn Trp Tyr Val Asp
145         150         155
160

Gly Val Glu Val His Asn Ala Lys Thr Lys Pro Arg Glu Glu Gln Phe
        165         170
175

Asn Ser Thr Tyr Arg Val Val Ser Val Leu Thr Val Leu His Gln Asp
       180         185         190

Trp Leu Asn Gly Lys Glu Tyr Lys Cys Lys Val Ser Asn Lys Gly Leu
     195         200         205

Pro Ser Ser Ile Glu Lys Thr Ile Ser Lys Ala Lys Gly Gln Pro Arg
    210         215         220

Glu Pro Gln Val Tyr Thr Leu Pro Pro Ser Gln Glu Glu Met Thr Lys
225         230         235
240

```
Asn Gln Val Ser Leu Thr Cys Leu Val Lys Gly Phe Tyr Pro Ser Asp
                        245                     250
255
```

```
Ile Ala Val Glu Trp Glu Ser Asn Gly Gln Pro Glu Asn Asn Tyr Lys
                        260                 265                     270
```

```
Thr Thr Pro Pro Val Leu Asp Ser Asp Gly Ser Phe Phe Leu Tyr Ser
            275                 280               285
```

```
Arg Leu Thr Val Asp Lys Ser Arg Trp Gln Glu Gly Asn Val Phe Ser
        290                 295                 300
```

```
Cys Ser Val Met His Glu Ala Leu His Asn His Tyr Thr Gln Lys Ser
305                     310                 315
320
```

```
Leu Ser Leu Ser Leu Gly
                325
```

<210> 18
<211> 51
<212> DNA
<213> Mus musculus

<220>
<223> C2 HuVH AF 4 humanised heavy chain variable region (CDR2)

<400> 18

```
agcatcaata gtaatggtgg tagcacctat tatccagaca gtgtgaaggg c
51
```

<210> 19
<211> 9
<212> DNA
<213> Mus musculus

<220>

<223> C2 HuVH AF 4 humanised heavy chain variable region (CDR3)

<400> 19

ggtgactac

9

<210> 20
<211> 49
<212> DNA
<213> Mus musculus

<220>
<223> C2 HuVK 1 humanised light chain variable region (CDR1)

<400> 20

agatctagtc agagccttgt atatagtaat ggagacacct atttacatt

49

<210> 21
<211> 635
<212> DNA
<213> artificial sequence

<220>
<223> artificial humanized C2 Hu VK 1 variable light chain

<400> 21

```
gatattgtga tgacccaatc tccactctcc ctgcctgtca ctcctggtga gcctgcctcc
60

atctcttgca gatctagtca gagccttgta tatagtaatg gagacaccta tttacattgg
120

tacctgcaga agccaggcca gtctccacag ctcctgatct acaaagtttc caaccgattt
180

tctggggtcc cagacaggtt cagtggcagt ggatcaggga cagatttcac actcaagatc
240

agcagagtgg aggctgagga tgtgggagtt tattactgct ctcaaagtac acatgttcct
300

tggacgttcg gccaaggcac caaggtggaa atcaaa
336
```

<210> 22
<211> 657
<212> DNA
<213> artificial sequence

<220>
<223> artificial humanized C2 light chain

<400> 22

```
gatattgtga tgacccaatc tccactctcc ctgcctgtca ctcctggtga gcctgcctcc
60
```

atctcttgca gatctagtca gagccttgta tatagtaatg gagacaccta tttacattgg

120

tacctgcaga agccaggcca gtctccacag ctcctgatct acaaagtttc caaccgattt

180

tctggggtcc cagacaggtt cagtggcagt ggatcaggga cagatttcac actcaagatc

240

agcagagtgg aggctgagga tgtgggagtt tattactgct ctcaaagtac acatgttcct

300

tggacgttcg gccaaggcac caaggtggaa atcaaaagga ctgtggctgc accatctgtc

360

ttcatcttcc cgccatctga tgagcagttg aaatctggaa ctgcctctgt tgtgtgcctg

420

ctgaataact tctatcccag agaggccaaa gtacagtgga aggtggataa cgccctccaa

480

tcgggtaact cccaggagag tgtcacagag caggacagca aggacagcac ctacagcctc

540

agcagcaccc tgacgctgag caaagcagac tacgagaaac acaaagtcta cgcctgcgaa

600

gtcacccatc agggcctgag ctcgcccgtc acaaagagct tcaacagggg agagtgt

657

<210> 23
<211> 321
<212> DNA
<213> Homo sapiens

<220>
<223> artificial humanized C2 light chain constant region

<400> 23

```
aggactgtgg ctgcaccatc tgtcttcatc ttcccgccat ctgatgagca gttgaaatct
60

ggaactgcct ctgttgtgtg cctgctgaat aacttctatc ccagagaggc caaagtacag
120

tggaaggtgg ataacgccct ccaatcgggt aactcccagg agagtgtcac agagcaggac
180

agcaaggaca gcacctacag cctcagcagc accctgacgc tgagcaaagc agactacgag
240

aaacacaaag tctacgcctg cgaagtcacc catcagggcc tgagctcgcc cgtcacaaag
300

agcttcaaca ggggagagtg t
321
```

<210> 24
<211> 798
<212> DNA
<213> artificial sequence

<220>
<223> artificial humanized C2 HuVH AF variable heavy chain

<400> 24

```
gaggtgcagc tggtcgagtc tggggggaggc ttagtgcagc ctggagggtc cctgagactc
60

tcctgtgcag cctctggatt cactttcagt agctatggca tgtcttgggt tcgccaggct
120

ccaggcaagg gtctcgaatt ggtcgcaagc atcaatagta atggtggtag cacctattat
180

ccagacagtg tgaagggccg attcaccatc tccagagaca tgccaagaa ctccctgtac
240

ctgcaaatga cagtctgag agctgaggac accgccgtgt attactgtgc aagtggtgac
300

tactggggcc aaggcaccac tgtcacagtc tcctca
336
```

<210> 25
<211> 1317
<212> DNA
<213> artificial sequence

<220>
<223> artificial humanized C2 heavy chain

<400> 25

```
gaggtgcagc tggtcgagtc tggggggaggc ttagtgcagc ctggagggtc cctgagactc
60

tcctgtgcag cctctggatt cactttcagt agctatggca tgtcttgggt tcgccaggct
120

ccaggcaagg gtctcgaatt ggtcgcaagc atcaatagta atggtggtag cacctattat
180

ccagacagtg tgaagggccg attcaccatc tccagagaca atgccaagaa ctccctgtac
240

ctgcaaatga acagtctgag agctgaggac accgccgtgt attactgtgc aagtggtgac
300

tactggggcc aaggcaccac tgtcacagtc tcctcagctt ccaccaaggg cccatccgtc
360

ttccccctgg cgccctgctc cagatcgacc tccgagagca cagccgccct gggctgcctg
420

gtcaaggact acttccccga accggtgacg gtgtcgtgga actcaggcgc cctgaccagc
480

ggcgtgcaca ccttccgggc tgtcctacag tcctcaggac tctactccct cagcagcgtg
540

gtgaccgtgc cctccagcag cttgggcacg aagacctaca cctgcaacgt agatcacaag
600

cccagcaaca ccaaggtgga caagagagtt gagtccaaat atggtccccc gtgtccccca
660

tgcccagcac ctgagttcct ggggggacca tcagtcttcc tgttcccccc aaaacccaag
720

gacactctca tgatctcccg gacccctgag gtcacgtgcg tggtggtgga cgtgagccag
780

gaagaccccg aggtccagtt caactggtac gtggatggcg tggaggtgca taatgccaag
840

acaaagccgc gggaggagca gttcaacagc acgtaccgtg tggtcagcgt cctcaccgtc
900

ctgcaccagg actggctgaa cggcaaggag tacaagtgca aggtctccaa caaaggcctc
960

ccgtcctcca tcgagaaaac catctccaaa gccaaagggc agccccgaga gccacaggtg
1020
```

```
tacaccctgc ccccatccca ggaggagatg accaagaacc aggtcagcct gacctgcctg
1080

gtcaaaggct tctaccccag cgacatcgcc gtggagtggg agagcaatgg gcagccggag
1140

aacaactaca agaccacgcc tcccgtcctc gattccgacg gctccttctt cctctacagc
1200

aggctaaccg tggacaagag caggtggcag gaggggaatg tcttctcatg ctccgtgatg
1260

catgaggctc tgcacaacca ctacacacag aagagcctct ccctgtctct gggtaaa
1317
```

<210> 26
<211> 981
<212> DNA
<213> Homo sapiens

<220>
<223> artificial humanized C2 heavy chain constant region

<400> 26

```
gcttccacca agggcccatc cgtcttcccc ctggcgccct gctccagatc gacctccgag
60

agcacagccg ccctgggctg cctggtcaag gactacttcc ccgaaccggt gacggtgtcg
120

tggaactcag gcgccctgac cagcggcgtg cacaccttcc cggctgtcct acagtcctca
180

ggactctact ccctcagcag cgtggtgacc gtgccctcca gcagcttggg cacgaagacc
240

tacacctgca acgtagatca caagcccagc aacaccaagg tggacaagag agttgagtcc
300

aaatatggtc ccccgtgtcc cccatgccca gcacctgagt tcctggggggg accatcagtc
360

ttcctgttcc ccccaaaacc caaggacact ctcatgatct cccggacccc tgaggtcacg
420

tgcgtggtgg tggacgtgag ccaggaagac cccgaggtcc agttcaactg gtacgtggat
480

ggcgtggagg tgcataatgc caagacaaag ccgcgggagg agcagttcaa cagcacgtac
540

cgtgtggtca gcgtcctcac cgtcctgcac caggactggc tgaacggcaa ggagtacaag
600

tgcaaggtct ccaacaaagg cctcccgtcc tccatcgaga aaaccatctc caaagccaaa
660
```

gggcagcccc gagagccaca ggtgtacacc ctgcccccat cccaggagga gatgaccaag
720

aaccaggtca gcctgacctg cctggtcaaa ggcttctacc ccagcgacat cgccgtggag
780

tgggagagca atgggcagcc ggagaacaac tacaagacca cgcctcccgt cctcgattcc
840

gacggctcct tcttcctcta cagcaggcta accgtggaca agagcaggtg caggagggg
900

aatgtcttct catgctccgt gatgcatgag gctctgcaca accactacac acagaagagc
960

ctctccctgt ctctgggtaa a
981

<210> 27
<211> 112
<212> PRT
<213> Mus musculus

<400> 27

Asp Val Val Met Thr Gln Thr Pro Leu Ser Leu Pro Val Ser Leu Gly
1               5                   10              15

Asp Gln Ala Ser Ile Ser Cys Arg Ser Ser Gln Ser Leu Val Tyr Ser
            20                  25                  30

Asn Gly Asp Thr Tyr Leu His Trp Tyr Leu Gln Lys Pro Gly Gln Ser
            35                  40                  45

Pro Lys Leu Leu Ile Tyr Lys Val Ser Asn Arg Phe Ser Gly Val Pro
        50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                        85                  90
95

Thr His Val Pro Trp Thr Phe Gly Gly Gly Thr Lys Leu Glu Ile Lys
                100                 105                 110

<210> 28
<211> 112
<212> PRT
<213> Mus musculus

86

<400> 28

```
Glu Val Gln Leu Val Glu Ser Gly Gly Gly Leu Val Gln Pro Gly Gly
1               5                   10
15

Ser Leu Lys Leu Ser Cys Ala Ala Ser Gly Phe Thr Phe Ser Ser Tyr
                20                  25                      30

Gly Met Ser Trp Val Arg Gln Thr Pro Asp Lys Arg Leu Glu Leu Val
            35                  40                  45

Ala Ser Ile Asn Ser Asn Gly Gly Ser Thr Tyr Tyr Pro Asp Ser Val
            50                  55                  60

Lys Gly Arg Phe Thr Ile Ser Arg Asp Asn Ala Lys Asn Thr Leu Tyr
65                      70                  75
80

Leu Gln Met Ser Ser Leu Lys Ser Glu Asp Thr Ala Met Tyr Tyr Cys
                85                  90
95

Ala Ser Gly Asp Tyr Trp Gly Gln Gly Ser Thr Leu Thr Val Ser Ser
            100                 105                     110
```

<210> 29
<211> 336
<212> DNA
<213> Mus musculus

<400> 29

gatgttgtga tgacccaaac tccactctcc ctgcctgtca gtcttggaga tcaagcctcc 60

atctcttgca gatctagtca gagccttgta tatagtaatg gagacaccta tttacattgg 120

tacctgcaga agccaggcca gtctccaaag ctcctgatct acaaagtttc caaccgattt 180

tctggggtcc cagacaggtt cagtggcagt ggatcaggga cagatttcac actcaagatc 240

agcagagtgg aggctgagga tctgggagtt tatttctgct ctcaaagtac acatgttcct 300

tggacgttcg gtggaggcac caagctagaa atcaaa 336

<210> 30
<211> 417
<212> DNA
<213> Mus musculus

<400> 30

atgaagttgc ctgttaggct gttggtgctg atgttctgga ttcctgcttc cagcagtgat 60

gttgtgatga cccaaactcc actctccctg cctgtcagtc ttggagatca agcctccatc 120

tcttgcagat ctagtcagag ccttgtatat agtaatggag acacctattt acattggtac 180

```
ctgcagaagc caggccagtc tccaaagctc ctgatctaca aagtttccaa ccgattttct
240

ggggtcccag acaggttcag tggcagtgga tcagggacag atttcacact caagatcagc
300

agagtggagg ctgaggatct gggagtttat ttctgctctc aaagtacaca tgttccttgg
360

acgttcggtg gaggcaccaa gctagaaatc aaacgggctg atgctgcacc aactgta
417
```

<210> 31
<211> 336
<212> DNA
<213> Mus musculus

<400> 31

```
gaggtgcagc tggtggagtc tggggggaggc ttagtgcagc ctggagggtc cctgaaactc
60

tcctgtgcag cctctggatt cactttcagt agctatggca tgtcttgggt tcgccagact
120

ccagacaaga ggctggaatt ggtcgcaagc atcaatagta atggtggtag cacctattat
180

ccagacagtg tgaagggccg attcaccatc tccagagaca atgccaagaa caccctgtac
240

ctgcaaatga gcagtctgaa gtctgaggac acagccatgt attactgtgc aagtggtgac
300

          tactggggcc aaggctccac tctcacagtc tcctca
          336
```

<210> 32
<211> 408
<212> DNA
<213> Mus musculus

<400> 32

```
atgrasttsg ggytcagmtt grttttcctt gcccttattt taaaaggtgt ccaatgtgag
60

gtgcagctgg tggagtctgg gggaggctta gtgcagcctg gagggtccct gaaactctcc
120

tgtgcagcct ctggattcac tttcagtagc tatggcatgt cttgggttcg ccagactcca
180

gacaagaggc tggaattggt cgcaagcatc aatagtaatg gtggtagcac ctattatcca
240

gacagtgtga agggccgatt caccatctcc agagacaatg ccaagaacac cctgtacctg
300

caaatgagca gtctgaagtc tgaggacaca gccatgtatt actgtgcaag tggtgactac
360

tggggccaag ctccactct cacagtctcc tcagccaaaa caacaccc
408
```

**Description**

**CROSS-REFERENCE TO RELATED PATENT APPLICATIONS**

This application claims priority to United States Provisional Application Serial No. 60/960,617, filed on October 5, 2007, which is incorporated herein by reference.

**BACKGROUND OF THE INVENTION**

The present invention is related to methods and compositions for diagnosis and treatment of amyloidosis, a group of disorders and abnormalities associated with amyloid protein such as Alzheimer's disease.

Amyloidosis is not a single disease entity, but rather a diverse group of progressive disease processes characterized by extracellular tissue deposits of a waxy, starch-like protein called amyloid, which accumulates in one or more organs or body systems. As the amyloid deposits accumulate, they begin to interfere with the normal function of the organ or body system. There are at least 15 different types of amyloidosis. The major forms are primary amyloidosis without known antecedent, secondary amyloidosis following some other condition, and hereditary amyloidosis.

Secondary amyloidosis occurs during chronic infection or inflammatory disease, such as tuberculosis, a bacterial infection called familial Mediterranean fever, bone infections (osteomyelitis), rheumatoid arthritis, inflammation of the small intestine (granulomatous ileitis), Hodgkin's disease, and leprosy.

Amyloid deposits include amyloid P (pentagonal) component (AP), a glycoprotein related to normal serum amyloid P (SAP), and sulphated glycosaminoglycans (GAG), complex carbohydrates of connective tissue. Amyloid protein fibrils, which account for about 90% of the amyloid material, comprise one of several different types of proteins. These proteins are capable of folding into so-called "beta-pleated" sheet fibrils, a unique protein configuration which exhibits binding sites for Congo red resulting in the unique staining properties of the amyloid protein.

Many diseases of aging are based on or associated with amyloid-like proteins and are characterized, in part, by the buildup of extracellular deposits of amyloid or amyloid-like material that contribute to the pathogenesis, as well as the progression of the disease. These diseases include, but are not limited to, neurological disorders such as Alzheimer's Disease (AD), Lewy body dementia (LBD), Down's syndrome, hereditary cerebral hemorrhage with amyloidosis (Dutch type); the Guam Parkinson-Dementia complex. Other diseases which are based on or associated with amyloid-like proteins are progressive supranuclear palsy, multiple sclerosis; Creutzfeld Jacob disease, Parkinson's disease, HIV-related dementia, ALS (amyotropic lateral sclerosis), Adult Onset Diabetes, senile cardiac amyloidosis, endocrine tumors, and others, including ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration, and macular degeneration, for example age-related macular degeneration; the optic nerve leading to optic nerve drusen, optic neuropathy and optic neuritis; and the cornea leading to lattice dystrophy.

Although pathogenesis of these diseases may be diverse, their characteristic deposits often contain many shared molecular constituents. To a significant degree, this may be attributable to the local activation of pro-inflammatory pathways thereby leading to the concurrent deposition of activated complement components, acute phase reactants, immune modulators, and other inflammatory mediators (McGeer et al., 1994).

Alzheimer's Disease (AD) is a neurological disorder primarily thought to be caused by amyloid plaques, an accumulation of abnormal deposit of proteins in the brain. The most frequent type of amyloid found in the brain of affected individuals is composed primarily of A$\beta$ fibrils. Scientific evidence demonstrates that an increase in the production and accumulation of beta-amyloid protein in plaques leads to nerve cell death, which contributes to the development and progression of AD. Loss of nerve cells in strategic brain areas, in turn, causes reduction in the neurotransmitters and impairment of memory. The proteins principally responsible for the plaque build up include amyloid precursor protein (APP) and two presenilins (presenilin I and presenilin II). Sequential cleavage of the amyloid precursor protein (APP), which is constitutively expressed and catabolized in most cells, by the enzymes $\beta$ and $\gamma$ secretase leads to the release of a 39 to 43 amino acid A$\beta$ peptide. The degradation of APPs likely increases their propensity to aggregate in plaques. It is especially the A$\beta$(1-42) fragment that has a high propensity of building aggregates due to two very hydrophobic amino acid residues at its C-terminus. The A$\beta$(1-42) fragment is therefore believed to be mainly involved and responsible for the initiation of neuritic plaque formation in AD and to have, therefore, a high pathological potential. There is therefore a need for agents to prevent the formation of amyloid plaques and to diffuse existing plaques in AD.

The symptoms of AD manifest slowly and the first symptom may only be mild forgetfulness. In this stage, individuals may forget recent events, activities, the names of familiar people or things and may not be able to solve simple math problems. As the disease progresses, symptoms are more easily noticed and become serious enough to cause people with AD or their family members to seek medical help. Mid-stage symptoms of AD include forgetting how to do simple tasks such as grooming, and problems develop with speaking, understanding, reading, or writing. Later stage AD patients may become anxious or aggressive, may wander away from home and ultimately need total care.

Presently, the only definite way to diagnose AD is to identify plaques and tangles in brain tissue in an autopsy after death of the individual. Therefore, doctors can only make a diagnosis of "possible" or "probable" AD while the person is still alive. Using current methods, physicians can diagnose AD correctly up to 90 percent of the time using several tools to diagnose "probable" AD. Physicians ask questions about the person's general health, past medical problems, and the history of any difficulties the person has carrying out daily activities. Behavioral tests of memory, problem solving, attention, counting, and language provide information on cognitive degeneration and medical tests such as tests of blood, urine, or spinal fluid, and brain scans can provide some further information.

The management of AD consists of medication-based and non-medication based treatments. Treatments aimed at changing the underlying course of the disease (delaying or reversing the progression) have so far been largely unsuccessful. Medicines that restore the deficit (defect), or malfunctioning, in the chemical messengers of the nerve cells (neurotransmitters), in particular the cholinesterase inhibitors (ChEIs) such as tacrine and rivastigmine, have been shown to improve symptoms. ChEIs impede the enzymatic degradation of neurotransmitters thereby increasing the amount of chemical messengers available to transmit the nerve signals in the brain.

For some people in the early and middle stages of the disease, the drugs tacrine (COGNEX®, Morris Plains, NJ), donepezil (ARICEPT®, Tokyo, JP), rivastigmine (EXELON®, East Hanover, NJ), or galantamine (REMINYL®, New Brunswick, NJ) may help prevent some symptoms from becoming worse for a limited time. Another drug, memantine (NAMENDA®, New York, NY), has been approved for treatment of moderate to severe AD. Medications are also available to address the psychiatric manifestations of AD. Also, some medicines may help control behavioral symptoms of AD such as sleeplessness, agitation, wandering, anxiety, and depression. Treating these symptoms often makes patients more comfortable and makes their care easier for caregivers. Unfortunately, despite significant treatment advances showing that this class of agents is consistently better than a placebo, the disease continues to progress, and the average effect on mental functioning has only been modest. Many of the drugs used in AD medication such as, for example, ChEIs also have side effects that include gastrointestinal dysfunction, liver toxicity and weight loss.

Cortical visual deficits are often associated with AD, despite the negative finding of impaired visual acuity or ocular disease. Post-mortem evidence from AD patients has shown pathological changes in the pre-cortical visual structures and a reduction in optic nerve fibers.

Visual processing dysfunction in AD is also associated with neurological changes and pathology within the ventral pathway, that extend from the retina with the P-ganglion cells through the parvocelluar layers of the lateral geniculate nucleus (LGN) reaching the inferotemporal cortex (IT), and the dorsal pathway, that extends from the retina with the M-ganglion cells through the magnocellular layers of the LGN, reaching the middle temporal cortex. Senile plaques of AD patients create abnormalities and dysfunctions within these cortical regions. Senile plaques also cause a loss in visual perception tasks, such as a dysfunction in the facial recognition of familiar people, a condition known as prosopagnosia.

Other diseases that are based on or associated with the accumulation and deposit of amyloid-like protein are mild cognitive impairment, Lewy body dementia (LBD), Down's Syndrome (trisomy 21), amyotrophic lateral sclerosis (ALS), inclusion-body myositis (IBM), and ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

Mild cognitive impairment (MCI) is a general term most commonly defined as a subtle but measurable memory disorder. A person with MCI experiences memory problems greater than normally expected with aging, but does not show other symptoms of dementia, such as impaired judgment or reasoning. MCI is a condition that frequently reflects a preclinical stage of AD.

The deposition of $\beta$-amyloid within the entorhinal cortex (EC) is believed to play a key role in the development of mild cognitive impairment (MCI) in the elderly. This is in line with the observation that the CSF-A A$\beta$(1-42) levels decline significantly once AD becomes clinically overt. In contrast to CSF- A$\beta$(1-42) CSF-tau levels are significantly increased in the MCI stage, and these values continue to be elevated thereafter, indicating that increased levels of CSF-tau may help in detecting MCI subjects who are predicted to develop AD.

Lewy body dementia (LBD) is a neurodegenerative disorder that can occur in persons older than 65 years of age, which typically causes symptoms of cognitive (thinking) impairment and abnormal behavioural changes. Symptoms can include cognitive impairment, neurological signs, sleep disorder, and autonomic failure. Cognitive impairment is the presenting feature of LBD in most cases. Patients have recurrent episodes of confusion that progressively worsen. The fluctuation in cognitive ability is often associated with shifting degrees of attention and alertness. Cognitive impairment and fluctuations of thinking may vary over minutes, hours, or days.

Lewy bodies are formed from phosphorylated and nonphosphorylated neurofilament proteins; they contain the synaptic protein alpha-synuclein as well as ubiquitin, which is involved in the elimination of damaged or abnormal proteins. In addition to Lewy Bodies, Lewy neurites, which are inclusion bodies in the cell processes of the nerve cells, may also be present. Amyloid plaques may form in the brains of patients afflicted with LBD, however they tend to be fewer in number than seen in patients with Alzheimer's disease. Neurofibrillary tangles, the other micropathological hallmark of AD, are not a main characteristic of LBD but are frequently present in addition to amyloid plaques.

Down's Syndrome (DS) or trisomy 21 is a genetic disorder caused by the presence of all or part of an extra chromosome 21 and is often associated with some impairment of cognitive ability and physical growth. DS is characterized by premature aging: most individuals affected by the disease develop Alzheimer's disease in their fifth decade, including deposits of the plaque-forming protein amyloid-beta that are often more severe than in most other Alzheimer's patients. Furthermore, many people with DS develop cataracts beginning in childhood and many suffer from congenital glaucoma. In humans, the gene for amyloid precursor protein, which is cleaved to form amyloid-beta, is located on chromosome 21. In individuals affected by DS both soluble and intracellular beta-amyloid accumulate before extracellular beta-amyloid, which is responsible for the formation of senile plaques. Increases in beta-amyloid levels in Down's Syndrome may reflect the increased expression and protein levels of beta-amyloid precursor protein cleavage enzyme 2 on chromosome 21.

Amyotrophic lateral sclerosis (ALS) is characterized by degeneration of upper and lower motor neurons. In some ALS patients, dementia or aphasia may be present (ALS-D). The dementia is most commonly a frontotemporal dementia (FTD), and many of these cases have ubiquitin-positive, tau-negative inclusions in neurons of the dentate gyrus and superficial layers of the frontal and temporal lobes.

Inclusion-body myositis (IBM) is a crippling disease usually found in people over age 50, in which muscle fibers develop inflammation and begin to atrophy-but in which the brain is spared and patients retain their full intellect. Two enzymes involved in the production of amyloid-ß protein were found to be increased inside the muscle cells of patients with this most common, progressive muscle disease of older people, in which amyloid-ß is also increased.

Another disease that is based on or associated with the accumulation and deposit of amyloid-like protein is macular degeneration.

Macular degeneration is a common eye disease that causes deterioration of the macula, which is the central area of the retina (the paper-thin tissue at the back of the eye where light-sensitive cells send visual signals to the brain). Sharp, clear, 'straight ahead' vision is processed by the macula. Damage to the macula results in the development of blind spots and blurred or distorted vision. Age-related macular degeneration (AMD) is a major cause of visual impairment in the United States and for people over age 65 it is the leading cause of legal blindness among Caucasians. Approximately 1.8 million Americans age 40 and older have advanced AMD, and another 7.3 million people with intermediate AMD are at substantial risk for vision loss. The government estimates that by 2020 there will be 2.9 million people with advanced AMD. Victims of AMD are often surprised and frustrated to find out how little is known about the causes and treatment of this blinding condition.

There are two forms of macular degeneration: dry macular degeneration and wet macular degeneration. The dry form, in which the cells of the macula slowly begin to break down, is diagnosed in 85 percent of macular degeneration cases. Both eyes are usually affected by dry AMD, although one eye can lose vision while the other eye remains unaffected. Drusen, which are yellow deposits under the retina, are common early signs of dry AMD. The risk of developing advanced dry AMD or wet AMD increases as the number or size of the drusen increases. It is possible for dry AMD to advance and cause loss of vision without turning into the wet form of the disease; however, it is also possible for early-stage dry AMD to suddenly change into the wet form.

The wet form, although it only accounts for 15 percent of the cases, results in 90 percent of the blindness, and is considered advanced AMD (there is no early or intermediate stage of wet AMD). Wet AMD is always preceded by the dry form of the disease. As the dry form worsens, some people begin to have abnormal blood vessels growing behind the macula. These vessels are very fragile and will leak fluid and blood (hence 'wet' macular degeneration), causing rapid damage to the macula.

The dry form of AMD will initially often cause slightly blurred vision. The center of vision in particular may then become blurred and this region grows larger as the disease progresses. No symptoms may be noticed if only one eye is affected. In wet AMD, straight lines may appear wavy and central vision loss can occur rapidly.

Diagnosis of macular degeneration typically involves a dilated eye exam, visual acuity test, and a viewing of the back of the eye using a procedure called fundoscopy to help diagnose AMD, and-if wet AMD is suspected-fluorescein angiography may also be performed. If dry AMD reaches the advanced stages, there is no current treatment to prevent vision loss. However, a specific high dose formula of antioxidants and zinc may delay or prevent intermediate AMD from progressing to the advanced stage. Macugen® (pegaptanib sodium injection), laser photocoagulation and photodynamic therapy can control the abnormal blood vessel growth and bleeding in the macula, which is helpful for some people who have wet AMD; however, vision that is already lost will not be restored by these techniques. If vision is already lost, low vision aids exist that can help improve the quality of life.

One of the earliest signs of age-related macular degeneration (AMD) is the accumulation of extracellular deposits known as drusen between the basal lamina of the retinal pigmented epithelium (RPE) and Bruch's membrane (BM). Recent studies conducted by Anderson et al. have confirmed that drusen contains amyloid beta. (Experimental Eye Research 78 (2004) 243-256).

Ongoing research continues with studies exploring environmental, genetic, and dietary factors that may contribute to AMD. New treatment strategies are also being explored, including retinal cell transplants, drugs that will prevent or slow down the progress of the disease, radiation therapy, gene therapies, a computer chip implanted in the retina that may help stimulate vision and agents that will prevent the growth of new blood vessels under the macula.

An important factor to consider when developing new drugs is the ease of use for the target patients. Oral drug delivery, specifically tablets, capsules and softgels, account for 70% of all dosage forms consumed because of patient convenience. Drug developers agree that patients prefer oral delivery rather than subjecting themselves to injections or other, more invasive forms of medicinal administration. Formulations resulting in low dosing intervals (i.e. once a day or sustained release) are also preferable. The ease of administering antibiotics in oral dosage forms results in an increase of patient compliance during treatment.

What is needed are effective methods and compositions for preventing or addressing the complications associated with amyloidosis, a group of diseases and disorders associated with amyloid plaque formation including secondary amyloidosis and age-related amyloidosis including, but not limited to, neurological disorders such as Alzheimer's Disease (AD), Lewy body dementia (LBD), Down's syndrome, hereditary cerebral hemorrhage with amyloidosis (Dutch type); the Guam Parkinson-Dementia complex; as well as other diseases which are based on or associated with amyloid-like proteins, such as progressive supranuclear palsy, multiple sclerosis; Creutzfeld Jacob disease, Parkinson's disease, HIV-related dementia, ALS (amyotropic lateral sclerosis), Adult Onset Diabetes; senile cardiac amyloidosis; endocrine tumors, and other diseases, including ocular diseases associated with pathological abnormalities/changes in the tissues of the visual system, particularly associated with amyloid-beta-related pathological abnormalities/changes in the tissues of the visual system, such as, for example, neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy. In particular what is needed are agents capable of counteracting the physiological manifestations of the disease such as the formation of plaques associated with aggregation of fibers of the amyloid or amyloid-like peptide.

Anti-amyloid antibodies elicited by the inoculation of A$\beta_{1-42}$ mixed with Freund complete or incomplete adjuvant were reported to reduce the amyloid burden in transgenic mice for human Alzheimer disease (Schenk et al., 1999). Intraperitoneal inoculation of tetrapalmitoylated A$\beta_{1-16}$ reconstituted in liposomes to NORBA transgenic mice elicited significant titers of anti-amyloid antibodies, which were reported to solubilize amyloid fibers and plaques *in vitro* and *in vivo.* (Nicolau et al., 2002).

A possible mechanism by which the dissolution of amyloid plaques and fibers occurred was first suggested by Bard et al., (2000), who concluded that the antibodies opsonized the plaques, which were subsequently destroyed by the macrophages of the microglia. De Mattos et al., (2001) indicated that an mAb directed against the central domain of $\beta$-amyloid was able to bind and completely sequester plasma amyloid. They argued that the presence of these mAbs in circulation shifted the equilibrium of A$\beta$ between brain and plasma, favoring the peripheral clearing and catabolism instead of deposition within the brain.

Prolonged human therapy with rodent antibodies may result in an antiglobulin response which is detectable at about 8-12 days after administration and reaches a peak at about 20-30 days. If such an antiglobulin response is encountered, the treatment must be discontinued after not more than about 10 days and re-treatment at a latter date is usually precluded because it will lead to rapid onset of a secondary antiglobulin response. Although rodent antibodies share a considerable degree of sequence conservation with that of human antibodies, there are many sequence differences between rodents and human antibodies sufficient for the rodent antibodies to be immunogenic in humans.

This problem may be overcome by generating antibodies directly in humans or by the creation of "humanized" (a.k.a. "reshaped" antibodies). Humanized antibodies have a variable region amino acid sequence that contains the rodent-derived CDRs interspersed into human or human-like framework sequences. Since the specificity of the humanized antibody is provided by the rodent-derived CDRs, their residues are to be used essentially unchanged with only minor modifications being allowable, which do not significantly interfere with the affinity and specificity of the antibody for its target antigen. Framework residues may be derived from any primate or, particularly, from any human variable region or may be a combination thereof and the resultant designed variable region would be considered reshaped.

To maximise the likelihood that affinity will be retained in the reshaped antibody it is important to make a proper selection of the framework region. It is known that the framework sequences serve to hold the CDRs in their correct spatial orientation for interaction with antigen, and that framework residues can sometimes even participate in antigen binding. In order to maintain the affinity of the antibody for its antigen it is advantageous to select human framework sequences that are most similar to the sequences of the rodent frameworks. It then may still be necessary to replace one or more amino acids in the human framework sequence with the corresponding residue in the rodent framework to avoid losses with the affinity. This replacement may be aided by computer modelling. Insert content of page 11A here

International Publication No. WO 2007/0688412 discloses mouse anti-beta-amyloid antibody mACI-01-AB7 C2, which antibody is of the IgG2b subclass and is capable of both disaggregation of existing plaques and prevention of the formation of new plaques.

International Publication No. WO 2006/039327 discloses compositions comprising anti-beta amyloid antibodies and methods for the prophylactic or therapeutic treatment of ophthalmic diseases characterized by amyloid deposition in the eye or optic nerve in a patient.

U.S. Patent No. 6,737,056 B1 discloses Fc region-containing polypeptides that have altered effector function as a consequence of one or more amino acid modifications in the Fc region thereof.

International Publication Nos. WO 2008/011348 and WO 2008/060364 disclose humanized antibodies, including the humanized antibody hC2, and the use of such antibodies for the treatment and diagnosis of amyloidosis.

International Publication No. WO 2008/156622 discloses humanized antibodies, including the humanized antibody hC2, with an IgG$_1$ Fc region containing one or more amino acid modifications.

The present invention provides novel methods and compositions comprising highly specific and highly effective antibodies, particularly chimeric antibodies including fragments thereof, more particularly partially or fully humanized antibodies including fragments thereof, having the ability to specifically recognize and bind to specific epitopes from a range of β-amyloid antigens, which may be presented to the antibody in a monomeric, dimeric, trimeric, etc, a polymeric form, in form of an aggregate, fibers, filaments or in the condensed form of a plaque. The antibodies enabled by the teaching of the present invention are particularly useful for the treatment of amyloidosis, a group of diseases and disorders associated with amyloid plaque formation including secondary amyloidosis and age-related amyloidosis including, but not limited to, neurological disorders such as Alzheimer's Disease (AD), Lewy body dementia (LBD), Down's syndrome, hereditary cerebral hemorrhage with amyloidosis (Dutch type), the Guam Parkinson-Dementia complex, as well as other diseases which are based on or associated with amyloid-like proteins such as progressive supranuclear palsy, multiple sclerosis; Creutzfeld Jacob disease, hereditary cerebral hemorrhage with amyloidosis Dutch type, Parkinson's disease, HIV-related dementia, ALS (amyotropic lateral sclerosis), Adult Onset Diabetes; senile cardiac amyloidosis; endocrine tumors, and other diseases, including ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

## SUMMARY OF THE INVENTION

In one embodiment, the invention relates to a chimeric antibody or a fragment thereof, a humanized antibody or a fragment thereof, which recognizes and binds to at least one distinct binding site, particularly to a least two distinct binding sites, and more particularly to at least three distinct binding sites on the β-amyloid protein wherein said one, said at least two and said at least three binding sites each comprise at least one or two consecutive amino acid residues predominantly involved in the binding of the antibody.

The embodiments of the present invention are represented by and defined in the claims.

Any reference to the invention or to aspects or embodiments of the invention mentioned herein, which exceed the scope of the invention as represented by or defined in the claims do not form part of the invention but represent background information only useful for understanding the invention.

The present invention provides in one embodiment a pharmaceutical composition for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis characterized by abnormal conjunctival vessels, keratoconjunctivitis sicca, papillary abnormalities, vitreous opacities and/or secondary glaucoma, and (iv) lattice dystrophy, but particularly for use in the treatment of glaucoma, comprising a therapeutically effective amount of a humanized antibody or a functional part thereof, which recognizes and binds to at least one distinct site on beta-amyloid protein, wherein the humanized antibody or functional part thereof comprises: (a) a heavy chain CDR1 having the amino acid sequence of SEQ ID NO: 1; a heavy chain CDR2 having the amino acid sequence of SEQ ID NO: 2; a heavy chain CDR3 having the amino acid sequence of SEQ ID NO: 3; a light chain CDR1 having the amino acid sequence of SEQ ID NO: 4; a light chain CDR2 having the amino acid sequence of SEQ ID NO:5, the amino acid sequence RVSNRFS, or the amino acid sequence KVSSRFS; and a light chain CDR3 having the amino acid sequence of SEQ ID NO: 6, and (b) an IgG1 Fc region with reduced binding to the Fc. gamma. R having an amino acid modification at any one or more of amino acid positions 238, 239, 248, 249, 252, 254,265, 268, 269, 270, 272, 278, 289, 292, 293, 294, 295, 296, 298, 301, 303, 322, 324, 327, 329, 333, 335, 338, 340, 373, 376, 382, 388, 389,414, 416, 419, 434, 435, 437, 438 or 439 of the IgG1 Fc region.

In a specific embodiment, the humanized antibody or a functional part thereof comprises a D265A mutation in the Fc region.

In another specific embodiment, the pharmaceutical composition of the invention as described herein is for use in the treatment of glaucoma selected from the group consisting of chronic open-angle glaucoma (COAG), acute angle closure glaucoma (AACG), mixed or combined mechanism glaucoma, normal tension glaucoma, congenital glaucoma, secondary glaucoma and exfoliative glaucoma.

In one embodiment, the invention relates to a humanized antibody or a functional part thereof, which recognizes and binds to at least one distinct site on beta-amyloid protein and comprises: (a) a heavy chain CDR1 having the amino acid sequence of SEQ ID NO: 1; a heavy chain CDR2 having the amino acid sequence of SEQ ID NO: 2; a heavy chain CDR3 having the amino acid sequence of SEQ ID NO: 3; a light chain CDR1 having the amino acid sequence of SEQ ID NO: 4; a light chain CDR2 having the amino acid sequence of SEQ ID NO:5, the amino acid sequence RVSNRFS, or the amino acid sequence KVSSRFS; and a light chain CDR3 having the amino acid sequence of SEQ ID NO: 6 and (b) an IgG1 Fc region with reduced binding to the Fc.gamma.R having an amino acid modification at any one or more of amino acid positions 238, 239, 248, 249, 252, 254, 265, 268, 269, 270, 272, 278, 289, 292, 293, 294, 295, 296, 298, 301, 303, 322, 324, 327, 329, 333, 335, 338, 340, 373, 376, 382, 388, 389, 414, 416, 419, 434. 435, 437, 438 or 439 of the IgG1 Fc region, for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis characterized by abnormal conjunctival vessels, keratoconjunctivitis sicca, papillary abnormalities, vitreous opacities and/or secondary glaucoma, and (iv) lattice dystrophy.

In another embodiment, the humanized antibody or a functional part thereof for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis characterized by abnormal conjunctiva vessels, keratoconjunctivitis sicca, papillary abnormalities, vitreous opacities and/or secondary glaucoma, and (iv) lattice dystrophy according to the preceding embodiment, wherein the humanized antibody or a functional part thereof comprises a D265A mutation in the Fc region.

The invention further relates to the humanized antibody or a functional part thereof for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis characterized by abnormal conjunctival vessels, keratoconjunctivitis sicca, papillary abnormalities, vitreous opacities and/or secondary glaucoma, and (iv) lattice dystrophy according to any of the preceding embodiments by reducing the plaque load or the amount of plaques in the retinal ganglion cell layer or by decreasing the total amount of soluble amyloid-$\beta$ in the retinal ganglion cell layer.

In one embodiment, the invention relates to the humanized antibody or a functional part thereof for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis characterized by abnormal conjunctival vessels, keratoconjunctivitis sicca, papillary abnormalities, vitreous opacities and/or secondary glaucoma, and (iv) lattice dystrophy according to any of the preceding embodiments by preventing, treating or alleviating the effects of glaucoma, optic neuritis ocular, amyloidosis and/or lattice dystrophy.

In one embodiment, the invention relates to the humanized antibody or a functional part thereof for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis characterized by abnormal conjunctival vessels, keratoconjunctivitis sicca, papillary abnormalities, vitreous opacities and/or secondary glaucoma, and (iv) lattice dystrophy according to any of the preceding embodiments by retaining or decreasing ocular pressure in the eyes of an animal suffering from glaucoma, optic neuritis, ocular amyloidosis and/or lattice dystrophy.

In one embodiment, the invention relates to the humanized antibody or a functional part thereof for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis characterized by abnormal conjunctival vessels, keratoconjunctivitis sicca, papillary abnormalities, vitreous opacities and/or secondary glaucoma, and (iv) lattice dystrophy according to any one of the preceding embodiments, wherein the humanized antibody or a functional part thereof comprises the light chain variable region of SEQ ID NO: 12 and the heavy chain variable region of SEQ ID NO: 15.

In a specific embodiment, the humanized antibody or functional part thereof for use according to any one of the preceding embodiments in the treatment of glaucoma, particularly glaucoma selected from the group consisting of chronic open-angle glaucoma (COAG), acute angle closure glaucoma (AACG), mixed or combined mechanism glaucoma, normal tension glaucoma, congenital glaucoma, secondary glaucoma and exfoliative glaucoma.

In one embodiment, the invention relates to the pharmaceutical composition for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis characterized by abnormal conjunctival vessels, keratoconjunctivitis sicca, papillary abnormalities, vitreous opacities and/or secondary glaucoma, and (iv) lattice dystrophy according to any one of the preceding embodiments, wherein the humanized antibody or a functional part thereof comprises the light chain variable region of SEQ ID NO: 12 and the heavy chain variable region of SEQ ID NO: 15.

In one embodiment, the invention relates to the humanized antibody or a functional part thereof, or the pharmaceutical composition for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis characterized by abnormal conjunctival vessels, keratoconjunctivitis sicca, papillary abnormalities, vitreous opacities and/or secondary glaucoma, and (iv) lattice dystrophy according to any one of any of the preceding embodiments, wherein the subject is a mammal, particularly a human.

The invention further relates to the use of a humanized antibody or a functional part thereof as defined in any one of the preceding embodiments, for the preparation of a medicament for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis characterized by abnormal conjunctival vessels, keratoconjunctivitis sicca, papillary abnormalities, vitreous opacities and/or secondary glaucoma, and (iv) lattice dystrophy, by

> i) reducing the plaque load in the retinal ganglion cell layer of a subject suffering from glaucoma, optic neuritis, ocular amyloidosis or lattice dystrophy;
>
> ii) reducing the amount of plaques in the retinal ganglion cell layer of a subject suffering from glaucoma, optic neuritis, ocular amyloidosis or lattice dystrophy;
>
> iii) decreasing the total amount of soluble amyloid-$\beta$ in the retinal ganglion cell layer of a subject suffering from glaucoma, optic neuritis, ocular amyloidosis or lattice dystrophy;
>
> iv) preventing, treating or alleviating the effects of glaucoma, optic neuritis, ocular amyloidosis or lattice dystrophy; or
>
> v) retaining or decreasing ocular pressure in the eyes of a subject suffering from glaucoma, optic neuritis, ocular amyloidosis or lattice dystrophy,

wherein the ocular amyloidosis is characterized by abnormal conjunctival vessels, keratoconjunctivitis sicca, pupillary abnormalities, vitreous opacities and/or secondary glaucoma.

The subject to be treated may be a mammal, particularly a human.

In particular, the chimeric antibody or a fragment thereof, or the humanized antibody or a fragment thereof according to the invention binds to at least two, particularly to at least three distinct binding sites on the $\beta$-amyloid protein wherein at least two of the three distinct binding sites comprise at least two consecutive amino acid residues predominantly involved in the binding of the antibody and at least one the three distinct binding sites comprise at least one amino acid residue.

The at least two distinct binding sites comprising at least two consecutive amino acid residues predominantly involved in the binding of the antibody are located in close proximity to each other on the antigen, separated and/or flanked by at least one amino acid residue not involved in antibody binding or to a significantly smaller extent as compared to said at least two consecutive amino acid residues, thus forming a conformational discontinuous epitope.

The at least three distinct binding sites comprising at least two consecutive amino acid residues and at least one amino acid residue, respectively, which are predominantly involved in the binding of the antibody are located in close proximity to each other on the epitope, separated and/or flanked by at least one amino acid residue not involved in antibody binding or to a significantly smaller extent as compared to the amino acid residues, which are predominantly involved in the binding of the antibody, thus forming a conformational discontinuous epitope.

In particular, a chimeric antibody or a fragment thereof, or a humanized antibody or a fragment thereof is provided, which recognizes and binds to at least one distinct binding site, particularly to a least two distinct binding sites, more particularly to at least three distinct binding sites on the $\beta$-amyloid protein wherein said at least one or said at least two distinct binding sites each comprise at least two consecutive amino acid residues predominantly involved in the binding of the antibody, wherein the at least two consecutive amino acid residues representing a first binding site are -Phe-Phe- embedded within the following core sequence (SEQ ID NO: 9):

$$\text{Xaa}_3 - \text{Phe} - \text{Phe} - \text{Xaa}_4 - \text{Xaa}_5 - \text{Xaa}_6,$$

wherein:

$\text{Xaa}_3$ is an amino acid residue selected from the group consisting of Ala, Val, Leu, norleucine, Met, Phe, and Ile;

$\text{Xaa}_4$ is an amino acid residue selected from the group consisting of Ala, Val, Leu, Ser and Ile;

$\text{Xaa}_5$ is an amino acid residue selected from the group consisting of Glu and Asp;

$\text{Xaa}_6$ is an amino acid residue selected from the group consisting of Glu and Asp; and wherein said amino acid residues $\text{Xaa}_3$ $\text{Xaa}_4$, $\text{Xaa}_5$ and $\text{Xaa}_6$ are not involved in antibody binding or to a significantly smaller extent as compared to the -Phe-Phe-binding site.

In another embodiment of the invention, a chimeric antibody or a fragment thereof, or a humanized antibody or a fragment thereof is provided, wherein:

$\text{Xaa}_3$ is Val or Leu, but particularly Val;

$\text{Xaa}_4$ is Ala or Val, but particularly Ala;

$\text{Xaa}_5$ is Glu or Asp, but particularly Glu;

$\text{Xaa}_6$ is Glu or Asp, but particularly Asp.

In particular, a chimeric antibody or a fragment thereof, or a humanized antibody or a fragment thereof is provided, which recognizes and binds to at least one distinct binding site, particularly to a least two distinct binding sites, more particularly to at least three distinct binding sites on the $\beta$-amyloid protein wherein said distinct binding sites comprise at least one and at least two consecutive amino acid residues, respectively, predominantly involved in the binding of the antibody, wherein the at least two consecutive amino acid residues representing a first binding site are -Phe-Phe- and the at least one amino acid residue is -His- embedded within the following core sequence:

$$- \text{Xaa}_1 - \text{His} - \text{Xaa}_3 - \text{Xaa}_4 - \text{Xaa}_5 - \text{Xaa}_6 - \text{Phe} - \text{Phe} - \text{Xaa}_7 - \text{Xaa}_8 - \text{Xaa}_9 -,$$

Wherein:

$\text{Xaa}_1$ is an amino acid residue selected from the group consisting of His, Asn, Gln, Lys and Arg;

$\text{Xaa}_3$ is an amino acid residue selected from the group consisting of Asn and Gln;

$\text{Xaa}_4$ is an amino acid residue selected from the group consisting of His, Asn, Gln, Lys and Arg;

$\text{Xaa}_5$ is an amino acid residue selected from the group consisting of Ala, Val, Leu, Ser and Ile;

$\text{Xaa}_6$ is an amino acid residue selected from the group consisting of Ala, Val, Leu, norleucine, Met, Phe, and Ile;

$\text{Xaa}_7$ is an amino acid residue selected from the group consisting of Ala, Val, Leu and Ile;

$\text{Xaa}_8$ is an amino acid residue selected from the group consisting of Glu and Asp;

$\text{Xaa}_9$ is an amino acid residue selected from the group consisting of Glu and Asp; and wherein said amino acid

residues $Xaa_1$, $Xaa_3$, $Xaa_6$, $Xaa_7$, $Xaa_8$ and $Xaa_9$, are not involved in antibody binding or to a smaller to significantly smaller extent as compared to the -His- and the -Phe-Phe- binding site, respectively.

In another embodiment of the invention, a chimeric antibody or a fragment thereof, or a humanized antibody or a fragment thereof is provided, wherein:

$Xaa_3$ is Gln or Asn, but particularly Gln;

$Xaa_4$ is Lys;

$Xaa_5$ is Leu;

$Xaa_6$ is Val or Leu, but particularly Val;

$Xaa_7$ is Ala or Val, but particularly Ala;

$Xaa_8$ is Glu or Asp, but particularly Glu; and

$Xaa_9$ is Asp or Glu, but particularly Asp.

In another embodiment of the invention, a chimeric antibody or a fragment thereof, or a humanized antibody or a fragment thereof is provided, which recognizes and binds to at least one distinct binding site, particularly to a least two distinct binding sites, more particularly to at least three distinct binding sites on the $\beta$-amyloid protein, wherein said at least one or said at least two distinct binding sites each comprise at least two consecutive amino acid residues predominantly involved in the binding of the antibody, wherein the at least two consecutive amino acid residues representing a second binding site are -Lys-Leu- embedded within the following core sequence (SEQ ID NO: 10):

$$Xaa_1 - Xaa_2 - Lys - Leu - Xaa_3,$$

wherein:

$Xaa_1$ is an amino acid residue selected from the group consisting of His, Asn, Gln Lys, and Arg;

$Xaa_2$ is an amino acid residue selected from the group consisting of Asn and Gln;

$Xaa_3$ is an amino acid residue selected from the group consisting of Ala, Val, Leu, norleucine, Met, Phe, and Ile; and wherein said amino acid residues $Xaa_2$, $Xaa_3$, are not involved in antibody binding or to a smaller to significantly smaller extent as compared to the -Lys-Leu- binding site.

In another embodiment of the invention, a chimeric antibody or a fragment thereof, or a humanized antibody or a fragment thereof is provided, which recognizes and binds to at least one distinct binding site, particularly to a least two distinct binding sites, more particularly to at least three distinct binding sites on the $\beta$-amyloid protein wherein said distinct binding sites comprise at least one and at least two consecutive amino acid residues, respectively, predominantly involved in the binding of the antibody, wherein the at least one and the at least two consecutive amino acids, which are separated by at least one amino acid residue not involved in antibody binding or to a significantly smaller extent as compared to the amino acid residues predominantly involved in the binding of the antibody, are -His- and -Lys-Leu-, respectively, embedded within the following core sequence:

$$His - Xaa_2 - Lys - Leu - Xaa_3 - Xaa_4 - Xaa_5 - Xaa_6 - Xaa_7 - Xaa_8 -,$$

wherein:

$Xaa_2$ is an amino acid residue selected from the group consisting of Asn and Gln;

$Xaa_3$ is an amino acid residue selected from the group consisting of Ala, Val, Leu, norleucine, Met, Phe, and Ile;

$Xaa_4$ is an amino acid residue selected from the group consisting of Ala, Val, Leu, norleucine, Met, Phe, and Ile;

$Xaa_5$ is an amino acid residue selected from the group consisting of Ala, Val, Leu, norleucine, Met, Phe, and Ile;

$Xaa_6$ is an amino acid residue selected from the group consisting of Ala, Val, Leu, Ser and Ile;

$Xaa_7$ is an amino acid residue selected from the group consisting of Glu and Asp;

$Xaa_8$ is an amino acid residue selected from the group consisting of Glu and Asp;

and wherein said amino acid residues $Xaa_2$, $Xaa_3$, $Xaa_6$, $Xaa_7$, $Xaa_8$, are not involved in antibody binding or to a smaller to significantly smaller extent as compared to the - His- and the -Lys-Leu- binding site, respectively.

In another embodiment of the invention, a chimeric antibody or a fragment thereof, or a humanized antibody or a fragment thereof is provided, wherein:

$Xaa_2$ is Gln or Asn, but particularly Gln;

$Xaa_3$ is Val or Leu, but particularly Val;

$Xaa_4$ is Phe;

$Xaa_5$ is Phe;

$Xaa_6$ is Ala or Val, but particularly Ala;

$Xaa_7$ is Glu or Asp, but particularly Glu; and

$Xaa_8$ is Asp or Glu, but particularly Asp.

In another embodiment of the invention, a chimeric antibody or a fragment thereof, or a humanized antibody or a fragment thereof is provided, which recognizes and binds to at least two distinct binding sites on the $\beta$-amyloid protein wherein said at least two distinct binding sites each comprise at least two consecutive amino acid residues predominantly involved in the binding of the antibody, wherein the at least two consecutive amino acids are separated by at least one amino acid residue not involved in antibody binding or to a significantly smaller extent than said consecutive amino acid residues, which are -Phe-Phe- and -Lys-Leu-, respectively, representing a first and second binding site embedded within the following core sequence:

$$Xaa_1 - Xaa_2 - Lys - Leu - Xaa_3 - Phe - Phe - Xaa_4 - Xaa_5 - Xaa_6,$$

wherein:

$Xaa_1$ is an amino acid residue selected from the group consisting of His, Asn, Gln Lys, and Arg;

$Xaa_2$ is an amino acid residue selected from the group consisting of Asn and Gln;

$Xaa_3$ is an amino acid residue selected from the group consisting of Ala, Val, Leu, norleucine, Met, Phe, and Ile;

$Xaa_4$ is an amino acid residue selected from the group consisting of Ala, Val, Leu, Ser and Ile;

$Xaa_5$ is an amino acid residue selected from the group consisting of Glu and Asp;

$Xaa_6$ is an amino acid residue selected from the group consisting of Glu and Asp; and wherein said amino acid residues $Xaa_2$, $Xaa_3$, $Xaa_4$, $Xaa_5$ and $Xaa_6$ are not involved in antibody binding or to a smaller to significantly smaller extent as compared to the -Lys-Leu- and -Phe-Phe- binding site, respectively.

In another embodiment of the invention, a chimeric antibody or a fragment thereof, or a humanized antibody or a fragment thereof is provided, which recognizes and binds to at least one distinct binding site, particularly to a least two distinct binding sites, more particularly to at least three distinct binding sites on the $\beta$-amyloid protein wherein said distinct binding sites comprise at least one and at least two consecutive amino acid residues, respectively, predominantly involved in the binding of the antibody, wherein the at least one and the at least two consecutive amino acids are separated by at least one amino acid residue not involved in antibody binding or to a significantly smaller extent as compared to the amino acid residues, which are predominantly involved in the binding of the antibody, and wherein said amino acid residues are -His- and -Phe-Phe- and -Lys-Leu-, respectively, embedded within the following core sequence:

His - Xaa$_2$ - Lys - Leu - Xaa$_3$ - Phe - Phe - Xaa$_4$ - Xaa$_5$ - Xaa$_6$,

wherein:

Xaa$_2$ is an amino acid residue selected from the group consisting of Asn and Gln;

Xaa$_3$ is an amino acid residue selected from the group consisting of Ala, Val, Leu, norleucine, Met, Phe, and Ile;

Xaa$_4$ is an amino acid residue selected from the group consisting of Ala, Val, Leu, Ser and Ile;

Xaa$_5$ is an amino acid residue selected from the group consisting of Glu and Asp;

Xaa$_6$ is an amino acid residue selected from the group consisting of Glu and Asp; and wherein said amino acid residues Xaa$_2$, Xaa$_3$, Xaa$_4$, Xaa$_5$, Xaa$_6$, are not involved in antibody binding or to a smaller to significantly smaller extent as compared to the - His-, the -Lys-Leu- and the -Phe-Phe- binding site, respectively.

In another embodiment of the invention, a chimeric antibody or a fragment thereof, or a humanized antibody or a fragment thereof is provided, wherein:

Xaa$_2$ is Gln or Asn, but particularly Gln;

Xaa$_3$ is Val or Leu, but particularly Val;

Xaa$_4$ is Ala or Val, but particularly Ala;

Xaa$_5$ is Glu or Asp, but particularly Glu; and

Xaa$_6$ is Asp or Glu, but particularly Asp.

In another embodiment of the invention, a chimeric antibody or a fragment thereof, or a humanized antibody or a fragment thereof is provided, which recognizes and binds to at least two distinct binding sites on the $\beta$-amyloid protein wherein said at least two distinct binding sites each comprise at least two consecutive amino acid residues predominantly involved in the binding of the antibody, wherein the at least two consecutive amino acids are separated by at least one amino acid residue not involved in antibody binding or to a significantly smaller extent than said consecutive amino acid residues, which are -Phe-Phe- and -Lys-Leu-, respectively, representing a first and second binding site embedded within the following core sequence:

Xaa$_1$ - Xaa$_2$ - Lys - Leu - Xaa$_3$ - Phe - Phe - Xaa$_4$ - Xaa$_5$ - Xaa$_6$,

wherein:

Xaa$_1$ is an amino acid residue selected from the group consisting of His, Asn, Gln, Lys and Arg;

Xaa$_2$ is an amino acid residue selected from the group consisting of Asn and Gln;

Xaa$_3$ is an amino acid residue selected from the group consisting of Val, Ala, Leu, Met, Phe, norleucine and Ile;

Xaa$_4$ is an amino acid residue selected from the group consisting of Ala, Val, Leu and Ile;

Xaa$_5$ is an amino acid residue selected from the group consisting of Glu and Asp;

Xaa$_6$ is an amino acid residue selected from the group consisting of Glu and Asp; and wherein said amino acid residues Xaa$_2$, Xaa$_3$, Xaa$_4$, Xaa$_5$, Xaa$_6$, are not involved in antibody binding or to a smaller to significantly smaller extent as compared to the - Lys-Leu- and the -Phe- Phe binding site, respectively.

In another embodiment of the invention, a chimeric antibody or a fragment thereof, or a humanized antibody or a fragment thereof is provided, wherein:

Xaa$_1$ is His or Arg, but particularly His;

Xaa$_2$ is Gln or Asn, but particularly Gln;

Xaa$_3$ is Val or Leu, but particularly Val;

Xaa$_4$ is Ala or Val, but particularly Ala;

Xaa$_5$ is Glu or Asp, but particularly Glu; and

Xaa$_6$ is Asp or Glu, but particularly Asp.

In one embodiment of the invention, a chimeric antibody or a fragment thereof, or a humanized antibody or a fragment thereof is provided which recognizes and binds to at least two distinct binding sites on the $\beta$-amyloid protein wherein said at least two distinct binding sites each comprise at least two consecutive amino acid residues predominantly involved in the binding of the antibody, which are - Phe - Phe -Ala - Glu -, particularly - Phe - Phe - Ala -,but especially - Phe - Phe - and - Lys - Leu -, respectively, and wherein said at least two distinct binding sites exhibit amino acid sequence -Val - Phe - Phe - Ala - Glu - Asp - shown in SEQ ID NO: 7 and amino acid sequence His - Gln - Lys - Leu - Val - shown in SEQ ID NO: 8, respectively.

In one embodiment of the invention, a chimeric antibody or a fragment thereof, or a humanized antibody or a fragment thereof is provided, which recognizes and binds to at least one distinct binding site, particularly to a least two distinct binding sites, more particularly to at least three distinct binding sites on the $\beta$-amyloid protein wherein the said at least one or said at least two distinct binding sites comprise at least one and at least two consecutive amino acid residues, respectively, predominantly involved in the binding of the antibody, which are - Phe - Phe - and - Lys - Leu -, and -His-, respectively, wherein said distinct binding sites are embedded in the amino acid sequence -Val - Phe - Phe - Ala - Glu-, and amino acid sequence - His - Gln - Lys - Leu - Val -, respectively.

In another embodiment of the invention, the chimeric antibody or a fragment thereof, or a humanized antibody or a fragment thereof comprises an antigen recognition and binding site which recognizes and binds to at least two distinct binding sites on the $\beta$-amyloid protein wherein said at least two distinct binding sites each comprise at least two consecutive amino acid residues within the amino acid sequence given in SEQ ID NOs: 7 and 8, respectively, wherein said consecutive amino acid residues, particularly -Phe- Phe- and -Lys-Leu-, are predominantly involved in the binding of the $\beta$-amyloid protein.

In a specific embodiment of the invention, the recognition and binding sites as defined herein before are forming a conformational discontinuous epitope localized in a region of the $\beta$-amyloid protein between amino acid residue 12 to 24, particularly between residues 14 to 23, more particularly between amino acid residues 14 and 20, wherein the at least two distinct recognition and binding sites each comprising at least 2 amino acid residues, are located at position 16 and 17 and at position 19 and 20, respectively, and wherein the at least one distinct recognition and binding site comprising at least 1 amino acid residue is located at position 14, which residues are predominantly involved in the binding of the $\beta$-amyloid protein and wherein said distinct recognition and binding sites are at least on one side flanked by amino acid residues, particularly residues 21 and 22, and separated by one amino acid residue located at position 15 and 18, which amino acid residues are not directly involved in the binding of the antigen or, at least, to a substantially smaller extent.

In still another embodiment of the invention the said at least three distinct recognition and binding sites are flanked on both sides by amino acid residues, particularly residues 12 and 13, and residues 21 and 22 and are separated by one amino acid residue located at position 15 and 18, which amino acid residues are not directly involved in the binding of the antigen or, at least, to a substantially smaller extent.

In a specific embodiment, said consecutive amino acid residues, particularly - Lys-Leu- at position 16 and 17 and -Phe- Phe- at position 19 and 20, which are predominantly involved in the binding of the $\beta$-amyloid protein, are embedded into the following core region:

| Val- | His- | His- | Gln- | Lys- | Leu- | Val- | Phe- | Phe- | Ala- | Glu- | Asp |
|------|------|------|------|------|------|------|------|------|------|------|-----|
| 12 | 13 | 14 | 15 | 16 | 17 | 18 | 19 | 20 | 21 | 22 | 23 |

In another specific embodiment, said amino acid residues, particularly -Lys-Leu- at position 16 and 17 and -Phe- Phe- at position 19 and 20, and -His- at position 14, which are predominantly involved in the binding of the $\beta$-amyloid protein, are embedded into the following core region:

| Val- | His- | His- | Gln- | Lys- | Leu- | Val- | Phe- | Phe- | Ala- | Glu- | Asp- | Val- | Gly- |
|------|------|------|------|------|------|------|------|------|------|------|------|------|------|
| 12 | 13 | 14 | 15 | 16 | 17 | 18 | 19 | 20 | 21 | 22 | 23 | 24 | 25 |

In another embodiment of the invention, a humanized antibody or a fragment thereof is provided which comprises in the light chain and heavy chain variable region, respectively, at least one CDR of non-human origin, particularly two CDRs of non-human origin, more particularly three CDR of non-human origin, embedded in one or more human- or primate-derived framework regions and, optionally, a constant region derived from a human or primate source antibody, which humanized antibody or fragment thereof is capable of specifically recognizing and binding $\beta$-amyloid protein, particularly a $\beta$-amyloid monomeric peptide, more particularly a $\beta$-amyloid polymeric peptide, even more particularly $\beta$-amyloid fibers, fibrils or filaments in isolation or as part of a $\beta$-amyloid plaque, at an epitope comprising the following amino acid sequence (SEQ ID NO: 11):

$$Xaa_1 - Xaa_2 - Lys - Leu - Xaa_3 - Phe - Phe - Xaa_4 - Xaa_5 - Xaa_6,$$

wherein:

$Xaa_1$ is an amino acid residue selected from the group consisting of His, Asn, Gln, but particularly His;

$Xaa_2$ is an amino acid residue selected from the group consisting of Asn and Gln, but particularly Gln; and

$Xaa_3$ is an amino acid residue selected from the group consisting of Val, Leu, and Ile, but particularly Val;

$Xaa_4$ is an amino acid residue selected from the group consisting of Ala and Val, but particularly Ala;

$Xaa_5$ is an amino acid residue selected from the group consisting of Glu and Asp, but particularly Glu;

$Xaa_6$ is an amino acid residue selected from the group consisting of Glu and Asp, but particularly Asp.

In still another embodiment of the invention, a humanized antibody or a fragment thereof is provided which comprises in the light chain and heavy chain variable region, respectively, at least one CDR of non-human origin, particularly two CDRs of non-human origin, more particularly three CDR of non-human origin, embedded in one or more human- or primate-derived framework regions and, optionally, a constant region derived from a human or primate source antibody, which humanized antibody or fragment thereof is capable of specifically recognizing and binding $\beta$-amyloid protein, particularly a $\beta$-amyloid monomeric peptide, more particularly a $\beta$-amyloid polymeric peptide, even more particularly $\beta$-amyloid fibers, fibrils or filaments in isolation or as part of a $\beta$-amyloid plaque, at an epitope comprising the following amino acid sequence:

$$His - Xaa_2 - Lys - Leu - Xaa_3 - Phe - Phe - Xaa_4 - Xaa_5 - Xaa_6,$$

wherein:

$Xaa_2$ is an amino acid residue selected from the group consisting of Asn and Gln, but particularly Gln; and

$Xaa_3$ is an amino acid residue selected from the group consisting of Val, Leu, and Ile, but particularly Val;

$Xaa_4$ is an amino acid residue selected from the group consisting of Ala and Val, but particularly Ala;

$Xaa_5$ is an amino acid residue selected from the group consisting of Glu and Asp, but particularly Glu;

$Xaa_6$ is an amino acid residue selected from the group consisting of Glu and Asp, but particularly Glu; and wherein said amino acid residues $Xaa_2$, $Xaa_3$, $Xaa_4$, $Xaa_5$, $Xaa_6$ are not involved in antibody binding or to a smaller extent as compared to the -His- and the -Lys-Leu- and the -Phe-Phe- binding site.

In a specific embodiment of the invention, the CDR of non-human origin is obtained from a donor antibody, but particularly from a murine donor antibody, raised against an antigen fragment which does not contain said distinct binding site. This shift in the epitopic region may have at least partially been caused by the use of a supramolecular antigenic construct comprising an antigenic peptide corresponding to the amino acid sequence of the $\beta$-amyloid peptide, particularly of $\beta$-amyloid peptide A$\beta_{1-16}$, modified with a hydrophilic moiety such as, for example, polyethylene glycol (PEG), wherein said hydrophilic moiety is covalently bound to each of the termini of the antigenic peptide through at least one, particularly one or two amino acids such as, for example, lysine, glutamic acid and cysteine or any other suitable amino acid or amino acid analogue capable of serving as a connecting device for coupling the hydrophilic moiety to the peptide fragment, as described herein below in the immunization process. When a PEG is used as the hydrophilic moiety, the free PEG termini are covalently bound to phosphatidylethanolamine or any other compound suitable to function as the anchoring element, for example, to embed the antigenic construct in the bilayer of a liposome as described herein.

In particular, the CDR of non-human origin is obtained from a murine donor antibody which exhibits the characteristic properties of ACI-01-Ab7C2 (also named "mC2" throughout the application) deposited 01 December 2005 with the Deutsche Sammlung von Mikroorganismen und Zellkulturen GmbH (DSMZ) in Braunschweig, Mascheroder Weg 1 B, 38124 Branuschweig, under the provisions of the Budapest Treaty under accession no DSM ACC2750).

In one embodiment of the invention, the CDR of non-human origin is obtained from murine donor antibody ACI-01-Ab7C2 (also named "mC2" throughout the application) deposited 01 December 2005 with the Deutsche Sammlung von Mikroorganismen und Zellkulturen GmbH (DSMZ) in Braunschweig, Mascheroder Weg 1 B, 38124 Branuschweig, under the provisions of the Budapest Treaty under accession no DSM ACC2750).

Also the use of lipid A as part of the immunization protocol may have contributed to a shift in the epitopic region.

In a specific embodiment, the invention relates to a humanized antibody or a fragment thereof comprising integrated into human- or primate-derived framework regions at least one peptide with an amino acid sequence selected from the group of sequences consisting of SEQ ID NO: 2 representing CDR2 and SEQ ID NO: 3 representing CDR3 of the Heavy Chain Variable Region (HCVR) and SEQ ID NO: 4 representing CDR1 of the Light Chain Variable Region (LCVR).

In another embodiment, the invention relates to a humanized antibody or a fragment thereof, wherein said humanized antibody comprises integrated into human-or primate-derived heavy chain framework regions at least one peptide with an amino acid sequence selected from the group of sequences consisting of SEQ ID NO: 2 representing CDR2 and SEQ ID NO: 3 representing CDR3 of the Heavy Chain Variable Region (HCVR).

In still another embodiment, the invention relates to a humanized antibody or a fragment thereof, wherein said humanized antibody comprises integrated into human-or primate-derived light chain framework regions a peptide with an amino acid sequence of SEQ ID NO: 4 representing CDR1 of the Light Chain Variable Region (LCVR).

In particular, the invention relates to a Light Chain Variable Region (LCVR) comprising integrated into human- or primate-derived framework regions at least one peptide with an amino acid sequence of SEQ ID NO: 4 representing CDR1 of the Light Chain Variable Region (LCVR).

In another specific embodiment, the invention relates to a Heavy Chain Variable Region (HCVR) comprising integrated into human- or primate-derived framework regions at least one peptide with an amino acid sequence selected from the group of sequences consisting of SEQ ID NO: 2 representing CDR2 and SEQ ID NO: 3 representing CDR3 of the Heavy Chain Variable Region (HCVR).

The invention further relates to a humanized antibody or a fragment thereof, which comprises integrated into human- or primate-derived framework regions at least two peptides, which peptides are different and exhibit an amino acid sequence selected from the group of sequences consisting of SEQ ID NO:1 representing CDR1, SEQ ID NO: 2 representing CDR2 and SEQ ID NO: 3 representing CDR3 of the Heavy Chain Variable Region (HCVR) and SEQ ID NO: 4 representing CDR1, SEQ ID NO: 5 representing CDR2 and SEQ ID NO: 6 representing CDR3 of the Light Chain Variable Region (LCVR) wherein the same CDR cannot be present twice in the antibody. In particular, if the at least two CDRs present are both CDRs of the Light Chain Variable Region (LCVR), at least on of said CDRs must be CDR1 represented by SEQ ID NO: 4.

Also comprised by the invention is a humanized antibody or a fragment thereof comprising integrated into human- or primate-derived heavy chain framework regions at least two peptides with an amino acid sequence selected from the group of sequences consisting of SEQ ID NO: 1 representing CDR1, SEQ ID NO: 2 representing CDR2 and SEQ ID NO: 3 representing CDR3 of the Heavy Chain Variable Region (HCVR), but particularly a humanized antibody or a fragment thereof wherein the same CDR cannot be present twice in the antibody.

In particular, the invention relates to a Heavy Chain Variable Region (HCVR) comprising integrated into human- or primate-derived heavy chain framework regions at least two peptides with an amino acid sequence selected from the group of sequences consisting of SEQ ID NO: 1 representing CDR1, SEQ ID NO: 2 representing CDR2 and SEQ ID NO: 3 representing CDR3 of the Heavy Chain Variable Region (HCVR).

In a further embodiment, the invention relates to a humanized antibody or a fragment thereof, comprising integrated into human- or primate-derived light chain framework regions at least two peptides with an amino acid sequence selected from the group of sequences consisting of SEQ ID NO: 4 representing CDR1, SEQ ID NO: 5 representing CDR2 and SEQ ID NO: 6 representing CDR3 of the Light Chain Variable Region (LCVR).

In particular, the invention relates to a Light Chain Variable Region (LCVR), which has integrated into human- or primate-derived light chain framework regions at least two peptides with an amino acid sequence selected from the group of sequences consisting of SEQ ID NO: 4 representing CDR1, SEQ ID NO: 5 representing CDR2 and SEQ ID NO: 6 representing CDR3 of the Light Chain Variable Region (LCVR), wherein the same CDR cannot be present twice in the antibody and, in particular, at least on of said CDRs must be CDR1 represented by SEQ ID NO: 4.

The invention also relates to a humanized antibody or a fragment thereof, comprising integrated into human- or primate-derived heavy chain framework regions peptides with an amino acid sequence of SEQ ID NO: 1 representing CDR1, SEQ ID NO: 2 representing CDR2 and SEQ ID NO: 3 representing CDR3 of the Heavy Chain Variable Region (HCVR), particularly in the order indicated above.

In particular, the invention relates to a Heavy Chain Variable Region (HCVR) comprising integrated into human- or primate-derived heavy chain framework regions peptides with an amino acid sequence of SEQ ID NO: 1 representing CDR1, SEQ ID NO: 2 representing CDR2 and SEQ ID NO: 3 representing CDR3 of the Heavy Chain Variable Region (HCVR), particularly in the order indicated above.

Also comprised by the invention is a humanized antibody or a fragment thereof comprising integrated into human- or primate-derived light chain framework regions peptides with an amino acid sequence of SEQ ID NO: 4 representing CDR1, SEQ ID NO: 5 representing CDR2 and SEQ ID NO: 6 representing CDR3 of the Light Chain Variable Region (LCVR), particularly in the order indicated above.

In particular, the invention relates to a Light Chain Variable Region (LCVR) comprising integrated into human- or primate-derived light chain framework regions peptides with an amino acid sequence of SEQ ID NO: 4 representing CDR1, SEQ ID NO: 5 representing CDR2 and SEQ ID NO: 6 representing CDR3 of the Light Chain Variable Region (LCVR), particularly in the order indicated above.

The invention also relates to a humanized antibody or a fragment thereof, which comprises integrated into human- or primate-derived framework regions at least three peptides with an amino acid sequence selected from the group of sequences consisting of SEQ ID NO: 1 representing CDR1 , SEQ ID NO: 2 representing CDR2 and SEQ ID NO: 3 representing CDR3 of the Heavy Chain Variable Region (HCVR) and SEQ ID NO: 4 representing CDR1, SEQ ID NO: 5 representing CDR2 and SEQ ID NO: 6 representing CDR3 of the Light Chain Variable Region (LCVR), but particularly a humanized antibody or a fragment thereof wherein the same CDR cannot be present twice in the antibody.

In another embodiment the invention relates to a humanized antibody or a fragment thereof, which antibody comprises integrated into human- or primate-derived framework regions at least four peptides with an amino acid sequence selected from the group of sequences consisting of SEQ ID NO: 1 representing CDR1 , SEQ ID NO: 2 representing CDR2 and SEQ ID NO:3 representing CDR3 of the Heavy Chain Variable Region (HCVR) and SEQ ID NO: 4 representing CDR1, SEQ ID NO: 5 representing CDR2 and SEQ ID NO: 6 representing CDR3 of the Light Chain Variable Region (LCVR), but particularly a humanized antibody or a fragment thereof wherein the same CDR cannot be present twice in the antibody.

In still anther embodiment, the invention relates to a humanized antibody or a fragment thereof, which comprises integrated into human- or primate-derived framework regions at least five peptides with an amino acid sequence selected from the group of sequences consisting of SEQ ID NO: 1 representing CDR1, SEQ ID NO: 2 representing CDR2 and SEQ ID NO:3 representing CDR3 of the Heavy Chain Variable Region (HCVR) and SEQ ID NO: 4 representing CDR1, SEQ ID NO: 5 representing CDR2 and SEQ ID NO: 6 representing CDR3 of the Light Chain Variable Region (LCVR), but particularly a humanized antibody or a fragment thereof wherein the same CDR cannot be present twice in the antibody.

In still anther embodiment, the invention relates to a humanized antibody or a fragment thereof, which comprises integrated into human- or primate-derived framework regions peptides with an amino acid sequence of SEQ ID NO: 1 representing CDR1, SEQ ID NO: 2 representing CDR2 and SEQ ID NO: 3 representing CDR3 of the Heavy Chain Variable Region (HCVR) and SEQ ID NO: 4 representing CDR1, SEQ ID NO: 5 representing CDR2 and SEQ ID NO: 6 representing CDR3 of the Light Chain Variable Region (LCVR).

In a specific embodiment, the invention relates to a humanized antibody, a Heavy Chain Variable Region (HCVR), or a fragment thereof, wherein said humanized antibody, Heavy Chain Variable Region (HCVR) or fragment thereof comprises integrated into human- or primate-derived heavy chain framework regions at least a peptide with an amino acid sequence of SEQ ID NO: 2 representing CDR2 of the Heavy Chain Variable Region (HCVR).

In another specific embodiment, the invention relates to a humanized antibody, a Heavy Chain Variable Region (HCVR) or a fragment thereof, wherein said humanized antibody, Heavy Chain Variable Region (HCVR) or fragment thereof comprises integrated into human- or primate-derived heavy chain framework regions at least a peptide with an amino acid sequence of SEQ ID NO: 3 representing CDR3 of the Heavy Chain Variable Region (HCVR).

In another specific embodiment, the invention relates to a humanized antibody, Heavy Chain Variable Region (HCVR) or a fragment thereof, which antibody, Heavy Chain Variable Region (HCVR) or fragment thereof comprises integrated into human-or primate-derived heavy chain framework regions at least two peptides with an amino acid sequence of SEQ ID NO: 1 representing CDR1 and SEQ ID NO: 2 representing CDR2 of the Heavy Chain Variable Region (HCVR).

In another specific embodiment, the invention relates to a humanized antibody, a Heavy Chain Variable Region (HCVR) or a fragment thereof, which antibody, Heavy Chain Variable Region (HCVR) or fragment thereof comprises integrated into human- or primate-derived heavy chain framework regions at least two peptides with an amino acid sequence of SEQ ID NO: 1 representing CDR1 and SEQ ID NO: 3 representing CDR3 of the Heavy Chain Variable Region (HCVR).

In another specific embodiment, the invention relates to a humanized antibody, a Heavy Chain Variable Region (HCVR) or a fragment thereof, which antibody, Heavy Chain Variable Region (HCVR) or fragment thereof comprises integrated into human- or primate-derived heavy chain framework regions at least two peptides with an amino acid sequence of SEQ ID NO: 2 representing CDR2 and SEQ ID NO: 3 representing CDR3 of the Heavy Chain Variable Region (HCVR).

In another specific embodiment, the invention relates to a humanized antibody, a Light Chain Variable Region (LCVR) or a fragment thereof, which antibody, Light Chain Variable Region (LCVR) or fragment thereof comprises integrated into human-or primate-derived heavy chain framework regions at least two peptides with an amino acid sequence of SEQ ID NO: 4 representing CDR1 and SEQ ID NO: 5 representing CDR2 of the Light Chain Variable Region (LCVR).

In another specific embodiment, the invention relates to a humanized antibody, a Light Chain Variable Region (LCVR) or a fragment thereof, which antibody, Light Chain Variable Region (LCVR) or fragment thereof comprises integrated into human-or primate-derived heavy chain framework regions at least two peptides with an amino acid sequence of SEQ ID NO: 4 representing CDR1 and SEQ ID NO: 6 representing CDR3 of the Light Chain Variable Region (LCVR).

Further comprised by the invention is a humanized antibody or a fragment thereof, wherein both the Heavy Chain Variable Region (HCVR) and the Light Chain Variable Region (LCVR) of the mouse C2 antibody each contributes at least one of its CDR regions to the at least two CDR regions of the humanized antibody. The resulting humanized antibody or a fragment thereof thus may comprise:

- at least an amino acid sequence of SEQ ID NO: 1 representing CDR1 (HCVR) in combination with an amino acid sequence of SEQ ID NO: 4 representing CDR1 (LCVR);

- at least an amino acid sequence of SEQ ID NO: 2 representing CDR2 (HCVR) in combination with an amino acid sequence of SEQ ID NO: 4 representing CDR1 (LCVR); -at least an amino acid sequence of SEQ ID NO: 3 representing CDR3 (HCVR) in combination with an amino acid sequence of SEQ ID NO: 4 representing CDR1 (LCVR);

- at least an amino acid sequence of SEQ ID NO: 1 representing CDR1 (HCVR) in combination with an amino acid sequence of SEQ ID NO: 5 representing CDR2 (LCVR); -at least an amino acid sequence of SEQ ID NO: 2 representing CDR2 (HCVR) in combination with an amino acid sequence of SEQ ID NO: 5 representing CDR2 (LCVR); -at least an amino acid sequence of SEQ ID NO:2 representing CDR2 (HCVR) in combination with an amino acid sequence of SEQ ID NO: 6 representing CDR3 (LCVR); -at least an amino acid sequence of SEQ ID NO:1 representing CDR1 (HCVR) in combination with an amino acid sequence of SEQ ID NO: 6 representing CDR3 (LCVR); -at least an amino acid sequence of SEQ ID NO: 3 representing CDR3 (HCVR) in combination with an amino acid sequence of SEQ ID NO: 5 representing CDR2 (LCVR); -at least an amino acid sequence of SEQ ID NO: 3 representing CDR3 (HCVR) in combination with an amino acid sequence of SEQ ID NO: 6 representing CDR3 (LCVR).

In still another embodiment, the invention relates to a chimeric antibody or a fragment thereof, or a humanized antibody or a fragment thereof as described herein before, which antibody comprises a light chain and/or a heavy chain constant region of human or primate origin.

In a further embodiment, the invention relates to a chimeric antibody or a fragment thereof, or a humanized antibody or a fragment thereof, wherein at least one, particularly at least one but not more than 5, more particularly at least one but not more than 4, even more particularly at least one but not more than 3, but especially at least one but not more than 2, of the amino acids representative of the light chain and/or heavy chain CDR regions as given in SEQ ID NOs: 1-6 is changed through a conservative substitution such that the antibody maintains its full functionality.

In particular, the invention relates to a chimeric antibody or a fragment thereof, or a humanized antibody or a fragment thereof, wherein in CDR2 of the light chain variable region (LCVR) as given in SEQ ID NO: 5, the Lys at Kabat position 50 is replaced by an amino acid residue selected from the group consisting of Arg, Gln and Glu, particularly by Arg.

In particular, the invention relates to a light chain variable region (LCVR) wherein in CDR2 as given in SEQ ID NO: 5, the Lys at Kabat position 50 is replaced by an amino acid residue selected from the group consisting of Arg, Gln and Glu, particularly by Arg.

In another embodiment, the invention relates to a chimeric antibody or a fragment thereof, or a humanized antibody or a fragment thereof, wherein in CDR2 of the light chain variable region (LCVR) as given in SEQ ID NO: 5, the Ser at Kabat position 53 is replaced by an amino acid residue selected from the group consisting of Asn or Thr, but particularly by Asn.

In particular, the invention relates to a light chain variable region (LCVR) wherein in CDR2 as given in SEQ ID NO: 5, the Ser at Kabat position 53 is replaced by an amino acid residue selected from the group consisting of Asn or Thr, but particularly by Asn.

In one embodiment of the invention, a chimeric antibody or a fragment thereof, or a humanized antibody or a fragment thereof is provided, wherein the Heavy Chain Variable Region (HCVR) has an amino acid sequence that is 90%, particularly 95%, more particularly 98% identical to the sequence given in SEQ ID NO: 15 and 16, respectively.

In another embodiment of the invention, a chimeric antibody or a fragment thereof, or a humanized antibody or a fragment thereof is provided, wherein the Light Chain Variable Region (LCVR) has an amino acid sequence that is 90%, particularly 95%, more particularly 98% identical to the sequence given in SEQ ID NO: 12 and 13, respectively.

In still another embodiment of the invention, a humanized antibody or a fragment thereof is provided, wherein at least two, but especially three, of the CDR regions of the Heavy Chain Variable Region (HCVR) have an amino acid sequence that is 90%, particularly 95%, more particularly 98% identical to the corresponding CDR region as given in SEQ ID NO: 1-3.

In a further embodiment of the invention, a humanized antibody or a fragment thereof is provided, wherein at least two, but especially three, of the CDR regions of the Light Chain Variable Region (LCVR) have an amino acid sequence that is 90%, particularly 95%, more particularly 98% identical to the corresponding CDR region as given in SEQ ID NO: 4-6.

In still another embodiment, the invention relates to a chimeric antibody or a fragment thereof, or a humanized antibody or a fragment thereof according to the present invention as described herein before wherein the Heavy Chain Variable Region (HCVR) has an amino acid sequence that is 90%, 91%, 92%, 93%, 94%, 95%, 96%, 97%, 98% or 99% identical to the sequence given in SEQ ID NO: 15 and 16, respectively.

In still another embodiment, the invention relates to a chimeric antibody or a fragment thereof, or a humanized antibody or a fragment thereof according to the present invention as described herein before wherein the Light Chain Variable Region (LCVR) has an amino acid sequence that is 90%, 91%, 92%, 93%, 94%, 95%, 96%, 97%, 98% or 99% identical to the sequence given in SEQ ID NO: 12 and 13, respectively.

In still another embodiment, the invention relates to a chimeric antibody or a fragment thereof, or a humanized antibody or a fragment thereof according to the present invention as described herein before, wherein at least one, particularly at least two, but especially three, of the CDR regions of the Heavy Chain Variable Region (HCVR) have an amino acid sequence that is 90%, 91%, 92%, 93%, 94%, 95%, 96%, 97%, 98% or 99% identical to the corresponding CDR region as given in SEQ ID NO: 1-3.

In still another embodiment, the invention relates to a chimeric antibody or a fragment thereof, or a humanized antibody or a fragment thereof according to the present invention as described herein before, wherein at least one, particularly at least two, but especially three, of the CDR regions of the Light Chain Variable Region (LCVR) have an amino acid sequence that is 90%, 91%, 92%, 93%, 94%, 95%, 96%, 97%, 98% or 99% identical to the corresponding CDR region as given in SEQ ID NO: 4-6.

In still another embodiment, the invention relates to a humanized antibody according to the present invention and as described herein before, wherein at least one of the amino acids representative of the acceptor framework sequences obtained from human germline $V_H$ and $V_K$ sequences, respectively is changed through a substitution to an amino acid from the corresponding region of murine antibody ACI-01-Ab7C2 or a substitution conservative thereto.

In particular, the invention relates to a humanized antibody, wherein the Trp in Kabat position 47 in the acceptor framework sequence obtained from human germline $V_H$ sequences of KABAT subgroup $V_H$III of the Heavy Chain Variable Region as shown in SEQ ID NO: 15 is replaced by an amino acid selected from the group consisting of Leu, norleucine, Ile, Val, Met, Ala, and Phe, particularly Leu and Ile, but especially Leu.

The invention further relates to a humanized antibody, wherein the Arg in Kabat position 94 in the acceptor framework sequence obtained from human germline $V_H$ sequences of KABAT subgroup $V_H$III of the Heavy Chain Variable Region as shown in SEQ ID NO: 15 is replaced by an amino acid selected from the group consisting of Ser and Thr, but especially by Ser.

In still another embodiment, the invention relates to a humanized antibody, wherein the Trp in Kabat position 47 in the acceptor framework sequence obtained from human germline $V_H$ sequences of KABAT subgroup $V_H$III of the Heavy Chain Variable Region as shown in SEQ ID NO: 15 is replaced by an amino acid selected from the group consisting of Leu, norleucine, He, Val, Met, Ala, and Phe, particularly Leu and He, but especially Leu and the Arg in Kabat position 94 is replaced by an amino acid selected from the group consisting of Ser and Thr, but especially by Ser.

The invention further relates to a humanized antibody, wherein the Gln in Kabat position 45 in the acceptor framework sequence obtained from human germline $V_K$ sequences of KABAT subgroup $V_K$II of the Light Chain Variable Region as shown in SEQ ID NO: 12 is replaced by an amino acid selected from the group consisting of Lys, Arg, Gln, and Asn, particularly by Lys and Arg, but especially by Lys.

The invention further relates to a humanized antibody, wherein the Leu in Kabat position 50 in the acceptor framework sequence obtained from human germline $V_K$ sequences of KABAT subgroup $V_K$II of the Light Chain Variable Region as shown in SEQ ID NO: 12 is replaced by an amino acid selected from the group consisting of Lys, Arg, Gln, and Asn, particularly by Lys and Arg, but especially by Lys.

The invention further relates to a humanized antibody, wherein the Tyr in Kabat position 87 in the acceptor framework sequence obtained from human germline $V_K$ sequences of KABAT subgroup $V_K$II of the Light Chain Variable Region as shown in SEQ ID NO: 12 is replaced by an amino acid selected from the group consisting of Phe, Leu, Val, Ile, and Ala, particularly by Leu and Phe, but especially by Phe.

In still another embodiment, the invention relates to a humanized antibody, wherein the Asn in Kabat position 53 in the acceptor framework sequence obtained from human germline $V_K$ sequences of KABAT subgroup $V_K$II of the Light Chain Variable Region as shown in SEQ ID NO: 12 may be replaced by an amino acid selected from the group consisting of Gln, His, Lys and Arg, but especially by His and Gln.

In still another embodiment, the invention relates to a humanized antibody, wherein the Trp in Kabat position 47 in the acceptor framework sequence obtained from human germline $V_H$ sequences of KABAT subgroup $V_H$III of the Heavy Chain Variable Region as shown in SEQ ID NO: 15 is replaced by an amino acid selected from the group consisting of Leu, norleucine, Ile, Val, Met, Ala, and Phe, particularly Leu and Ile, but especially Leu and the Arg in Kabat position 94 in the acceptor framework sequence obtained from human germline $V_H$ sequences of KABAT subgroup $V_H$III of the Heavy Chain Variable Region as shown in SEQ ID NO: 15 is replaced by an amino acid selected from the group consisting of Ser and Thr, but especially by Ser, and the Tyr in Kabat position 87 in the acceptor framework sequence obtained from human germline $V_K$ sequences of KABAT subgroup $V_K$II of the Light Chain Variable Region as shown in SEQ ID NO: 12 is replaced by an amino acid selected from the group consisting of Phe, Leu, Val, Ile, and Ala, particularly by Leu and Phe, but especially by Phe.

In still another embodiment, the invention relates to a humanized antibody, wherein the Trp in Kabat position 47 in the acceptor framework sequence obtained from human germline $V_H$ sequences of KABAT subgroup $V_H$III of the Heavy Chain Variable Region as shown in SEQ ID NO: 15 is replaced by an amino acid selected from the group consisting of Leu, norleucine, Ile, Val, Met, Ala, and Phe, particularly Leu and Ile, but especially Leu.

In still another embodiment, the invention relates to a humanized antibody, wherein the Arg in Kabat position 94 in the acceptor framework sequence obtained from human germline $V_H$ sequences of KABAT subgroup $V_H$III of the Heavy Chain Variable Region as shown in SEQ ID NO: 15 is replaced by an amino acid selected from the group consisting of Ser and Thr, but especially by Ser.

In still another embodiment, the invention relates to a humanized antibody, wherein the Trp in Kabat position 47 in the acceptor framework sequence obtained from human germline $V_H$ sequences of KABAT subgroup $V_H$III of the Heavy Chain Variable Region as shown in SEQ ID NO: 15 is replaced by Leu and Ile, but especially Leu and the Arg in Kabat position 94 in the acceptor framework sequence obtained from human germline $V_H$ sequences of KABAT subgroup $V_H$III of the Heavy Chain Variable Region as shown in SEQ ID NO: 15 is replaced by Ser.

In still another embodiment, the invention relates to a humanized antibody, wherein the Tyr in Kabat position 87 in the acceptor framework sequence obtained from human germline $V_K$ sequences of KABAT subgroup $V_K$II of the Light Chain Variable Region as shown in SEQ ID NO: 12 is replaced by Phe.

In still another embodiment, the invention relates to a humanized antibody, wherein the Trp in Kabat position 47 in the acceptor framework sequence obtained from human germline $V_H$ sequences of KABAT subgroup $V_H$III of the Heavy Chain Variable Region as shown in SEQ ID NO: 15 is replaced by Leu and Ile, but especially Leu, and the Arg in Kabat position 94 in the acceptor framework sequence obtained from human germline $V_H$ sequences of KABAT subgroup $V_H$III of the Heavy Chain Variable Region as shown in SEQ ID NO: 15 is replaced by Ser and the Tyr in Kabat position 87 in the acceptor framework sequence obtained from human germline $V_K$ sequences of KABAT subgroup $V_K$II of the Light Chain Variable Region as shown in SEQ ID NO: 12 is replaced by Phe.

In a specific embodiment, the invention relates to the light chain variable region of SEQ ID NO: 12.

In another specific embodiment of the invention, a humanized antibody is provided, which comprises the light chain variable region of SEQ ID NO: 12.

In a specific embodiment, the invention relates to the light chain variable region including signal sequences as shown in SEQ ID NO: 13.

In another specific embodiment of the invention, a humanized antibody is provided, which comprises the complete light chain variable region including signal sequences as shown in SEQ ID NO: 13.

In another specific embodiment of the invention, a humanized antibody is provided, which comprises the light chain variable region of SEQ ID NO: 12 and the light chain constant region of SEQ ID NO: 14.

In another specific embodiment of the invention, a humanized antibody is provided, which comprises the complete light chain variable region of SEQ ID NO: 13 and the light chain constant region of SEQ ID NO: 14.

In a specific embodiment, the invention relates to the heavy chain variable region of SEQ ID NO: 15.

In another specific embodiment of the invention, a humanized antibody is provided, which comprises the heavy chain variable region of SEQ ID NO: 15.

In a specific embodiment, the invention relates to the heavy chain variable region including signal sequences as shown in SEQ ID NO: 16.

In another specific embodiment of the invention, a humanized antibody is provided, which comprises the complete heavy chain variable region including signal sequences as shown in SEQ ID NO: 16.

In another specific embodiment of the invention, a humanized antibody is provided, which comprises the heavy chain variable region of SEQ ID NO: 15 and the heavy chain constant region of SEQ ID NO: 17.

In another specific embodiment of the invention, a humanized antibody is provided, which comprises the heavy chain variable region of SEQ ID NO: 16 and the heavy chain constant region of SEQ ID NO: 17.

In one embodiment the humanized antibody according to the invention and as described herein, upon co-incubation with an A$\beta$ monomeric peptide having at least 30, particularly at least 35, more particularly at least 38, even more particularly at least 40 amino acid residues and/or an A$\beta$ polymeric soluble amyloid peptide comprising a plurality of said A$\beta$ monomeric units, but especially with an A$\beta_{1-42}$ monomeric and/or an A$\beta$ polymeric soluble amyloid peptide comprising a plurality of said A$\beta_{1-42}$ monomeric units, particularly at a molar concentration ratio of antibody to A$\beta$1-42 of up to 1:1000, but especially at a molar concentration ratio of between 1:10 and 1:100, inhibits the aggregation of the A$\beta$ monomers to high molecular polymeric fibrils.

In particular, the co-incubation of the antibody according to the invention with amyloid monomeric and/or polymeric soluble amyloid peptides is carried out for 24 hours to 60 hours, particularly for 30 hours to 50 hours, more particularly for 48 hours, but especially 24 hours, at a temperature of between 28°C and 40°C, particularly of between 32°C and 38°C, more particularly at 37°C.

In a specific embodiment of the invention, co-incubation with amyloid monomeric and/or polymeric soluble amyloid peptides is accomplished for 24 hours at a temperature of 37°C.

In particular, the antibody, particularly the humanized antibody according to the invention including any functionally equivalent antibody or functional parts thereof binds to A$\beta_{1-42}$ monomeric peptide and/or A$\beta$ polymeric soluble amyloid peptide comprising a plurality of said A$\beta_{1-42}$ monomeric units and, upon co-incubation with A$\beta_{1-42}$ monomeric peptide and/or A$\beta$ polymeric soluble amyloid peptide comprising a plurality of said A$\beta_{1-42}$ monomeric units inhibits the aggregation of the A$\beta$ monomers and/or polymers to high molecular polymeric fibrils.

In one embodiment, the antibody, particularly the humanized antibody according to the invention including any functionally equivalent antibody or functional parts thereof inhibits the aggregation of the A$\beta$ monomers and/or A$\beta$ soluble polymers comprising a plurality of said A$\beta$ monomeric units to high molecular polymeric fibrils by at least 50%, particularly by at least 60%, particularly by at least 65%, more particularly by at least 75%, even more particularly by at least 80%, but especially by at least 85%-90%, or more as compared to the respective amyloid peptide monomers incubated in buffer (control), at a molar concentration ratio of antibody to A$\beta$1-42 of up to 1:1000, particularly at a molar concentration ratio of between 1:10 and 1:100, but especially at a molar concentration ratio of 1:10.

In a specific embodiment of the invention, the antibody, particularly the humanized antibody according to the invention including any functionally equivalent antibody or functional parts thereof inhibits the aggregation of the A$\beta$ monomers and/or A$\beta$ soluble polymers comprising a plurality of said A$\beta$ monomeric units to high molecular polymeric fibrils by at least 30% at a molar concentration ratio of antibody to A$\beta$1-42 of 1:100.

In another specific embodiment of the invention, the antibody, particularly the humanized antibody according to the invention including any functionally equivalent antibody or functional parts thereof inhibits the aggregation of the A$\beta$ monomers and/or A$\beta$ soluble polymers comprising a plurality of said A$\beta$ monomeric units to high molecular polymeric fibrils by at least 80% at a molar concentration ratio of antibody to A$\beta$1-42 of 1:10.

Binding of the antibodies according to the invention and as described herein to amyloidogenic monomeric and/or polymeric peptides but, particularly, to the amyloid form (1-42) leads to inhibition of the aggregation of monomeric and/or polymeric amyloidogenic peptides to high molecular fibrils or filaments. Through the inhibition of the aggregation of amyloidogenic monomeric and/or polymeric peptides the antibodies according to the present invention are capable of preventing or slowing down the formation of amyloid plaques, particularly the amyloid form (1-42), which is know to become insoluble by change of secondary conformation and to be the major part of amyloid plaques in brains of diseased animals or humans.

The aggregation inhibition potential of the antibody according to the invention may be determined by any suitable method known in the art, particularly by density-gradient ultracentrifugation followed by an SDS-PAGE sedimentation analysis on a preformed gradient and/or by a thioflavin T (Th-T) fluorescent assay.

In one embodiment, the invention relates to an antibody, particularly a humanized antibody as described herein including any functionally equivalent antibody or functional parts thereof, which antibody, upon co-incubation, particularly at a molar concentration ratio of between 1:10 and 1:1000, more particularly at a ratio of 1:100 with preformed high molecular polymeric amyloid fibrils or filaments formed by the aggregation of A$\beta$ monomeric peptides having at least 30, particularly at least 35, more particularly at least 38, even more particularly at least 40 amino acid residues and, but especially A$\beta_{1-42}$ monomeric peptides, is capable of disaggregating the preformed polymeric fibrils or filaments by at least 20%, particularly by at least 30%, more particularly by at least 35%%, even more particularly by at least 40%, but especially by at least 50% or more.

In a specific embodiment of the invention, the aggregation inhibition and the disaggregation potential of the antibody, respectively, is determined by density-gradient ultracentrifugation followed by a SDS-PAGE sedimentation analysis on a preformed gradient.

In another specific embodiment of the invention, the aggregation inhibition and the disaggregation potential of the antibody, respectively, is determined by thioflavin T (Th-T) fluorescent assay.

In another specific embodiment, the antibody according to the invention is co-incubated with amyloid preformed high molecular polymeric amyloid fibrils or filaments for 12 hours to 36 hours, particularly for 18 hours to 30 hours, more particularly for 24 hours at a temperature of between 28°C and 40°C, particularly of between 32°C and 38°C, more particularly at 37°C.

In particular, the co-incubation with preformed high molecular polymeric amyloid fibrils or filaments is done for 24 hours at a temperature of 37°C.

In a specific embodiment of the invention, the antibody, particularly the humanized antibody according to the invention including any functionally equivalent antibody or functional parts thereof is capable of disaggregating the preformed polymeric fibrils or filaments by at least 24% at a molar concentration ratio of antibody to A$\beta$1-42 of 1:100.

In another specific embodiment of the invention, the antibody, particularly the humanized antibody according to the invention including any functionally equivalent antibody or functional parts thereof is capable of disaggregating the preformed polymeric fibrils or filaments by at least 32% at a molar concentration ratio of antibody to A$\beta$1-42 of 1:10.

Through the disaggregation of amyloidogenic polymeric fibrils or filaments the antibodies according to the present invention are capable of preventing or slowing down the formation of amyloid plaques which leads to an alleviation of the symptoms associated with the disease and a delay or reversal of its progression.

Accordingly, it is a further embodiment of the invention to provide an antibody, particularly a humanized antibody, including any functionally equivalent antibody or functional parts thereof as described herein, which antibody is capable of decreasing the total amount of A$\beta$ in the brain of an animal, particularly a mammal, but especially a human suffering from a disease or condition leading to increased concentration of A$\beta$ in the brain.

In another embodiment, the invention relates to a humanized antibody according to the invention and as described herein before, which antibody is bi-effective in that it exhibits both an aggregation inhibition property as well as a disaggregation property, particularly paired with a high degree of conformational sensitivity.

In particular, the invention relates to a chimeric antibody or a fragment thereof, or a humanized antibody or a fragment thereof according to the invention and as described herein before, which antibody, upon co-incubation with amyloid monomeric and/or polymeric soluble amyloid peptides, particularly with $\beta$-amyloid monomeric peptides such as, for example, A$\beta$ monomeric peptides 1-39; 1-40, 1-41, or 1-42, and/or a polymeric soluble $\beta$-amyloid peptide comprising a plurality of said A$\beta$ monomeric units, but especially with an A$\beta_{1-42}$ monomeric and/or an A$\beta$ polymeric soluble amyloid peptide comprising a plurality of said A$\beta_{1-42}$ monomeric units, inhibits the aggregation of the A$\beta$ monomers into high molecular polymeric fibrils or filaments and, in addition, upon co-incubation with preformed high molecular polymeric amyloid fibrils or filaments formed by the aggregation of amyloid monomeric peptides, particularly $\beta$-amyloid monomeric peptides such as, for example, A$\beta$ monomeric peptides 1-39; 1-40, 1-41, or 1-42, but especially A$\beta_{1-42}$ monomeric peptides, is capable of disaggregating the preformed polymeric fibrils or filaments.

In another aspect, the invention relates to a chimeric antibody or a fragment thereof, or a humanized antibody or a fragment thereof according to the present invention and as described herein before, which antibody is capable of inducing a transition of the $\beta$-sheet conformation towards an $\alpha$-helix and/or a random coil conformation, but particularly a random coil conformation, even more particularly a random coil conformation at a given location in the molecule, especially in the environment of Tyr 10 and Val12 of the A$\beta$ protein, which leads to an increase of the random coil conformation at the expense of the $\beta$-sheet conformation and an improved solubilization of the preformed high molecular polymeric amyloid fibrils or filaments. In particular the decrease of the $\beta$-sheet conformation amounts to at least 30%, particularly to at least 35%, and more particularly to at least 40% and more as compared to the respective preformed amyloid polymeric fibrils or filaments incubated in buffer (control).

The antibody's potential in inducing a transition in the secondary structure is determined by solid state 13C NMR spectroscopy but, in particular, by measuring the integral intensities of the conformations of Tyr 10 and Val 12 C$\beta$ in the A$\beta_{1-42}$ peptide.

In a further embodiment of the invention, a chimeric antibody or a fragment thereof, or a humanized antibody or a fragment thereof according to the present invention and as described herein before, is provided comprising at least one light chain or a fragment thereof or at least one heavy chain or a fragment thereof, wherein said antibody or fragment binds to an A$\beta$ monomer with a binding affinity of at least about $1 \times 10^{-7}$ to at least about $1 \times 10^{-12}$, particularly of at least about 1 x $10^{-8}$ to at least about 1 x $10^{-11}$, more particularly of at least about 1 x $10^{-9}$ to at least about 1 x $10^{-10}$, even more particularly of at least about 1 x $10^{-8}$ to at least about 2 x $10^{-8}$., but, preferably, does not show any significant cross-reactivity with amyloid precursor protein (APP).

In another embodiment of the invention, a chimeric antibody or a fragment thereof, or a humanized antibody or a fragment thereof according to the present invention and as described herein before, is provided comprising at least one light chain or a fragment thereof or at least one heavy chain or a fragment thereof, wherein said antibody or fragment binds to an A$\beta$ fiber, fibril or filament with a binding affinity of at least about 1 x $10^{-7}$ to at least about 1 x $10^{-12}$, particularly of at least about 1 x $10^{-8}$ to at least about 1 x $10^{-11}$, more particularly of at least about 1 x $10^{-9}$ to at least about 1 x $10^{-10}$, even more particularly of at least about 2 x $10^{-9}$ to at least about 5 x $10^{-9}$, but, preferably, does not show any significant cross-reactivity with amyloid precursor protein (APP).

In another embodiment, the antibody according to the invention and as described herein before or a fragment thereof, exhibits an binding affinity to an A$\beta$ fiber, fibril or filament which is at least 10 times, particularly at least 15 times, more particularly at least 20 times, but especially at least 25 times higher than the binding affinity to an A$\beta$ monomer.

In still another embodiment, a chimeric antibody or a fragment thereof, or a humanized antibody or a fragment thereof is provided as described herein before, which antibody substantially binds to aggregated A$\beta$, including A$\beta$ plaques, in the mammalian, particularly the human brain but, preferably, does not show any significant cross-reactivity with amyloid precursor protein (APP).

In another aspect of the invention, the chimeric antibody or a fragment thereof, or a humanized antibody or a fragment thereof is provided as described herein before, which antibody substantially binds to soluble polymeric amyloid, particularly amyloid $\beta$ (A$\beta$), including A$\beta$ monomers, in the mammalian, particularly the human brain but, preferably, does not show any significant cross-reactivity with amyloid precursor protein (APP).

Further provided is a chimeric antibody or a fragment thereof, or a humanized antibody or a fragment thereof according to the invention and as described herein before, which antibody significantly reduces A$\beta$ plaque burden in the mammalian, particularly the human brain. This can be achieved by either binding of the antibody to the plaque or by shifting the equilibrium between amyloid, particularly amyloid $\beta$ (A$\beta$), in its insoluble and aggregated state towards its soluble form by disaggregating fibers to soluble poly- and monomeric forms by inducing a shift in conformation and binding and stabilizing the disaggregated and solubilized amyloid forms, particularly amyloid $\beta$ (A$\beta$) forms, in the tissue and/or body fluids of a subject, particularly a mammal, and even more particularly a human, and in particular in the brain of the subject. Through the activity of the antibody according to the invention the peripheral clearing and catabolism is thus favored rather than deposition within the tissue and/or body fluids of the subject, particularly the brain. The beneficial effect of the antibody according to the invention can thus be obtained without binding of the antibody to the plaque.

Through this stabilizing activity, the antibody according to the invention is able to neutralize the toxic effects of the polymeric and less aggregated soluble amyloid protein, particularly amyloid $\beta$ (A$\beta$) protein, in the tissue and/or body fluids of a subject, particularly a mammal, and even more particularly a human, and in particular in the brain of the subject. In a specific embodiment of the invention the antibody according to the invention may thus achieve its beneficial effects without necessarily binding aggregated amyloid beta in the brain of the subject.

In a further aspect of the invention a humanized antibody or a fragment thereof according to the present invention and as described herein before, is provided comprising at least one light chain or a fragment thereof or at least one heavy chain or a fragment thereof incorporating at least one, particularly two and more particularly three CDR regions obtained form a mouse donor antibody, particularly from mouse antibody ACI-01-Ab7C2 (named "mC2" and "hC2" for the humanized C2 antibody, throughout the application) deposited 01 December 2005 with the Deutsche Sammlung von Mikroorganismen und Zellkulturen GmbH (DSMZ) in Braunschweig, Mascheroder Weg 1 B, 38124 Braunschweig, under accession no DSM ACC2750, wherein said antibody or fragment thereof has an affinity to the A$\beta$ antigen which is at least 5 times, particularly at least 8 times, more particularly at least 10 times, but especially at least 15 times higher than that of the mouse donor antibody.

The antibody of this invention can be, in one embodiment, a whole antibody (e.g., with two full length light chains and two full length heavy chains) of any isotype and subtype (e.g., IgM, IgD, IgG1, IgG2, IgG3, IgG4, IgE, IgA1 and IgA2); but especially an antibody of the IgG4 isotype; alternatively, in another embodiment, it can be an antigen-binding fragment (e.g., Fab, F(ab')$_2$, and Fv) of a whole antibody.

The invention thus also relates to antigen-binding fragments of the antibodies described herein. In one embodiment of the invention, the fragment is selected from the group consisting of a Fab fragment, a Fab' fragment, a F(ab)$_2$ fragment, and a F$_v$ fragment, including the products of an Fab immunoglobulin expression library and epitope-binding fragments of any of the antibodies and fragments mentioned above.

In another embodiment, the antibody or antigen-binding fragment of the invention is conjugated to polyethylene glycol. In yet another embodiment, the constant region of the antibody of the invention is modified to reduce at least one constant region-mediated biological effector function relative to an unmodified antibody. In still another embodiment, the antibody or antigen-binding fragment of the invention comprises an Fc region having an altered effector function.

The invention further relates to a nucleotide molecule comprising a nucleotide sequence encoding a chimeric antibody or a fragment thereof, or a humanized antibody or a fragment thereof according to the invention and as disclosed herein before.

In particular, the invention relates to a nucleotide molecule comprising a nucleotide sequence encoding a stretch of contiguous amino acid molecules as given in SEQ ID NO: 2 and 3, respectively, or the complementary sequence, representing the Complementarity Determining Regions (CDRs) 2 and 3 of the Heavy Chain Variable Region (HCVR).

More particularly, the invention relates to a nucleotide molecule comprising a nucleotide sequence encoding a stretch of contiguous amino acid molecules as given in SEQ ID NO: 4, or the complementary sequence, representing the Complementarity Determining Regions (CDRs) 1 of the Light Chain Variable Region (LCVR).

In another embodiment of the invention a nucleotide molecule is provided comprising a nucleotide sequence as given in SEQ ID NO: 18 and SEQ ID NO: 19, or the complementary sequence, encoding the amino acid sequence of CDR 2 and CDR 3, respectively, of the Heavy Chain Variable Region (HCVR).

In another embodiment of the invention a nucleotide molecule is provided comprising a nucleotide sequence as given in SEQ ID NO: 20, or the complementary sequence, encoding the nucleotide sequence of CDR 1 of the Light Chain Variable Region (LCVR).

In another embodiment of the invention a nucleotide molecule is provided comprising a nucleotide sequence of SEQ ID NO: 21, or the complementary sequence, encoding the light chain variable region.

In another embodiment of the invention a nucleotide molecule is provided comprising a nucleotide sequence of SEQ ID NO: 22, or the complementary sequence, encoding the complete light chain variable region including signal sequences.

In another embodiment of the invention a nucleotide molecule is provided comprising a nucleotide sequence encoding the light chain variable region of SEQ ID NO: 22 and the light chain constant region of SEQ ID NO: 23. The invention also comprises the complementary strand of said nucleotide molecule.

In another embodiment of the invention a nucleotide molecule is provided comprising a nucleotide sequence of SEQ ID NO: 24 encoding the heavy chain variable region. The invention also comprises the complementary strand of said nucleotide molecule.

In another embodiment of the invention a nucleotide molecule is provided comprising a nucleotide sequence of SEQ ID NO: 25 encoding the complete heavy chain variable region including signal sequences. The invention also comprises the complementary strand of said nucleotide molecule.

In another embodiment of the invention a nucleotide molecule is provided comprising a nucleotide sequence encoding the heavy chain variable region of SEQ ID NO: 25 and the heavy chain constant region of SEQ ID NO: 26. The invention also comprises the complementary strand of said nucleotide molecule.

Also comprised by the present invention is a nucleotide sequence which hybridizes to one of the above-described antibody-encoding nucleotide sequences of the invention, particularly to the complementary strand thereof, either in isolation or as part of larger nucleotide molecule.

In particular, the invention relates to a nucleotide sequence that hybridizes under conventional hybridization conditions, particularly under stringent hybridization conditions, to any of the nucleotide sequences given in SEQ ID NOs: 18-26 and 29-32, particularly to the complementary strand thereof.

In another embodiment of the invention an expression vector is provided comprising the nucleic acid molecule according to the invention and as mentioned herein before.

In another embodiment of the invention a cell is provided comprising an expression vector comprising the nucleic acid according to the invention and as mentioned herein before.

In still another embodiment, the invention relates to a composition comprising the antibody according to the invention, but particularly a chimeric antibody or a fragment thereof, or a humanized antibody or a fragment thereof according to the invention and as described herein before including any functionally equivalent antibody or any derivative or functional parts thereof, in a therapeutically effective amount, in particular a composition which is a pharmaceutical or therapeutic composition, optionally further comprising a pharmaceutically acceptable carrier.

In another embodiment of the invention, said composition comprises the antibody in a therapeutically effective amount.

Further comprised by the invention is a composition comprising an antibody, particularly a monoclonal antibody according to the invention, but particularly a chimeric antibody or a fragment thereof, or a humanized antibody or a fragment thereof according to the invention and as described herein before including any functionally equivalent antibody or any derivative or functional parts thereof, in a therapeutically effective amount and, optionally, a further biologically active substance and/or a pharmaceutically acceptable carrier and/or a diluent and/or an excipient.

In particular, the invention relates to a composition or mixture, wherein the further biologically active substance is a compound used in the medication of amyloidosis, a group of diseases and disorders associated with amyloid or amyloid-like protein such as the $A\beta$ protein involved in Alzheimer's disease.

In another embodiment of the invention, the other biologically active substance or compound may also be a therapeutic agent that may be used in the treatment of amyloidosis caused by amyloid $\beta$ or may be used in the medication of other neurological disorders.

The other biologically active substance or compound may exert its biological effect by the same or a similar mechanism as the antibody according to the invention or by an unrelated mechanism of action or by a multiplicity of related and/or unrelated mechanisms of action.

Generally, the other biologically active compound may include neutron-transmission enhancers, psychotherapeutic drugs, acetylcholine esterase inhibitors, calcium-channel blockers, biogenic amines, benzodiazepine tranquillizers, acetylcholine synthesis, storage or release enhancers, acetylcholine postsynaptic receptor agonists, monoamine oxidase-A or -B inhibitors, N-methyl-D-aspartate glutamate receptor antagonists, non-steroidal anti-inflammatory drugs, antioxidants, and serotonergic receptor antagonists.

More particularly, the invention relates to a composition or mixture comprising at least one compound selected from the group consisting of compounds effective against oxidative stress, anti-apoptotic compounds, metal chelators, inhibitors of DNA repair such as pirenzepin and metabolites, 3-amino-1-propanesulfonic acid (3APS), 1,3-propanedisulfonate (1,3PDS), $\alpha$-secretase activators, $\beta$ and $\gamma$-secretase inhibitors, tau proteins, neurotransmitter, $\beta$-sheet breakers, attractants for amyloid beta clearing/depleting cellular components, inhibitors of N-terminal truncated amyloid beta including pyroglutamated amyloid beta 3-42, anti-inflammatory molecules, or cholinesterase inhibitors (ChEIs) such as tacrine, rivastigmine, donepezil, and/or galantamine, M1 agonists and other drugs including any amyloid or tau modifying drug and nutritive supplements, and nutritive supplements, together with an antibody according to the present invention and, optionally, a pharmaceutically acceptable carrier and/or a diluent and/or an excipient.

The invention further relates to a composition or mixture, wherein the compound is a cholinesterase inhibitor (ChEIs), particularly a mixture, wherein the compound is one selected from the group consisting of tacrine, rivastigmine, donepezil, galantamine, niacin and memantine.

In a further embodiment, the compositions according to the invention may comprise niacin or memantine together with an antibody according to the present invention and, optionally, a pharmaceutically acceptable carrier and/or a diluent and/or an excipient.

In still another embodiment of the invention compositions are provided that comprise "atypical antipsychotics" such as clozapine, ziprasidone, risperidone, aripiprazole or olanzapine, for the treatment of positive and negative psychotic symptoms including hallucinations, delusions, thought disorders (manifested by marked incoherence, derailment, tangentiality), and bizarre or disorganized behavior, as well as anhedonia, flattened affect, apathy, and social withdrawal, together with an antibody, particularly a monoclonal antibody according to the invention, more particularly a chimeric antibody or a fragment thereof, or a humanized antibody or a fragment thereof according to the invention and as described herein, and, optionally, a pharmaceutically acceptable carrier and/or a diluent and/or an excipient.

In a specific embodiment of the invention, the compositions and mixtures according to the invention and as described herein before comprise the antibody and the biologically active substance, respectively, in a therapeutically effective amount.

Other compounds that can be suitably used in mixtures in combination with the antibody according to the present invention are described in WO 2004/058258 (see especially pages 16 and 17) including therapeutic drug targets (page 36-39), alkanesulfonic acids and alkanolsulfuric acids (pages 39-51), cholinesterase inhibitors (pages 51-56), NMDA receptor antagonists (pages 56-58), estrogens (pages 58-59), non-steroidal anti-inflammatory drugs (pages 60-61), antioxidants (pages 61-62), peroxisome proliferators-activated receptor (PPAR) agonists (pages 63-67), cholesterol-lowering agents (pages 68-75); amyloid inhibitors (pages 75-77), amyloid formation inhibitors (pages 77-78), metal chelators (pages 78-79), anti-psychotics and anti-depressants (pages 80-82), nutritional supplements (pages 83-89) and compounds increasing the availability of biologically active substances in the brain (see pages 89-93) and prodrugs (pages 93 and 94).

In another embodiment, the invention relates to a composition comprising the antibody, particularly a monoclonal antibody according to the invention, more particularly a chimeric antibody or a fragment thereof, or a humanized antibody or a fragment thereof according to the invention and as described herein before and/or the biologically active substance in a therapeutically effective amount.

The invention further relates to the use of an antibody, particularly a monoclonal antibody according to the invention, more particularly a chimeric antibody or a fragment thereof, or a humanized antibody or a fragment thereof according to the invention and as described herein before and/or a functional part thereof and/or a pharmaceutical composition, or a mixture comprising said antibody, for the preparation of a medicament for treating or alleviating the effects of amyloidosis, a group of diseases and disorders associated with amyloid plaque formation, including secondary amyloidosis and age-related amyloidosis, and neurological disorders, such as Alzheimer's Disease (AD), Lewy body dementia (LBD), Down's syndrome, hereditary cerebral hemorrhage with amyloidosis (Dutch type), the Guam Parkinson-Dementia complex, as well as other diseases which are based on or associated with amyloid-like proteins, such as progressive supranuclear palsy, multiple sclerosis; Creutzfeld Jacob disease, Parkinson's disease, HIV-related dementia, ALS (amyotropic lateral sclerosis), Adult Onset Diabetes; senile cardiac amyloidosis; endocrine tumors, and other diseases, including ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

Also comprised by the present invention is a method for the preparation of an antibody, particularly a monoclonal antibody according to the invention, more particularly a chimeric antibody or a fragment thereof, or a humanized antibody or a fragment thereof according to the invention and as described herein before and/or a functional part thereof and/or a pharmaceutical composition, or a mixture comprising said antibody and/or a functional part thereof, particularly in a therapeutically effective amount, comprising formulating an antibody, particularly a monoclonal antibody according to the invention, more particularly a chimeric antibody or a fragment thereof, or a humanized antibody or a fragment thereof according to the invention in a pharmaceutically acceptable form, for use in a method of preventing, treating or alleviating the effects of amyloidosis, a group of diseases and disorders associated with amyloid plaque formation, including secondary amyloidosis and age-related amyloidosis, and neurological disorders such as Alzheimer's Disease (AD), Lewy body dementia (LBD), Down's syndrome, hereditary cerebral hemorrhage with amyloidosis (Dutch type), the Guam Parkinson-Dementia complex, as well as other diseases which are based on or associated with amyloid-like proteins, such as progressive supranuclear palsy, multiple sclerosis; Creutzfeld Jacob disease, Parkinson's disease, HIV-related dementia, ALS (amyotropic lateral sclerosis), Adult Onset Diabetes; senile cardiac amyloidosis; endocrine tumors, and other diseases, including ocular diseases associated with pathological abnormalities/changes in the tissues of the visual system, particularly associated with amyloid-beta-related pathological abnormalities/changes in the tissues of the visual system, such as neuronal degradation. The pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

Further comprised by the present invention is a method for preventing, treating or alleviating the effects of amyloidosis, a group of diseases and disorders associated with amyloid plaque formation including secondary amyloidosis and age-related amyloidosis, and neurological disorders such as Alzheimer's Disease (AD), Lewy body dementia (LBD), Down's syndrome, hereditary cerebral hemorrhage with amyloidosis (Dutch type), the Guam Parkinson-Dementia complex, as well as other diseases which are based on or associated with amyloid-like proteins, such as progressive supranuclear palsy, multiple sclerosis; Creutzfeld Jacob disease, Parkinson's disease, HIV-related dementia, ALS (amyotropic lateral sclerosis), Adult Onset Diabetes; senile cardiac amyloidosis; endocrine tumors, and other diseases, including ocular diseases associated with pathological abnormalities/changes in the tissues of the visual system, particularly associated with amyloid-beta-related pathological abnormalities/changes in the tissues of the visual system, such as neuronal degradation, wherein the pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy, by administering an antibody and/or a functional part thereof, more particularly a humanized antibody and/or a functional part thereof, or a composition or mixture comprising such an antibody and/or a functional part thereof, to a subject, including a mammal, or a human affected by such a disorder, in a therapeutically effective amount.

It is also an object of the invention to provide a method for the treatment of amyloidosis, a group of diseases and disorders associated with amyloid plaque formation including secondary amyloidosis and age-related amyloidosis including, but not limited to, neurological disorders such as Alzheimer's Disease (AD), and other diseases, including ocular diseases associated with pathological abnormalities/changes in the tissues of the visual system, particularly associated with amyloid-beta-related pathological abnormalities/changes in the tissues of the visual system, such as neuronal degradation, wherein the pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy, by administering to the subject, particularly a mammal or a human affected by such a disorder, an antibody, particularly a pharmaceutical composition according to the invention and as described herein.

In a specific embodiment the invention provides a method for retaining or increasing cognitive memory capacity but, particularly, for restoring the cognitive memory capacity of a subject, particularly a mammal or a human, suffering from memory impairment by administering to the subject, particularly a mammal or a human in need thereof, an antibody, particularly a pharmaceutical or therapeutic composition according to the invention and as described herein before.

It is a further object of the invention to provide a therapeutic composition and a method of producing such a composition as well as a method for the treatment of amyloidosis, a group of diseases and disorders associated with amyloid plaque formation including secondary amyloidosis and age-related amyloidosis including, but not limited to, neurological disorders such as Alzheimer's Disease (AD) and other diseases, including ocular diseases associated with pathological abnormalities/changes in the tissues of the visual system, particularly associated with amyloid-beta-related pathological abnormalities/changes in the tissues of the visual system, such as neuronal degradation. The pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

The invention further relates to a method of diagnosis of an amyloid-associated disease or condition comprising detecting the immunospecific binding of an antibody or an active fragment thereof to an epitope of the amyloid protein in a sample or *in situ* which includes the steps of:

(a) bringing the sample or a specific body part or body area suspected to contain the amyloid protein into contact with an antibody, particularly a monoclonal antibody according to the invention, but particularly a chimeric antibody or a fragment thereof, or a humanized antibody or a fragment thereof according to the invention and as described herein before, and/or a functional part thereof, which antibody binds an epitope of the amyloid protein;

(b) allowing the antibody and/or a functional part thereof, to bind to the amyloid protein to form an immunological complex;

(c) detecting the formation of the immunological complex; and

(d) correlating the presence or absence of the immunological complex with the presence or absence of amyloid protein in the sample or specific body part or area of the subject.

Also comprised is a method of determining the extent of amyloidogenic plaque burden in a tissue and/or body fluids comprising

(a) obtaining a sample representative of the tissue and/or body fluids under investigation;

(b) testing said sample for the presence of amyloid protein with an antibody, particularly a monoclonal antibody according to the invention, more particularly a chimeric antibody or a fragment thereof, or a humanized antibody or a fragment thereof according to the invention and as described herein before, and/or a functional part thereof;

(c) determining the amount of antibody bound to the protein; and

(d) calculating the plaque burden in the tissue and/or body fluids.

In particular, the invention relates to a method of determining the extent of amyloidogenic plaque burden in a tissue and/or body fluids in need thereof, wherein the formation of the immunological complex in step c) is determined such that presence or absence of the immunological complex correlates with presence or absence of amyloid protein.

In another embodiment of the invention, a test kit for detection and diagnosis of amyloid-associated diseases and conditions in a subject is provided comprising an antibody, particularly a monoclonal antibody according to the invention, but particularly a chimeric antibody or a fragment thereof, or a humanized antibody or a fragment thereof according to the invention and as described herein before, and/or a functional part thereof.

In particular, the invention relates to a test kit for detection and diagnosis of amyloid-associated diseases and conditions comprising a container holding one or more antibodies according to the present invention, and/or a functional part thereof, and instructions for using the antibodies for the purpose of binding to amyloid protein to form an immunological complex and detecting the formation of the immunological complex such that presence or absence of the immunological complex correlates with presence or absence of amyloid protein.

In another aspect, the invention provides methods and compositions for preventing, treating, or detecting a disease associated with amyloidosis in a subject in need thereof using immunoglobulins as described herein that further comprise a variant Fc region, wherein said variant Fc region comprises at least one amino acid modification relative to a wild-type Fc region. The Fc region mediates the effector function of the antibody or fragment thereof. By modulating the ability of the Fc portion of the antibody or fragment thereof to bind to or activate its receptor, it is possible to abrogate or enhance the effector function of the antibody or fragment thereof.

Thus, in another aspect, the invention provides an antibody or fragment thereof of the invention further comprising a variant Fc region comprising at least one amino acid mutation which decreases effector function. In one such aspect, the at least one amino acid mutation decreases glycosylation of the antibody or fragment thereof. In another such aspect, the at least one amino acid mutation decreases binding to a cognate Fc receptor. In another such aspect, the at least one amino acid mutation decreases activation of a cognate Fc receptor upon binding of the antibody or fragment thereof. In one such aspect, the variant Fc region is a variant IgG1 Fc region. In one such aspect, the antibody or fragment thereof comprises a D265A mutation in the Fc region.

In another aspect, the invention provides an antibody or fragment thereof of the invention further comprising a variant Fc region comprising at least one amino acid mutation which increases effector function. In one such aspect, the at least one amino acid mutation enhances glycosylation of the antibody or fragment thereof. In another such aspect, the at least one amino acid mutation increases binding to a cognate Fc receptor. In another such aspect, the at least one amino acid mutation increases activation of a cognate Fc receptor upon binding of the antibody or fragment thereof. These and other objects, features and advantages of the present invention will become apparent after a review of the following detailed description of the disclosed embodiment and the appended claims.

**BRIEF DESCRIPTION OF THE DRAWINGS**

Figure 1 (Example 2): Chimeric antibody heavy chain expression vector

Figure 2 (Example 2): Chimeric antibody light chain expression vector

Figures 3-1 and 3-2 (Example 2): Expression Cassette of the mouse light chain variable region of the Chimeric Antibody

Figures 4-1 and 4-2 (Example 2): Expression Cassette of the mouse heavy chain variable region of the Chimeric Antibody

Figure 5 (Example 5.2): Comparison of the mouse heavy chain variable region to the closest murine germ line sequence

Figure 6 (Example 8): Activity of purified humanized C2 antibodies

Figure 7 (Example 9): Binding activity of antibodies produced by transient expression of C2 modified CDRL2 constructs in conjunction with C2 chimeric heavy chain, compared to chimeric antibody C2ChVHAF/ChVK, produced by transient transfection and purified antibody.

Figure 8 (Example 11): Results of Immunohistochemical Binding Assay with chimeric antibody AF (IgG4) and humanized antibody H4K1 (IgG4)

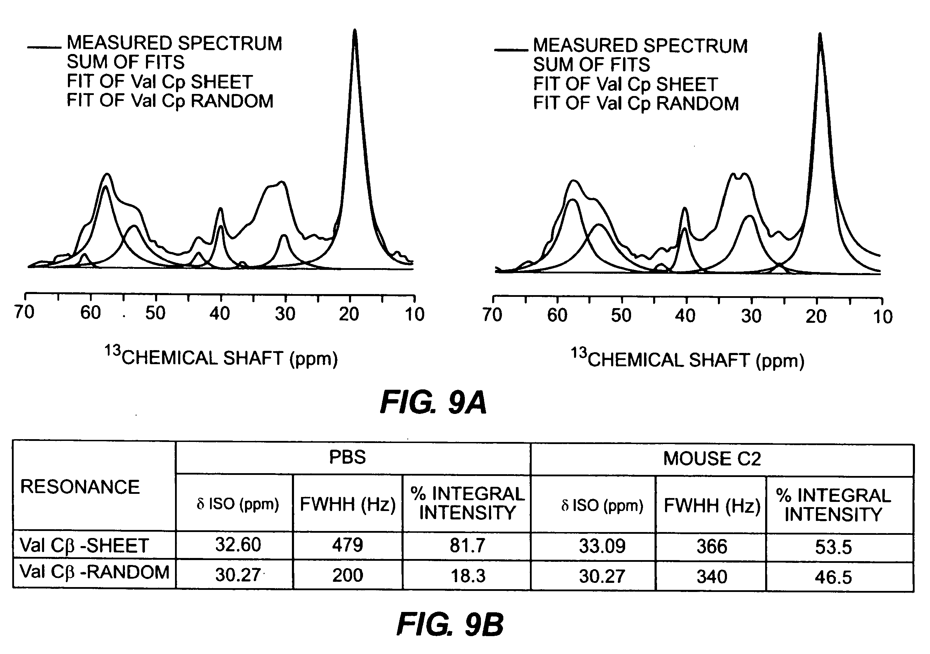

Figure 9 (Example 12): Functionality of mC2 on Amyloid fibers. (A) Comparison of 13C CPMAS spectra and fits for U-13C Tyr10 and Val12 labelled amyloid $\beta$1-42 fibers incubated with PBS (left served as control) or ACI-7-C2 (right) for 24 hours and then lyophilized. The peak at c33 ppm corresponds to the beta sheet conformation of the fibers whilst the peak at 30 ppm is a result of random coil conformation. (B) Comparison of the fitted parameters for the two conformations of Val12 C$\beta$. The fitted chemical shifts for the two conformations are quite similar but the integral intensities are very different, reflecting a reduction in the original beta sheet conformation by approximately 35% (1-(53.5/81.7)), in agreement with the value obtained from the fluorescent measurement

Figure 10 (Example 12): Binding Affinity of humanized C2 in ELISA.

Figure 11 (Example 14): Conformation-specific binding of mC2 to different classes of Amyloid Protein. Pellet preparation in the legend to this figure refers to A$\beta_{1-42}$ fibers, supernatant preparation refers to amyloid monomers.

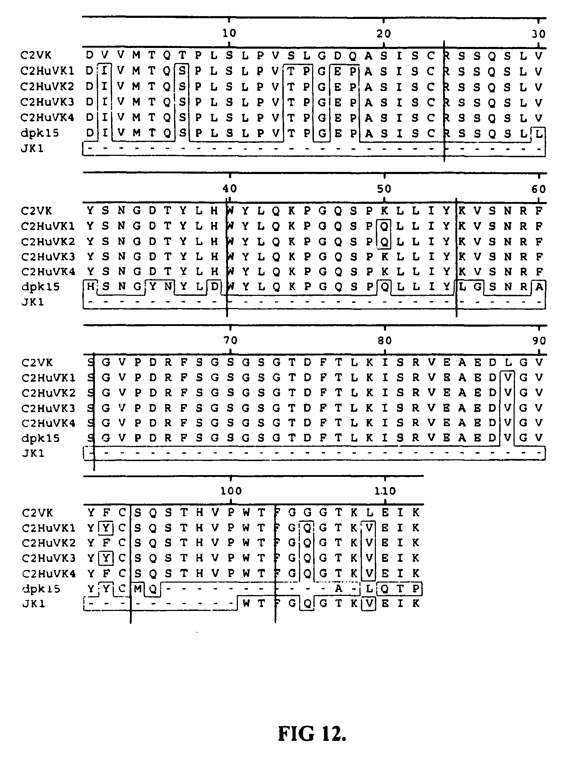

Figure 12: Humanized C2 VK sequences compared to murine sequence and human acceptor sequences DPK15 AND J$_K$1

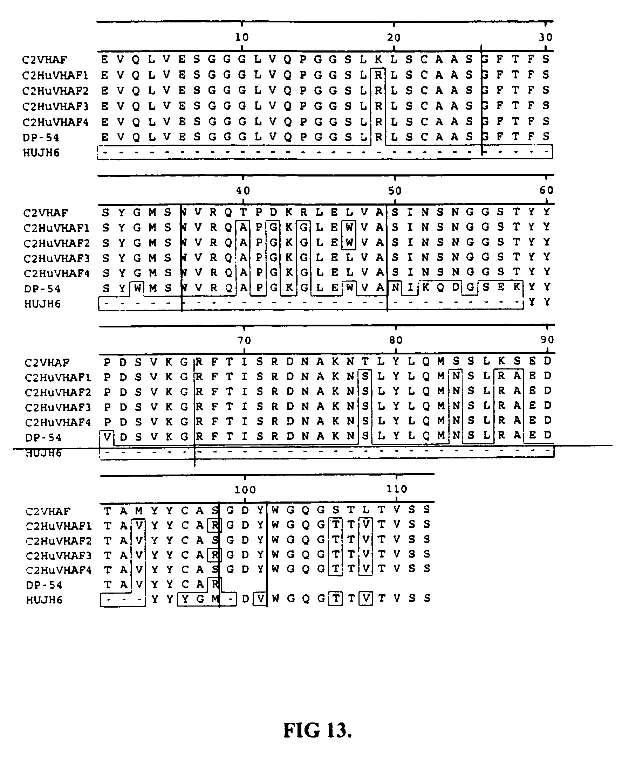

Figure 13: Humanized C2 VH sequences compared to murine sequence and human acceptor sequences DP54 AND J$_H$6

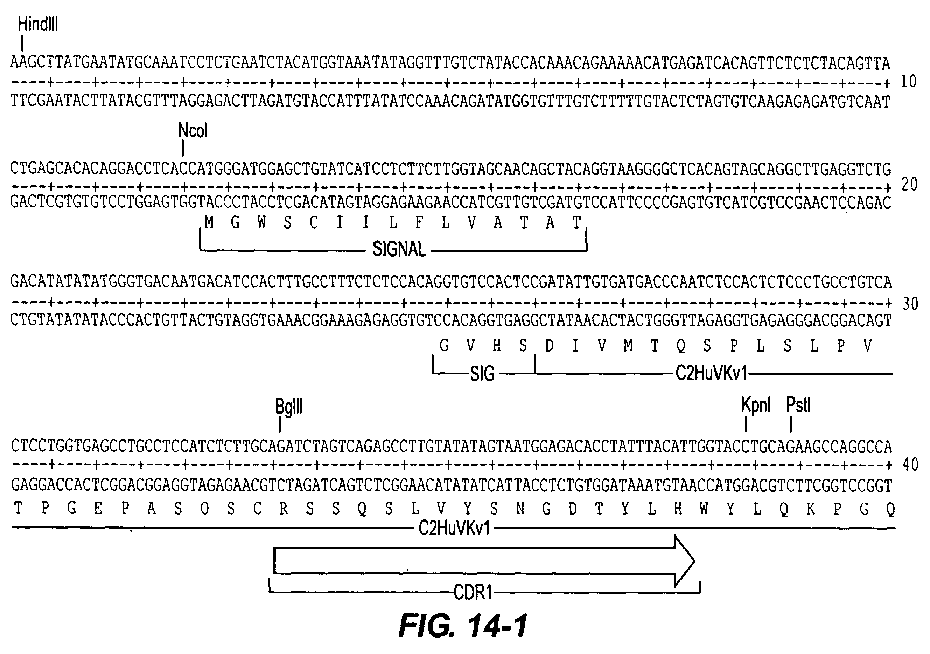

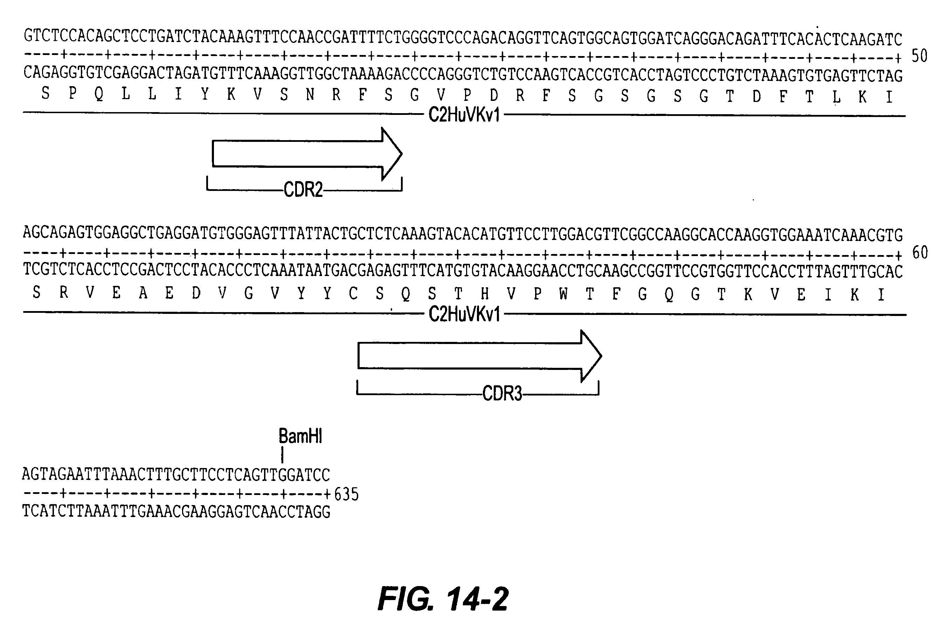

Figures 14-1 and 14-2: Complete DNA and protein sequence of light chain variable region of C2 humanized antibody, C2HuVK1

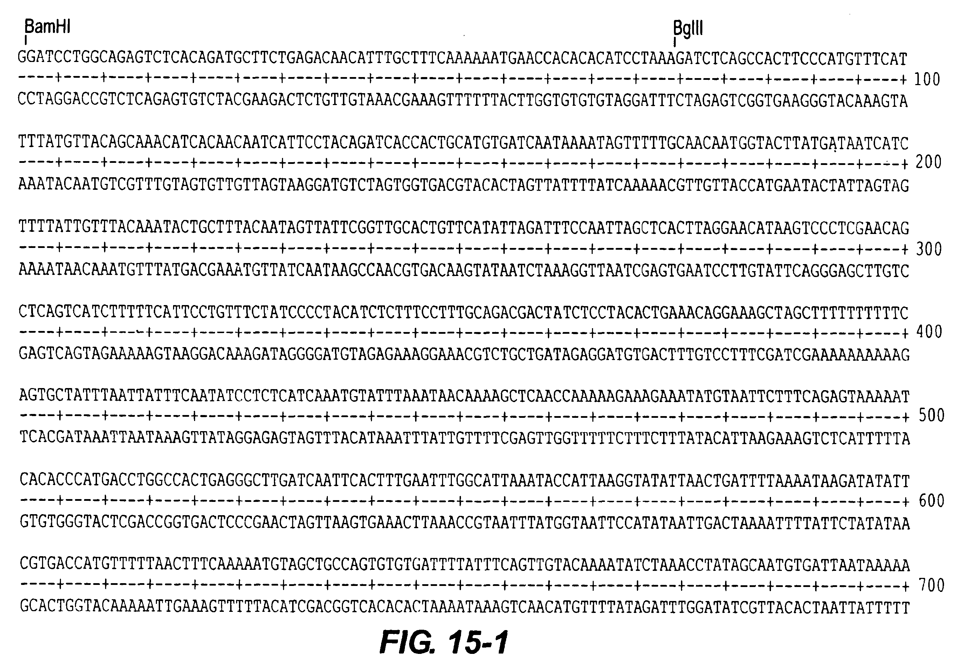

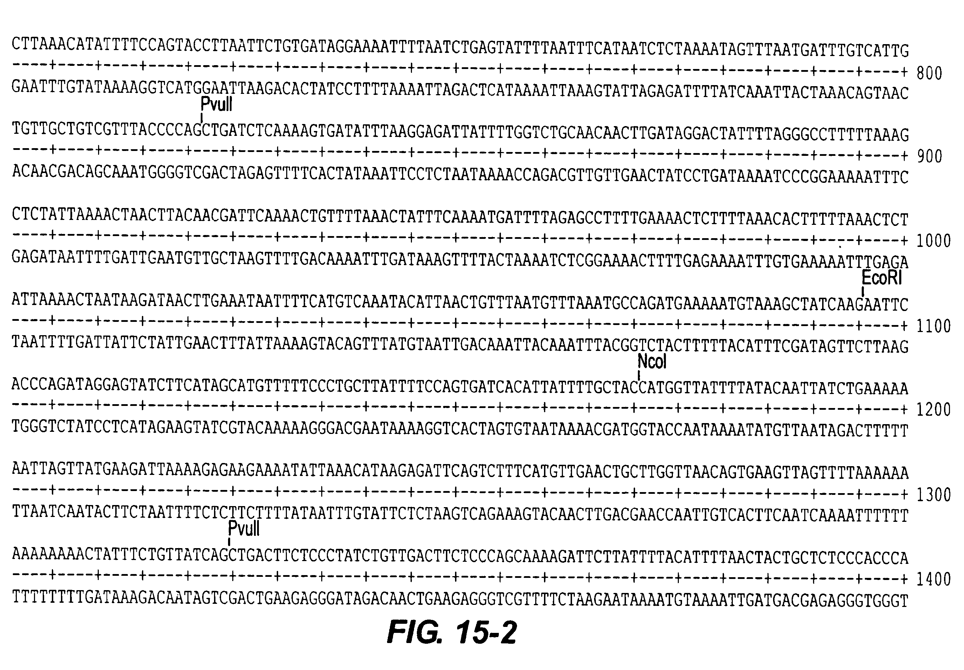

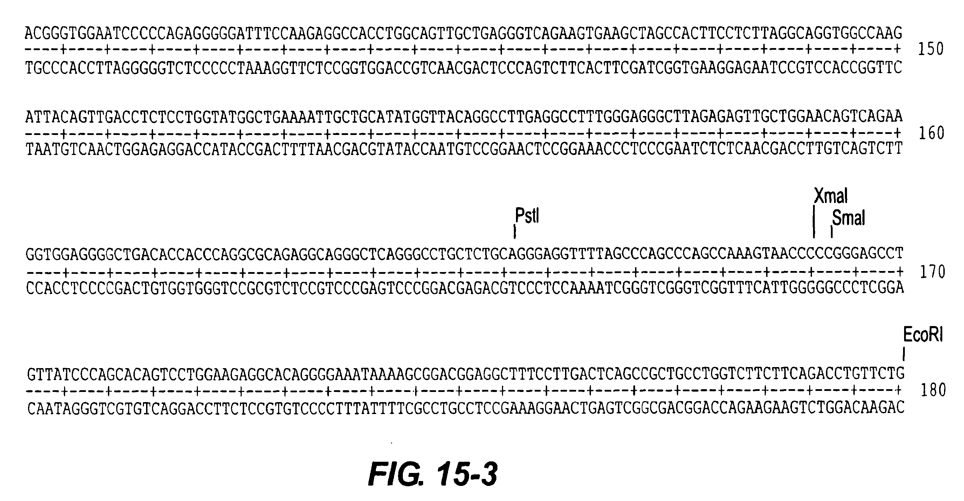

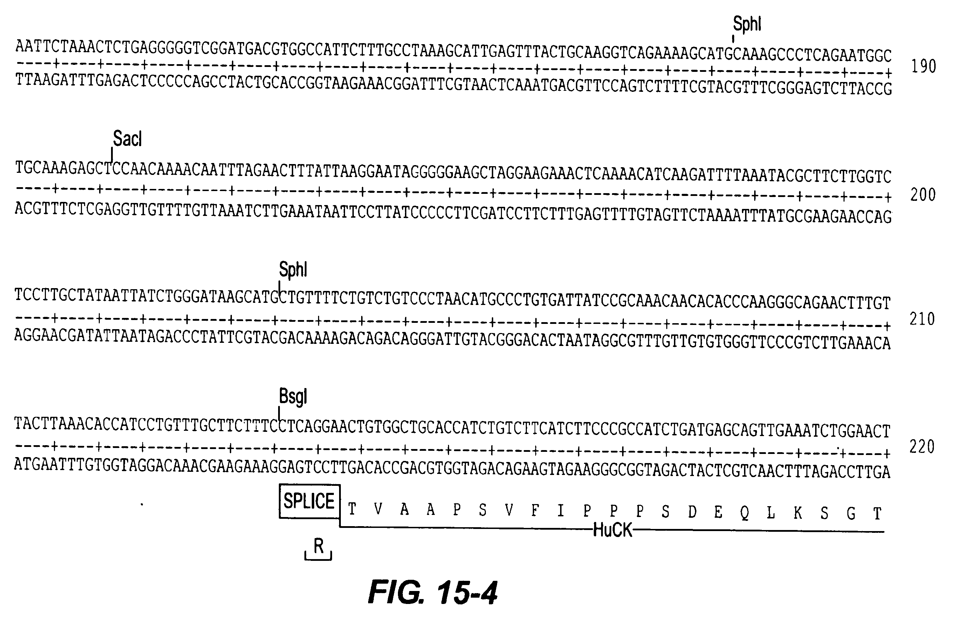

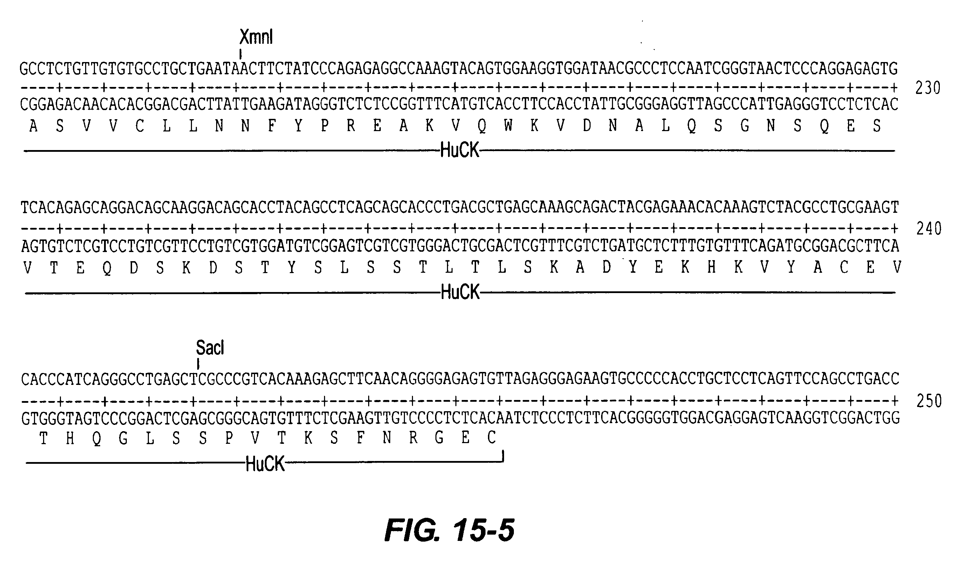

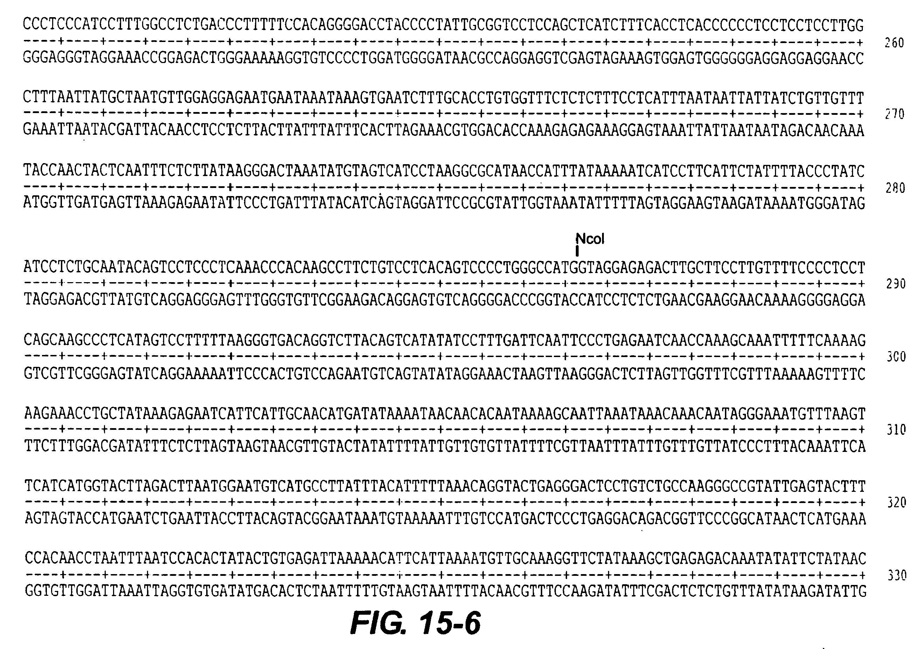

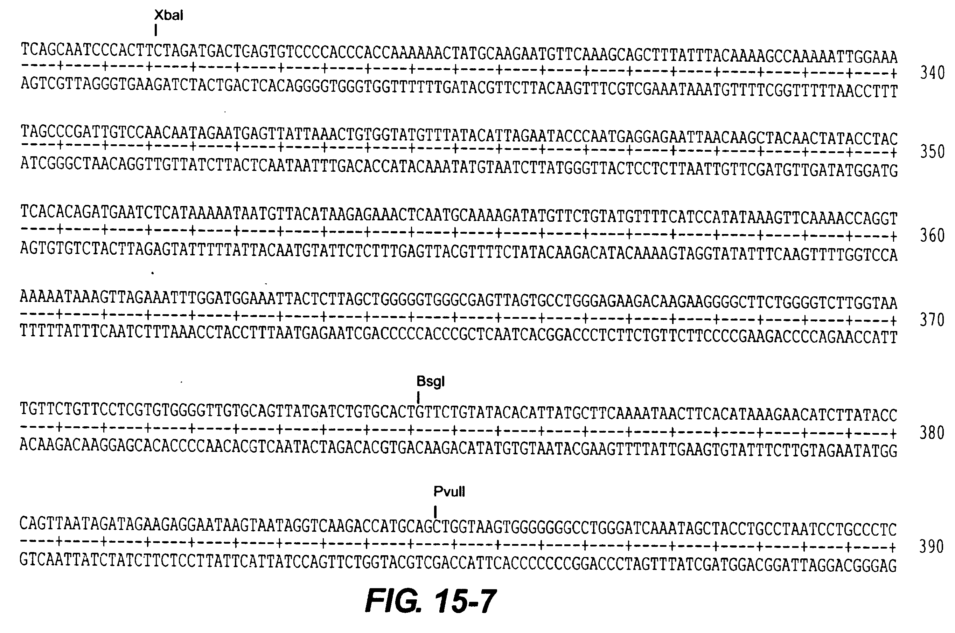

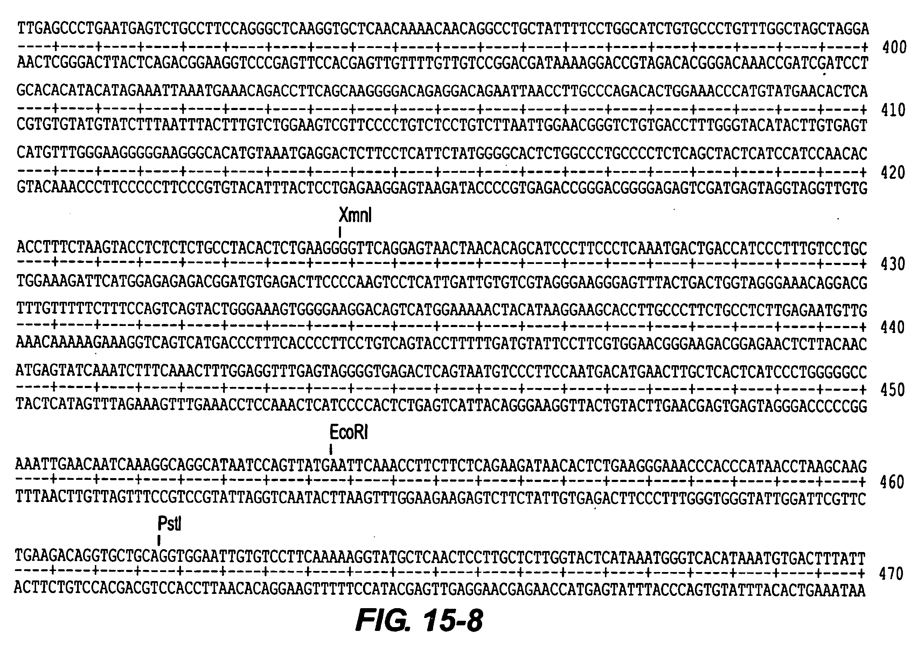

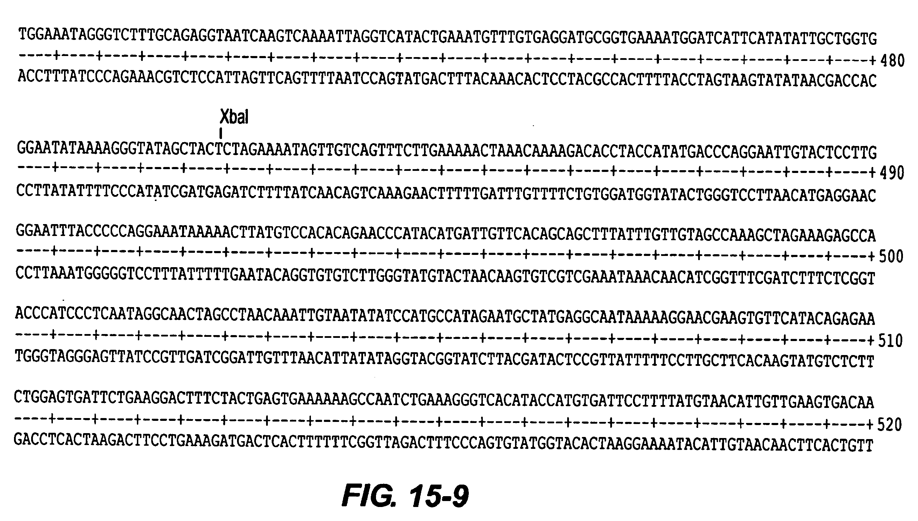

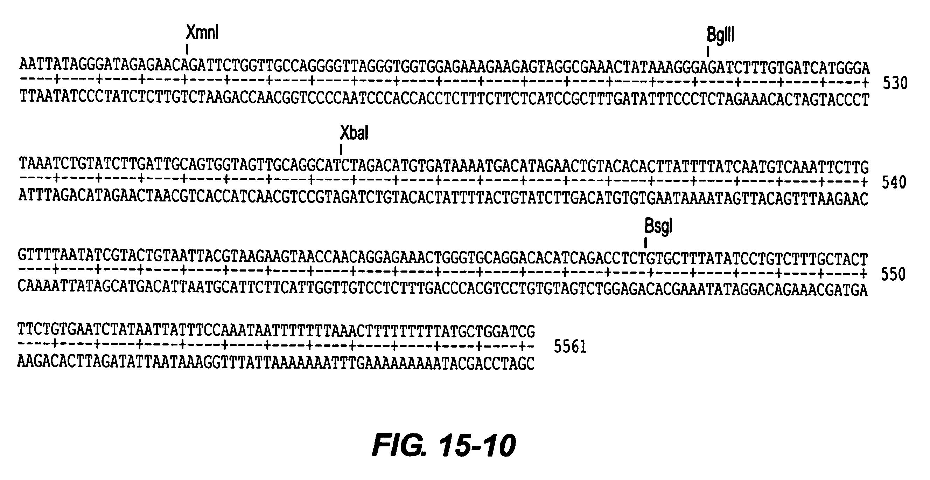

Figures 15-1-15-10: Complete DNA and protein sequence of light chain constant region (human C Kappa) of humanized C2 antibody

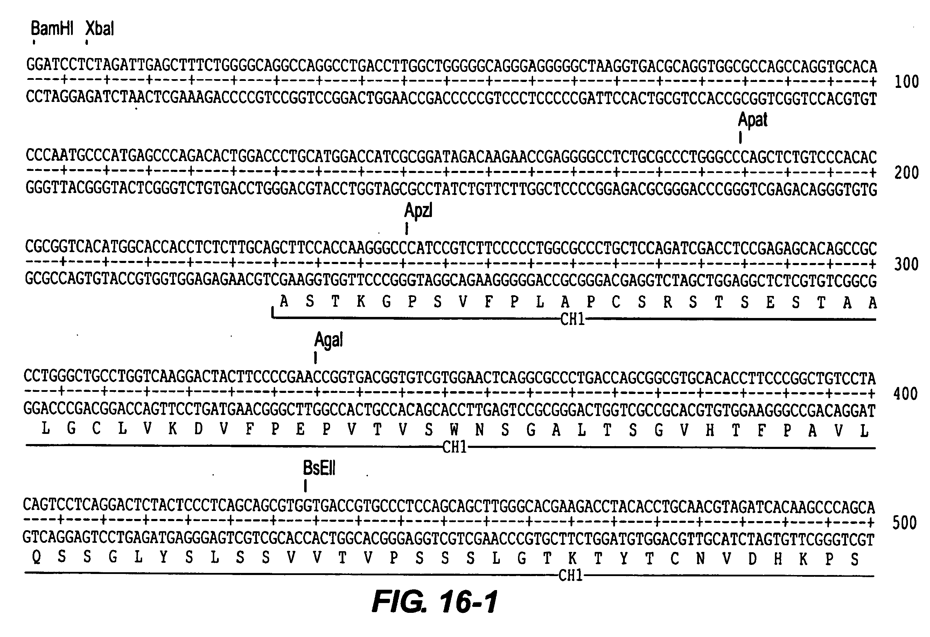

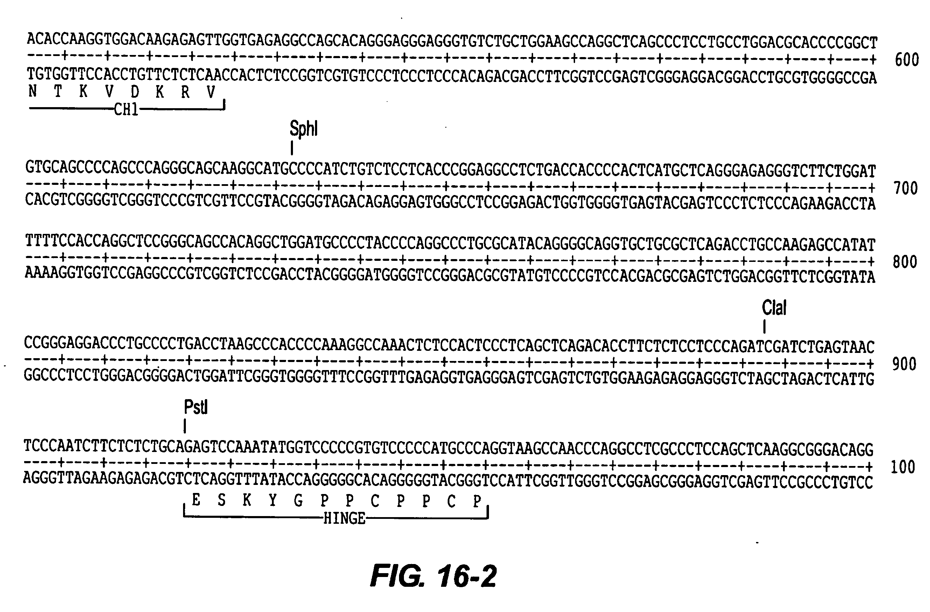

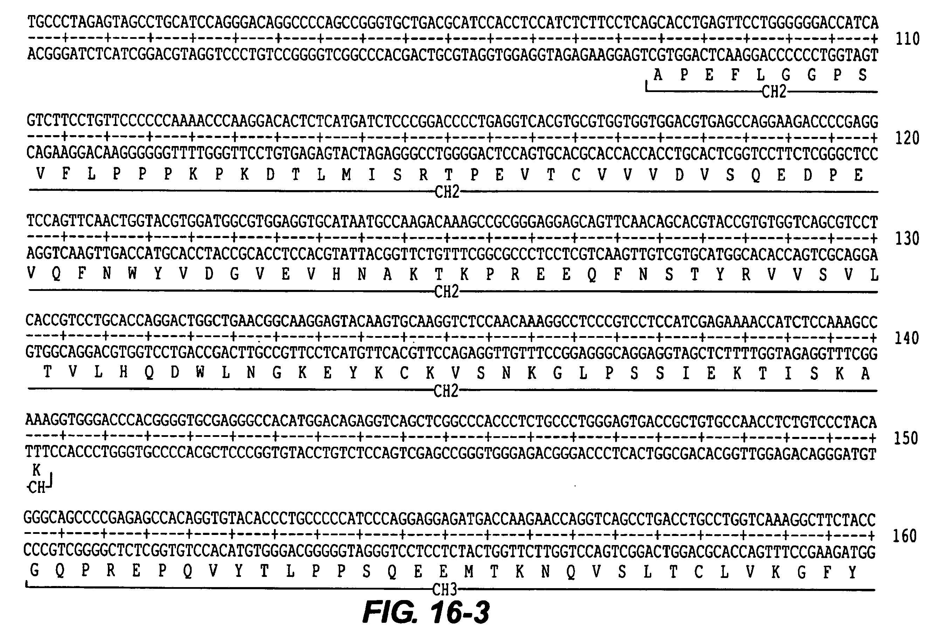

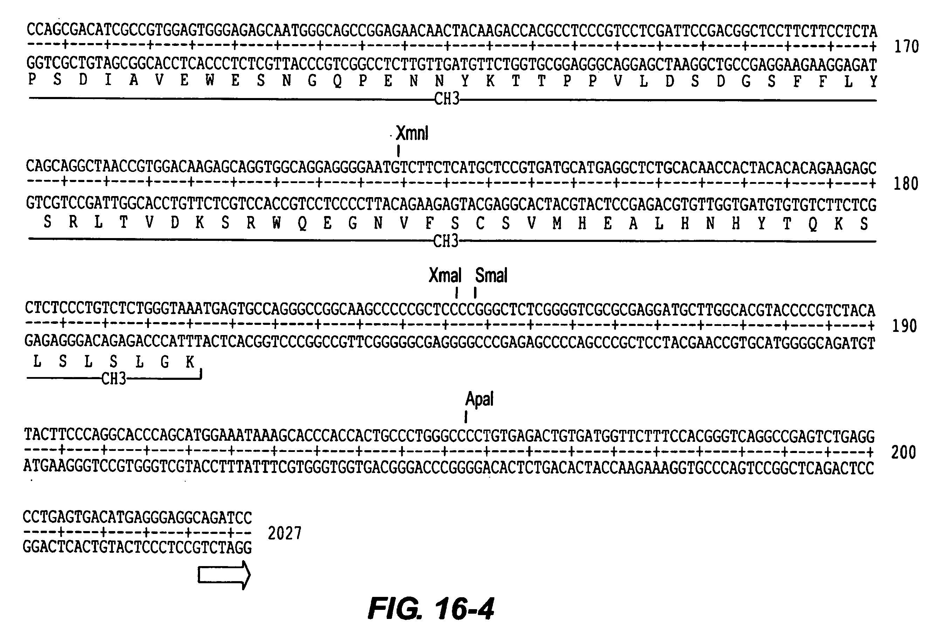

Figures 16-1-16-4: Complete DNA and protein sequence of heavy chain constant region (human IgG4 ser228-pro) of humanized C2 antibody

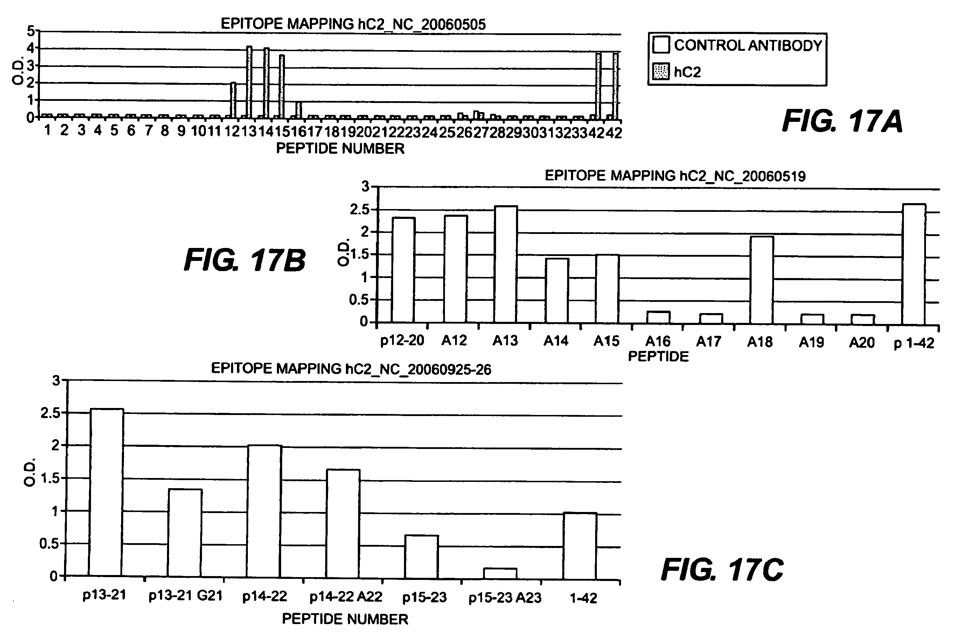

Figure 17A-C (Example 15): Epitope Mapping of humanized monoclonal antibody hC2 performed by ELISA. Results are expressed as OD. (A) Binding of hC2 to overlapping peptides of A$\beta_{1-42}$. Binding to the complete A$\beta_{1-42}$ and binding to a non-binding chimaeric control antibody were used as positive and negative controls, respectively. The peptide number corresponds to the amino acid in the A$\beta_{1-42}$ sequence on which the peptide starts. (B) Binding of hC2 to A$\beta_{12-20}$ and alanine-substituted A$\beta_{12-20}$. Binding to the complete A$\beta_{1-42}$ was used as positive control. The peptide number corresponds to the amino acid that is substituted by alanine. (C) Binding of hC2 to the peptides A$\beta$ 13-21, 13-21G21, 14-22, 14-22A22, 15-23 and 15-23A23. Binding to the complete A$\beta_{1-42}$ was used as positive control.

Figure 18 (Example 13): Results of aggregation assay experiments

Figure 19 (Example 13): Results of disaggregation assay experiments

Figure 20: (Example 16): Results of neuroprotection experiments with humanized antibody C2.

## BRIEF DESCRIPTION OF THE SEQUENCES

| | |
|---|---|
| SEQ ID NO: 1 | Amino acid sequence of C2 HuVH AF 4 humanized heavy chain variable region (CDR1). |
| SEQ ID NO: 2 | Amino acid sequence of C2 HuVH AF 4 humanized heavy chain variable region (CDR2). |
| SEQ ID NO: 3 | Amino acid sequence of C2 HuVH AF 4 humanized heavy chain variable region (CDR3). |
| SEQ ID NO: 4 | Amino acid sequence of C2 HuVK 1 humanized light chain variable region (CDR1). |
| SEQ ID NO: 5 | Amino acid sequence of C2 HuVK 1 humanized light chain variable region (CDR2). |
| SEQ ID NO: 6 | Amino acid sequence of C2 HuVK 1 humanized light chain variable region (CDR3). |
| SEQ ID NO: 7 | Amino acid sequence of A$\beta$ epitope region 2. |
| SEQ ID NO: 8 | Amino acid sequence of A$\beta$ epitope region 1. |
| SEQ ID NO: 9 | Amino acid sequence of A$\beta$ epitope region 2 modified. |
| SEQ ID NO: 10 | Amino acid sequence of A$\beta$ epitope region 1 modified. |
| SEQ ID NO: 11 | Amino acid sequence of Epitope region modified complete. |
| SEQ ID NO: 12 | Amino acid sequence of C2 HuVK 1 humanized light chain variable region. |
| SEQ ID NO: 13 | Amino acid sequence of C2 humanized light chain. |
| SEQ ID NO: 14 | Amino acid sequence of humanized C2 light chain constant region. |
| SEQ ID NO: 15 | Amino acid sequence of C2 HuVH AF 4 humanized heavy chain variable region. |
| SEQ ID NO: 16 | Amino acid sequence of C2 humanized heavy chain. |
| SEQ ID NO: 17 | Amino acid sequence of IG GAMMA-4 CHAIN C REGION-modified. |
| SEQ ID NO: 18 | Nucleotide sequence of CDR2 of C2 HuVH AF 4 humanised heavy chain variable region. |
| SEQ ID NO: 19 | Nucleotide sequence of CDR3 of C2 HuVH AF 4 humanised heavy chain variable region. |
| SEQ ID NO: 20 | Nucleotide sequence of CDR1 of C2 HuVK 1 humanised light chain variable region. |
| SEQ ID NO: 21 | Nucleotide sequence of C2 HuVK 1 humanized light chain variable region. |
| SEQ ID NO: 22 | Nucleotide sequence of C2 humanized light chain. |
| SEQ ID NO: 23 | Nucleotide sequence of C2 humanized light chain constant region. |
| SEQ ID NO: 24 | Nucleotide sequence of C2 HuVH AF 4 humanized heavy chain variable region. |
| SEQ ID NO: 25 | Nucleotide sequence of C2 humanized heavy chain. |

(continued)

SEQ ID NO: 26    Nucleotide sequence of C2 humanized heavy chain constant region.
SEQ ID NO: 27    Amino acid sequence of Mouse C2 Light Chain Variable Region.
SEQ ID NO: 28    Amino acid sequence of Mouse C2 Heavy Chain Variable Region.
SEQ ID NO: 29    Nucleotide sequence of Mouse C2 Light Chain Variable Region.
SEQ ID NO: 30    Nucleotide sequence of Mouse C2 Light Chain.
SEQ ID NO: 31    Nucleotide sequence of Mouse C2 Heavy Chain Variable Region.
SEQ ID NO: 32    Nucleotide sequence of Mouse C2 Heavy Chain.

## DETAILED DESCRIPTION OF THE INVENTION

The antibodies according to the present invention including any functionally equivalent antibodies or functional parts thereof, or, more particularly, a humanized antibody including any functionally equivalent antibody or functional parts thereof, as described herein can be used for the treatment of ocular diseases associated with pathological abnormalities/changes in the tissues of the visual system of a subject, particularly associated with amyloid-beta-related pathological abnormalities/changes in the tissues of the visual system, such as, for example, neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

In particular, a composition, particularly a therapeutic composition comprising an antibody, particularly a humanized antibody including any functionally equivalent antibody or functional parts thereof, as described herein in a therapeutically effective amount can be used for the treatment of ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

In another embodiment, the composition according to the invention to be used in the treatment of ocular diseases associated with pathological abnormalities/changes in the tissues of the visual system, particularly associated with amyloid-beta-related pathological abnormalities/changes in the tissues of the visual system, such as neuronal degradation wherein said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy, is provided in the form of a mixture, wherein the antibody and the other biologically active substance are intermixed in or with the same pharmaceutically acceptable solvent and/or carrier or the antibody and the other biologically active substance may be provided separately as part of a separate composition, which may be offered separately or together in the form of a kit of parts.

Glaucoma is a group of diseases of the optic nerve involving loss of retinal ganglion cells (RGCs) in a characteristic pattern of optic neuropathy. Glaucoma is often, but not always, accompanied by an increased eye pressure, which may be a result of blockage of the circulation of aqueous, or its drainage.

Although raised intraocular pressure is a significant risk factor for developing glaucoma, no threshold of intraocular pressure can be defined which would be determinative for causing glaucoma.

The damage may also be caused by poor blood supply to the vital optic nerve fibers, a weakness in the structure of the nerve, and/or a problem in the health of the nerve fibers themselves.

Untreated glaucoma leads to permanent damage of the optic nerve and resultant visual field loss, which can progress to blindness.

RGCs are the nerve cells that transmit visual signals from the eye to the brain. Caspase-3 and Caspase-8, two major enzymes in the apoptotic process, are activated in the process leading to apoptosis of RGCs. Caspase-3 cleaves amyloid precursor protein (APP) to produce neurotoxic fragments, including Amyloid $\beta$. Without the protective effect of APP, Amyloid $\beta$ accumulation in the retinal ganglion cell layer results in the death of RGCs and irreversible loss of vision.

The different types of glaucomas are classified as open-angle glaucomas, if the condition is chronic, or closed-angle glaucomas, if acute glaucoma occurs suddenly. Glaucoma usually affects both eyes, but the disease can progress more rapidly in one eye than in the other.

Chronic open-angle glaucoma (COAG), also known as primary open angle glaucoma (POAG), is the most common type of glaucoma. COAG is caused by microscopic blockage in the trabecular meshwork, which decreases the drainage of the aqueous outflow into the Schlemm's canal and raises the intraocular pressure (IOP). POAG usually affects both eyes and is strongly associated with age and a positive family history. Its frequency increases in elderly people as the eye drainage mechanism may gradually become clogged with aging. The increase in intraocular pressure in subjects affected by chronic open-angle glaucoma is not accompanied by any symptoms until the loss is felt on the central visual area.

Acute Angle Closure Glaucoma (AACG) or closed-angle glaucoma is a relatively rare type of glaucoma characterized by a sudden increase in intraocular pressure to 35 to 80 mmHg, leading to severe pain and irreversible loss of vision. The sudden pressure increase is caused by the closing of the filtering angle and blockage of the drainage channels. Individuals with narrow angles have an increased risk for a sudden closure of the angle. AACG usually occurs monocularly, but the risk exists in both eyes. Age, cataract and pseudoexfoliation are also risk factors since they are associated with enlargement of the lens and crowding or narrowing of the angle. A sudden glaucoma attack may be associated with severe eye pain and headache, inflamed eye, nausea, vomiting, and blurry vision.

Mixed or Combined Mechanism Glaucoma is a mixture or combination of open and closed angle glaucoma. It affects patients with acute ACG whose angle opens after laser iridotomy, but who continue to require medications for IOP control, as well as patients with POAG or pseudoexfoliative glaucoma who gradually develop narrowing of the angle.

Normal tension glaucoma (NTG), also known as low tension glaucoma (LTG), is characterized by progressive optic nerve damage and loss of peripheral vision similar to that seen in other types of glaucoma; however, the intraocular pressure is the normal range or even below normal.

Congenital (infantile) glaucoma is a relatively rare, inherited type of open-angle glaucoma. Insufficient development of the drainage area results in increased pressure in the eye that can lead to the loss of vision from optic nerve damage and to an enlarged eye. Early diagnosis and treatment are critical to preserve vision in infants and children affected by the disease.

Secondary glaucoma may result from an ocular injury, inflammation in the iris of the eye (iritis), diabetes, cataract, or use of steroids in steroid-susceptible individuals. Secondary glaucoma may also be associated with retinal detachment or retinal vein occlusion or blockage.

Pigmentary glaucoma is characterized by the detachment of granules of pigment from the iris. The granules cause blockage of the drainage system of the eye, leading to elevated intraocular pressure and damage to the optic nerve.

Exfoliative glaucoma (pseudoexfoliation) is characterized by deposits of flaky material on the anterior capsule and in the angle of the eye. Accumulation of the flaky material blocks the drainage system and raises the eye pressure.

Diagnosis of glaucoma may be made using various tests. Tonometry determines the pressure in the eye by measuring the tone or firmness of its surface. Several types of tonometers are available for this test, the most common being the applanation tonometer. Pachymetry determines the thickness of the cornea which, in turn, measures intraocular pressure. Gonioscopy allows examination of the filtering angle and drainage area of the eye. Gonioscopy can also determine if abnormal blood vessels may be blocking the drainage of the aqueous fluid out of the eye. Ophthalmoscopy allows examination of the optic nerve and can detect nerve fiber layer drop or changes in the optic disc, or indentation (cupping) of this structure, which may be caused by increased intraocular pressure or axonal drop out. Gonioscopy is also useful in assessing damage to the nerve from poor blood flow or increased intraocular pressure. Visual Field testing maps the field of vision, subjectively, which may detect signs of glaucomatous damage to the optic nerve. This is represented by specific patterns of visual field loss. Ocular coherence tomography, an objective measure of nerve fiber layer loss, is carried out by looking at the thickness of the optic nerve fiber layer (altered in glaucoma) via a differential in light transmission through damaged axonal tissue.

Optic nerve drusen are globular concretions of protein and calcium salts which are felt to represent secretions through congenitally altered vascular structures affecting the axonal nerve fiber layer. These accumulations occur in the peripapillary nerve fiber layer and are felt to damage the nerve fiber layer either directly by compression or indirectly through disruptions of the vascular supply to the nerve fiber layer. They usually become visible after the first decade of life in affected individuals. They occur most often in both eyes but may also affect one eye, and may cause mild loss of peripheral vision over many years.

Optic neuropathy is a disease characterized by damage to the optic nerve caused by demyelination, blockage of blood supply, nutritional deficiencies, or toxins. Demyelinating optic neuropathies (see optic neuritis below) are typically caused by an underlying demyelinating process such as multiple sclerosis. Blockage of the blood supply, known as ischemic optic neuropathy, can lead to death or dysfunction of optic nerve cells. Non-arteritic ischemic optic neuropathy usually occurs in middle-aged people. Risk factors include high blood pressure, diabetes and atherosclerosis. Arteritic ischemic optic neuropathy usually occurs in older people following inflammation of the arteries (arteritis), particularly the temporal artery (temporal arteritis). Loss of vision may be rapid or develop gradually over 2 to 7 days and the damage may be to one or both eyes. In people with optic neuropathy caused by exposure to a toxin or to a nutritional deficiency, both eyes are usually affected.

About 40% of people with non-arteritic ischemic optic neuropathy experience spontaneous improvement over time. Non-arteritic ischemic optic neuropathy is treated by controlling blood pressure, diabetes and cholesterol levels. Arteritic ischemic optic neuropathy is treated with high doses of corticosteroids to prevent loss of vision in the second eye.

Optic neuritis is associated with mild or severe vision loss in one or both eyes and may be caused by a systemic demyelinating process (see above), viral infection, vaccination, meningitis, syphilis, multiple sclerosis and intraocular inflammation (uveitis). Eye movement may be painful and vision may deteriorate with repeat episodes. Diagnosis involves examination of the reactions of the pupils and determining whether the optic disk is swollen. Magnetic resonance imaging (MRI) may show evidence of multiple sclerosis or, rarely, a tumor pressing on the optic nerve, in which case vision improves once the tumor pressure is relieved. Most cases of optic neuritis improve over a few months without treatment. In some cases, treatment with intravenous corticosteroids may be necessary.

A cataract is an opacity that develops in the crystalline lens of the eye or in its envelope. Cataracts typically cause progressive vision loss and may cause blindness if left untreated. In the Morgagnian Cataract, the cataract cortex progressively liquefies to form a milky white fluid and may cause severe inflammation if the lens capsule ruptures and leaks. If left untreated, the cataract may also cause phacomorphic glaucoma. Cataracts may be congenital in nature or caused by genetic factors, advanced age, long-term ultraviolet exposure, exposure to radiation, diabetes, eye injury or physical trauma.

Extra-capsular (ECCE) surgery is the most effective treatment to treat cataract. In the surgery, the lens is removed, but the majority of the lens capsule is left intact. Phacoemulsification, a small incision on the side of the cornea, is typically used to break up the lens before extraction.

Ocular amyloidosis is a hereditary disorder associated with Type I Familial Amyloidotic Polyneuropathy (FAP) and characterized by abnormal conjunctival vessels, keratoconjunctivitis sicca, pupillary abnormalities and, in some cases, vitreous opacities and secondary glaucoma. Type I FAP is associated with mutations in transthyretin (TTR), a tetrameric plasma protein (prealbumin) synthesized in the liver, the retinal pigment epithelium2 and thechoroid plexus of the brain. Different mutations cause transthyretin to polymerize into a pleated structure of amyloid fibril, leading to hereditary amyloidosis. The most frequent mutation is TTR-met303, in which methionine replaces valine at position 30 in transthyretin.

Type IV FAP is associated with lattice corneal dystrophy (LCD). Lattice corneal dystrophy is an inherited, primary, usually bilateral corneal amyloidosis characterized by the presence of refractile lattice lines with a double contour in the corneal stroma. LCD type I (Biber-Haab-Dimmer) is an autosomal dominant, bilaterally symmetrical corneal disorder characterized by the presence of numerous translucent fine lattice lines with white dots and faint haze in the superficial and middle layers of the central stroma. The symptoms start during the first or second decades of life, causing a progressive loss of vision. Most patients require a corneal transplant by 40 years of age. LCD type II is associated with systemic amyloidosis (Meretoja's syndrome) and is characterized by the presence of thick lattice lines in the limbus, central cornea and stroma. Vision is not affected until later in life. LCD type III affect middle-age people and is characterized by the presence of thick lattice lines that extend from limbus to limbus. LCD type III A is characterized by the accumulation of amyloid deposits in the stroma and the presence of ribbons of amyloid between the stroma and Bowman's layer, LCD type III A differs from LCD type III because of the presence of corneal erosions, the occurrence in whites and the autosomal dominant inheritance pattern.

Down's Syndrome (DS) or trisomy 21 is the most common genetic disorder with an incidence of about 1:700 live births, and is often associated with various congenital anomalies. The disorder, which is caused by the presence of an extra chromosome 21, is associated with premature deposits of the plaque-forming protein amyloid-beta and development of Alzheimer's disease by middle age. Furthermore, many people affected by DS suffer from cataracts beginning in childhood and many suffer from congenital glaucoma. Since the gene for amyloid precursor protein, which is cleaved to form amyloid beta, is located on the long arm of chromosome 21 in humans, overexpression of this gene may lead to increased levels of amyloid precursor protein and amyloid deposition in Down's syndrome.

There is no cure for glaucoma. Medications for the treatment of glaucoma include agents that decrease production of the aqueous humor in the eye, such as beta blockers (Timoptic, Betoptic), carbonic anhydrase inhibitors (Trusopt, Azopt), and alpha agonists(Alphagan, Iopidine), and agents that redirect drainage of the aqueous humor through a different pathway at the back of the eye, such as prostaglandin (Xalatan). Laser surgeries include trabeculoplasty, a procedure that helps the aqueous humor leave the eye more efficiently. According to the Glaucoma Foundation, nearly 80% of patients respond well enough to the procedure to delay or avoid further surgery. However, pressure increases again in the eyes of half of all patients within two years after laser surgery, according to the National Eye Institute. Incisional surgery is performed if medication and initial laser treatments are unsuccessful in reducing pressure within the eye. One type of surgery, a trabeculectomy, creates an opening in the wall of the eye so that aqueous humor can drain. However, about one-third of trabeculectomy patients develop cataracts within five years, according to the Glaucoma Foundation. If the trabeculectomy fails, additional incisional procedures include placing a drainage tube into the eye between the cornea and iris and the use of a laser or freezing treatment to destroy tissue in the eye that makes aqueous humor. Surgery may save the remaining vision in the patient, but it does not improve sight. Vision may actually be worse following surgery.

Age-related macular degeneration (AMD) is a major cause of blindness among Caucasians over age 65. Although much progress has been made recently in macular degeneration research, there are no treatments that rescue neuronal cell death that occurs during the course of the disease. There are also no definitive treatments for other ocular diseases associated with amyloid beta-related neuronal degradation, such as cortical visual deficits, optic nerve drusen, optic neuropathy, optic neuritis, ocular amyloidosis and lattice dystrophy.

Accordingly, there is an urgent need in the art for improved treatment options for subjects affected by ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy. The present invention satisfies this need, by providing solutions that target the process that causes an ocular disease associated with amyloid beta-related neuronal degradation in a subject affected by the disease.

Further to this object, the invention relates to the use of an antibody according to the invention and as described herein, particularly a humanized monoclonal antibody including any functionally equivalent antibody or functional parts thereof and/or of a pharmaceutical composition according to the invention and as described herein, or of a mixture according to the invention and as described herein for the preparation of a medicament for treating or alleviating the effects of ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

In one embodiment, the invention relates to a pharmaceutical composition or a mixture according to the invention and as described herein using an antibody according to the invention and as described herein, particularly a humanized monoclonal antibody including any functionally equivalent antibody or functional parts thereof for use in treating or alleviating the effects of ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

In another embodiment, the invention provides a medicament comprising a humanized monoclonal antibody including any functionally equivalent antibody or functional parts thereof, a pharmaceutical composition or a mixture comprising the antibody according to the invention and as described herein in a therapeutically effective amount, for preventing, treating or alleviating the effects of ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

In one aspect of the invention, a method is provided for reducing the plaque load in the retinal ganglion cell layer of a subject, particularly a mammal, but especially a human suffering from an ocular disease associated with pathological abnormalities/changes in the tissues of the visual system, particularly associated with amyloid-beta-related pathological abnormalities/changes in the tissues of the visual system, such as neuronal degradation, comprising administering to the subject, particularly a mammal, more particularly a human in need of such a treatment, a therapeutically effective amount of an antibody, particularly a humanized monoclonal antibody including any functionally equivalent antibody or functional parts thereof, or a composition or a mixture according to the invention and as described herein.

The pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy. In a further aspect of the invention the humanized monoclonal antibody used in such methods is the humanized C2 or a functional part thereof as described herein. In particular, the humanized monoclonal antibody is produced from mouse antibody ACI-01-Ab7C2, deposited on December 1, 2005, with the Deutsche Sammlung von Mikroorganismen und Zellkulturen GmbH (DSMZ) in Braunsweig, Mascheroder Weg 1 B, 38124 Braunschweig, under accession number DSM ACC2750. In particular, the plaque load is reduced by at least 20 %, particularly by at least 25%, more particularly by at least 30%, even more particularly by more than 30%.

In another aspect of the invention, a method is provided for reducing the amount of plaques in the retinal ganglion cell layer of a subject, particularly a mammal, but especially a human suffering from an ocular disease associated with pathological abnormalities/changes in the tissues of the visual system, particularly associated with amyloid-beta-related pathological abnormalities/changes in the tissues of the visual system, such as neuronal degradation, wherein said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy, comprising administering to the subject, particularly a mammal, more particularly a human in need of such a treatment, a therapeutically effective amount of an antibody, particularly a humanized monoclonal antibody including any functionally equivalent antibody or functional parts thereof, or a composition or a mixture according to the invention and as described herein. In a further aspect of the invention the humanized monoclonal antibody used in such methods is the humanized C2 or a functional part thereof as described herein. In particular, the humanized monoclonal antibody is produced from mouse antibody ACI-01-Ab7C2, deposited on December 1, 2005, with the Deutsche Sammlung von Mikroorganismen und Zellkulturen GmbH (DSMZ) in Braunsweig, Mascheroder Weg 1 B, 38124 Braunschweig, under accession number DSM ACC2750. In particular, the amount of plaques in the brain is reduced by at least 10 %, particularly by at least 15%, more particularly by more than 15%.

In still another aspect of the invention, a method is provided for decreasing the total amount of soluble A$\beta$ in the retinal ganglion cell layer of a subject, particularly a mammal, but especially a human suffering from an ocular disease associated with pathological abnormalities/changes in the tissues of the visual system, particularly associated with amyloid-beta-related pathological abnormalities/changes in the tissues of the visual system, such as neuronal degradation, wherein said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy, comprising administering to the subject, particularly a mammal, more particularly a human in need of such a treatment, a therapeutically effective amount of an antibody, particularly a humanized monoclonal antibody including any functionally equivalent antibody or functional parts thereof, or a composition or a mixture according to the invention and as described herein. In a further aspect of the invention the humanized monoclonal antibody used in such methods is the humanized C2 or a functional part thereof as described herein. In particular, the humanized monoclonal antibody is produced from mouse antibody ACI-01-Ab7C2, deposited on December 1, 2005, with the Deutsche Sammlung von Mikroorganismen und Zellkulturen GmbH (DSMZ) in Braunsweig, Mascheroder Weg 1 B, 38124 Braunschweig, under accession number DSM ACC2750.

In another aspect of the invention, a method is provided for preventing, treating or alleviating the effects of an ocular disease associated with pathological abnormalities/changes in the tissues of the visual system, particularly associated with amyloid-beta-related pathological abnormalities/changes in the tissues of the visual system, such as neuronal degradation, wherein said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy, in an animal, mammal, or a human affected by the ocular disease associated with amyloid beta-related neuronal degradation, by administering a therapeutically effective amount of an antibody, particularly a humanized monoclonal antibody, including any functionally equivalent antibody or functional parts thereof, or a composition or a mixture according to the invention and as described herein to a subject, particularly a mammal, more particularly a human in need of such a treatment. In a further aspect of the invention the humanized monoclonal antibody used in such methods is the humanized C2 or a functional part thereof as described herein. In particular, the humanized monoclonal antibody is produced from mouse antibody ACI-01-Ab7C2, deposited on December 1, 2005, with the Deutsche Sammlung von Mikroorganismen und Zellkulturen GmbH (DSMZ) in Braunsweig, Mascheroder Weg 1 B, 38124 Braunschweig, under accession number DSM ACC2750.

In another aspect of the invention, a method is provided for diagnosing an ocular disease associated with pathological abnormalities/changes in the tissues of the visual system, particularly associated with amyloid-beta-related pathological abnormalities/changes in the tissues of the visual system, such as neuronal degradation, in a subject comprising detecting the immunospecific binding of an antibody, particularly a humanized monoclonal antibody, including any functionally equivalent antibody or functional parts thereof, or a composition or a mixture according to the invention and as described herein, to an epitope of the amyloid protein in a sample or in situ which includes the steps of: (a) bringing the sample or a specific body part or body area suspected to contain the amyloid protein into contact with an antibody according to the invention, which antibody binds a conformational epitope of the amyloid protein; (b) allowing the antibody to bind to the amyloid protein to form an immunological complex; (c) detecting the formation of the immunological complex, particularly such that presence or absence of the immunological complex correlates with presence or absence of amyloid protein; and (d) correlating the presence or absence of the immunological complex with the presence or absence of amyloid protein in the sample or specific body part or area. In a further aspect of the invention the humanized monoclonal antibody used in such methods is the humanized C2 or a functional part thereof as described herein. In particular, the humanized monoclonal antibody is produced from mouse antibody ACI-01-Ab7C2, deposited on December 1, 2005, with the Deutsche Sammlung von Mikroorganismen und Zellkulturen GmbH (DSMZ) in Braunsweig, Mascheroder Weg 1 B, 38124 Braunschweig, under accession number DSM ACC2750.

In a further aspect of the invention, a method is provided for diagnosing a predisposition to an ocular disease associated with pathological abnormalities/changes in the tissues of the visual system, particularly associated with amyloid-beta-related pathological abnormalities/changes in the tissues of the visual system, such as neuronal degradation, in a subject comprising detecting the specific binding of an antibody, particularly a humanized monoclonal antibody, including any functionally equivalent antibody or functional parts thereof, or a composition or a mixture according to the invention and as described herein, to an epitope of the amyloid protein in a sample or in situ which includes the steps of: (a) bringing the sample or a specific body part or body area suspected to contain the amyloid protein into contact with the antibody, wherein the antibody binds a conformational epitope of the amyloid protein; (b) allowing the antibody to bind to any amyloid protein in the sample to form an immunological complex; (c) detecting the formation of the immunological complex; (d) correlating the presence or absence of the immunological complex with the presence or absence of amyloid protein in the sample or specific body part or area, (e) and comparing the amount of said immunological complex to a normal control value, wherein an increase in the amount of the complex compared to a normal control value indicates that the subject is suffering from or is at risk of developing an ocular disease associated with pathological abnormalities/changes in the tissues of the visual system, particularly associated with amyloid-beta-related pathological abnormalities/changes in the tissues of the visual system. In a further aspect of the invention the humanized monoclonal antibody used in such methods is the humanized C2 or a functional part thereof as described herein. In particular, the humanized monoclonal antibody is produced from mouse antibody ACI-01-Ab7C2, deposited on December 1, 2005, with the Deutsche Sammlung von Mikroorganismen und Zellkulturen GmbH (DSMZ) in Braunsweig, Mascheroder Weg 1 B, 38124 Braunschweig, under accession number DSM ACC2750.

In another aspect of the invention, a method is provided for monitoring minimal residual ocular disease associated with pathological abnormalities/changes in the tissues of the visual system, particularly associated with amyloid-beta-related pathological abnormalities/changes in the tissues of the visual system, such as neuronal degradation, in a subject following treatment with a pharmaceutical composition according to the invention, wherein the method comprises: (a) bringing a sample or a specific body part or body area suspected to contain the amyloid protein into contact with an antibody, particularly a humanized monoclonal antibody, including any functionally equivalent antibody or functional parts thereof, or a composition or a mixture according to the invention and as described herein, which antibody binds an epitope of the amyloid protein; (b) allowing the antibody to bind to the amyloid protein to form an immunological complex; (c) detecting the formation of the immunological complex; (d) correlating the presence or absence of the immunological complex with the presence or absence of amyloid protein in the sample or specific body part or area; and (e) comparing the amount of the immunological complex to a normal control value, wherein an increase in the amount of the complex compared to a normal control value indicates that the subject still suffers from a minimal residual ocular disease associated with pathological abnormalities/changes in the tissues of the visual system, particularly associated with amyloid-beta-related pathological abnormalities/changes in the tissues of the visual system. In a further aspect of the invention the humanized monoclonal antibody used in such methods is the humanized C2 or a functional part thereof as described herein. In particular, the humanized monoclonal antibody is produced from mouse antibody ACI-01-Ab7C2, deposited on December 1, 2005, with the Deutsche Sammlung von Mikroorganismen und Zellkulturen GmbH (DSMZ) in Braunsweig, Mascheroder Weg 1 B, 38124 Braunschweig, under accession number DSM ACC2750.

In yet another aspect of the invention a method is provided for predicting responsiveness of a subject being treated with a pharmaceutical composition according to the invention comprising the steps of: (a) bringing a sample or a specific body part or body area suspected to contain an amyloid protein into contact with an antibody, particularly a humanized monoclonal antibody, including any functionally equivalent antibody or functional parts thereof, or a composition or a mixture according to the invention and as described herein, which antibody binds an epitope of the amyloid protein; (b) allowing the antibody to bind to the amyloid protein to form an immunological complex; (c) detecting the formation of the immunological complex; (d) correlating the presence or absence of the immunological complex with the presence or absence of amyloid protein in the sample or specific body part or area, and (e) comparing the amount of the immunological complex before and after onset of the treatment, wherein a decrease in the amount of the immunological complex indicates that the subject has a high potential of being responsive to the treatment. In a further aspect of the invention the humanized monoclonal antibody used in such methods is the humanized C2 or a functional part thereof as described herein. In particular, the humanized monoclonal antibody is produced from mouse antibody ACI-01-Ab7C2, deposited on December 1, 2005, with the Deutsche Sammlung von Mikroorganismen und Zellkulturen GmbH (DSMZ) in Braunsweig, Mascheroder Weg 1 B, 38124 Braunschweig, under accession number DSM ACC2750.

In another aspect of the invention a method is provided for retaining or decreasing ocular pressure in the eyes of a subject, specifically a mammal, more specifically a human suffering from an ocular disease associated with pathological abnormalities/changes in the tissues of the visual system, particularly associated with amyloid-beta-related pathological abnormalities/changes in the tissues of the visual system, such as neuronal degradation, wherein said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy, comprising administering to the subject, particularly a mammal, more particularly a human in need of such a treatment, a therapeutically effective amount of an antibody, particularly a humanized monoclonal antibody including any functionally equivalent antibody or functional parts thereof, or a composition or a mixture according to the invention and as described herein. In a further aspect of the invention the humanized monoclonal antibody used in such methods is the humanized C2 or a functional part thereof as described herein. In particular, the humanized monoclonal antibody is produced from mouse antibody ACI-01-Ab7C2, deposited on December 1, 2005, with the Deutsche Sammlung von Mikroorganismen und Zellkulturen GmbH (DSMZ) in Braunsweig, Mascheroder Weg 1 B, 38124 Braunschweig, under accession number DSM ACC2750.

## DEFINITIONS

The terms "polypeptide", "peptide", and "protein", as used herein, are interchangeable and are defined to mean a biomolecule composed of amino acids linked by a peptide bond.

The terms "a", "an" and "the" as used herein are defined to mean "one or more" and include the plural unless the context is inappropriate.

The language "diseases and disorders which are caused by or associated with amyloid or amyloid-like proteins" includes, but is not limited to, diseases and disorders caused by the presence or activity of amyloid-like proteins in monomeric, fibril, or polymeric state, or any combination of the three. Such diseases and disorders include, but are not limited to, amyloidosis, endocrine tumors, and other diseases, including ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

The phrase "ocular diseases associated with pathological abnormalities/changes in the tissues of the visual system, particularly associated with amyloid-beta-related pathological abnormalities/changes in the tissues of the visual system, such as neuronal degradation" refers to pathological abnormalities associated with aberrant $\beta$-amyloid function or deposition resulting in neuronal degradation, that may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

The term "amyloidosis" refers to a group of diseases and disorders associated with amyloid plaque formation including, but not limited to, secondary amyloidosis and age-related amyloidosis such as diseases including, but not limited to, neurological disorders such as Alzheimer's Disease (AD), including diseases or conditions characterized by a loss of cognitive memory capacity such as, for example, mild cognitive impairment (MCI), Lewy body dementia (LBD), Down's syndrome, hereditary cerebral hemorrhage with amyloidosis (Dutch type); the Guam Parkinson-Dementia complex; as well as other diseases which are based on or associated with amyloid-like proteins such as progressive supranuclear palsy, multiple sclerosis; Creutzfeld Jacob disease, Parkinson's disease, HIV-related dementia, ALS (amyotropic lateral sclerosis), inclusion-body myositis (IBM), Adult Onset Diabetes; senile cardiac amyloidosis, and other diseases, including ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

The terms "detecting" or "detected" as used herein mean using known techniques for detection of biologic molecules such as immunochemical or histological methods and refer to qualitatively or quantitatively determining the presence or concentration of the biomolecule under investigation.

"Polymeric soluble amyloid" refers to multiple aggregated monomers of amyloid peptides, or of amyloid-like peptides, or of modified or truncated amyloid peptides or of other derivates of amyloid peptides forming oligomeric or polymeric structures which are soluble in the mammalian or human body more particularly in the brain, but particularly to multiple aggregated monomers of amyloid $\beta$ (A$\beta$) or of modified or truncated amyloid $\beta$ (A$\beta$) peptides or of derivatives thereof, which are soluble in the mammalian or human body more particularly in the brain.

"Amyloid $\beta$, A$\beta$ or $\beta$-amyloid" is an art recognized term and refers to amyloid $\beta$ proteins and peptides, amyloid $\beta$ precursor protein (APP), as well as modifications, fragments and any functional equivalents thereof. In particular, by amyloid $\beta$ as used herein is meant any fragment produced by proteolytic cleavage of APP but especially those fragments which are involved in or associated with the amyloid pathologies including, but not limited to, A$\beta_{1-38}$, A$\beta_{1-39}$, A$\beta_{1-40}$, A$\beta_{1-41}$ A$\beta_{1-42}$ and A$\beta_{1-43}$.

The structure and sequences of the amyloid $\beta$ peptides as mentioned above are well known to those skilled in the art and methods of producing said peptides or of extracting them from brain and other tissues are described, for example, in Glenner and Wong, Biochem Biophys Res Comm129, 885-890 (1984). Moreover, amyloid $\beta$ peptides are also commercially available in various forms.

By "isolated" is meant a biological molecule free from at least some of the components with which it naturally occurs.

The terms "antibody" or "antibodies" as used herein are art-recognized terms and are understood to refer to molecules or active fragments of molecules that bind to known antigens, particularly to immunoglobulin molecules and to immunologically active portions of immunoglobulin molecules, i.e., molecules that contain a binding site that specifically binds an antigen. An immunoglobulin is a protein comprising one or more polypeptides substantially encoded by the immunoglobulin kappa and lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Also subclasses of the heavy chain are known. For example, IgG heavy chains in humans can be any of IgG1, IgG2, IgG3 and IgG4 subclass. The immunoglobulin according to the invention can be of any class (IgG, IgM, IgD, IgE, IgA and IgY) or subclass (IgG1, IgG2, IgG3, IgG4, IgA1 and IgA2) of immunoglobulin molecule.

As used herein, the phrase "specifically binds" in reference to an antibody means that the antibody binds to its target antigen with greater affinity that it does to a structurally different antigen(s).

A typical immunoglobulin structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain ($V_L$) and variable heavy chain ($V_H$) refer to these light and heavy chains respectively.

Antibodies exist as full length intact antibodies or as a number of well-characterized fragments produced by digestion with various peptidases or chemicals. Thus, for example, pepsin digests an antibody below the disulfide linkages in the hinge region to produce F(ab')$_2$, a dimer of Fab which itself is a light chain joined to $V_H$-CH$_1$ by a disulfide bond. The F(ab')$_2$ may be reduced under mild conditions to break the disulfide linkage in the hinge region thereby converting the F(ab')$_2$ dimer into a Fab' monomer. The Fab' monomer is essentially a Fab fragment with part of the hinge region (see, Fundamental Immunology, W. E. Paul, ed., Raven Press, N.Y. (1993), for a more detailed description of other antibody fragments). While various antibody fragments are defined in terms of the digestion of an intact antibody, one of skill will appreciate that any of a variety of antibody fragments may be synthesized de novo either chemically or by utilizing recombinant DNA methodology. Thus, the term antibody, as used herein also includes antibody fragments either produced by the modification of whole antibodies or synthesized de novo or antibodies and fragments obtained by using

EP 2 205 632 B1

recombinant DNA methodologies.

"Antibodies" are intended within the scope of the present invention to include monoclonal antibodies, polyclonal antibodies, chimeric, single chain, bispecific, simianized, human and humanized antibodies as well as active fragments thereof. Examples of active fragments of molecules that bind to known antigens include separated light and heavy chains, Fab, Fab/c, Fv, Fab', and $F(ab')_2$ fragments, including the products of an Fab immunoglobulin expression library and epitope-binding fragments of any of the antibodies and fragments mentioned above.

These active fragments can be derived from an antibody of the present invention by a number of techniques. For example, monoclonal antibodies can be cleaved with an enzyme, such as pepsin, and subjected to HPLC gel filtration. The appropriate fraction containing Fab fragments can then be collected and concentrated by membrane filtration and the like. For further description of general techniques for the isolation of active fragments of antibodies, see for example, Khaw, B. A. et al. J. Nucl. Med. 23:1011-1019 (1982); Rousseaux et al. Methods Enzymology, 121:663-69, Academic Press, 1986.

Recombinantly made antibodies may be conventional full length antibodies, active antibody fragments known from proteolytic digestion, unique active antibody fragments such as Fv or single chain Fv (scFv), domain deleted antibodies, and the like. An Fv antibody is about 50 Kd in size and comprises the variable regions of the light and heavy chain. A single chain Fv ("scFv") polypeptide is a covalently linked VH::VL heterodimer which may be expressed from a nucleic acid including VH- and VL-encoding sequences either joined directly or joined by a peptide-encoding linker. See Huston, et al. (1988) Proc. Nat. Acad. Sci. USA, 85:5879-5883. A number of structures for converting the naturally aggregated, but chemically separated light and heavy polypeptide chains from an antibody V region into an scFv molecule which will fold into a three dimensional structure substantially similar to the structure of an antigen-binding site. See, e.g. U.S. Patent Nos. 5,091,513, 5,132,405 and 4,956,778.

The combining site refers to the part of an antibody molecule that participates in antigen binding. The antigen binding site is formed by amino acid residues of the N-terminal variable ("V") regions of the heavy ("H") and light ("L") chains. The antibody variable regions comprise three highly divergent stretches referred to as "hypervariable regions" or "complementarity determining regions" (CDRs) which are interposed between more conserved flanking stretches known as "framework regions" (FRs). In an antibody molecule, the three hypervariable regions of a light chain (LCDR1, LCDR2, and LCDR3) and the three hypervariable regions of a heavy chain (HCDR1, HCDR2 and HCDR3) are disposed relative to each other in three dimensional space to form an antigen binding surface or pocket. The antibody combining site therefore represents the amino acids that make up the CDRs of an antibody and any framework residues that make up the binding site pocket.

The identity of the amino acid residues in a particular antibody that make up the combining site can be determined using methods well known in the art. For example, antibody CDRs may be identified as the hypervariable regions originally defined by Kabat et al. (see, "Sequences of Proteins of Immunological Interest," E. Kabat et al., U.S. Department of Health and Human Services; Johnson, G and Wu, TT (2001) Kabat Database and its applications: future directions. Nucleic Acids Research, 29: 205-206; http://immuno.bme.nwa.edu). The positions of the CDRs may also be identified as the structural loop structures originally described by Chothia and others, (see Chothia and Lesk, J. Mol. Biol. 196, 901 (1987), Chothia et al., Nature 342, 877 (1989), and Tramontano et al., J. Mol. Biol. 215, 175 (1990)). Other methods include the "AbM definition" which is a compromise between Kabat and Chothia and is derived using Oxford Molecular's AbM antibody modeling software (now Accelrys) or the "contact definition" of CDRs by Macallum et al., ("Antibody-antigen interactions: contact analysis and binding site topography" J Mol Biol. 1996 Oct 11;262(5):732-45). The following chart identifies CDRs based upon various known definitions.

| Loop | Kabat | AbM | Chothia | Contact |
|---|---|---|---|---|
| L1 | L24 -- L34 | L24 -- L34 | L24 -- L34 | L30 -- L36 |
| L2 | L50 -- L56 | L50 -- L56 | L50 -- L56 | L46 -- L55 |
| L3 | L89 -- L97 | L89 -- L97 | L89 -- L97 | L89 -- L96 |
| H1 (Kabat Numbering) | H31 -- H35B | H26 -- H35B | H26 -- H32..34 | H30 -- H35B |
| H1 (Chothia Numbering) | H31 -- H35 | H26 -- H35 | H26 -- H32 | H30 -- H35 |
| H2 | H50 -- H65 | H50 -- H58 | H52 -- H56 | H4 -- H58 |
| H3 | H95 -- H102 | H95 -- H102 | H9 -- H102 | H9 -- H101 |

General guidelines by which one may identify the CDRs in an antibody from sequence alone are as follows:

LCDR1:

Start - Approximately residue 24.

Residue before is always a Cys.

Residue after is always a Trp. Typically TRP is followed with TYR-GLN, but also may be followed by LEU-GLN, PHE-GLN, or TYR-LEU.

Length is 10 to 17 residues.

LCDR2:

Start - 16 residues after the end of L1.

Sequence before is generally ILE-TYR, but also may be VAL-TYR, ILE-LYS, or ILE-PHE.

Length is generally 7 residues.

LCDR3:

Start - generally 33 residues after end of L2.

Residue before is a Cys.

Sequence after is PHE-GLY-X-GLY.

Length is 7 to 11 residues.

HCDR1:

Start - at approximately residue 26 (four residues after a CYS) [Chothia / AbM definition] Kabat definition starts 5 residues later.

Sequence before is CYS-X-X-X.

Residues after is a TRP, typically followed by VAL, but also followed by ILE, or ALA.

Length is 10 to 12 residues under AbM definition while Chothia definition excludes the last 4 residues.

HCDR2:

Start - 15 residues after the end of Kabat /AbM definition of CDR-H1.

Sequence before typically LEU-GLU-TRP-ILE-GLY (SEQ ID NO. 1), but a number of variations are possible.

Sequence after is LYS/ARG-LEU/ILE/VAL/PHE/THR/ALA-THR/SER/ILE/ALA

Length is 16 to 19 residues under Kabat definition (AbM definition ends 7 residues earlier).

HCDR3:

Start -33 residues after end of CDR-H2 (two residues after a CYS).

Sequence before is CYS-X-X (typically CYS-ALA-ARG).

Sequence after is TRP-GLY-X-GLY.

Length is 3 to 25 residues.

The identity of the amino acid residues in a particular antibody that are outside the CDRs, but nonetheless make up part of the combining site by having a side chain that is part of the lining of the combining site (i.e., it is available to linkage through the combining site), can be determined using methods well known in the art such as molecular modeling and X-ray crystallography. See e.g., Riechmann et al., (1988) Nature, 332:;323-327.

Chimeric antibodies are those in which one or more regions of the antibody are from one species of animal and one or more regions of the antibody are from a different species of animal. A preferred chimeric antibody is one which includes regions from a primate immunoglobulin. A chimeric antibody for human clinical use is typically understood to have variable regions from a non-human animal, e.g. a rodent, with the constant regions from a human. In contrast, a humanized antibody uses CDRs from the non-human antibody with most or all of the variable framework regions from and all the constant regions from a human immunoglobulin. A human chimeric antibody is typically understood to have the variable regions from a rodent. A typical human chimeric antibody has human heavy constant regions and human light chain constant regions with the variable regions of both the heavy and light coming from a rodent antibody. A chimeric antibody may include some changes to a native amino acid sequence of the human constant regions and the native rodent variable region sequence. Chimeric and humanized antibodies may be prepared by methods well known in the art including CDR grafting approaches (see, e.g., U.S. Patent Nos. 5,843,708; 6,180,370; 5,693,762; 5,585,089; 5,530,101), chain shuffling strategies (see e.g., U.S. Patent No. 5,565,332; Rader et al., Proc. Natl. Acad. Sci. USA (1998) 95:8910-8915), molecular modeling strategies (U.S. Patent No. 5,639,641), and the like.

A "humanized antibody" as used herein in the case of a two chain antibody is one where at least one chain is humanized. A humanized antibody chain has a variable region where one or more of the framework regions are human. A humanized antibody which is a single chain is one where the chain has a variable region where one or more of the framework regions are human. The non-human portions of the variable region of the humanized antibody chain or fragment thereof is derived from a non-human source, particularly a non-human antibody, typically of rodent origin. The non-human contribution to the humanized antibody is typically provided in form at least one CDR region which is interspersed among framework regions derived from one (or more) human immunoglobulin(s). In addition, framework support residues may be altered to preserve binding affinity.

The humanized antibody may further comprise constant regions (e.g., at least one constant region or portion thereof, in the case of a light chain, and preferably three constant regions in the case of a heavy chain). The constant regions of a humanized antibody if present generally are human.

Methods to obtain "humanized antibodies" are well known to those skilled in the art. (see, e.g., Queen et al., Proc. Natl Acad Sci USA, 86:10029-10032 (1989), Hodgson et al., Bio/Technology, 9:421 (1991)).

A "humanized antibody" may also be obtained by a novel genetic engineering approach that enables production of affinity-matured human-like polyclonal antibodies in large animals such as, for example, rabbits and mice. See, e.g. U.S. Pat No. 6,632,976.

The phrase "constant region" (CR) as used herein refers to constant regions genes of the immunoglobulin. The constant region genes encode the portion of the antibody molecule which confers effector functions. For Chimeric human antibodies and humanized antibodies, typically non-human (*e.g.*, murine), constant regions are substituted by human constant regions. The constant regions of the subject chimeric or humanized antibodies are typically derived from human immunoglobulins. The heavy chain constant region can be selected from any of the five isotypes: alpha, delta, epsilon, gamma or mu. Further, heavy chains of various subclasses (such as the IgG subclasses of heavy chains) are responsible for different effector functions and thus, by choosing the desired heavy chain constant region, antibodies with desired effector function can be produced. Constant regions that may be used within the scope of this invention are gamma 1 (IgG1), particularly an Fc region of the gamma 1 (IgG1) isotype, gamma 3 (IgG3) and especially gamma 4 (IgG4). The light chain constant region can be of the kappa or lambda type, preferably of the kappa type. In one embodiment the light chain constant region is the human kappa constant chain (Heiter et al. (1980) Cell 22:197-207) and the heavy constant chain is the human IgG4 constant chain.

The phrase "Fc region" is used to define a C-terminal region of an immunoglobulin heavy chain. The "Fc region" may be a native sequence Fc region or a variant Fc region. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The Fc region of an immunoglobulin generally comprises two constant domains, CH2 and CH3.

A "functional Fc region" possesses an "effector function" of a native sequence Fc region. Exemplary "effector functions" include C1q binding; complement dependent cytotoxicity; Fc receptor binding; antibody-dependent cell-mediated cytotoxicity (ADCC); phagocytosis; down regulation of cell surface receptors (*e.g.* B cell receptor; BCR), etc. Such effector functions generally require the Fc region to be combined with a binding domain (*e.g.* an antibody variable domain) and can be assessed using various assays as herein disclosed, for example. A functional Fc region generally includes two heavy chain CH2 and CH3 containing polypeptides which are in association.

A "native sequence Fc region" comprises an amino add sequence identical to the amino acid sequence of an Fc region found in nature and naturally occurring variants thereof.

A "variant Fc region" comprises an amino acid sequence which differs from that of a native sequence Fc region by virtue of at least one "amino acid modification" as herein defined. Preferably, the variant Fc region has at least one amino acid substitution compared to a native sequence Fc region or to the Fc region of a parent polypeptide, e.g. from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith.

An "amino acid modification" refers to a change in the amino acid sequence of a predetermined amino acid sequence. Exemplary modifications include an amino acid substitution, insertion and/or deletion. The preferred amino acid modification herein is a substitution.

An "amino acid modification at" a specified position, *e.g.* of the Fc region, refers to the substitution or deletion of the specified residue, or the insertion of at least one amino acid residue adjacent the specified residue. By insertion "adjacent" a specified residue is meant insertion within one to two residues thereof. The insertion may be N-terminal or C-terminal to the specified residue.

An "amino acid substitution" refers to the replacement of at least one existing amino acid residue in a predetermined amino acid sequence with another different "replacement" amino acid residue. The replacement residue or residues may be "naturally occurring amino acid residues" *(i.e.* encoded by the genetic code) and selected from the group consisting of: alanine (Ala); arginine (Arg); asparagine (Asn); aspartic acid (Asp); cysteine (Cys); glutamine (Gln); glutamic acid (Glu); glycine (Gly); histidine (His); Isoleucine (Ile): leucine (Leu); lysine (Lys); methionine (Met); phenylalanine (Phe); proline (Pro): serine (Ser); threonine (Thr); tryptophan (Trp); tyrosine (Tyr); and valine (Val). Preferably, the replacement residue is not cysteine. Substitution with one or more non-naturally occurring amino acid residues is also encompassed by the definition of an amino acid substitution herein.

A "non-naturally occurring amino acid residue" refers to a residue, other than those naturally occurring amino acid residues listed above, which is able to covalently bind adjacent amino acid residues(s) in a polypeptide chain. Examples of non-naturally occurring amino acid residues include norleucine, omithine, norvaline, homoserine and other amino acid residue analogues such as those described in Ellman et al. Meth. Enzym. 202:301-336 (1991). To generate such non-naturally occurring amino acid residues, the procedures of Noren et al. Science 244:182 (1989) and Ellman et al., supra, can be used. Briefly, these procedures involve chemically activating a suppressor tRNA with a non-naturally occurring amino acid residue followed by in vitro transcription and translation of the RNA.

An "amino acid insertions" refers to the incorporation of at least one amino acid into a predetermined amino acid sequence. While the insertion will usually consist of the insertion of one or two amino acid residues, the present application contemplates larger "peptide insertions," e.g. insertion of about three to about five or even up to about ten amino acid residues. The inserted residue(s) may be naturally occurring or non-naturally occurring as disclosed above.

An "amino acid deletion" refers to the removal of at least one amino acid residue from a predetermined amino acid sequence.

"Antibody-dependent cell-mediated cytotoxicity" or "ADCC" refers to a form of cytotoxicity in which secreted antibody bound to target antigen expressed by target cells is recognized by Fc receptors (FcRs) present on certain cytotoxic cells (e.g. Natural Killer (NK) cells, neutrophils, and macrophages) enabling these cytotoxic effector cells to bind specifically to recognize the antibody coated target cell and subsequently kill the target cell with cytotoxins. The primary cells for mediating ADCC, NK cells, express Fc.gamma.RIII only, whereas monocytes express Fc.gamma.RI, Fc.gamma.RII and Fc.gamma.RIII. FcR expression on hematopoietic cells is summarized in Table 3 on page 464 of Ravetch and Kinet, Annu. Rev. Immunol 9:457-92 (1991). To assess ADCC activity of a molecule of interest, an in vitro ADCC assay, such as that described in U.S. Pat. Nos. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. PNAS (USA) 95:652-656 (1998).

"Immune effector cells" are leukocytes which express one or more FcRs and perform effector functions. Preferably, the cells express at least Fc.gamma.RIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source thereof, e.g. from blood or PBMCs as described herein.

"Complement dependent cytotoxicity" or "CDC" refers to complement dependent lysis of a target cell which has been bound by antibody reactive with antigen expressed by the target cell. Activation of the classical complement pathway is initiated by the binding of the first component of the complement system (C1q) to antibodies (of the appropriate subclass) which are bound to their cognate antigen. To assess complement activation, a CDC assay, e.g. as described in Gazzano-Santoro et al., J. Immunol. Methods 202:163 (1996), maybe performed.

A polypeptide with a variant IgG Fc with "altered" FcR binding affinity or ADCC activity is one which has either enhanced or reduced FcR binding activity (Fc.gamma.R or FcRn) and/or ADCC activity compared to a parent polypeptide or to a polypeptide comprising a native sequence Fc region. The variant Fc which "exhibits increased binding" to an FcR binds at least one FcR with better affinity than the parent polypeptide. The improvement in binding compared to a parent polypeptide may be about 3 fold, preferably about 5, 10, 25, 50, 60, 100, 150, 200, up to 500 fold, or about 25% to 1000% improvement in binding. The polypeptide variant which "exhibits decreased binding" to an FcR, binds at least one FcR with worse affinity than a parent polypeptide. The decrease in binding compared to a parent polypeptide may be about 40% or more decrease in binding. Such Fc variants which display decreased binding to an FcR may possess little or no appreciable binding to an FcR, e.g., 0-20% binding to the FcR compared to a native sequence IgG Fc region, e.g. as determined in the Examples herein.

The polypeptide comprising a variant Fc region which "exhibits increased ADCC" or mediates antibody-dependent cell-mediated cytotoxicity (ADCC) in the presence of human effector cells more effectively than a polypeptide having wild type IgG Fc is one which in vitro or in vivo is substantially more effective at mediating ADCC, when the amounts of polypeptide with variant Fc region and the polypeptide with wild type Fc region used in the assay are essentially the same (all other factors being equal). Generally, such variants will be identified using an in vitro ADCC assay, but other assays or methods for determining ADCC activity, e.g. in an animal model etc, are contemplated. The preferred variant is from about 5 fold to about 100 fold, e.g. from about 25 to about 50 fold, more effective at mediating ADCC than the wild type Fc.

The term "monoclonal antibody" is also well recognized in the art and refers to an antibody that is the product of a single cloned antibody producing cell. Monoclonal antibodies are typically made by fusing a normally short-lived, antibody-producing B cell to a fast-growing cell, such as a cancer cell (sometimes referred to as an "immortal" cell). The resulting hybrid cell, or hybridoma, multiplies rapidly, creating a clone that produces the antibody.

For the purpose of the present invention, "monoclonal antibody" is also to be understood to comprise antibodies that are produced by a mother clone which has not yet reached full monoclonality.

"Functionally equivalent antibody" is understood within the scope of the present invention to refer to an antibody which substantially shares at least one major functional property with an antibody mentioned above and herein described comprising: binding specificity to the $\beta$-amyloid protein, particularly to the A$\beta_{1-42}$ protein, and more particularly to the 16-21 epitope region of the A$\beta_{1-42}$ protein, immunoreactivity *in vitro*, inhibition of aggregation of the A$\beta_{1-42}$ monomers into high molecular polymeric fibrils and/or disaggregation of preformed A$\beta_{1-42}$ polymeric fibrils, and/or a $\beta$-sheet breaking property and alleviating the effects of amyloidosis, a group of diseases and disorders associated with amyloid plaque formation including secondary amyloidosis and age-related amyloidosis such as diseases including, but not limited to, neurological disorders such as Alzheimer's Disease (AD), Lewy body dementia (LBD), Down's syndrome, hereditary cerebral hemorrhage with amyloidosis (Dutch type); the Guam Parkinson-Dementia complex; as well as other diseases which are based on or associated with amyloid-like proteins such as progressive supranuclear palsy, multiple sclerosis; Creutzfeld Jacob disease, Parkinson's disease, HIV-related dementia, ALS (amyotropic lateral sclerosis), Adult Onset Diabetes; senile cardiac amyloidosis; endocrine tumors, and other diseases, including ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy, when administered prophylactically or therapeutically. The antibodies can be of any class such as IgG, IgM, or IgA, etc or any subclass such as IgG1, IgG2a, etc and other subclasses mentioned herein above or known in the art, but particularly of the IgG4 class. Further, the antibodies can be produced by any method, such as phage display, or produced in any organism or cell line, including bacteria, insect, mammal or other type of cell or cell line which produces antibodies with desired characteristics, such as humanized antibodies. The antibodies can also be formed by combining a Fab portion and an Fc region from different species.

The term "hybridize" as used refers to conventional hybridization conditions, preferably to hybridization conditions at which 5xSSPE, 1% SDS, 1xDenhardts solution is used as a solution and/or hybridization temperatures are between 35°C and 70°C, preferably 65°C. After hybridization, washing is preferably carried out first with 2xSSC, 1% SDS and subsequently with 0.2xSSC at temperatures between 35°C and 70°C, preferably at 65°C (regarding the definition of SSPE, SSC and Denhardts solution see Sambrook et al. loc. cit.). Stringent hybridization conditions as for instance described in Sambrook et al., supra, are particularly preferred. Particularly preferred stringent hybridization conditions are for instance present if hybridization and washing occur at 65°C as indicated above. Non-stringent hybridization conditions, for instance with hybridization and washing carried out at 45°C are less preferred and at 35°C even less.

"Homology" between two sequences is determined by sequence identity. If two sequences which are to be compared with each other differ in length, sequence identity preferably relates to the percentage of the nucleotide residues of the shorter sequence which are identical with the nucleotide residues of the longer sequence. Sequence identity can be determined conventionally with the use of computer programs such as the Bestfit program (Wisconsin Sequence Analysis Package, Version 8 for Unix, Genetics Computer Group, University Research Park, 575 Science Drive Madison, WI 53711). Bestfit utilizes the local homology algorithm of Smith and Waterman, Advances in Applied Mathematics 2 (1981), 482-489, in order to find the segment having the highest sequence identity between two sequences. When using Bestfit or another sequence alignment program to determine whether a particular sequence has for instance 95% identity with a reference sequence of the present invention, the parameters are preferably so adjusted that the percentage of identity is calculated over the entire length of the reference sequence and that homology gaps of up to 5% of the total number of the nucleotides in the reference sequence are permitted. When using Bestfit, the so-called optional parameters are preferably left at their preset ("default") values. The deviations appearing in the comparison between a given sequence and the above-described sequences of the invention may be caused for instance by addition, deletion, substitution, insertion or recombination. Such a sequence comparison can preferably also be carried out with the program "fasta20u66" (version 2.0u66, September 1998 by William R. Pearson and the University of Virginia; see also W.R. Pearson (1990), Methods in Enzymology 183, 63-98, appended examples and http://workbench.sdsc.edu/). For this purpose, the "default" parameter settings may be used.

The antibody according to the invention may be an immunoglobulin or antibody, which is understood to have each of its binding sites identical (if multivalent) or, in the alternative, may be a "bispecific" or "bifunctional antibody".

A "bispecific" or "bifunctional antibody" is an artificial hybrid antibody having two different heavy/light chain pairs and two different binding sites. Bispecific antibodies can be produced by a variety of methods including fusion of hybridomas or linking of Fab' fragments. See, *e.g.,* Songsivilai & Lachmann, Clin. Exp. Immunol. 79:315-321 (1990); Kostelny et al., J. Immunol. 148, 1547-1553 (1992).

The term "fragment" refers to a part or portion of an antibody or antibody chain comprising fewer amino acid residues than an intact or complete antibody or antibody chain. Fragments can be obtained *via* chemical or enzymatic treatment of an intact or complete antibody or antibody chain. Fragments can also be obtained by recombinant means. Exemplary fragments include Fab, Fab', F(ab')2, Fabc and/or Fv fragments. The term "antigen-binding fragment" refers to a polypeptide fragment of an immunoglobulin or antibody that binds antigen or competes with intact antibody *(i.e.,* with the intact antibody from which they were derived) for antigen binding *(i.e.,* specific binding).

Binding fragments are produced by recombinant DNA techniques, or by enzymatic or chemical cleavage of intact immunoglobulins. Binding fragments include Fab, Fab', F(ab')$_2$, Fabc, Fv, single chains, and single-chain antibodies.

The term "fragment" also refers to a peptide or polypeptide comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least contiguous 90 amino acid residues, at least contiguous 100 amino acid residues, at least contiguous 125 amino acid residues, at least 150 contiguous amino acid residues, at least contiguous 175 amino acid residues, at least contiguous 200 amino acid residues, or at least contiguous 250 amino acid residues of the amino acid sequence of another polypeptide. In a specific embodiment, a fragment of a polypeptide retains at least one function of the polypeptide.

The term "antigen" refers to an entity or fragment thereof which can bind to an antibody. An immunogen refers to an antigen which can elicit an immune response in an organism, particularly an animal, more particularly a mammal including a human. The term antigen includes regions known as antigenic determinants or epitopes which refers to a portion of the antigen (which are contacted or which play a significant role in supporting a contact reside in the antigen responsible for antigenicity or antigenic determinants.

As used herein, the term "soluble" means partially or completely dissolved in an aqueous solution.

Also as used herein, the term "immunogenic" refers to substances which elicit the production of antibodies, T-cells and other reactive immune cells directed against an antigen of the immunogen.

An immune response occurs when an individual produces sufficient antibodies, T-cells and other reactive immune cells against administered immunogenic compositions of the present invention to moderate or alleviate the disorder to be treated.

The term immunogenicity as used herein refers to a measure of the ability of an antigen to elicit an immune response (humoral or cellular) when administered to a recipient. The present invention is concerned with approaches that reduce the immunogenicity of the subject human chimeric or humanized antibodies.

Humanized antibody of reduced immunogenicity refers to a humanized antibody exhibiting reduced immunogenicity relative to the parent antibody, e.g., the murine antibody.

Humanized antibody substantially retaining the binding properties of the parent antibody refers to a humanized antibody which retains the ability to specifically bind the antigen recognized by the parent antibody used to produce such humanized antibody. Preferably the humanized antibody will exhibit the same or substantially the same antigen-binding affinity and avidity as the parent antibody. Ideally, the affinity of the antibody will not be less than 10% of the parent antibody affinity, more preferably not less than about 30%, and most preferably the affinity will not be less than 50% of the parent antibody. Methods for assaying antigen-binding affinity are well known in the art and include half-maximal binding assays, competition assays, and Scatchard analysis. Suitable antigen binding assays are described in this application.

A "back mutation" is a mutation introduced in a nucleotide sequence which encodes a humanized antibody, the mutation results in an amino acid corresponding to an amino acid in the parent antibody (e.g., donor antibody, for example, a murine antibody). Certain framework residues from the parent antibody may be retained during the humanization of the antibodies of the invention in order to substantially retain the binding properties of the parent antibody, while at the same time minimizing the potential immunogenicity of the resultant antibody. In one embodiment of the invention, the parent antibody is of mouse origin. For example, the back mutation changes a human framework residue to a parent murine residue. Examples of framework residues that may be back mutated include, but are not limited to, canonical residues, interface packing residues, unusual parent residues which are close to the binding site, residues in the "Vernier Zone" (which forms a platform on which the CDRs rest) (Foote & Winter, 1992, J. Mol. Biol. 224, 487-499), and those close to CDR H3.

As used herein, a "conservative change" refers to alterations that are substantially conformationally or antigenically neutral, producing minimal changes in the tertiary structure of the mutant polypeptides, or producing minimal changes in the antigenic determinants of the mutant polypeptides, respectively, as compared to the native protein. When referring to the antibodies and antibody fragments of the invention, a conservative change means an amino acid substitution that does not render the antibody incapable of binding to the subject receptor. Those of ordinary skill in the art will be able to predict which amino acid substitutions can be made while maintaining a high probability of being conformationally and antigenically neutral. Such guidance is provided, for example in Berzofsky, (1985) Science 229:932-940 and Bowie et al. (1990) Science 247:1306-1310. Factors to be considered that affect the probability of maintaining conformational and antigenic neutrality include, but are not limited to: (a) substitution of hydrophobic amino acids is less likely to affect antigenicity because hydrophobic residues are more likely to be located in a protein's interior; (b) substitution of physiochemically similar, amino acids is less likely to affect conformation because the substituted amino acid structurally mimics the native amino acid; and (c) alteration of evolutionarily conserved sequences is likely to adversely affect conformation as such conservation suggests that the amino acid sequences may have functional importance. One of ordinary skill in the art will be able to assess alterations in protein conformation using well-known assays, such as, but not limited to microcomplement fixation methods (Wasserman et al. (1961) J. Immunol. 87:290-295; Levine et al. (1967) Meth. Enzymol. 11:928-936) and through binding studies using conformation-dependent monoclonal antibodies (Lewis et al. (1983) Biochem. 22:948-954).

Further, the phrase "therapeutically effective amount" refers to the amount of antibody which, when administered to a human or animal, which is sufficient to result in a therapeutic effect in said human or animal. The effective amount is readily determined by one of skill in the art following routine procedures.

As used herein, the terms "treat," "prevent," "preventing," and "prevention" refer to the prevention of the recurrence or onset of one or more symptoms of a disorder in a subject resulting from the administration of a prophylactic or therapeutic agent.

## Construction of Humanized Antibodies

The present invention may be understood more readily by reference to the following detailed description of specific embodiments included herein. Although the present invention has been described with reference to specific details of certain embodiments, thereof, it is not intended that such details should be regarded as limitations upon the scope of the invention.

The present invention provides novel methods and compositions comprising highly specific and highly effective antibodies having the ability to specifically recognize and bind to specific epitopes from a range of $\beta$-amyloid antigens. The antibodies enabled by the teaching of the present invention are particularly useful for the treatment of amyloidosis, a group of diseases and disorders associated with amyloid plaque formation including secondary amyloidosis and age-related amyloidosis including, but not limited to, neurological disorders such as Alzheimer's Disease (AD), Lewy body dementia (LBD), Down's syndrome, hereditary cerebral hemorrhage with amyloidosis (Dutch type); the Guam Parkinson-Dementia complex; as well as other diseases which are based on or associated with amyloid-like proteins such as progressive supranuclear palsy, multiple sclerosis; Creutzfeld Jacob disease, hereditary cerebral hemorrhage with amyloidosis Dutch type, Parkinson's disease, HIV-related dementia, ALS (amyotropic lateral sclerosis), Adult Onset Diabetes; senile cardiac amyloidosis; endocrine tumors, and other diseases, including ocular diseases associated with pathological abnormalities/changes in the tissues of the visual system, particularly associated with amyloid-beta-related pathological abnormalities/changes in the tissues of the visual system, such as neuronal degradation. Said pathological abnormalities may occur, for example, in different tissues of the eye, such as the visual cortex leading to cortical visual deficits; the anterior chamber and the optic nerve leading to glaucoma; the lens leading to cataract due to beta-amyloid deposition; the vitreous leading to ocular amyloidosis; the retina leading to primary retinal degeneration and macular degeneration, for example age-related macular degeneration; the optic nerve leading to optic nerve drusen, optic neuropathy and optic neuritis; and the cornea leading to lattice dystrophy.

A fully humanized or reshaped variable region according to the present invention may be created within the scope of the invention by first designing a variable region amino acid sequence that contains non-human-, particularly rodent-derived CDRs, but especially CDRs derived from murine antibody ACI-01-Ab7C2 (named "mC2" throughout the application and deposited 01 December 2005 with the Deutsche Sammlung von Mikroorganismen und Zellkulturen GmbH (DSMZ) in Braunschweig, Mascheroder Weg 1 B, 38124 Branuschweig, under the provisions of the Budapest Treaty and given accession no DSM ACC2750) embedded in human-derived framework sequences. The non-human-, particularly the rodent-derived CDRs, which may be obtained from the antibody according to the present invention, provide the desired specificity. Accordingly, these residues are to be included in the design of the reshaped variable region essentially unchanged. Any modifications should thus be restricted to a minimum and closely watched for changes in the specificity and affinity of the antibody. On the other hand, framework residues in theory can be derived from any human variable region.

In order to create a reshaped antibody which shows an acceptable or an even improved affinity, a human framework sequences should be chosen, which is equally suitable for creating a reshaped variable region and for retaining antibody affinity.

In order to achieve this goal, the best-fit strategy was developed. As it is known that the framework sequences serve to hold the CDRs in their correct spatial orientation for interaction with antigen, and that framework residues can sometimes even participate in antigen binding, this strategy aims at minimizing changes that may negatively effect the three-dimensional structure of the antibody by deriving the human framework sequence used for antibody reshaping from the human variable region that is most homologous or similar to the non-human-, particularly the rodent-derived variable region. This will also maximise the likelihood that affinity will be retained in the reshaped antibody.

At its simplest level, the "best fit" strategy involves comparing the donor rodent V-region with all known human V-region amino acid sequences, and then selecting the most homologous to provide the acceptor framework regions for the humanization exercises. In reality there are several other factors which should be considered, and which may influence the final selection of acceptor framework regions. Molecular modelling predictions may be used in this regard prior to any experimental work in an attempt to maximise the affinity of the resultant reshaped antibody. Essentially, the goal of the modelling is to predict which key residues (if any) of the most homologous human framework should be left as in the rodent to obtain the best affinity in the reshaped antibody.

In one embodiment of the invention, the CDRs are obtainable from mouse monoclonal antibody, particularly from mouse monoclonal antibody ACI-01-Ab7C2 (named "mC2" throughout the application) described in co-pending application EP 05 02 7092.5 filed 12.12.2005.

Hybridoma cells FP-12H3-C2, producing mouse monoclonal antibody ACI-01-Ab7C2 (named "mC2" and hC2 for the humanized C2 antibody, throughout the application) were deposited 01 December 2005 in co-pending application no EP05027092.5 with the Deutsche Sammlung von Mikroorganismen und Zellkulturen GmbH (DSMZ) in Braunschweig, Mascheroder Weg 1 B, 38124 Braunschweig, under the provisions of the Budapest Treaty and given accession no DSM ACC2750.

The mouse antibody may be raised against a supramolecular antigenic construct comprising an antigenic peptide corresponding to the amino acid sequence of the $\beta$-amyloid peptide, particularly of $\beta$-amyloid peptide $A\beta_{1-15}$, $A\beta_{1-16}$ and $A\beta_{1-16}(\Delta 14)$, modified with a hydrophobic moiety such as, for example, palmitic acid or a hydrophilic moiety such as, for example, polyethylene glycol (PEG) or a combination of both, wherein the hydrophobic and hydrophilic moiety, respectively, is covalently bound to each of the termini of the antigenic peptide through at least one, particularly one or two amino acids such as, for example, lysine, glutamic acid and cysteine or any other suitable amino acid or amino acid analogue capable of serving as a connecting device for coupling the hydrophobic and hydrophilic moiety to the peptide fragment. When a PEG is used as the hydrophilic moiety, the free PEG termini is covalently bound to phosphatidylethanolamine or any other compound suitable to function as the anchoring element, for example, to embed the antigenic construct in the bilayer of a liposome.

In particular, a mouse antibody may be raised against a supramolecular antigenic construct comprising an antigenic peptide corresponding to the amino acid sequence of the $\beta$-amyloid peptide $A\beta_{1-16}$ modified with a hydrophilic moiety such as, for example, polyethylene glycol (PEG) hydrophilic moiety is covalently bound to each of the termini of the antigenic peptide through at least one, particularly one or two amino acids such as, for example, lysine, glutamic acid and cysteine or any other suitable amino acid or amino acid analogue capable of serving as a connecting device for coupling the hydrophobic and hydrophilic moiety to the peptide fragment. When a PEG is used as the hydrophilic moiety, the free PEG termini are covalently bound to phosphatidylethanolamine or any other compound suitable to function as the anchoring element, for example, to embed the antigenic construct in the bilayer of a liposome.

In an embodiment of the invention, a chimeric antibody or a fragment thereof, or a humanized antibody or a fragment thereof is provided which comprises in the variable region at least one CDR of non-human origin embedded in one or more human- or primate-derived framework regions and combined with a constant region derived from a human or primate source antibody, which chimeric antibody or a fragment thereof, or a humanized antibody or a fragment thereof is capable of specifically recognizing and binding $\beta$-amyloid monomeric peptide.

The CDRs contain the residues most likely to bind antigen and must be retained in the reshaped antibody. CDRs are defined by sequence according to Kabat et al., Sequence of Proteins of Immunological Interest, 5th Edition, The United States Department of Health and Human Services, The United States Government Printing Office, 1991. CDRs fall into canonical classes (Chothia et al, 1989 Nature, 342, 877-883) where key residues determine to a large extent the structural conformation of the CDR loop. These residues are almost always retained in the reshaped antibody.

In the process for preparing a humanized antibody according to the invention, the amino acid sequences of the C2 heavy chain and light chain variable regions ($V_H$ and $V_K$) are compared to rodent antibody $V_H$ and $V_K$ sequences in the NCBI and Kabat databases.

The closest match mouse germ line gene to C2 $V_K$ is bb1, Locus MMU231201, (Schable *et al*, 1999). A comparison reveals that two amino acids differ from this germ line sequence, both located within CDRL1. Mature murine antibodies with similar, but not identical, sequence can be found. Several have an identical CDRL2 and identical CDRL3, but the CDRL1 of C2 seems to be unique. Comparison with human germ line $V_K$ sequences shows that genes from subgroup $V_K$II are the best match for C2 $V_K$ (Cox *et al*, 1994). C2 $V_K$ can thus be assigned to Kabat subgroup Mu$V_K$II.Sequence.

DPK15 together with the human J region Hu$J_K$1 may be selected to provide the acceptor framework sequences for the humanized $V_K$.

The residues at the interface between the variable light and heavy chains have been defined (Chothia et al, 1985 J. Mol. Biol., 186, 651-663). These are usually retained in the reshaped antibody. The Phe at position 87 of mouse C2 $V_K$ is unusual at the interface, where a Tyr is more common in the $V_K$II subgroup, indicating that this framework residue may be important for antibody activity. Tyr 87 is present in the human germline and humanized C2VK.

The humanized $V_K$ sequences thus may be designed such that the C2HuVK1 consists of mouse C2 $V_K$ CDRs with frameworks from DPK 15 and human $J_K$1. In a specific embodiment of the invention, murine residues may be substituted in the human framework region at positions 45, and/or 87, and/or 50 and/or 53. Residue 45 may be involved in supporting the conformation of the CDRs. Residue 87 is located at the interface of the $V_H$ and $V_K$ domains. Therefore these residues may be critical for maintenance of antibody binding.

The closest match mouse germ line gene to C2 $V_H$ AF is VH7183, Locus AF120466, (Langdon *et al*, 2000). Comparison with human germ line $V_H$ sequences shows that genes from subgroup $V_H$III are the best match for C2 $V_H$. C2 $V_H$ AF can be assigned to Kabat subgroup Mu$V_H$IIID. Sequence DP54 together with the human J region Hu$J_H$6 can be selected to provide the acceptor framework sequences for the humanized $V_H$.

The comparison shows that there are nine amino acid differences between the C2 VH sequences and the human acceptor germ line sequence DP54 and $J_H$6, most being located within CDRH2. Mature murine antibodies with identical or similar (one residue different) CDRH1 or with similar CDRH2 (one residue different) are found, but none with all three CDRs identical to C2 $V_H$ AF. CDRH3 of C2 antibody is unusually short, consisting of only three residues. However, other antibodies are found in the database with CDRH3 of this length. Residue 47 of C2 $V_H$ is Leu rather than the more common Trp, and residue 94 is Ser rather than the normal Arg, indicating that these framework residues may be important for antibody activity.

Various humanized $V_H$ sequences may be designed. C2HuVH1 consists of C2 $V_H$ AF CDRs with frameworks from DP54 and Hu$J_H$6. In a specific embodiment of the invention, murine residues may be substituted in the human framework region at positions 47 or 94 or both. Residue 47 in framework 2 makes contact both with the CDRs and with the $V_K$ domain. Residue 94 may be involved in supporting the conformation of the CDRs. Therefore these residues may be critical for maintenance of antibody binding.

Different HCVR and LCVR regions may be designed which comprise the non-human CDRs obtainable from the donor antibody, for example, a murine antibody, embedded into the native or modified human- or primate-derived framework regions. The modification may particularly concern an exchange of one or more amino acid residues within the framework region by non-human residues, particularly murine residues, more commonly found in this position in the respective subgroups or by residues which have similar properties to the ones more commonly found in this position in the respective subgroups.

The modification of the framework region the framework sequences serve to hold the CDRs in their correct spatial orientation for interaction with antigen, and that framework residues can sometimes even participate in antigen binding. In one embodiment of the invention measures are taken to further adapt the selected human framework sequences to make them most similar to the sequences of the rodent frameworks in order to maximise the likelihood that affinity will be retained in the reshaped antibody.

Accordingly, murine residues in the human framework region may be substituted. In particular, murine residues may be substituted in the human framework region of the Heavy Chain Variable (HCVR) region at positions 47 or 94 or both and in the human framework region of the Light Chain Variable (LCVR) region at positions 45 and/or 87 and/or 50 and/or 53, respectively.

The residues found in the above indicated positions in the human framework region may be exchanged by murine residues more commonly found in this position in the respective subgroups. In particular, the Trp in Kabat position 47 in the human- or primate-derived framework region of the Heavy Chain Variable Region as shown in SEQ ID NO: 15 may be replaced by an Leu or by an amino acid residue that has similar properties and the substitution of which leads to alterations that are substantially conformationally or antigenically neutral, producing minimal changes in the tertiary structure of the mutant polypeptides, or producing minimal changes in the antigenic determinants. In particular, the Trp in Kabat position 47 in the human- or primate-derived framework region of the Heavy Chain Variable Region as shown in SEQ ID NO: 15 may further be replaced by an amino acid selected from the group consisting of norleucine, Ile, Val, Met, Ala, and Phe, particularly by Ile. Alternative conservative substitutions may be contemplated which are conformationally and antigenically neutral.

The Arg in Kabat position 94 in the human- or primate-derived framework region of the Heavy Chain Variable Region as shown in SEQ ID NO: 15 may be replaced by Ser or by an amino acid residue that has similar properties and the substitution of which leads to alterations that are substantially conformationally or antigenically neutral, producing minimal changes in the tertiary structure of the mutant polypeptides, or producing minimal changes in the antigenic determinants. In particular, the Arg in Kabat position 94 in the human- or primate-derived framework region of the Heavy Chain Variable Region as shown in SEQ ID NO: 15 may alternatively be replaced by Thr.

In another embodiment of the invention, both residues may be replaced in the humanized antibody.

The Gln in Kabat position 45 in the human- or primate-derived framework region of the Light Chain Variable Region as shown in SEQ ID NO: 12 may be replaced by Lys or by an amino acid residue that has similar properties and the substitution of which leads to alterations that are substantially conformationally or antigenically neutral, producing minimal changes in the tertiary structure of the mutant polypeptides, or producing minimal changes in the antigenic determinants. In particular, the Gln in Kabat position 45 in the human- or primate-derived framework region of the Light Chain Variable Region as shown in SEQ ID NO: 12 may be replaced by an amino acid selected from the group consisting of Arg, Gln, and Asn, particularly by Arg.

The Leu in Kabat position 50 in the human- or primate-derived framework region of the Light Chain Variable Region as shown in SEQ ID NO: 12 may be replaced by Lys or by an amino acid residue that has similar properties and the substitution of which leads to alterations that are substantially conformationally or antigenically neutral, producing minimal changes in the tertiary structure of the mutant polypeptides, or producing minimal changes in the antigenic determinants. In particular, the Leu in Kabat position 50 in the human- or primate-derived framework region of the Light Chain Variable Region as shown in SEQ ID NO: 12 may be replaced by an amino acid selected from the group consisting of Arg, Gln, and Asn, particularly by Arg.

The Asn in Kabat position 53 in the human- or primate-derived framework region of the Light Chain Variable Region as shown in SEQ ID NO: 12 may be replaced by His and Gln or by an amino acid residue that has similar properties and the substitution of which leads to alterations that are substantially conformationally or antigenically neutral, producing minimal changes in the tertiary structure of the mutant polypeptides, or producing minimal changes in the antigenic determinants. In particular, the Asn in Kabat position 53 in the human- or primate-derived framework region of the Light Chain Variable Region as shown in SEQ ID NO: 12 may be replaced by an amino acid selected from the group consisting of Gln, His, Lys and Arg.

The Thr in Kabat position 87 in the human- or primate-derived framework region of the Light Chain Variable Region as shown in SEQ ID NO: 12 may be replaced by Phe or by an amino acid residue that has similar properties and the substitution of which leads to alterations that are substantially conformationally or antigenically neutral, producing minimal changes in the tertiary structure of the mutant polypeptides, or producing minimal changes in the antigenic determinants. In particular, the Tyr in Kabat position 87 in the human- or primate-derived framework region of the Light Chain Variable Region as shown in SEQ ID NO: 12 may be replaced by an amino acid selected from the group consisting of Leu, Val, Ile, and Ala, particularly by Leu.

The so obtained variable region comprising at least one CDR of non-human origin embedded in one or more human- or primate-derived framework regions may then be combined with a constant region derived from a human or primate source antibody, particularly with human IgG4 or κ constant regions respectively. The IgG4 constant region may be modified by, for example, changing Serine at position 228 in the hinge region to Proline (HuIgG4 Ser-Pro). This mutation stabilizes the interchain disulphide bond and prevents the formation of half molecules that may occur in native human IgG4 preparations. The IgG4 constant region may be further modified by deletion of the terminal Lys in position 439 as shown in SEQ ID NO: 16.

The modified variable regions may be constructed by method known in the art such as, for example overlapping PCR recombination. The expression cassettes for the chimeric antibody, C2 ChV$_H$ AF and C2 ChV$_K$, may be used as templates for mutagenesis of the framework regions to the required sequences. Sets of mutagenic primer pairs are synthesized encompassing the regions to be altered. The humanized V$_H$ and V$_K$ expression cassettes produced may be cloned into appropriate cloning vectors know in the art such as, for example, pUC19. After the entire DNA sequence is confirmed to be correct for each V$_H$ and V$_K$, the modified heavy and light chain V-region genes can be excised from the cloning vector as expression cassettes and transferred to appropriate expression vectors.

## MUTATING Fc REGION

The present invention provides methods for making a polypeptide variant, particularly an antibody comprising a variant region. Such an antibody comprising, for example, a variant Fc region may be used for treating a disease or disorder, such as amyloidosis.

The "parent" "starting" or "nonvariant" polypeptide is prepared using techniques available in the art for generating polypeptides comprising, for example, an Fc region. In a preferred embodiment of the invention, the parent polypeptide is an antibody and exemplary methods for generating antibodies are described in more detail in the following sections. The parent polypeptide may, however, be any other polypeptide comprising an Fc region, e.g. an immunoadhesin. Methods for making immunoadhesins are elaborated in more detail below.

In an alternative embodiment, a variant Fc region may be generated according to the methods herein disclosed and this "variant Fc region" can be fused to a heterologous polypeptide of choice, such as an antibody variable domain or binding domain of a receptor or ligand.

The parent polypeptide comprises an Fc region. Generally the Fc region of the parent polypeptide will comprise a native sequence Fc region, and preferably a human native sequence Fc region. However, the Fc region of the parent polypeptide may have one or more pre-existing amino acid sequence alterations or modifications from a native sequence Fc region. For example, the C1q binding activity of the Fc region may have been previously altered (other types of Fc region modifications are described in more detail below). In a further embodiment the parent polypeptide Fc region is "conceptual" and, while it does not physically exist, the antibody engineer may decide upon a desired variant Fc region amino acid sequence and generate a polypeptide comprising that sequence or a DNA encoding the desired variant Fc region amino acid sequence.

In a preferred embodiment of the invention, however, a nucleic acid encoding an Fc region of a parent polypeptide is available and this nucleic acid sequence is altered to generate a variant nucleic acid sequence encoding the Fc region variant D265A.

DNA encoding an amino acid sequence variant of the starting polypeptide is prepared by a variety of methods known in the art. These methods include, but are not limited to, preparation by site-directed (or oligonucleotide-mediated) mutagenesis, PCR mutagenesis, and cassette mutagenesis of an earlier prepared DNA encoding the polypeptide.

Site-directed mutagenesis is a preferred method for preparing substitution variants. This technique is well known in the art (see, e.g., Carter et al. Nucleic Acids Res. 13:4431-4443 (1985) and Kunkel et al., Proc. Natl. Acad. Sci. USA 82:488 (1987)). Briefly, in carrying out site-directed mutagenesis of DNA, the starting DNA is altered by first hybridizing an oligonucleotide encoding the desired mutation to a single strand of such starting DNA. After hybridization, a DNA polymerase is used to synthesize an entire second strand, using the hybridized oligonucleotide as a primer, and using the single strand of the starting DNA as a template. Thus, the oligonucleotide encoding the desired mutation is incorporated in the resulting double-stranded DNA.

PCR mutagenesis is also suitable for making amino acid sequence variants of the starting polypeptide. See Higuchi, in PCR Protocols, pp. 177-183 (Academic Press, 1990); and Vallette et al., Nuc. Acids Res. 17:723-733 (1989). Briefly, when small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template.

Another method for preparing variants, cassette mutagenesis, is based on the technique described by Wells et al., Gene 34:315-323 (1985). The starting material is the plasmid (or other vector) comprising the starting polypeptide DNA to be mutated. The codon(s) in the starting DNA to be mutated are identified. There must be a unique restriction endonuclease site on each side of the identified mutation site(s). If no such restriction sites exist, they may be generated using the above-described oligonucleotide-mediated mutagenesis method to introduce them at appropriate locations in the starting polypeptide DNA. The plasmid DNA is cut at these sites to linearize it. A double-stranded oligonucleotide encoding the sequence of the DNA between the restriction sites but containing the desired mutation(s) is synthesized using standard procedures, wherein the two strands of the oligonucleotide are synthesized separately and then hybridized together using standard techniques. This double-stranded oligonucleotide is referred to as the cassette. This cassette is designed to have 5' and 3' ends that are compatible with the ends of the linearized plasmid, such that it can be directly ligated to the plasmid. This plasmid now contains the mutated DNA sequence.

Alternatively, or additionally, the desired amino acid sequence encoding a polypeptide variant can be determined, and a nucleic acid sequence encoding such amino acid sequence variant can be generated synthetically.

The amino acid sequence of the parent polypeptide is modified in order to generate a variant Fc region with altered Fc receptor binding affinity or activity in vitro and/or in vivo and/or altered antibody-dependent cell-mediated cytotoxicity (ADCC) activity in vitro and/or in vivo and/or altered cell mediated cytoxicity (CDC) activity in vitro and/or in vivo.

Generally, the modification entails one or more amino acid substitutions. The substitution may, for example, be a "conservative substitution." Substantial modifications in the biological properties of the Fc region may be accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;

(2) neutral hydrophilic: cys, ser, thr;

(3) acidic: asp, glu;

(4) basic: asn, gln, his, lys, arg;

(5) residues that influence chain orientation: gly, pro; and

(6) aromatic: trp, tyr, phe.

Aside from amino acid substitutions, the present invention contemplates other modifications of the parent region amino acid sequence in order to generate, for example, an Fc region variant with altered effector function.

One may, for example, delete one or more amino acid residues of the Fc region in order to reduce binding to an FcR. Generally, one will delete one or more of the Fc region residues identified herein as effecting FcR binding in order to generate such an Fc region variant. Generally, no more than one to about ten Fc region residues will be deleted according to this embodiment of the invention. The Fc region herein comprising one or more amino acid deletions will preferably retain at least about 80%, and preferably at least about 90%, and most preferably at least about 95%, of the parent Fc region or of a native sequence human Fc region.

By introducing the appropriate amino acid sequence modifications in a parent Fc region, for example, one can generate a variant Fc region which (a) mediates antibody-dependent cell-mediated cytotoxicity (ADCC) in the presence of human effector cells more or less effectively and/or (b) binds an Fc gamma receptor (Fc.gamma.R) with more or less affinity than the parent polypeptide. Such Fc region variants will generally comprise at least one amino acid modification in the Fc region. Combining amino acid modifications is thought to be particularly desirable. For example, the variant Fc region may include two, three, four, five, etc substitutions therein, e.g. of the specific Fc region positions identified herein.

For example, in the IgG1 context, an Fc region variant can be generated with reduced binding to the Fc.gamma.R by introducing an amino acid modification at any one or more of amino acid positions 238, 239, 248, 249, 252, 254, 265, 268, 269, 270, 272, 278, 289, 292, 293, 294, 295, 296, 298, 301, 303, 322, 324, 327, 329, 333, 335, 338, 340, 373, 376, 382, 388, 389, 414, 416, 419, 434, 435, 437, 438 or 439 of the Fc region. See, e.g. Presta U.S. Pat. No. 6,737,056.

IgG1 variants which display reduced binding to Fc.gamma.RI, include those comprising an Fc region amino acid modification at any one or more of amino acid positions 238, 265, 269, 270, 327 or 329. See, e.g. Presta U.S. Pat. No. 6,737,056.

IgG1 variants which display reduced binding to Fc.gamma.RII include those comprising an Fc region amino acid modification at any one or more of amino acid positions 238, 265, 269, 270, 292, 294, 295, 298, 303, 324, 327, 329, 333, 335, 338, 373, 376, 414, 416, 419, 435, 438 or 439. See, e.g. Presta U.S. Pat. No. 6,737,056.

IgG1 Fc region variants which display reduced binding to Fc.gamma.RIII include those comprising an Fc region amino acid modification at any one or more of amino acid positions 238, 239, 248, 249, 252, 254, 265, 268, 269, 270, 272, 278, 289, 293, 294, 295, 296, 301, 303, 322, 327, 329, 338, 340, 373, 376, 382, 388, 389, 416, 434, 435 or 437. See, e.g. Presta U.S. Pat. No. 6,737,056.

It will be understood by one of ordinary skill in the art that similar effects can be obtained by varying specific residues in other Ig Fc regions, though the numbering of the residues may be different. See, e.g. Presta U.S. Pat. No. 6,737,056.

One can design an Fc region with altered effector function, e.g., by modifying Clq binding and/or FcR binding and thereby changing CDC activity and/or ADCC activity. For example, one can generate a variant Fc region with improved C1q binding and improved Fc.gamma.RIII binding; e.g. having both improved ADCC activity and improved CDC activity. Alternatively, where one desires that effector function be reduced or ablated, one may engineer a variant Fc region with reduced CDC activity and/or reduced ADCC activity. In other embodiments, one may increase only one of these activities, and optionally also reduce the other activity, e.g. to generate an Fc region variant with improved ADCC activity, but reduced CDC activity and vice versa. See, e.g. Presta U.S. Pat. No. 6,737,056.

With respect to further amino acid sequence alterations, any cysteine residue not involved in maintaining the proper conformation of the polypeptide variant also may be substituted, generally with serine, to improve the oxidative stability of the molecule and prevent aberrant cross linking. See, e.g. Presta U.S. Pat. No. 6,737,056.

Another type of amino acid substitution serves to alter the glycosylation pattern of the polypeptide. This may be achieved by deleting one or more carbohydrate moieties found in the polypeptide, and/or adding one or more glycosylation sites that are not present in the polypeptide. Glycosylation of polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Addition of glycosylation sites to the polypeptide is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original polypeptide (for O-linked glycosylation sites). An exemplary glycosylation variant has an amino acid substitution of residue Asn 297 of the heavy chain. See, e.g. Presta U.S. Pat. No. 6,737,056.

Moreover, the class, subclass or allotype of the Fc region may be altered by one or more further amino acid substitutions to generate an Fc region with an amino acid sequence more homologous to a different class, subclass or allotype as desired. For example, a murine Fc region may be altered to generate an amino acid sequence more homologous to a human Fc region; a human non-A allotype IgG1 Fc region may be modified to achieve a human A allotype IgG1 Fc region etc. In one embodiment, the amino modification(s) herein which alter FcR binding and/or ADCC activity are made in the CH2 domain of the Fc region and the CH3 domain is deleted or replaced with another dimerization domain. Preferably, however, the CH3 domain is retained (aside from amino acid modifications therein which alter effector function as herein disclosed). See, e.g. Presta U.S. Pat. No. 6,737,056.

## BINDING ASSAYS

The ability of the polypeptide variant to bind an FcR may be evaluated. Where the FcR is a high affinity Fc receptor, such as Fc.gamma.RI, FcRn or Fc.gamma.RIIIA-V158, binding can be measured by titrating monomeric polypeptide variant and measuring bound polypeptide variant using an antibody which specifically binds to the polypeptide variant in a standard ELISA format See, e.g. Presta U.S. Pat. No. 6,737,056. FcR binding assays for low affinity FcRs are well-known in the art and are described in, *inter alia*, Presta U.S. Pat. No. 6,737,056.

To assess ADCC activity of the polypeptide variant, an in vitro ADCC assay may be performed using varying effector:target ratios. Useful "effector cells" for such assays include peripheral blood mononuclear cells (PBMC) and Natural Killer (NK) cells. Alternatively, or additionally, ADCC activity of the polypeptide variant may be assessed in vivo, e.g., in a animal model such as that disclosed in Clynes et al. PNAS (USA) 95:652-656 (1998).

## EXPRESSION VECTORS

Any suitable expression vector may be used to practice the invention. For example, one of ordinary skill in the art would be able to express IgG1 in, for instance, *pSVgpt.* Expression vector *pSVgpt* is based on pSV$_2$*gpt* (Mulligan and Berg, 1980) and includes the ampicillin resistance gene for selection in bacterial cells, the *gpt* gene for selection in mammalian cells, the murine heavy chain immunoglobulin enhancer region, genomic sequence encoding the constant region gene and SV40 poly A sequences. The heavy chain variable region for expression is inserted as a *Hin*dIII to *Bam*HI fragment.

Expression vector pSVhyg includes the ampicillin resistance gene for selection in bacterial cells, the *hyg* gene for selection in mammalian cells, the murine heavy chain immunoglobulin enhancer region, genomic sequence encoding the kappa constant region gene and including the kappa enhancer and SV40 poly A sequences. The light chain variable region for expression is inserted as a *Hin*dIII to *Bam*HI fragment.

The DNA sequence is then to be confirmed to be correct for the humanized V$_H$ and V$_K$ in the expression vectors.

For antibody production the humanized heavy and light chain expression vectors may be introduced into appropriate production cell lines know in the art such as, for example, NS0 cells. Introduction of the expression vectors may be accomplished by co-transfection via electroporation or any other suitable transformation technology available in the art. Antibody producing cell lines can then be selected and expanded and humanized antibodies purified. The purified antibodies can then be analyzed by standard techniques such as SDS-PAGE.

## ANTIBODY WITH IMPROVED AFFINITY, SPECIFICITY, STABILITY

The CDRL2 sequence ("KVSNRFS") of the mouse C2 antibody may be modified slightly without adversely affecting antibody activity. Conservative substitutions may be made through exchange of R for K at position 50 and S for N at position 53. The two alternative CDRL2 sequences are therefore "RVSNRFS" and "KVSSRFS", respectively. These are incorporated into the murine $V_K$ sequence with no other changes, as C2 VK-R and C2 VK-S, respectively.

The affinity, specificity and stability of an antibody according to the invention as described herein before or a fragment thereof can be modified by change of its glycosylation profile or pattern resulting in improved therapeutic values.

To achieve this change in glycosylation pattern, host cells may be engineered such that they are capable of expressing a preferred range of a glycoprotein-modifying glycosyl transferase activity which increases complex N-linked oligosaccharides carrying bisecting GlcNAc. Further, modified glycoforms of glycoproteins may be obtained, for example antibodies, including whole antibody molecules, antibody fragments, or fusion proteins that include a region equivalent to the Fc region of an immunoglobulin, having an enhanced Fc-mediated cellular cytotoxicity.

Methods of obtaining antibodies with modified glycosylation pattern are known to those skilled in the art and described, for example, in EP1071700, US2005272128, Ferrara et al (2006) J Biol Chem 281(8), 5032-5036); Ferrara et al (2006) Biotechnology and Bioengineering 93(5), 851-861.

## PHARMACEUTICAL PREPARATION AND ADMINISTRATION

The antibodies according to the invention, but particularly a monoclonal antibody according the invention, can be prepared in a physiologically acceptable formulation and may comprise a pharmaceutically acceptable carrier, diluent and/or excipient using known techniques. For example, the antibody according to the invention and as described herein before including any functionally equivalent antibody or functional parts thereof, in particular, the monoclonal antibody including any functionally equivalent antibody or functional parts thereof is combined with a pharmaceutically acceptable carrier, diluent and/or excipient to form a therapeutic composition. Suitable pharmaceutical carriers, diluents and/or excipients are well known in the art and include, for example, phosphate buffered saline solutions, water, emulsions such as oil/water emulsions, various types of wetting agents, sterile solutions, etc.

Formulation of the pharmaceutical composition according to the invention can be accomplished according to standard methodology know to those skilled in the art.

The compositions of the present invention may be administered to a subject in the form of a solid, liquid or aerosol at a suitable, pharmaceutically effective dose. Examples of solid compositions include pills, creams, and implantable dosage units. Pills may be administered orally. Therapeutic creams may be administered topically. Implantable dosage units may be administered locally, for example, at a tumor site, or may be implanted for systematic release of the therapeutic composition, for example, subcutaneously. Examples of liquid compositions include formulations adapted for injection intramuscularly, subcutaneously, intravenously, intra-arterially, and formulations for topical and intraocular administration. Examples of aerosol formulations include inhaler formulations for administration to the lungs.

The compositions may be administered by standard routes of administration. In general, the composition may be administered by topical, oral, rectal, nasal, interdermal, intraperitoneal, or parenteral (for example, intravenous, subcutaneous, or intramuscular) routes. In addition, the composition may be incorporated into sustained release matrices such as biodegradable polymers, the polymers being implanted in the vicinity of where delivery is desired, for example, at the site of a tumor. The method includes administration of a single dose, administration of repeated doses at predetermined time intervals, and sustained administration for a predetermined period of time.

A sustained release matrix, as used herein, is a matrix made of materials, usually polymers which are degradable by enzymatic or acid/base hydrolysis or by dissolution. Once inserted into the body, the matrix is acted upon by enzymes and body fluids. The sustained release matrix desirably is chosen by biocompatible materials such as liposomes, polylactides (polylactide acid), polyglycolide (polymer of glycolic acid), polylactide co-glycolide (copolymers of lactic acid and glycolic acid), polyanhydrides, poly(ortho)esters, polypeptides, hyaluronic acid, collagen, chondroitin sulfate, carboxylic acids, fatty acids, phospholipids, polysaccharides, nucleic acids, polyamino acids, amino acids such phenylalanine, tyrosine, isoleucine, polynucleotides, polyvinyl propylene, polyvinylpyrrolidone and silicone. A preferred biodegradable matrix is a matrix of one of either polylactide, polyglycolide, or polylactide co-glycolide (co-polymers of lactic acid and glycolic acid).

It is well know to those skilled in the pertinent art that the dosage of the composition will depend on various factors such as, for example, the condition of being treated, the particular composition used, and other clinical factors such as weight, size, sex and general health condition of the patient, body surface area, the particular compound or composition to be administered, other drugs being administered concurrently, and the route of administration.

The composition may be administered in combination with other compositions comprising an biologically active substance or compound, particularly at least one compound selected from the group consisting of compounds against oxidative stress, anti-apoptotic compounds, metal chelators, inhibitors of DNA repair such as pirenzepin and metabolites, 3-amino-1-propanesulfonic acid (3APS), 1,3-propanedisulfonate (1,3PDS), $\alpha$-secretase activators, $\beta$- and $\gamma$-secretase inhibitors, tau proteins, neurotransmitter, $\beta$-sheet breakers, attractants for amyloid beta clearing / depleting cellular components, inhibitors of N-terminal truncated amyloid beta including pyroglutamated amyloid beta 3-42, anti-inflammatory molecules, "atypical antipsychotics" such as, for example clozapine, ziprasidone, risperidone, aripiprazole or olanzapine or cholinesterase inhibitors (ChEIs) such as tacrine, rivastigmine, donepezil, and/or galantamine, M1 agonists and other drugs including any amyloid or tau modifying drug and nutritive supplements such as, for example, vitamin B12, cysteine, a precursor of acetylcholine, lecithin, choline, Ginkgo biloba, acyetyl-L-carnitine, idebenone, propentofylline, or a xanthine derivative, together with an antibody according to the present invention and, optionally, a pharmaceutically acceptable carrier and/or a diluent and/or an excipient and procedures for the treatment of diseases.

Proteinaceous pharmaceutically active matter may be present in amounts between 1 ng and 10 mg per dose. Generally, the regime of administration should be in the range of between 0.1 $\mu$g and 10 mg of the antibody according to the invention, particularly in a range 1.0 $\mu$g to 1.0 mg, and more particularly in a range of between 1.0 $\mu$g and 100 $\mu$g, with all individual numbers falling within these ranges also being part of the invention. If the administration occurs through continuous infusion a more proper dosage may be in the range of between 0.01 $\mu$g and 10 mg units per kilogram of body weight per hour with all individual numbers falling within these ranges also being part of the invention.

Administration will generally be parenterally, e.g., intravenously. Preparations for parenteral administration include sterile aqueous or non-aqueous solutions, suspensions and emulsions. Non-aqueous solvents include without being limited to it, propylene glycol, polyethylene glycol, vegetable oil such as olive oil, and injectable organic esters such as ethyl oleate. Aqueous solvents may be chosen from the group consisting of water, alcohol/aqueous solutions, emulsions or suspensions including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose) and others. Preservatives may also be present such as, for example, antimicrobials, anti-oxidants, chelating agents, inert gases, etc.

The pharmaceutical composition may further comprise proteinaceous carriers such as, for example, serum albumin or immunoglobulin, particularly of human origin. Further biologically active agents may be present in the pharmaceutical composition of the invention dependent on its the intended use.

When the binding target is located in the brain, certain embodiments of the invention provide for the antibody or active fragment thereof to traverse the blood-brain barrier. Certain neurodegenerative diseases are associated with an increase in permeability of the blood-brain barrier, such that the antibody or active fragment thereof can be readily introduced to the brain. When the blood-brain barrier remains intact, several art-known approaches exist for transporting molecules across it, including, but not limited to, physical methods, lipid-based methods, and receptor and channel-based methods.

Physical methods of transporting the antibody or active fragment thereof across the blood-brain barrier include, but are not limited to, circumventing the blood-brain barrier entirely, or by creating openings in the blood-brain barrier. Circumvention methods include, but are not limited to, direct injection into the brain (see, e.g., Papanastassiou et al., Gene Therapy 9: 398-406 (2002)) and implanting a delivery device in the brain (see, e.g., Gill et al., Nature Med. 9: 589-595 (2003); and Gliadel Wafers™, Guildford Pharmaceutical). Methods of creating openings in the barrier include, but are not limited to, ultrasound (see, e.g., U.S. Patent Publication No. 2002/0038086), osmotic pressure (e.g., by administration of hypertonic mannitol (Neuwelt, E. A., Implication of the Blood-Brain Barrier and its Manipulation, Vols 1 & 2, Plenum Press, N.Y. (1989))), permeabilization by, e.g., bradykinin or permeabilizer A-7 (see, e.g., U.S. Patent Nos. 5,112,596, 5,268,164, 5,506,206, and 5,686,416), and transfection of neurons that straddle the blood-brain barrier with vectors containing genes encoding the antibody or antigen-binding fragment (see, e.g., U.S. Patent Publication No. 2003/0083299).

Lipid-based methods of transporting the antibody or active fragment thereof across the blood-brain barrier include, but are not limited to, encapsulating the antibody or active fragment thereof in liposomes that are coupled to antibody binding fragments that bind to receptors on the vascular endothelium of the blood-brain barrier (see, e.g., U.S. Patent Application Publication No. 20020025313), and coating the antibody or active fragment thereof in low-density lipoprotein particles (see, e.g., U.S. Patent Application Publication No. 20040204354) or apolipoprotein E (see, e.g., U.S. Patent Application Publication No. 20040131692).

Receptor and channel-based methods of transporting the antibody or active fragment thereof across the blood-brain barrier include, but are not limited to, using glucocorticoid blockers to increase permeability of the blood-brain barrier (see, e.g., U.S. Patent Application Publication Nos. 2002/0065259, 2003/0162695, and 2005/0124533); activating potassium channels (see, e.g., U.S. Patent Application Publication No. 2005/0089473), inhibiting ABC drug transporters (see, e.g., U.S. Patent Application Publication No. 2003/0073713); coating antibodies with a transferrin and modulating activity of the one or more transferrin receptors (see, e.g., U.S. Patent Application Publication No. 2003/0129186), and cationizing the antibodies (see, e.g., U.S. Patent No. 5,004,697).

## DETECTION/DIAGNOSIS

In a further embodiment the present invention provides methods and kits for the detection and diagnosis of amyloid-associated diseases or conditions. These methods include known immunological methods commonly used for detecting or quantifying substances in biological samples or in an *in situ* condition.

Diagnosis of an amyloid-associated disease or condition in a patient may be achieved by detecting the immunospecific binding of a monoclonal antibody or an active fragment thereof to an epitope of the amyloid protein in a sample or *in situ,* which includes bringing the sample or a specific body part or body area suspected to contain the amyloid protein into contact with an antibody which binds an epitope of the amyloid protein, allowing the antibody to bind to the amyloid protein to form an immunological complex, detecting the formation of the immunological complex and correlating the presence or absence of the immunological complex with the presence or absence of amyloid protein in the sample or specific body part or area.

Biological samples that may be used in the diagnosis of an amyloid-associated disease or condition are, for example, fluids such as serum, plasma, saliva, gastric secretions, mucus, cerebrospinal fluid, lymphatic fluid, aqueous humor of the eye and the like or tissue or cell samples obtained from an organism such as neural, brain, cardiac or vascular tissue. For determining the presence or absence of the amyloid protein in a sample any immunoassay known to those of ordinary skill in the art. (See Harlow and Lane, Antibodies: A Laboratory Manual (Cold Spring Harbor Laboratory, New York 1988 555-612) may be used such as, for example, assays which utilize indirect detection methods using secondary reagents for detection, ELISA's and immunoprecipitation and agglutination assays. A detailed description of these assays is, for example, given in WO96/13590 to Maertens and Stuyver, Zrein et al. (1998) and WO96/29605.

For *in situ* diagnosis, the antibody or any active and functional part thereof may be administered to the organism to be diagnosed by methods known in the art such as, for example, intravenous, intranasal, intraperitoneal, intracerebral, intraarterial injection such that a specific binding between the antibody according to the invention with an eptitopic region on the amyloid protein may occur. The antibody/antigen complex may be detected through a label attached to the antibody or a functional fragment thereof.

The immunoassays used in diagnostic applications typically rely on labelled antigens, antibodies, or secondary reagents for detection. These proteins or reagents can be labelled with compounds generally known to those skilled in the art including enzymes, radioisotopes, and fluorescent, luminescent and chromogenic substances including colored particles, such as colloidal gold and latex beads. Of these, radioactive labelling can be used for almost all types of assays and with most variations. Enzyme-conjugated labels are particularly useful when radioactivity must be avoided or when quick results are needed. Fluorochromes, although requiring expensive equipment for their use, provide a very sensitive method of detection. Antibodies useful in these assays include monoclonal antibodies, polyclonal antibodies, and affinity purified polyclonal antibodies.

Alternatively, the antibody may be labelled indirectly by reaction with labelled substances that have an affinity for immunoglobulin, such as protein A or G or second antibodies. The antibody may be conjugated with a second substance and detected with a labelled third substance having an affinity for the second substance conjugated to the antibody. For example, the antibody may be conjugated to biotin and the antibody-biotin conjugate detected using labelled avidin or streptavidin. Similarly, the antibody may be conjugated to a hapten and the antibody-hapten conjugate detected using labelled anti-hapten antibody.

Those of ordinary skill in the art will know of these and other suitable labels which may be employed in accordance with the present invention. The binding of these labels to antibodies or fragments thereof can be accomplished using standard techniques commonly known to those of ordinary skill in the art. Typical techniques are described by Kennedy, J. H., et al., 1976 (Clin. Chim. Acta 70:1-31), and Schurs, A. H. W. M., et al. 1977 (Clin. Chim Acta 81:1-40). Coupling techniques mentioned in the latter are the glutaraldehyde method, the periodate method, the dimaleimide method.

Current immunoassays utilize a double antibody method for detecting the presence of an analyte, wherein. The antibody is labeled indirectly by reactivity with a second antibody that has been labeled with a detectable label. The second antibody is preferably one that binds to antibodies of the animal from which the monoclonal antibody is derived. In other words, if the monoclonal antibody is a mouse antibody, then the labeled, second antibody is an anti-mouse antibody. For the monoclonal antibody to be used in the assay described below, this label is preferably an antibody-coated bead, particularly a magnetic bead. For the polyclonal antibody to be employed in the immunoassay described herein, the label is preferably a detectable molecule such as a radioactive, fluorescent or an electrochemiluminescent substance.

An alternative double antibody system often referred to as fast format systems because they are adapted to rapid determinations of the presence of an analyte, may also be employed within the scope of the present invention. The system requires high affinity between the antibody and the analyte. According to one embodiment of the present invention, the presence of the amyloid protein is determined using a pair of antibodies, each specific for amyloid protein. One of said pairs of antibodies is referred to herein as a "detector antibody" and the other of said pair of antibodies is referred to herein as a "capture antibody". The monoclonal antibody of the present invention can be used as either a capture antibody or a detector antibody. The monoclonal antibody of the present invention can also be used as both capture and detector antibody, together in a single assay. One embodiment of the present invention thus uses the double antibody sandwich method for detecting amyloid protein in a sample of biological fluid. In this method, the analyte (amyloid protein) is sandwiched between the detector antibody and the capture antibody, the capture antibody being irreversibly immobilized onto a solid support. The detector antibody would contain a detectable label, in order to identify the presence of the antibody-analyte sandwich and thus the presence of the analyte.

Exemplary solid phase substances include, but are not limited to, microtiter plates, test tubes of polystyrene, magnetic, plastic or glass beads and slides which are well known in the field of radioimmunoassay and enzyme immunoassay. Methods for coupling antibodies to solid phases are also well known to those skilled in the art. More recently, a number of porous material such as nylon, nitrocellulose, cellulose acetate, glass fibers and other porous polymers have been employed as solid supports.

The present invention also relates to a diagnostic kit for detecting amyloid protein in a biological sample comprising a composition as defined above. Moreover, the present invention relates to the latter diagnostic kit which, in addition to a composition as defined above, also comprises a detection reagent as defined above. The term "diagnostic kit" refers in general to any diagnostic kit known in the art. More specifically, the latter term refers to a diagnostic kit as described in Zrein *et al.* (1998).

It is still another object of the present invention to provide novel immunoprobes and test kits for detection and diagnosis of amyloid-associated diseases and conditions comprising antibodies according to the present invention. For immunoprobes, the antibodies are directly or indirectly attached to a suitable reporter molecule, e.g., an enzyme or a radionuclide. The test kit includes a container holding one or more antibodies according to the present invention and instructions for using the antibodies for the purpose of binding to amyloid protein to form an immunological complex and detecting the formation of the immunological complex such that presence or absence of the immunological complex correlates with presence or absence of amyloid protein.

## EXAMPLES

*Materials*

The development and preparation of mouse monoclonal antibody ACI-01-Ab7C2 (named "mC2" and hC2 for the humanized C2 antibody, throughout the application) is described in co-pending application EP 05 02 7092.5 filed 12.12.2005,

Hybridoma cells FP-12H3-C2, producing mouse monoclonal antibody ACI-01-Ab7C2 (named "mC2" and hC2 for the humanized C2 antibody, throughout the application) were deposited 01 December 2005 in co-pending application no EP05027092.5 with the Deutsche Sammlung von Mikroorganismen und Zellkulturen GmbH (DSMZ) in Braunschweig, Mascheroder Weg 1 B, 38124 Braunschweig, under the provisions of the Budapest Treaty and given accession no DSM ACC2750.

Hybridoma cells were cultured in Dulbecco's modified Eagle Medium (DMEM) supplemented with 10% foetal bovine serum and antibiotics (Penicillin/Streptomycin). The isotype of the antibody produced was checked and found to be mouse IgG2b/kappa, as expected.

*Assay*

An ELISA for binding to Amyloid Beta provided a reliable measure of the potency of C2 antibodies. Positive control antibodies, murine FP-12H3-C2 antibody (Genovac Lot No: AK379/01), and standard Chemicon antibody 1560 (Lot no: 0508008791).

*Choice of human constant regions*

As immune system recruitment is not desirable for the clinical antibody candidate, the selected human constant region for the heavy chain was human IgG4, modified to change Serine at position 228 in the hinge region to Proline (HuIgG4 Ser-Pro). This mutation stabilizes the interchain disulphide bond and prevents the formation of half molecules that may occur in native human IgG4 preparations. The antibody expressed from the production cell lines will also have the terminal lysine removed. The sequences of human constant regions HuIgG4 Ser-Pro and human Kappa are given in SEQ ID NO: 17 and 14, respectively.

## Example 1 Cloning and Sequencing of Antibody Variable Regions

Total RNA was prepared from $3 \times 10^6$ hybridoma cells (one T175 flask) using the Qiagen RNeasy mini kit (Cat No: 74104). RNA was eluted in 50$\mu$L water and checked on a 1.2% agarose gel. The conditioned medium from the cells was retained and a sample used for testing in the antibody activity assay.

$V_H$ and $V_K$ cDNAs were prepared using reverse transcriptase with mouse IgG and $\kappa$ constant region primers. The first strand cDNAs were amplified by PCR using a large set of signal sequence primers. The amplified DNAs were gel-purified and cloned into the vector pGem® T Easy (Promega). The $V_H$ and $V_K$ clones obtained were screened for inserts of the expected size by PCR and the DNA sequence of selected clones determined by automated DNA sequencing. The locations of the complementarity determining regions (CDRs) in the sequences were determined with reference to other antibody sequences (Kabat EA *et al.*, 1991). The numbering convention of Kabat for antibody variable regions is used throughout this application; hence residue numbers may differ from the strict linear number.

The DNA sequence and deduced amino acid sequence for mC2 $V_K$ is shown in SEQ ID NO: 29 and 27, respectively. Four clones gave this identical productive sequence. A non-productive aberrant $V_K$ sequence that arises from the hybridoma fusion partner was also found in a number of clones.

For mC2 $V_H$, two different productive sequences were isolated. The mC2 $V_H$ AF sequence (see SEQ ID NO: 30) was found in a total of 29 clones, with 14 single base pair changes in individual clones. The mC2 $V_H$ B sequence was found in a total of 8 clones. Five of these represented the majority sequence, with the other 3 clones being variations on this. It is possible that these similar $V_H$ B sequences arose as an artifact of the PCR amplification. A non-productive aberrant $V_H$ was also obtained from the C2 hybridoma and is attributed to defective V-D-J joining.

In order to determine which is the correct active mC2 $V_H$, two chimeric antibodies were prepared with the two different $V_H$ sequences, AF and B, combined with the mC2 $V_K$, to be tested for the correct antibody activity.

**Example 2 Construction of Chimeric Antibody Genes**

A human chimeric antibody in its most common form consists of human constant regions linked to murine (or other non-human) variable regions. A chimeric antibody provides a very useful tool, firstly for confirmation that the correct variable regions have been identified, secondly for use as a control antibody in antigen binding assays with the same effector functions and utilizing the same secondary detection reagents as a humanized or engineered antibody, and also may be used to investigate the pharmacokinetic and other properties of the human constant regions with reference to the particular target for the antibody.

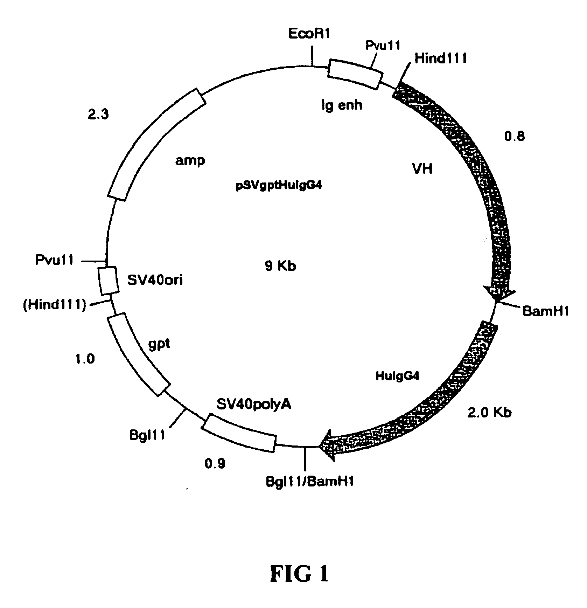

Two chimeric heavy chain expression vectors were constructed consisting of mC2 $V_H$ AF or mC2 $V_H$ B variable regions linked to HuIgG4 (Ser-Pro) constant region in the expression vector *pSVgpt* (Figure 1). This is based on pSV$_2$*gpt* (Mulligan and Berg, 1980) and includes the ampicillin resistance gene for selection in bacterial cells, the *gpt* gene for selection in mammalian cells, the murine heavy chain immunoglobulin enhancer region, genomic sequence encoding the constant region gene and SV40 poly A sequences. The heavy chain variable region for expression is inserted as a *Hin*dIII to *Bam*HI fragment.

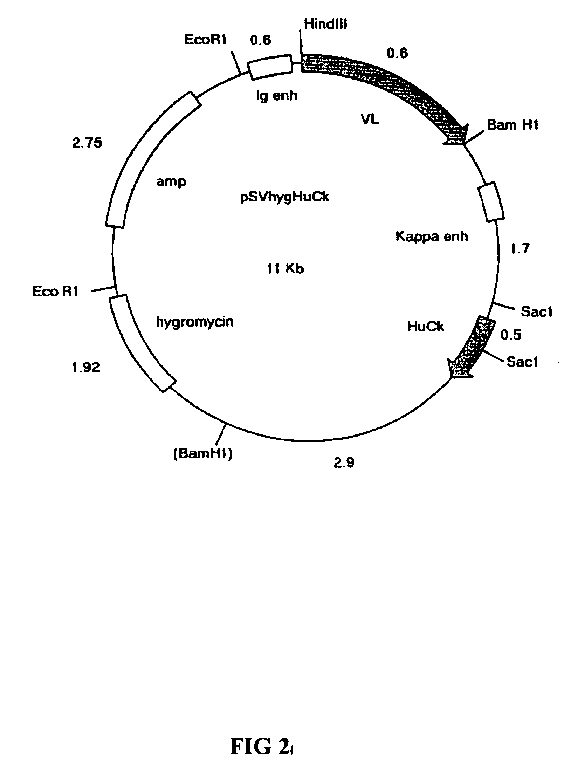

A chimeric light chain vector was constructed consisting of C2 VK linked to human C Kappa constant region in the expression vector pSVhyg (Figure 2). pSVhyg includes the ampicillin resistance gene for selection in bacterial cells, the *hyg* gene for selection in mammalian cells, the murine heavy chain immunoglobulin enhancer region, genomic sequence encoding the kappa constant region gene and including the kappa enhancer and SV40 poly A sequences. The light chain variable region for expression is inserted as a *Hin*dIII to *Bam*HI fragment.

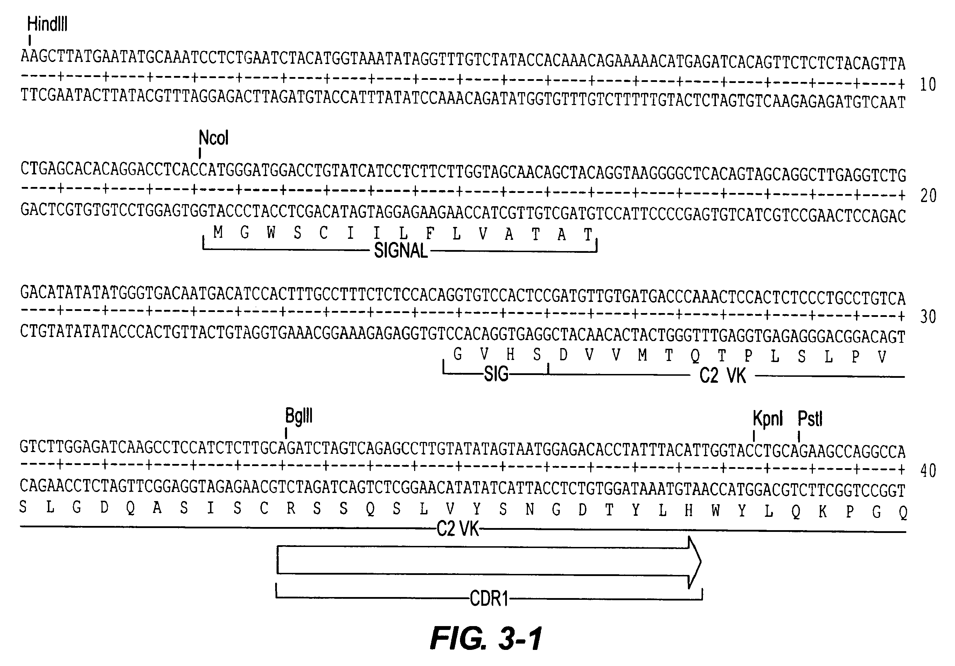

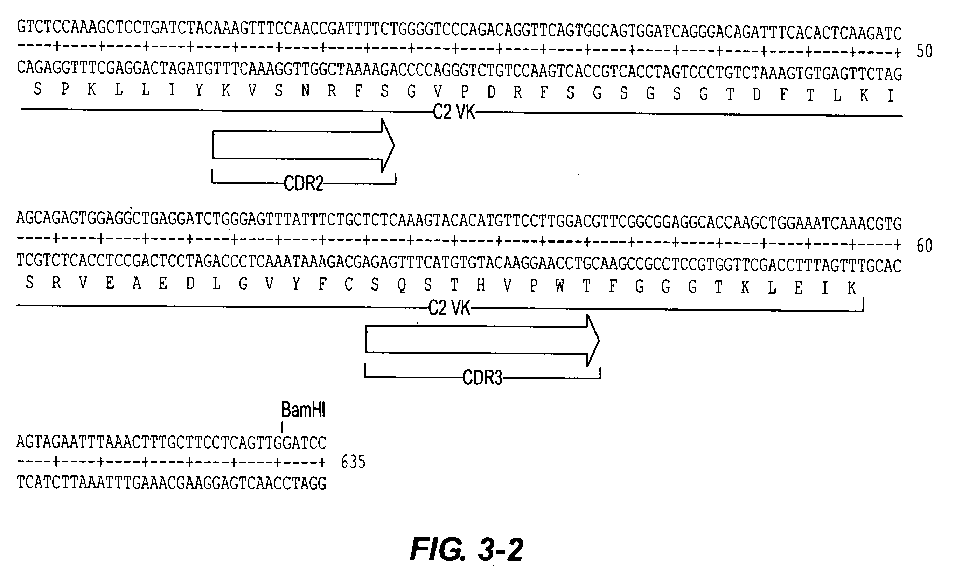

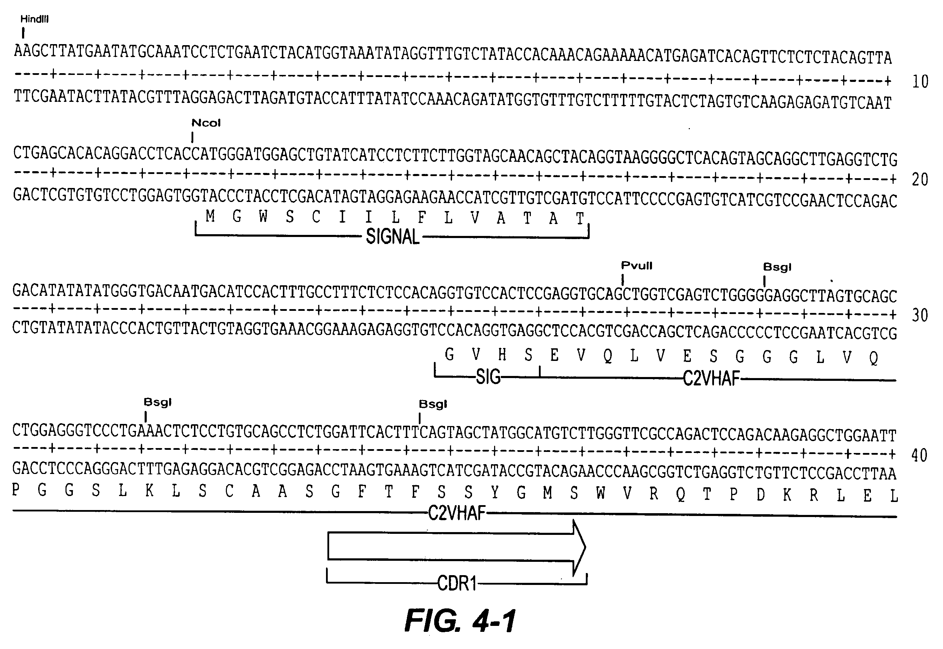

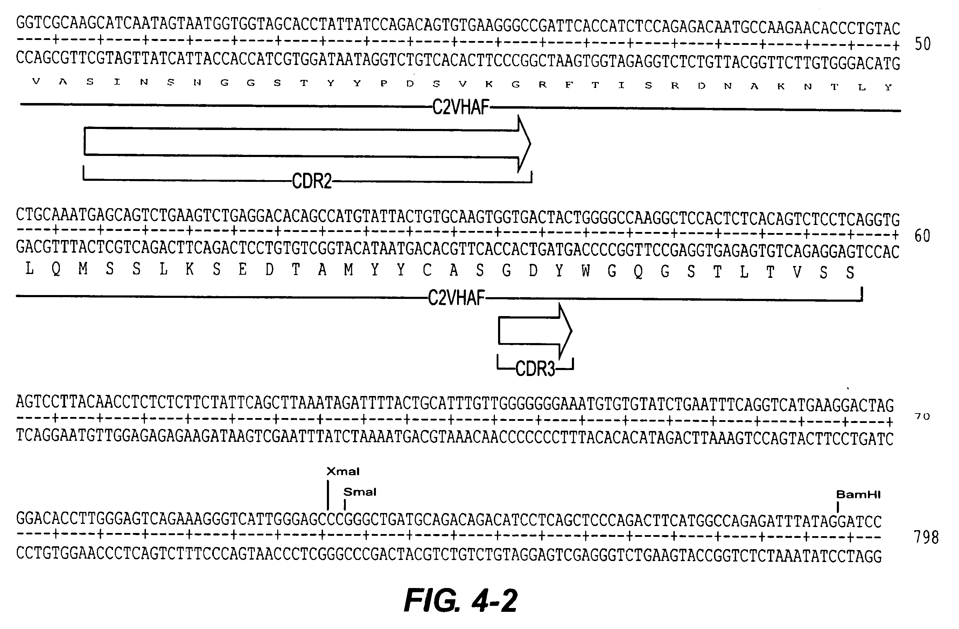

Expression cassettes for the murine C2 VH and VK sequences were constructed by addition of 5' flanking sequence including the leader signal peptide, leader intron and the murine immunoglobulin promoter, and 3' flanking sequence including the splice site and intron sequence, using the vectors VH-PCR1 and VK-PCR1 as templates (Riechmann et al., 1988). The DNA sequence was confirmed to be correct for the VH and VK in the chimeric expression vectors. The DNA and amino acid sequences of the VH and VK genes in the expression cassettes are shown in Figures 3 and 4.

**Example 3 Expression of Chimeric Antibodies**

*3.1 Expression in stable cell lines*

The host cell line for antibody expression was NS0, a non-immunoglobulin producing mouse myeloma, obtained from the European Collection of Animal Cell Cultures, Porton UK (ECACC No 85110503). The heavy and light chain expression vectors were co-transfected into NS0 cells by electroporation. Colonies expressing the *gpt* gene were selected in Dulbecco's Modified Eagle's Medium (DMEM) supplemented with 10% foetal bovine serum (FBS), 0.8 μg/ml mycophenolic acid and 250 μg/ml xanthine. Transfected cell clones were screened for production of human antibody by ELISA for human IgG. Cell lines secreting antibody were expanded and the highest producers selected and frozen down in liquid nitrogen. The best producing cell lines for each antibody were expanded in medium as above but with only 5% FBS. Chimeric antibodies were purified using Prosep®-A (Bioprocessing Ltd). The concentration was determined by ELISA for human IgGκ antibody. The antibodies were also analyzed by SDS-PAGE.

*3.2 Transient expression of chimeric antibodies*

To expedite the testing of the different chimeric antibodies, transient expression was used to produce quickly small quantities of cell supernatant containing recombinant antibody for testing. The mC2 $V_H$ and $V_K$ expression cassettes were transferred to vectors based on pcDNA3.1 (Invitrogen) for transient expression. The heavy chain vector included a human IgG constant region. The light chain vector included a human kappa constant region. Both mC2 $V_H$ AF and mC2 $V_H$ B were transfected with mC2 $V_K$ into human embryonic kidney (HEK 298) cells with Lipofectamine 2000 reagent (Invitrogen Cat No: 11668) according to the protocol supplied by the manufacturer. Conditioned medium was harvested from cells 3 days after transfection. The amount of antibody produced was determined by ELISA for human IgGκ antibody.

**Example 4 Activity of Chimeric C2 Antibodies**

*4.1 Activity of chimeric C2 antibodies produced by transient transfection*

Samples of conditioned medium from transient transfection for the two different chimeric antibodies were tested in the ELISA for binding to Amyloid Beta. The results clearly indicate that the C2 VH AF is the correct sequence. The C2 $V_H$ AF/C2 $V_K$ chimeric antibody binds well in the assay, but the C2 $V_H$ B/C2 $V_K$ does not show any binding at all. The Chemicon 1560 murine control antibody showed good binding, but binding by the purified murine C2 antibody supplied was low. It should be noted that a different secondary antibody was employed for the murine antibodies with the mouse constant regions compared to the chimeric antibodies with human constant regions, so the results are not directly comparable. Conditioned medium from the C2 hybridoma was later found to give a good result in the assay.

*4.2 Activity of purified chimeric C2 antibodies*

The two different C2 chimeric antibodies were purified from stable NS0 cell lines as described and tested using the Amyloid Beta ELISA. The results obtained are in accordance with the results obtained with transiently expressed antibody. The C2 ChVH AF/ChVK antibody binds well in the ELISA and the C2 ChVH B/ChVK antibody does not bind at all.

**Example 5 Design of Humanized C2 Antibody Genes**

The mC2 $V_H$ and $V_K$ amino acid sequences were compared to rodent antibody $V_H$ and $V_K$ sequences in the NCBI and Kabat databases.

*5.1 Light chain variable region*

The closest match mouse germ line gene to mC2 $V_K$ is bb1, Locus MMU231201, (*Schable et al*, 1999). Only two amino acids differ from this germ line sequence, both located within CDRL1. Mature murine antibodies with similar, but not identical, sequence are found. Several have an identical CDRL2 and identical CDRL3, but the CDRL1 of mC2 seems to be unique. mC2 $V_K$ can be assigned to Kabat subgroup $MuV_K II$. Position 87 of mC2 $V_K$ is F rather than the Y that is more common in the subgroup, indicating that this framework residue may be important for antibody activity. Comparison with human germ line $V_K$ sequences shows that genes from subgroup $V_K II$ are the best match for mC2 $V_K$ (Cox *et al*, 1994). Sequence DPK15 together with the human J region $HuJ_K 1$ were selected to provide the acceptor framework sequences for the humanized $V_K$.

Four humanized $V_K$ sequences were designed. C2HuVK1 consists of mC2 $V_K$ CDRs with frameworks from DPK 15 and human $J_K 1$. In versions 2, 3 and 4 murine residues have been substituted in the framework at positions 45 or 87 or both. Residue 45 may be involved in supporting the conformation of the CDRs. Residue 87 is located at the interface of the $V_H$ and $V_K$ domains. Therefore these residues may be critical for maintenance of antibody binding.

The positions and changes that have been made in the light chain framework regions are shown in Table 1. A comparison of the humanized sequences with mC2 $V_K$ sequence, and with DPK15 and human $J_K 1$.

*5.2 Heavy chain variable region*

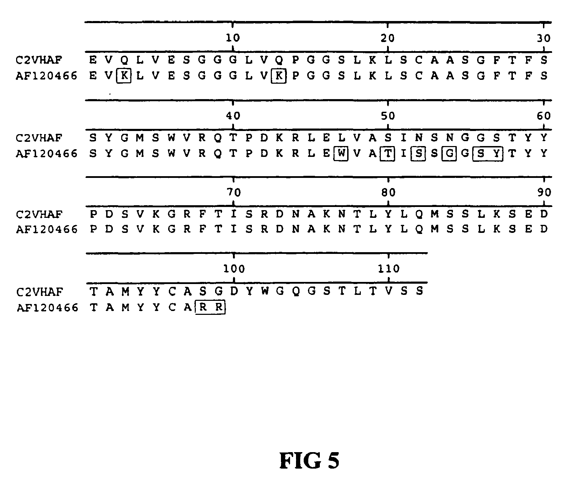

The closest match mouse germ line gene to mC2 $V_H$ AF is VH7183, Locus AF120466, (Langdon *et al*, 2000). The comparison is shown in Figure 5. Nine amino acids differ from this germ line sequence, most being located within CDR2. Mature murine antibodies with identical or similar (one residue different) CDR1 or with similar CDR2 (one residue different) are found, but none with all three CDRs identical to mC2 $V_H$ AF. CDR3 of mC2 antibody is unusually short, consisting of only three residues. However, other antibodies are found in the database with CDR3 of this length. mC2 $V_H$ AF can be assigned to Kabat subgroup $MuV_H IIID$. Residue 47 of mC2 $V_H$ is L rather than the more common W, and residue 94 is S rather than the normal R, indicating that these framework residues may be important for antibody activity. Comparison with human germ line $V_H$ sequences shows that genes from subgroup $V_H III$ are the best match for mC2 $V_H$. Sequence DP54 together with the human J region $HuJ_H 6$ was selected to provide the acceptor framework sequences for the humanized $V_H$.

Four humanized $V_H$ sequences were designed. C2HuVH1 consists of mC2 $V_H$ AF CDRs with frameworks from DP54 and HuJ$_H$6. In versions 2, 3 and 4 murine residues have been substituted in the framework at positions 47 or 94 or both. Residue 47 in framework 2 makes contact both with the CDRs and with the $V_K$ domain. Residue 94 may be involved in supporting the conformation of the CDRs. Therefore these residues may be critical for maintenance of antibody binding.

The positions and changes that have been made in the heavy chain framework regions are shown in Table 2.

## Example 6 Construction of Humanized Antibody Genes

The modified variable regions were constructed by the method of overlapping PCR recombination. The expression cassettes for the chimeric antibody, C2 ChV$_H$ AF and C2 ChV$_K$, were used as templates for mutagenesis of the framework regions to the required sequences. Sets of mutagenic primer pairs were synthesized encompassing the regions to be altered. The humanized $V_H$ and $V_K$ expression cassettes produced were cloned into pUC19 and the entire DNA sequence was confirmed to be correct for each $V_H$ and $V_K$. The modified heavy and light chain V-region genes were excised from pUC19 as *Hind*III to *Bam*HI expression cassettes. These were transferred to the expression vectors *pSVgpt* and pSV*hyg* which include human IgG4 Ser-pro or κ constant regions respectively, as for the chimeric antibody vectors. The DNA sequence was confirmed to be correct for the humanized $V_H$ and $V_K$ in the expression vectors.

## Example 7 Expression of Humanized Antibodies

### 7.1 Expression in stable cell lines

The humanized heavy and light chain expression vectors were co-transfected into NS0 cells by electroporation, as for the expression of chimeric antibodies. Antibody producing cell lines were selected and expanded and humanized antibodies purified, exactly as for the chimeric antibody. The purified antibodies were analyzed by SDS-PAGE.

### 7.2 Transient expression of humanized antibodies

To expedite testing of the different humanized $V_H$ and $V_K$ constructs, the C2 humanized $V_H$ and $V_K$ expression cassettes were also transferred to the vectors for transient expression described in section 7.2. The four humanized C2 $V_K$ constructs were co-transfected with the chimeric C2 $V_H$ construct into HEK293 cells. Similarly, the four humanized C2 $V_H$ constructs were co-transfected with the chimeric C2 $V_K$ construct into HEK293 cells. Conditioned medium was harvested from cells three days after transfection. The amount of antibody produced was determined by ELISA for human IgGκ antibody.

## Example 8 Activity of Humanized C2 Antibodies

### 8.1 Activity of humanized C2 antibodies produced by transient transfection

Samples of conditioned medium from the transient transfection were tested in the Amyloid Beta ELISA. The results obtained clearly indicate that the humanized VH constructs C2 HuVH AF versions 2 and 4 are functional when combined with the chimeric C2 kappa chain, and are comparable to the chimeric C2 antibody in the assay. In contrast, the antibodies containing C2 HuVH AF versions 1 and 3 combined with the chimeric C2 kappa chain show no binding at all in the assay. This indicates that the substitution of the murine residue at position 94 is essential for antibody activity. Antibodies containing the chimeric C2 heavy chain combined with the four humanized C2 kappa chains all showed good binding, comparable to the chimeric antibody, in the ELISA.

### 8.2 Activity of purified humanized C2 antibodies

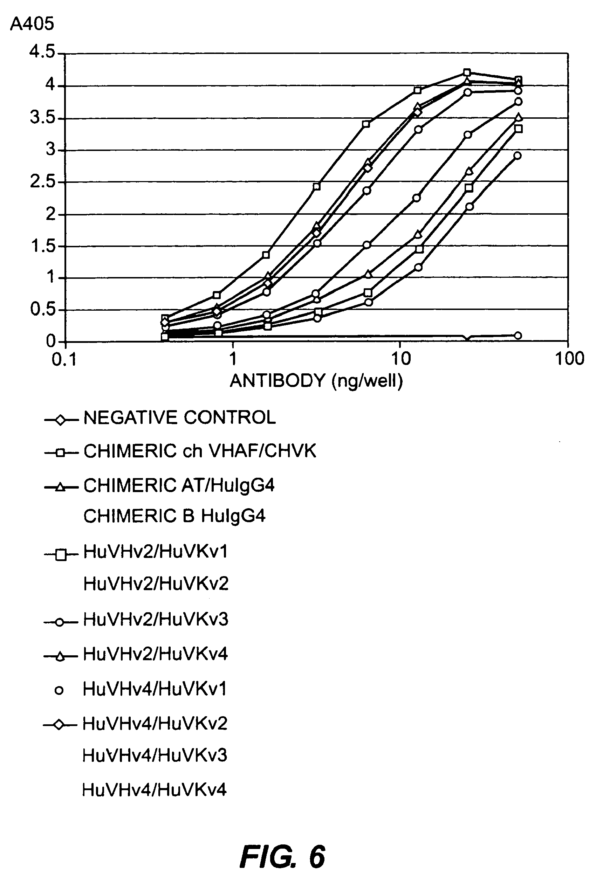

Eight different humanized C2 antibodies comprising all combinations of two humanized heavy chains and four humanized light chains were purified from stable NS0 cell lines as described and tested using the Amyloid Beta ELISA (figure 6).

The results obtained clearly indicate that C2 HuVH4 antibodies perform better in the assay than C2 HuVH2 antibodies. Of the C2 HuVH2 antibodies, C2 HuVH2/HuVK3 shows the best binding activity, but this is approximately 2 fold reduced compared to the chimeric control antibody C2 ChVHAF/ChVK. C2 HuVH2/HuVK2 activity is four to five fold reduced compared to the control. The activities of the antibodies comprising C2HuVH4 with the four different humanized light chains are similar. The highest activity is observed for C2HuVH4/HuVK1 and all four antibodies are close to the control chimeric antibody in the assay.

**Example 9 Modifications to CDRL2**

*9.1 Design light chain with modified CDR 2*

As noted above, many antibodies share the same CDRL2 sequence ("KVSNRFS") as the C2 antibody. It was decided to test whether CDRL2 could be modified slightly without adversely affecting antibody activity. Two conservative substitutions were selected: R for K at position 50 and S for N at position 53. The two alternative CDRL2 sequences are therefore "RVSNRFS" and "KVSSRFS". These were incorporated into the murine $V_K$ sequence with no other changes, as mC2 VK-R and mC2 VK-S respectively.

*9.2 Transient expression of modified CDRL2 antibody*

The two C2 light chain constructs with modified CDRL2 described in Section 11.2.1 were cloned into the light chain vector for transient expression. Each was co-transfected with the chimeric C2 $V_H$ vector into HEK293 cells. Conditioned medium was harvested from cells three days after transfection. The amount of antibody produced was determined by ELISA for human IgGκ antibody.

*9.3 Activity of C2 antibody with modified CDRL2*

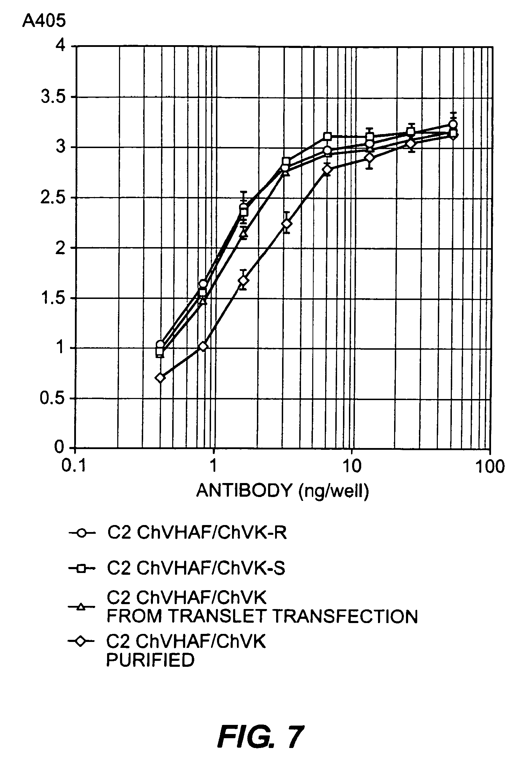

Samples of conditioned medium from the transient transfection of mC2 $V_K$s with modified CDRL2 combined with mC2 $V_H$ were tested in the Amyloid Beta ELISA.(figure 7) Both the VK-R and the VK-S antibodies are comparable to the chimeric C2 antibody, indicating that the individual modifications to CDRL2 chosen do not markedly affect the activity of the antibody in the assay.

**Example 10 Affinity Determination**

To assess the binding specificity and affinity of mouse (ACI-01-Ab-7-C2), chimeric (AF) and humanized antibodies (H4K1; H4K4), BIACORE.RTM, analysis was performed using amyloid beta 1-42 monomers and fibers as antigen immobilized on a CM5 chip. BIACORE.RTM. technology utilizes changes in the refractive index at the surface layer upon binding of the antibody to the antigen immobilized on the layer. Binding is detected by surface plasmon resonance (SPR) of laser light refracting from the surface. Analysis of the signal kinetics on rate and off rate allows the discrimination between non-specific and specific interaction. The concentration of antibody used was in the range of 0.05 μM to 1.0 μM.

**Table 1:** Binding specificity and affinity of mouse (ACI-01-Ab-7-C2) chimeric (AF) and humanized antibodies (H4K1; H4K4) for amyloid beta 1-42 monomers and fibers

| | Monomers | | | Fibers | | |
|---|---|---|---|---|---|---|
| | $k_a$(1/Ms) | $k_d$(1/s) | KD (M) | $k_a$(1/Ms) | $k_d$(1/s) | KD (M) |
| Mouse ACI-01-Ab-7-C2 | 1,8E+04 | 2,7E-03 | 1,5E-07 | 2,4E+04 | 9,9E-04 | 4,1E-08 |
| chimeric AF | 4,7E+04 | 9,5E-04 | 2E-08 | 5,1E+04 | 3,3E-04 | 6,5E-09 |
| humanized H4K1 | 5,0E+04 | 9,5E-04 | 1,9E-08 | 4,9E+04 | 2,3E-04 | 4,7E-09 |
| humanized H4K4 | 2,5E+04 | 4,4E-04 | 1,8E-08 | 1,3E+05 | 3,0E-04 | 2,3E-09 |

**Example 11 Immunhistochemical Binding Assay**

*11.1 Human brain sections*

Brains from healthy, non-demented pre-AD and AD patients were obtained from the Universitätsklinik in Bonn after ethical approval. Brains were fixed in formaldehyde and the hippocampus region was dehydrated, embedded in paraffin and 5 μm sections were cut with a microtome. Paraffin sections were stored at RT until use. For fresh material, 5 μm cryosections were cut with a cryostat and sections stored at -80°C until use.

*11.2 Immunohistochemistry*

Paraffin sections were deparaffinized and rehydrated by bathing slides in xylene followed by 100% ethanol, 90% ethanol and 70% ethanol. Background was decreased by 30 minutes incubation in $10\% H_2O_2$, 10% methanol in water. Antigen retrieval was obtained by incubating the slides in 100% formic acid for 3 minutes. After 3 washes in Tris buffered saline (TBS, pH 7.5), non-specific labeling was blocked by a 2 hour incubation of the slides in 10 % BSA, 0.25% Triton X-100 in TBS. After washing (3 washes in TBS) blocking of endogenous antibodies was performed by adding a non-labeled anti-human IgG (Biomeda) and incubating slides in humid chambers overnight at RT. After another 3 washes, the primary human anti amyloid antibody was added to the slides and incubated another 24 hours at RT. Following washing, an alkaline phosphatase labeled secondary anti human IgG (Sigma) was added to the slides and incubated for 2 hours at RT. After washing, slides were developed with Liquid permanent Red (Dakocytomation) washed with water and air-dried before mounting with permanent mounting media (corbitbalsam).

Cryosections were fixed in methanol for 30 minutes at -80°C and background decreased by adding $H_2O_2$ to the cold methanol to a final concentration of 10% and incubating for 30 minutes at RT. After 3 washes in Tris buffered saline (TBS, pH7.5), non-specific labeling was blocked by a 2 hour incubation of the slides in 10 % BSA, 0.25% Triton X 100 in TBS as above and the same staining procedure as above was carried out.

Sections were examined with a Leica DMLB microscope and photographed using a Leica DC500 camera and Leica FireCam1.2.0 software.

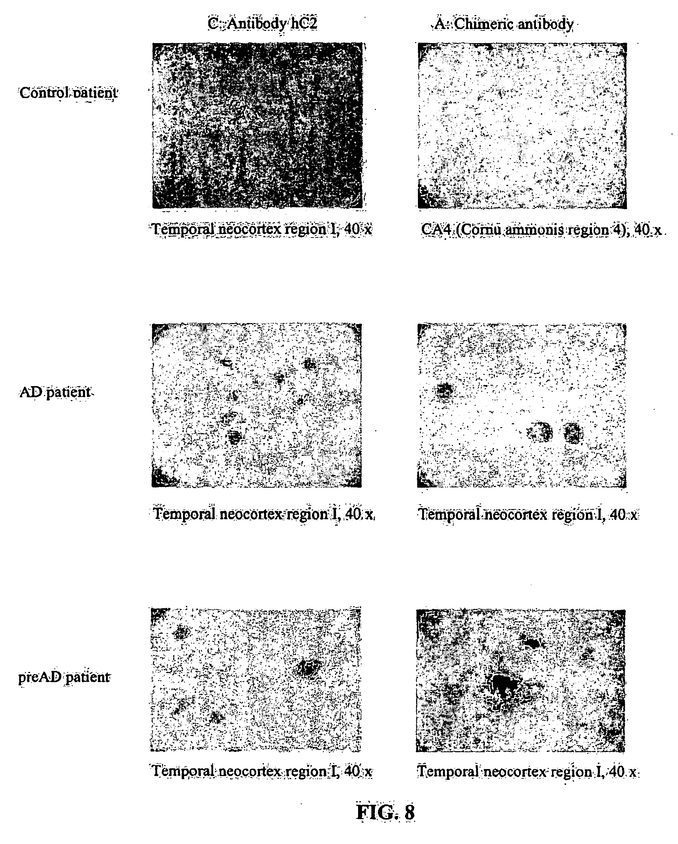

Both human antibodies A and C labeled plaques of brains from AD disease patients (figure 8). Both diffuse and cored plaques were labeled. Moreover, diffuse plaques in non -demented pre-AD patients could also be detected by the A and C antibodies. Amyloid in cerebral amyloid angiopathy (CAA) was labeled with both antibodies and some staining of neurons which may correspond to intracellular amyloid was also detected. No labeling was seen on control brains from healthy patient. Plaques could be detected on paraffin sections pretreated with formic acid but no plaques were labeled on paraffin sections without formic acid pretreatment and on cryosections fixed in methanol. The human antibody B did not detect plaques on paraffin sections and the mouse antibody did not stain either paraffin or cryosections of human brains.

Abbreviations:

A = binding chimeric antibody AF (IgG4)

B = non-binding chimeric antibody B (IgG4)

C = binding humanized antibody H4K1 (IgG4)

Mouse = ACI-01-Ab-C2 mouse antibody (IgG2b)

**Example 12 Functionality of mC2 on Amyloid Fibers**

*12.1 Modification of Conformation of $A\beta$1-42 Fibers and Initiation of Disaggregation after Binding of the mC2 antibody*

In order to evaluate the mechanism by which the antibody is capable to disaggregate preformed beta-amyloid ($A\beta_{1-42}$) fibers, a head-to-head comparison of Thioflavin-T (Th-T) fluorescent assay was performed measuring disaggregation and solid-state Nuclear Magnetic Resonance (NMR) of U-$^{13}$C Tyrosine10 and Valine12-labeled $A\beta$1-42 peptide, analysing secondary conformation (figure 9A). The mC2 antibody solubilized 35.4 % of the preformed $A\beta$1-42 fibers and simultaneously induced a shift in secondary conformation from beta sheet to random coiled. The reduction in the population of the beta sheet conformation with respect to the random coil is of the order of 35% and is therefore in close agreement with that measured using fluorescence Th-T assay (figure 9B). These data indicate that the binding of the mC2 antibody initiates a transition of the secondary structure which potentially causes a destabilization of the parallel intermolecular arrangement of the beta sheets affecting a break of elongated fibers into smaller fragments.

*12.2 Conformation-dependent Binding Affinity of mC2 antibody*

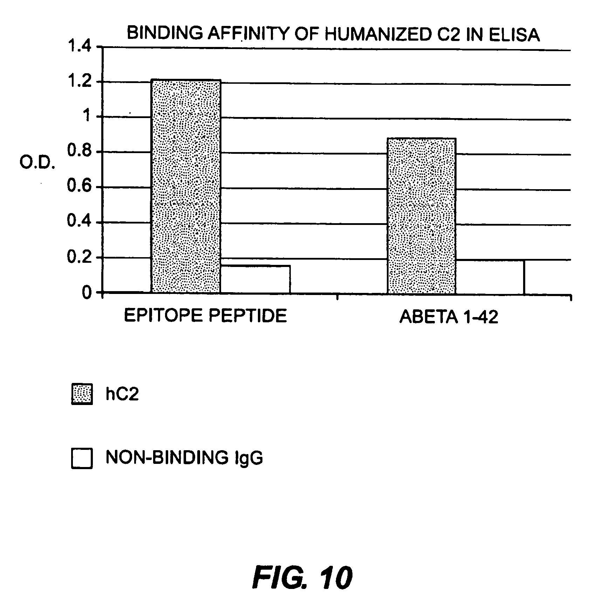

Since it is well known in the scientific literature that a proportion of the antibody-antigen binding energy can be used for energy-dependent modification of the conformation of an antigen (Blond and Goldberg, 1987), a comparison experiment of the binding affinity of the C2 antibody to the whole $A_{\beta 1\text{-}42}$ protein and to a smaller, nine amino acid long, peptide comprising the antibody's epitope was performed (figure 10). For this comparison the affinities of the humanized antibody C2 were analyzed by ELISA using biotinylated peptides covering the complete amino-acid sequence of the C2's epitope (produced by Mimotopes and purchased from ANAWA Trading SA) and a biotinylated complete $A_{\beta}1\text{-}42$ peptide (Bachem). The analysis was done according to the manufacturer's (Mimotopes) instructions. As demonstrated in Figure 10, the antibody binds with a 36.0% higher affinity to the peptide comprising its specific epitope (aminoacids 13-21 of the $A_{\beta 1\text{-}42}$ sequence) than to the whole $A_{\beta}1\text{-}42$ protein. It is therefore suggested that the difference in binding affinity energy was used for the energy-consuming transition of the secondary conformation of the amyloid protein to present the antigen in a more acceptable position for the antibody interaction. This explains why the affinity of the antibody is lower for the native (the whole amyloid protein) than for the isolated subunit.

**Example 13 Effects of the anti-amyloid hC2 on the aggregation of amyloid beta 1-42 peptide**

To evaluate the ability of the humanized anti-human amyloid beta monoclonal antibody hC2 to mediate anti-aggregating and disaggregating effects on amyloid beta ($A\beta$) a thioflavin T spectrofluorescence assay was accomplished.

*13.1 Inhibition of Aggregation Assay*

$A\beta 1\text{-}42$ lyophilized powder was reconstituted in hexafluoroisopropanol (HFIP) to 1 mM. The peptide solution was sonicated for 15 min. at room temperature, agitated overnight, and aliquots made into non-siliconized microcentrifuge tubes. The HFIP was then evaporated under a stream of argon. The resulting peptide film was vacuum dried for 10 min and stored at -80°C until used.

To assay for the antibody-mediated inhibition of $A\beta 1\text{-}42$ aggregation the hC2 antibody was pre-diluted in PBS and an assay solution containing the following components was made in a non-siliconized incubation tube: 3.3 or 0.33 mM pre-diluted antibody, 10 mM thioflavin T, 33 mM $A\beta 1\text{-}42$, and 8.2% DMSO. Therefore the final molar ratios of antibody to $A\beta 1\text{-}42$ were 1:10 and 1:100. Appropriate control solutions were also prepared. The solutions were then incubated for 24 hrs at 37°C, and the spectrofluorescence (relative fluorescence units; RFU) read in six replicates in black 384-well plates (Perkin-Elmer) on a Perkin-Elmer FluoroCount spectrofluorometer. The spectrofluorescence was then measured and % disaggregation calculated as described below.

*13.2 Disaggregation Assay*

To assay for antibody-mediated disaggregation of pre-aggregated $A\beta 1\text{-}42$, a low-molecular weight $A\beta_{1\text{-}42}$, prepared as described above, was made up as a 110 mM solution in 27% DMSO and 1x PBS. This solution was then allowed to aggregate at 37°C for 24 hrs after which the following were added: 3.3 or 0.33 mM pre-diluted antibody, and 10 mM thioflavin T. This resulted in a molar ratio of 1:10 and 1:100 antibody to $A\beta_{1\text{-}42}$. This solution was then incubated for additional 24 hrs at 37°C. The spectrofluorescence was then measured and % disaggregation calculated as described below.

*13.3 Calculation*

Inhibition of aggregation or disaggregation is expressed as mean % inhibition or disaggregation, respectively, $\pm$ standard error of the mean (SEM) according to the following equation:

$$\% \text{ Inhibition} = \frac{(\text{RFU of pos contrl} - \text{RFU of neg contrl}) - (\text{RFU of sample with } A\beta 1\text{-}42 - \text{RFU of sample without } A\beta 1\text{-}42)}{(\text{RFU of pos contrl} - \text{RFU of neg contrl})} \times 100\%$$

*13.4 Result*

*13.4.1 Inhibition of $A\beta 1\text{-}42$ aggregation*

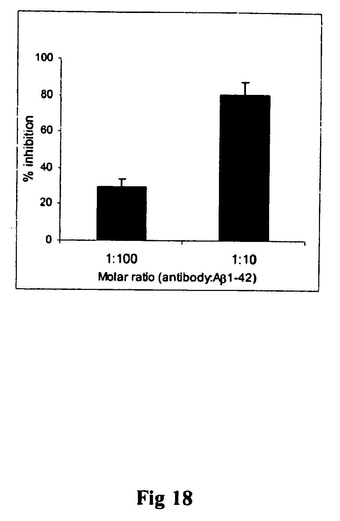

Inhibition of $A\beta_{1\text{-}42}$ aggregation using the hC2 antibody is shown in Table 1 and Figure 18. At an antibody to $A\beta_{1\text{-}42}$ molar ratio of 1:100 the inhibition averaged 30% (2 independent experiments), whereas at a 1:10 molar ratio the inhibition was 80% (2 independent experiments; *see* Table 2).

**Table 2.** hC2-mediated inhibition of $A\beta_{1-42}$ aggregation at a 1:100 and 1:10 antibody to $A\beta_{1-42}$ molar ratios.

| Antibody | Molar ratio (antibody to $A\beta_{1-42}$) | |
|---|---|---|
| | 1:100 | 1:10 |
| hC2 | 30.0 ± 4.1% | 80.4 ± 6.9% |

### 13.4.2 Disaggregation of pre-aggregated $A\beta1$-42

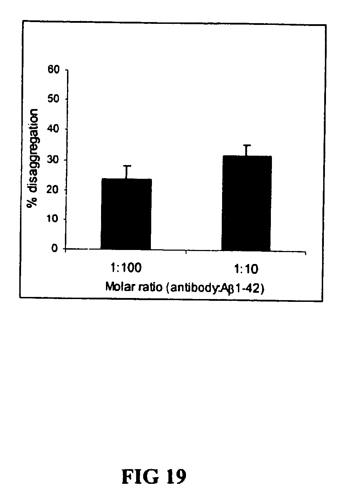

Disaggregation of pre-aggregated $A\beta_{1-42}$ using the hC2 antibody is shown in Table 2 and Figure 19. At an antibody to $A\beta_{1-42}$ molar ratio of 1:100 the disaggregation averaged 24%, whereas at a 1:10 molar ratio the disaggregation was 32% (3 independent experiments; see Table 3).

**Table 3.** hC2-mediated disaggregation of pre-aggregated $Ab_{1-42}$ at a 1:100 and 1:10 antibody to $A\beta_{1-42}$ molar ratios.

| Antibody | Molar ratio (antibody to $A\beta_{1-42}$) | |
|---|---|---|
| | 1:100 | 1:10 |
| hC2 | 23.9 ± 4.4% | 31.9 ± 3.5% |

Using the thioflavin T assay, the bi-functional properties of the anti-$A\beta$ humanized antibody hC2 can be demonstrated, namely to inhibit the aggregation of $A\beta_{1-42}$ into pathogenic protofibrillar conformation and in addition to disaggregate preformed $A\beta_{1-42}$ protofibrils. hC2 inhibited $A\beta_{1-42}$ aggregation by 80% at an antibody to $A\beta_{1-42}$ molar ratio of 1:10. The ability of hC2 to disaggregate pre-aggregated protofibrils of $A\beta_{1-42}$ at a 1:10 molar ratio was shown to be 32%.

### Example 14: Conformation-specific binding of mC2 to different classes of Amyloid Protein

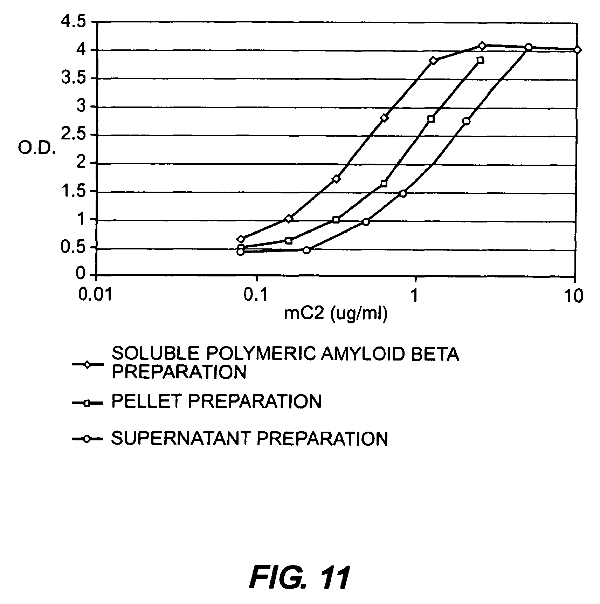

In order to evaluate the specificity of mC2 to different stages of polymerized amyloid protein, monomeric, polymeric soluble and fibrillic amyloid, an ELISA coated with these different stages of polymeric beta-amyloid was performed (figure 11). Monomers were prepared according to a modified method published by Klein (2002), soluble polymeric amyloid beta according to Barghorn *et al.* (2005), whereas fibers were performed by incubation of amyloid (Bachem, Switzerland) with a final concentration of $1\mu g/\mu l$ in Tris/HCl pH 7.4 at 37°C for 5 days followed by a centrifugation step (10,000 rpm for 5 minutes). Then amyloid polymers were coated on an ELISA plates with a final concentration of $55\mu g/ml$ and binding affinity ELISA by using an anti-mouse IgG monoclonal antibody (Jackson) labelled with alkaline phosphate was performed. As demonstrated in Figure 11 the mC2 antibody binds with higher affinity to soluble polymeric amyloid beta than to fibers and with the lowest to monomers. These data indicate that the antibody's binding is influenced by the amyloid epitope and by the conformation of the different amyloid aggregates.

### Example 15: Epitope mapping of AC Immune's monoclonal antibody hC2

Epitope mapping of the humanized monoclonal antibody hC2 was performed by ELISA using three different peptide libraries. One library comprised a total of 33 biotinylated peptides covering the complete amino acid (aa) sequence of $A\beta1$-42 (produced by Mimotopes and purchased from ANAWA Trading SA), the second library contains biotinylated peptides using peptide 12 (aa12-20 of $A\beta$) from the first peptide library and substituting each aa in the sequence by an alanine (see table 3 below), and the third library contains biotinylated peptides 13, 14, or 15 (aa 13-21, 14-22 or 15-23 of $A\beta$) and substituting in each case the last amino acids to an alanine or to a glycine for aa 21 which is already an alanine (see table 4 below). A biotinylated complete $A\beta_{1-42}$ peptide was used as positive control (Bachem). Epitope mapping was done according to the manufacturer's (Mimotopes) instructions. Briefly, Streptavidin coated plates (NUNC) were blocked with 0.1% BSA in PBS overnight at 4°C. After washing with PBS-0.05% Tween 20, plates were coated for 1 hour at RT with the different peptides from the library, diluted in 0.1 % BSA, 0.1 % Sodium Azide in PBS to a final concentration of 10 $\mu$M. After washing, plates were incubated for 1 hour at RT with the hC2 antibody or a non $A\beta$. binding chimeric IgG4 antibody diluted to 200 ng/ml in 2% BSA, 0.1% Sodium Azide in PBS. Plates were washed again and incubated with alkaline phosphatase conjugated goat anti human IgG for 1h at RT. After final washing, plates were incubated with phosphatase substrate (pNPP) and read at 405 nm using an ELISA plate reader.

The humanized monoclonal antibody hC2 bound specifically to peptides 12,13,14,15 and 16 of the first peptide library (Figure 17A). These peptides comprise aa 12-20, 13-21, 14-22, 15-23 and 16-24 respectively of $A\beta_{1-42}$, suggesting that the epitope lies in region 12-24 of $A\beta$. A second library with alanine substitutions was used to determine the critical aa for binding to Aβ12-20 (VHHQKLVFF). The binding of the hC2 antibody is lost completely when amino acids 16, 17, 19 or 20 are substituted by an alanine, indicating that these aa are absolutely critical for binding of the antibody to Aβ. The binding of the hC2 antibody is partially lost when aa 15 and 18 are substituted.

The binding was also almost completely lost when aa 14 was substituted for an alanine, indicating that aa 14 is also very important for binding (Figure 17B).

Finally, a third library was used to determine whether aa 21, 22 or 23 are critical for binding to the epitope. The binding of the antibody to aa 15-23 was reduced when aa 23 was substituted for an alanine, indicating that aa 23 is also important for binding. The binding was partially lost when aa 21 was substituted for a glycine and slightly lost when aa 22 was substituted for an alanine (Figure 17C).

## Example 16: Neuroprotection by the hC2 Antibody

The ability of antibody hC2 to protect neurons from Abeta oligomer-induced degeneration was assessed in an in vitro assay. Embryonic day 16.5-17.5 mouse cortical neurons were isolated, dissociated, and cultured in vitro in N3-F12 media. The cells were grown for nine days in total, and were fed on day 3 and on the day that Abeta oligomer, or Abeta oligomer plus anti-Abeta antibody hC2 was added. At day five ("4 days Abeta") or day six ("3 days Abeta"), certain wells of cells were treated with either 2 µM Abeta oligomer alone, or a combination of 2 µM Abeta oligomer and 50 µg/mL anti-Abeta antibody hC2.

The Abeta oligomer was prepared by dissolving Abeta 1-42 (rPeptide) in HFIP, from which Abeta peptides were aliquoted into 10µl aliquots at 1mg/ml and then evaporated in a fume hood for 30 minutes and peptide films were stored at -80C until use. Upon use, the peptide film was dissolved in 10µl of DMSO, then 78.6µl of HAMS F12, and the Abeta peptide solution was incubated at 4C for 24-48 hours (25µM final concentration of Abeta).

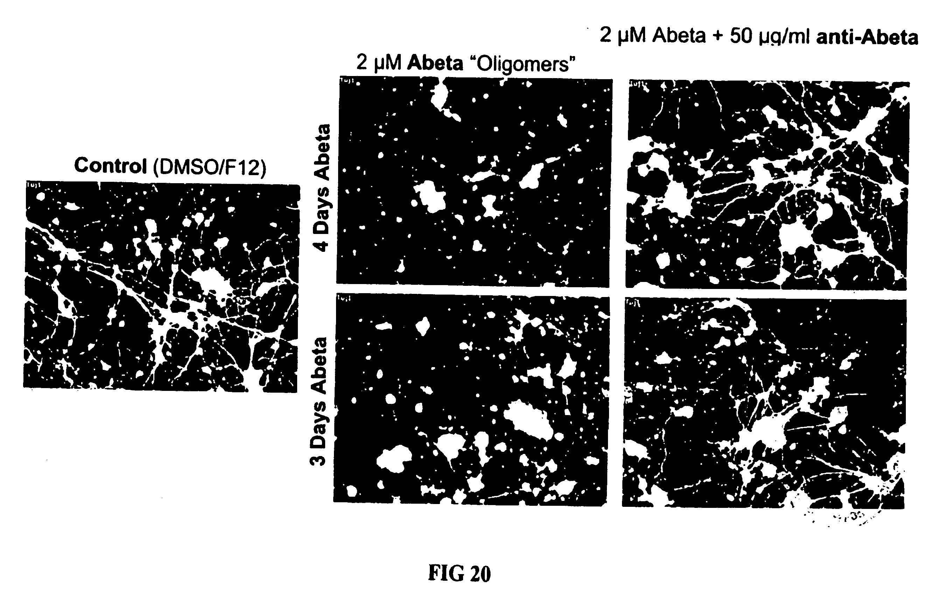

For control cells, DMSO-F12 alone was added at the same volume as Abeta-DMSO at day 5, and the cells were cultured for an additional 4 days without any additional treatment. On day 9, neurons from all culture conditions were fixed and stained with Tuj1 (an anti-beta-tubulin antibody), followed by staining with secondary antibodies labeled with FITC to visualize microtubules, and thus neuronal processes in general. The results are shown in Figure 20.

Untreated mouse embryonic cortical neurons showed normal morphology after nine days of culture (Figure 20, leftmost panel). Treatment of the cells with Abeta oligomer for three days induced axon degeneration and caused a decrease in the total number of axons (Figure 20, lower center panel), and this effect was even more pronounced at four days of treatment (Figure 20, upper center panel). In contrast, the cells treated with the combination of Abeta oligomer and anti-Abeta antibody hC2 looked similar to control cells (Figure 20, upper and lower right panels). These results indicate that anti-Abeta antibody hC2 was able to protect embryonic mouse cortical neurons from Abeta oligomer-induced degeneration.

**Table 4:** Positions and changes made in the humanized C2 light chain framework regions

| Position Light chain | 45 | 87 | 50 | 53 |
|---|---|---|---|---|
| Mouse C2V$_K$ | K | F | K | N |
| Humanized C2HuV$_K$1 | Q | Y | K | N |
| Humanized C2HuV$_K$2 | Q | F | K | N |
| Humanized C2HuV$_K$3 | K | Y | K | N |
| Humanized C2HuV$_K$4 | K | F | K | N |
| Human Germline dpk15 | Q | Y | L | N |
| Mouse C2V$_K$-R | | | R | |
| Mouse C2V$_K$-S | | | | S |

**Table 5:** Positions and changes made in the humanized C2 heavy chain framework regions

| Position Heavy chain | 47 | 94 |
|---|---|---|
| Mouse C2VHAF | L | S |
| Humanized C2HuVHAF1 | W | R |
| Humanized C2HuVHAF2 | W | S |

(continued)

| Position Heavy chain | 47 | 94 |
|---|---|---|
| Humanized C2HuVHAF3 | L | R |
| Humanized C2HuVHAF4 | L | S |
| Human Germline DP-54 | W | R |

A total of 8 different antibodies were constructed with light chains Humanized C2HuV$_K$1, C2HuV$_K$2, C2HuV$_K$3, C2HuV$_K$4 and heavy chains C2HuVHAF4 and C2HuVHAF2.

**Table 6.** Summary of peptides used in the second library

aa that are important for binding are marked in italics and underscore and aa absolutely critical for binding are marked in italics and bold.

| p12-20 | V | H | H | Q | K | L | V | F | F |
|---|---|---|---|---|---|---|---|---|---|
| A12 | **A** | H | H | Q | K | L | V | F | F |
| A13 | V | **A** | H | Q | K | L | V | F | F |
| A14 | V | H | **A** | Q | K | L | V | F | F |
| A15 | V | H | H | **A** | K | L | V | F | F |
| A16 | V | H | H | Q | **A** | L | V | F | F |
| A17 | V | H | H | Q | K | **A** | V | F | F |
| A18 | V | H | H | Q | K | L | **A** | F | F |
| A19 | V | H | H | Q | K | L | V | **A** | F |
| A20 | V | H | H | Q | K | L | V | F | **A** |
| aa no. | 12 | 13 | **14** | <u>15</u> | **16** | **17** | <u>18</u> | **19** | **20** |

**Table 7.** Summary of peptides used in the third library.

aa that are important for binding are marked in italics and underscore and aa absolutely critical for binding are marked in italics and bold

| p13-21 | | H | H | Q | K | L | V | F | F | A | | |
|---|---|---|---|---|---|---|---|---|---|---|---|---|
| p13-21 | G21 | H | H | Q | K | L | V | F | F | **G** | | |
| p14-22 | | | H | Q | K | L | V | F | F | A | E | |
| p14-22 | A22 | | H | Q | K | L | V | F | F | A | **A** | |
| p15-23 | | | | Q | K | L | V | F | F | A | E | D |
| p15-23 | A23 | | | Q | K | L | V | F | F | A | E | **A** |
| aa no. | | 13 | **14** | <u>15</u> | **16** | **17** | <u>18</u> | **19** | **20** | 21 | 22 | <u>23</u> |

## Example 17 Effect of the hC2 antibody on cultured retinal ganglion cell (RGC) apoptosis

To assess the in vitro capacity of the hC2 antibody to reduce retinal ganglion cell (RGC) death related to ocular diseases associated with pathological abnormalities/changes in the tissues of the visual system, particularly associated with amyloid-beta-related pathological abnormalities/changes in the tissues of the visual system, such as, for example, neuronal degradation, cultured RGCs from rats and mice are used.

To isolate the cells, at sacrifice the animals are anesthetized, their eyes are removed and the retina is dissected and incubated in 2 mg/ml papain solution for 25 minutes at 37°C to break down the extracellular matrix. At the end of treatment, the cells are washed three times with RCG medium in the presence of a protease inhibitor to stop the papain action. The tissue is then triturated by passing it quickly up and down through a Pasteur pipette until the cells are dispersed. A commercially available Coulter counter is used to determine cell density in the cell suspension, before culturing the cells in 95% air/5% $CO_2$ at 37°C.

In order to mimic the damage from ocular diseases associated with pathological abnormalities/changes in the tissues of the visual system, particularly associated with amyloid-beta-related pathological abnormalities/changes in the tissues of the visual system, such as, for example, neuronal degradation, and assess the preventive effect of the hC2 antibody, the cells are incubated with L-glutamate for three days in the presence or absence of the hC2 antibody. Cells cultured in buffer alone serve as control.

To determine RGC survival, at the end of the incubation period the cells are fixed with 3.7% formaldehyde in phosphate buffered saline (PBS) at room temperature for 30 minutes, rinsed three times in PBS and incubated for 1 hour in PBS containing RGC specific markers Thy1.1 or NF-L antibody. The antibody is then removed by washing and the cells are incubated for 30 minutes with the fluorescence-labeled secondary antibodies goat anti-mouse IgG, goat anti-rabbit IgG or rabbit anti-goat IgG. At the end of the incubation, the cells are washed, stained for 5 minutes with DAPI solution and rinsed. Surviving RGCs are counted by fluorescence microscopy and the number of cells present after incubation with the hC2 antibody are compared to control.

**Example 18 - Effect of the hC2 antibody on retinal ganglion cell (RGC) apoptosis in vivo**

To assess the in vivo capacity of the hC2 antibody to reduce retinal ganglion cell (RGC) death in individuals affected by ocular diseases associated with pathological abnormalities/changes in the tissues of the visual system, particularly associated with amyloid-beta-related pathological abnormalities/changes in the tissues of the visual system, such as, for example, neuronal degradation, rats and mice are used for a 2 to a 16 week long induced intra-ocular pressure (IOP) study. Retinal ganglion cell death is measured at the end of the study by both *in vivo* imaging and histological endpoint analysis.

In order to mimic the increase in intra-ocular pressure associated with certain ocular diseases associated with pathological abnormalities/changes in the tissues of the visual system, particularly associated with amyloid-beta-related pathological abnormalities/changes in the tissues of the visual system, such as, for example, neuronal degradation, glaucoma in particular, the animals are first anesthetized with intraperitoneal ketamine (75 mg/kg) and xylazine (5 mg/kg) and topical proparacaine 1% eye drops. Two alternative methods are then used to artificially elevate IOP in one eye (unilaterally) in rats and mice. In the first method, the anesthetized animals receive an injection of India ink into the anterior chamber followed by laser-induced photocoagulation of the dye in the trabecular meshwork with a 532-nm diode laser at the slit lamp perpendicular to the trabeculae and parallel to the iris. The animals receive an initial treatment of 40 to 50 spots of 50 $\mu$m size, 0.4 W, and 0.6 second duration. In the second method to artificially increase IOP, the anesthetized animals receive a 50 $\mu$l injection of hypertonic saline solution into the episcleral veins in one eye using a microneedle with a force just sufficient to blanch the vein.

To measure IOP, a commercially available handheld tonomer (TonoLab) is used. The measurements are taken while the animals are under anesthesia as the average of 12 readings immediately before laser treatment, 1, 4 and 7 days after treatment, and then weekly for the duration of the experiment. If, at an interval of one week, the difference in the IOP between the two eyes of the animals is less than 6 mm Hg, the animals are not further included in the study.

In order to evaluate the preventive effect of the hC2 antibody on RGC apoptosis, half of the animals receiving the IOP-inducing treatment receive an intravitreal or intravenous injection of the hC2 antibody at the time of IOP elevation. Half of the animals serve as control. The functional RGCs in the entire retina of eyes with IOP elevation are imaged and counted, and then compared to the number present in the contralateral eyes in the same animals. The difference in RGC number between the two eyes represents cells that have been lost as a result of IOP elevation in the surgical eye. Analysis of changes in this differential value assists in the identification of protective effects elicited by the hC2 antibody.

The number of RGCs is measured by histological endpoint analysis at 2, 4, 8 and 16 weeks after induced elevation of IOP. The retinas of the animals are fixed in 4% paraformaldehyde and stained in sections or whole mount using the RGC specific marker Brn3b. Multiple studies have demonstrated that loss of Brn3b staining correlates with loss of function in RGCs. To confirm the accuracy of histological RGC labeling, this method may be used in conjunction with backlabeling of the optic nerve from the SCN with DiASP or Fluorogold in a subset of animals to identify RCGs which maintain an intact, functional axon that has not lost connectivity with targets in the brain.

As a secondary endpoint, apoptosis of RGCs is also measured in a subset of eyes. Fluorescently labeled annexin V is used to label apoptotic cells by intravitreal injection of the protein one hour prior to sacrifice of the animal. Retinas are prepared as above and imaging of annexin V is conducted in conjunction with imaging of histological endpoints.

Reference List

Barghorn S, Nimmrich V, Striebinger A, Krantz C, Keller P, Janson B, Bahr M, Schmidt M, Bitner RS, Harlan J, Barlow E, Ebert U, Hillen H (2005) Globular amyloid beta-peptide oligomer - a homogenous and stable neuropathological protein in Alzheimer's disease. J Neurochem 95:834-847.
Blond and Goldberg, 1987, PNAS March 1, 1987 Vol. 84 | no. 5 | 1147-1151
Cox JPL, Tomlinson IM and Winter G. Eur. J. Immunol. 1994; 24: 827-836. A directory of human germ-line V$\kappa$

segments reveals a strong bias in their usage.

Kabat EA, Wu TT, Perry HM, Gottesman KS, Foeller C. Sequences of proteins of Immunological Interest, US Department of Health and Human Services, 1991.

Klein WL (2002) Abeta toxicity in Alzheimer's disease: globular soluble polymeric amyloid beta (ADDLs) as new vaccine and drug targets. Neurochem Int 41(5):345-352.

Langdon SD, Inaioki M, Kelsoe G. and Tedder TF. Immunogenetics 2000; 51: 241-245. Germline sequences of V(H)7183 gene family members in C57BL/6 mice demonstrate natural selection of particular sequences during recent evolution

Mulligan RC and Berg P. Science 1980; 209: 1422-1427. Expression of a bacterial gene in mammalian cells.

Riechmann L, Clark M, Waldmann H, Winter G, Nature 1988; 332: 323-327. Reshaping human antibodies for therapy.

Schable KF, Thiebe R, Bensch A, Brensing-Kueppers J, Heim V, Kirschbaum T, Lamm R, Ohnrich M, Pourrajabi S, Roschenthaler F, Schwendinger J, Wichelhaus D, Zocher I and Zachau HG. Eur. J. Immunol. 1999; 29: 2082-2086. Characteristics of the immunoglobulin V kappa genes, pseudogenes, relics and orphons in the mouse genome.

Tomlinson IM, Walter G, Marks JD, Llewelyn MB and Winter G. J. Mol. Biol. 1992; 227: 776-798. The repertoire of human germline VH sequences reveals about 50 groups of VH segments with different hypervariable loops

**Claims**

1.  A pharmaceutical composition for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis **characterized by** abnormal conjunctival vessels, keratoconjunctivitis sicca, papillary abnormalities, vitreous opacities and/or secondary glaucoma, and (iv) lattice dystrophy, comprising a therapeutically effective amount of a humanized antibody or functional part thereof, which recognizes and binds to at least one distinct site on beta-amyloid protein, wherein the humanized antibody or functional part thereof comprises: (a) a heavy chain CDR1 having the amino acid sequence of SEQ ID NO: 1; a heavy chain CDR2 having the amino acid sequence of SEQ ID NO: 2; a heavy chain CDR3 having the amino acid sequence of SEQ ID NO: 3; a light chain CDR1 having the amino acid sequence of SEQ ID NO: 4; a light chain CDR2 having the amino acid sequence of SEQ ID NO:5, the amino acid sequence RVSNRFS, or the amino acid sequence KVSSRFS; and a light chain CDR3 having the amino acid sequence of SEQ ID NO: 6, and (b) an IgG1 Fc region with reduced binding to the Fc.gamma.R having an amino acid modification at any one or more of amino acid positions 238, 239, 248, 249, 252, 254, 265, 268, 269, 270, 272, 278, 289, 292, 293, 294, 295, 296, 298, 301, 303, 322, 324, 327, 329, 333, 335, 338, 340, 373, 376, 382, 388, 389, 414, 416, 419, 434, 435, 437, 438 or 439 of the IgG1 Fc region.

2.  The pharmaceutical composition for use according to claim 1, wherein the humanized antibody or functional part thereof comprises a D265A mutation in the Fc region.

3.  The pharmaceutical composition for use according to claim 1 or 2 in the treatment of glaucoma.

4.  The pharmaceutical composition for use according to claim 3, wherein the glaucoma is selected from the group consisting of chronic open-angle glaucoma (COAG), acute angle closure glaucoma (AACG), mixed or combined mechanism glaucoma, normal tension glaucoma, congenital glaucoma, secondary glaucoma and exfoliative glaucoma.

5.   A humanized antibody or functional part thereof, which recognizes and binds to at least one distinct site on beta-amyloid protein and comprises: (a) a heavy chain CDR1 having the amino acid sequence of SEQ ID NO: 1; a heavy chain CDR2 having the amino acid sequence of SEQ ID NO: 2; a heavy chain CDR3 having the amino acid sequence of SEQ ID NO: 3; a light chain CDR1 having the amino acid sequence of SEQ ID NO: 4; a light chain CDR2 having the amino acid sequence of SEQ ID NO:5, the amino acid sequence RVSNRFS, or the amino acid sequence KVSSRFS; and a light chain CDR3 having the amino acid sequence of SEQ ID NO: 6 and (b) an IgG1 Fc region with reduced binding to the Fc.gamma.R having an amino acid modification at any one or more of amino acid positions 238, 239, 248, 249, 252, 254, 265, 268, 269, 270, 272, 278, 289, 292, 293, 294, 295, 296, 298, 301, 303, 322, 324, 327, 329, 333, 335, 338, 340, 373, 376, 382, 388, 389, 414, 416, 419, 434, 435, 437, 438 or 439 of the IgG1 Fc region, for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis **characterized by** abnormal conjunctival vessels, keratoconjunctivitis sicca, papillary abnormalities, vitreous opacities and/or secondary glaucoma, and (iv) lattice dystrophy.

6.   The humanized antibody or functional part thereof for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis **characterized by** abnormal conjunctival vessels, keratoconjunctivitis sicca, papillary abnormalities, vitreous opacities and/or secondary glaucoma, and (iv) lattice dystrophy according to claim 5, wherein the humanized antibody or functional part thereof comprises a D265A mutation in the Fc region.

7.   The humanized antibody or functional part thereof for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis **characterized by** abnormal conjunctival vessels, keratoconjunctivitis sicca, papillary abnormalities, vitreous opacities and/or secondary glaucoma, and (iv) lattice dystrophy according to claim 5 or 6 by reducing the plaque load or the amount of plaques in the retinal ganglion cell layer.

8.   The humanized antibody or functional part thereof for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis **characterized by** abnormal conjunctival vessels, keratoconjunctivitis sicca, papillary abnormalities, vitreous opacities and/or secondary glaucoma, and (iv) lattice dystrophy according to claim 5 or 6 by decreasing the total amount of soluble amyloid-$\beta$ in the retinal ganglion cell layer.

9.   The humanized antibody or functional part thereof for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis **characterized by** abnormal conjunctival vessels, keratoconjunctivitis sicca, papillary abnormalities, vitreous opacities and/or secondary glaucoma, and (iv) lattice dystrophy according to claim 5 or 6 by preventing, treating or alleviating the effects of glaucoma, optic neuritis, ocular amyloidosis and/or lattice dystrophy.

10.  The humanized antibody or functional part thereof for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis **characterized by** abnormal conjunctival vessels, keratoconjunctivitis sicca, papillary abnormalities, vitreous opacities and/or secondary glaucoma, and (iv) lattice dystrophy according to claim 5 or 6 by retaining or decreasing ocular pressure in the eyes of an animal suffering from glaucoma, optic neuritis, ocular amyloidosis and/or lattice dystrophy.

11.  The humanized antibody or functional part thereof for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis **characterized by** abnormal conjunctival vessels, keratoconjunctivitis sicca, papillary abnormalities, vitreous opacities and/or secondary glaucoma, and (iv) lattice dystrophy according to any one of claims 5 to 10, wherein the humanized antibody or functional part thereof comprises the light chain variable region of SEQ ID NO: 12 and the heavy chain variable region of SEQ ID NO: 15.

12.  The humanized antibody or functional part thereof for use according to any one of claims 5 to 11 in treatment of glaucoma.

13. The humanized antibody or functional part thereof for use in the treatment of glaucoma according to any one of claims 5 to 11, wherein the glaucoma is selected from the group consisting of chronic open-angle glaucoma (COAG), acute angle closure glaucoma (AACG), mixed or combined mechanism glaucoma, normal tension glaucoma, congenital glaucoma, secondary glaucoma and exfoliative glaucoma.

14. The pharmaceutical composition for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis **characterized by** abnormal conjunctival vessels, keratoconjunctivitis sicca, papillary abnormalities, vitreous opacities and/or secondary glaucoma, and (iv) lattice dystrophy according to any one of claims 1 to 4, wherein the humanized antibody or functional part thereof comprises the light chain variable region of SEQ ID NO: 12 and the heavy chain variable region of SEQ ID NO: 15.

15. The humanized antibody or functional part thereof, or the pharmaceutical composition for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis **characterized by** abnormal conjunctival vessels, keratoconjunctivitis sicca, papillary abnormalities, vitreous opacities and/or secondary glaucoma, and (iv) lattice dystrophy according to any one of claims 1 to 14, wherein the subject is a mammal.

16. The humanized antibody or functional part thereof, or the pharmaceutical composition for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis **characterized by** abnormal conjunctival vessels, keratoconjunctivitis sicca, papillary abnormalities, vitreous opacities and/or secondary glaucoma, and (iv) lattice dystrophy according to claim 15, wherein the mammal is a human.

17. Use of a humanized antibody or functional part thereof as defined in any one of claims 5 to 16, for the preparation of a medicament for use in the treatment of a subject suffering from an ocular disease associated with amyloid-beta-related pathological abnormalities/changes in the tissues of the visual system selected from (i) glaucoma, (ii) optic neuritis, (iii) ocular amyloidosis **characterized by** abnormal conjunctival vessels, keratoconjunctivitis sicca, papillary abnormalities, vitreous opacities and/or secondary glaucoma, and (iv) lattice dystrophy by

i) reducing the plaque load in the retinal ganglion cell layer of a subject suffering from glaucoma, optic neuritis, ocular amyloidosis or lattice dystrophy;
ii) reducing the amount of plaques in the retinal ganglion cell layer of a subject suffering from glaucoma, optic neuritis, ocular amyloidosis or lattice dystrophy;
iii) decreasing the total amount of soluble amyloid-$\beta$ in the retinal ganglion cell layer of a subject suffering from glaucoma, optic neuritis, ocular amyloidosis or lattice dystrophy;
iv) preventing, treating or alleviating the effects of glaucoma, optic neuritis, ocular amyloidosis or lattice dystrophy; or
v) retaining or decreasing ocular pressure in the eyes of a subject suffering from glaucoma, optic neuritis, ocular amyloidosis or lattice dystrophy,

wherein the ocular amyloidosis is **characterized by** abnormal conjunctival vessels, keratoconjunctivitis sicca, pupillary abnormalities, vitreous opacities and/or secondary glaucoma.

18. The use of claim 17, wherein the subject is a mammal.

19. The use of claim 18, wherein the subject is a human.

**Patentansprüche**

1. Pharmazeutische Zusammensetzung zur Verwendung bei der Behandlung eines Subjekts, das an einer Augenkrankheit leidet, die mit pathologischen Anomalien/Veränderungen in den Geweben des Sehsystems, welche mit Amyloid-beta in Zusammenhang stehen, assoziiert ist, ausgewählt aus (i) Glaukom, (ii) optischer Neuritis, (iii) okularer Amyloidose, die durch abnormale Bindehautgefäße, das Syndrom des trockenen Auges, papilläre Anomalien, Glaskörpertrübungen und/oder sekundäres Glaukom gekennzeichnet ist, sowie (iv) Gitterdystrophie, wobei sie eine therapeutisch wirksame Menge eines humanisierten Antikörpers oder eines funktionellen Teils davon umfasst, welcher mindestens eine bestimmte Stelle an einem beta-Amyloidprotein erkennt und an diese bindet, wobei der humanisierte Antikörper oder der funktionelle Teil davon Folgendes umfasst: (a) eine CDR1 der schweren Kette mit der Aminosäuresequenz der SEQ ID NO:1; eine CDR2 der schweren Kette mit der Aminosäuresequenz der SEQ ID NO:2; eine CDR3 der schweren Kette mit der Aminosäuresequenz der SEQ ID NO:3; eine CDR1 der leichten Kette mit der Aminosäuresequenz der SEQ ID NO:4; eine CDR2 der leichten Kette mit der Aminosäuresequenz der SEQ ID NO:5, die Aminosäuresequenz RVSNRFS, oder die Aminosäuresequenz KVSSRFS; und eine CDR3 der leichten Kette mit der Aminosäuresequenz der SEQ ID NO:6, sowie (b) eine Fc-Region von IgG1, die eine verringerte Bindung an Fc.gamma.R aufweist und an einer oder mehreren beliebigen der Aminosäurepositionen 238, 239, 248, 249, 252, 254, 265, 268, 269, 270, 272, 278, 289, 292, 293, 294, 295, 296, 298, 301, 303, 322, 324, 327, 329, 333, 335, 338, 340, 373, 376, 382, 388, 389, 414, 416, 419, 434, 435, 437, 438 oder 439 der Fc-Region des IgG1 eine Aminosäuremodifikation aufweist.

2. Pharmazeutische Zusammensetzung zur Verwendung nach Anspruch 1, wobei der humanisierte Antikörper oder der funktionelle Teil davon eine D265A-Mutation in der Fc-Region aufweist.

3. Pharmazeutische Zusammensetzung zur Verwendung nach Anspruch 1 oder 2 bei der Behandlung von Glaukom.

4. Pharmazeutische Zusammensetzung zur Verwendung nach Anspruch 3, wobei das Glaukom aus der Gruppe ausgewählt ist, die aus chronischem Offenwinkelglaukom (Chronic Open-Angle Glaucoma/COAG), akutem Engwinkelglaukom (Acute Angle Closure Glaucoma/AACG), Glaukom mit gemischtem oder kombiniertem Mechanismus, Normaldruckglaukom, angeborenem Glaukom, sekundärem Glaukom und Exfoliationsglaukom besteht.

5. Humanisierter Antikörper oder funktioneller Teil davon, welcher mindestens eine bestimmte Stelle an einem beta-Amyloidprotein erkennt und an diese bindet und Folgendes umfasst: (a) eine CDR1 der schweren Kette mit der Aminosäuresequenz der SEQ ID NO:1; eine CDR2 der schweren Kette mit der Aminosäuresequenz der SEQ ID NO:2; eine CDR3 der schweren Kette mit der Aminosäuresequenz der SEQ ID NO:3; eine CDR1 der leichten Kette mit der Aminosäuresequenz der SEQ-ID No.: 4; eine CDR2 der leichten Kette mit der Aminosäuresequenz der SEQ ID NO:5, die Aminosäuresequenz RVSNRFS, oder die Aminosäuresequenz KVSSRFS; und eine CDR3 der leichten Kette mit der Aminosäuresequenz der SEQ ID NO:6 sowie (b) eine Fc-Region von IgG1, die eine verringerte Bindung an Fc. gamma. R aufweist und an einer oder mehreren beliebigen der Aminosäurepositionen 238, 239, 248, 249, 252, 254, 265, 268, 269, 270, 272, 278, 289, 292, 293, 294, 295, 296, 298, 301, 303, 322, 324, 327, 329, 333, 335, 338, 340, 373, 376, 382, 388, 389, 414, 416, 419, 434, 435, 437, 438 oder 439 der Fc-Region des IgG1 eine Aminosäuremodifikation aufweist, zur Verwendung bei der Behandlung eines Subjekts, das an einer Augenkrankheit leidet, die mit pathologischen Anomalien/Veränderungen in den Geweben des Sehsystems, welche mit Amyloid-beta in Zusammenhang stehen, assoziiert ist, ausgewählt aus (i) Glaukom, (ii) optischer Neuritis, (iii) okularer Amyloidose, die durch abnormale Bindehautgefäße, das Syndrom des trockenen Auges, papilläre Anomalien, Glaskörpertrübungen und/oder sekundäres Glaukom gekennzeichnet ist, sowie (iv) Gitterdystrophie.

6. Humanisierter Antikörper oder funktioneller Teil davon, zur Verwendung bei der Behandlung eines Subjekts, das an einer Augenkrankheit leidet, die mit pathologischen Anomalien/Veränderungen in den Geweben des Sehsystems, welche mit Amyloid-beta in Zusammenhang stehen, assoziiert ist, ausgewählt aus (i) Glaukom, (ii) optischer Neuritis, (iii) okularer Amyloidose, die durch abnormale Bindehautgefäße, das Syndrom des trockenen Auges, papilläre Anomalien, Glaskörpertrübungen und/oder sekundäres Glaukom gekennzeichnet ist, sowie (iv) Gitterdystrophie, nach Anspruch 5, wobei der humanisierte Antikörper oder der funktionelle Teil davon eine D265A-Mutation in der Fc-Region umfasst.

7. Humanisierter Antikörper oder funktioneller Teil davon, zur Verwendung bei der Behandlung eines Subjekts, das an einer Augenkrankheit leidet, die mit pathologischen Anomalien/Veränderungen in den Geweben des Sehsystems, welche mit Amyloid-beta in Zusammenhang stehen, assoziiert ist, ausgewählt aus (i) Glaukom, (ii) optischer Neuritis, (iii) okularer Amyloidose, die durch abnormale Bindehautgefäße, das Syndrom des trockenen Auges, papilläre Anomalien, Glaskörpertrübungen und/oder sekundäres Glaukom gekennzeichnet ist, sowie (iv) Gitterdystrophie, nach Anspruch 5 oder 6, durch Verringern der Plaquebelastung oder der Menge an Plaques in der Ganglienzellschicht der Netzhaut.

8. Humanisierter Antikörper oder funktioneller Teil davon, zur Verwendung bei der Behandlung eines Subjekts, das an einer Augenkrankheit leidet, die mit pathologischen Anomalien/Veränderungen in den Geweben des Sehsystems, welche mit Amyloid-beta in Zusammenhang stehen, assoziiert ist, ausgewählt aus (i) Glaukom, (ii) optischer Neuritis, (iii) okularer Amyloidose, die durch abnormale Bindehautgefäße, das Syndrom des trockenen Auges, papilläre Anomalien, Glaskörpertrübungen und/oder sekundäres Glaukom gekennzeichnet ist, sowie (iv) Gitterdystrophie, nach Anspruch 5 oder 6, durch Senken der Gesamtmenge an löslichem Amyloid-β in der Ganglienzellschicht der Netzhaut.

9. Humanisierter Antikörper oder funktioneller Teil davon, zur Verwendung bei der Behandlung eines Subjekts, das an einer Augenkrankheit leidet, die mit pathologischen Anomalien/Veränderungen in den Geweben des Sehsystems, welche mit Amyloid-beta in Zusammenhang stehen, assoziiert ist, ausgewählt aus (i) Glaukom, (ii) optischer Neuritis, (iii) okularer Amyloidose, die durch abnormale Bindehautgefäße, das Syndrom des trockenen Auges, papilläre Anomalien, Glaskörpertrübungen und/oder sekundäres Glaukom gekennzeichnet ist, sowie (iv) Gitterdystrophie, nach Anspruch 5 oder 6, durch Verhüten, Behandeln oder Lindern der Auswirkungen von Glaukom, optischer Neuritis, okularer Amyloidose und/oder Gitterdystrophie.

10. Humanisierter Antikörper oder funktioneller Teil davon, zur Verwendung bei der Behandlung eines Subjekts, das an einer Augenkrankheit leidet, die mit pathologischen Anomalien/Veränderungen in den Geweben des Sehsystems, welche mit Amyloid-beta in Zusammenhang stehen, assoziiert ist, ausgewählt aus (i) Glaukom, (ii) optischer Neuritis, (iii) okularer Amyloidose, die durch abnormale Bindehautgefäße, das Syndrom des trockenen Auges, papilläre Anomalien, Glaskörpertrübungen und/oder sekundäres Glaukom gekennzeichnet ist, sowie (iv) Gitterdystrophie, nach Anspruch 5 oder 6, durch Aufrechterhalten oder Verringern des Augendrucks in den Augen eines Tieres, welches an Glaukom, optischer Neuritis, okularer Amyloidose und/oder Gitterdystrophie leidet.

11. Humanisierter Antikörper oder funktioneller Teil davon, zur Verwendung bei der Behandlung eines Subjekts, das an einer Augenkrankheit leidet, die mit pathologischen Anomalien/Veränderungen in den Geweben des Sehsystems, welche mit Amyloid-beta in Zusammenhang stehen, assoziiert ist, ausgewählt aus (i) Glaukom, (ii) optischer Neuritis, (iii) okularer Amyloidose, die durch abnormale Bindehautgefäße, das Syndrom des trockenen Auges, papilläre Anomalien, Glaskörpertrübungen und/oder sekundäres Glaukom gekennzeichnet ist, sowie (iv) Gitterdystrophie, nach einem beliebigen der Ansprüche 5 bis 10, wobei der humanisierte Antikörper oder der funktionelle Teil davon die variable Region der leichten Kette der SEQ ID NO:12 und die variable Region der schweren Kette der SEQ ID NO:15 umfasst.

12. Humanisierter Antikörper oder funktioneller Teil davon zur Verwendung nach einem beliebigen der Ansprüche 5 bis 11 bei der Behandlung von Glaukom.

13. Humanisierter Antikörper oder funktioneller Teil davon zur Verwendung bei der Behandlung von Glaukom nach einem beliebigen der Ansprüche 5 bis 11, wobei das Glaukom aus der Gruppe ausgewählt ist, die aus chronischem Offenwinkelglaukom (Chronic Open-Angle Glaucoma/COAG), akutem Engwinkelglaukom (Acute Angle Closure Glaucoma/AACG), Glaukom mit gemischtem oder kombiniertem Mechanismus, Normaldruckglaukom, angeborenem Glaukom, sekundärem Glaukom und Exfoliationsglaukom besteht.

14. Pharmazeutische Zusammensetzung zur Verwendung bei der Behandlung eines Subjekts, das an einer Augenkrankheit leidet, die mit pathologischen Anomalien/Veränderungen in den Geweben des Sehsystems, welche mit Amyloid-beta in Zusammenhang stehen, assoziiert ist, ausgewählt aus (i) Glaukom, (ii) optischer Neuritis, (iii) okularer Amyloidose, die durch abnormale Bindehautgefäße, das Syndrom des trockenen Auges, papilläre Anomalien, Glaskörpertrübungen und/oder sekundäres Glaukom gekennzeichnet ist, sowie (iv) Gitterdystrophie, nach einem beliebigen der Ansprüche 1 bis 4, wobei der humanisierte Antikörper oder der funktionelle Teil davon die variable Region der leichten Kette der SEQ ID NO:12 und die variable Region der schweren Kette der SEQ ID NO:15 umfasst.

15. Humanisierter Antikörper oder funktioneller Teil davon oder pharmazeutische Zusammensetzung, zur Verwendung bei der Behandlung eines Subjekts, das an einer Augenkrankheit leidet, die mit pathologischen Anomalien/Veränderungen in den Geweben des Sehsystems, welche mit Amyloid-beta in Zusammenhang stehen, assoziiert ist, ausgewählt aus (i) Glaukom, (ii) optischer Neuritis, (iii) okularer Amyloidose, die durch abnormale Bindehautgefäße, das Syndrom des trockenen Auges, papilläre Anomalien, Glaskörpertrübungen und/oder sekundäres Glaukom gekennzeichnet ist, sowie (iv) Gitterdystrophie, nach einem beliebigen der Ansprüche 1 bis 14, wobei das Subjekt ein Säugetier ist.

16. Humanisierter Antikörper oder funktioneller Teil davon oder pharmazeutische Zusammensetzung, zur Verwendung bei der Behandlung eines Subjekts, das an einer Augenkrankheit leidet, die mit pathologischen Anomalien/Veränderungen in den Geweben des Sehsystems, welche mit Amyloid-beta in Zusammenhang stehen, assoziiert ist, ausgewählt aus (i) Glaukom, (ii) optischer Neuritis, (iii) okularer Amyloidose, die durch abnormale Bindehautgefäße, das Syndrom des trockenen Auges, papilläre Anomalien, Glaskörpertrübungen und/oder sekundäres Glaukom gekennzeichnet ist, sowie (iv) Gitterdystrophie, nach Anspruch 15, wobei das Säugetier ein Mensch ist.

17. Verwendung eines humanisierten Antikörpers oder eines funktionellen Teils davon wie in einem beliebigen der Ansprüche 5 bis 16 definiert, zur Herstellung eines Arzneimittels zur Verwendung bei der Behandlung eines Subjekts, das unter einer Augenkrankheit leidet, die mit pathologischen Anomalien/Veränderungen in den Geweben des Sehsystems, welche mit Amyloid-beta in Zusammenhang stehen, assoziiert ist, ausgewählt aus (i) Glaukom, (ii) optischer Neuritis, (iii) okularer Amyloidose, die durch abnormale Bindehautgefäße, das Syndrom des trockenen Auges, papilläre Anomalien, Glaskörpertrübungen und/oder sekundäres Glaukom gekennzeichnet ist, sowie (iv) Gitterdystrophie, durch

i) Verringern der Plaquebelastung in der Ganglienzellenschicht der Netzhaut eines Subjekts, welches an Glaukom, optischer Neuritis, okularer Amyloidose oder Gitterdystrophie leidet;
ii) Verringern der Menge an Plaques in der Ganglienzellenschicht der Netzhaut eines Subjekts, welches an Glaukom, optischer Neuritis, okularer Amyloidose oder Gitterdystrophie leidet;
iii) Senken der Gesamtmenge an löslichem Amyloid-β in der Ganglienzellenschicht der Netzhaut eines Subjekts, welches an Glaukom, optischer Neuritis, okularer Amyloidose oder Gitterdystrophie leidet ;
iv) Verhüten, Behandeln oder Lindern der Auswirkungen von Glaukom, optischer Neuritis, okularer Amyloidose oder Gitterdystrophie ; oder
v) Aufrechterhalten oder Senken des Augendrucks in den Augen eines Subjekts, das an Glaukom, optischer Neuritis, okularer Amyloidose oder Gitterdystrophie leidet ,

wobei die okulare Amyloidose durch abnormale Bindehautgefäße, das Syndrom des trockenen Auges, Anomalien der Pupille, Glaskörpertrübungen und/oder sekundäres Glaukom gekennzeichnet ist.

18. Verwendung nach Anspruch 17, wobei das Subjekt ein Säugetier ist.

19. Verwendung nach Anspruch 18, wobei das Subjekt ein Mensch ist.

**Revendications**

1. Composition pharmaceutique pour utilisation dans le traitement d'un sujet souffrant d'une affection oculaire, associée à des anomalies/modifications pathologiques liées à la protéine bêta-amyloïde dans les tissus du système visuel et choisie parmi (i) le glaucome, (ii) la névrite optique, (iii) l'amyloïdose oculaire **caractérisée par** des vaisseaux conjonctivaux anormaux, une kératoconjonctivite sèche, des anomalies papillaires, des opacités du corps vitré et/ou un glaucome secondaire, et (iv) une dystrophie grillagée, comprenant une quantité thérapeutiquement efficace d'un anticorps humanisé ou d'une partie fonctionnelle de celui-ci, qui reconnaît et se lie à au moins un site distinct sur la protéine bêta-amyloïde, dans laquelle l'anticorps humanisé ou la partie fonctionnelle de celui-ci comprend : (a) une chaîne lourde CDR1 ayant la séquence d'acides aminés SEQ ID NO : 1 ; une chaîne lourde CDR2 ayant la séquence d'acides aminés SEQ ID NO : 2 ; une chaîne lourde CDR3 ayant la séquence d'acides aminés SEQ ID NO : 3 ; une chaîne légère CDR1 ayant la séquence d'acides aminés SEQ ID NO : 4 ; une chaîne légère CDR2 ayant la séquence d'acides aminés SEQ ID NO : 5, la séquence d'acides aminés RVSNRFS ou la séquence d'acides aminés KVSSRFS ; et une chaîne légère CDR3 ayant la séquence d'acides aminés SEQ ID NO : 6, et (b) une région Fc d'IgG1 présentant une liaison réduite à Fc gamma R et présentant une modification d'acide aminé au niveau de l'une quelconque ou de plusieurs des positions d'acides aminés suivantes : 238, 239, 248, 249, 252, 254, 265, 268, 269, 270, 272, 278, 289, 292, 293, 294, 295, 296, 298, 301, 303, 322, 324, 327, 329, 333, 335, 338, 340, 373, 376, 382, 388, 389, 414, 416, 419, 434, 435, 437, 438 ou 439 de la région Fc d'IgG1.

2. Composition pharmaceutique pour utilisation selon la revendication 1, dans laquelle l'anticorps humanisé ou la partie fonctionnelle de celui-ci comprend une mutation D265A dans la région Fc.

3. Composition pharmaceutique pour utilisation selon la revendication 1 ou 2 dans le traitement du glaucome.

4. Composition pharmaceutique pour utilisation selon la revendication 3, le glaucome étant choisi dans le groupe constitué du glaucome chronique à angle ouvert (GCAO), du glaucome aigu à angle fermé (GAAF), du glaucome à mécanisme mixte ou combiné, du glaucome à pression normale, du glaucome congénital, du glaucome secondaire et du glaucome exfoliatif.

**5.** Anticorps humanisé ou partie fonctionnelle de celui-ci, qui reconnaît et se lie à au moins un site distinct sur la protéine bêta-amyloïde et comprend : (a) une chaîne lourde CDR1 ayant la séquence d'acides aminés SEQ ID NO : 1 ; une chaîne lourde CDR2 ayant la séquence d'acides aminés SEQ ID NO : 2 ; une chaîne lourde CDR3 ayant la séquence d'acides aminés SEQ ID NO : 3 ; une chaîne légère CDR1 ayant la séquence d'acides aminés SEQ ID NO : 4 ; une chaîne légère CDR2 ayant la séquence d'acides aminés SEQ ID NO : 5, la séquence d'acides aminés RVSNRFS ou la séquence d'acides aminés KVSSRFS ; et une chaîne légère CDR3 ayant la séquence d'acides aminés SEQ ID NO : 6, et (b) une région Fc d'IgG1 présentant une liaison réduite à FC gamma R et présentant une modification d'acide aminé au niveau de l'une quelconque ou de plusieurs des positions d'acides aminés suivantes : 238, 239, 248, 249, 252, 254, 265, 268, 269, 270, 272, 278, 289, 292, 293, 294, 295, 296, 298, 301, 303, 322, 324, 327, 329, 333, 335, 338, 340, 373, 376, 382, 388, 389, 414, 416, 419, 434, 435, 437, 438 ou 439 de la région Fc d'IgG1, pour utilisation dans le traitement d'un sujet souffrant d'une affection oculaire, associée à des anomalies/modifications pathologiques liées à la protéine bêta-amyloïde dans les tissus du système visuel et choisie parmi (i) le glaucome, (ii) la névrite optique, (iii) l'amyloïdose oculaire **caractérisée par** des vaisseaux conjonctivaux anormaux, une kératoconjonctivite sèche, des anomalies papillaires, des opacités du corps vitré et/ou un glaucome secondaire, et (iv) une dystrophie grillagée.

**6.** Anticorps humanisé ou partie fonctionnelle de celui-ci pour utilisation dans le traitement d'un sujet souffrant d'une affection oculaire, associée à des anomalies/modifications pathologiques liées à la protéine bêta-amyloïde dans les tissus du système visuel et choisie parmi (i) le glaucome, (ii) la névrite optique, (iii) l'amylaïdose oculaire **caractérisée par** des vaisseaux conjonctivaux anormaux, une kératoconjonctivite sèche, des anomalies papillaires, des opacités du corps vitré et/ou un glaucome secondaire, et (iv) une dystrophie grillagée, selon la revendication 5, l'anticorps humanisé ou la partie fonctionnelle de celui-ci comprenant une mutation D265A dans la région Fc.

**7.** Anticorps humanisé ou partie fonctionnelle de celui-ci pour utilisation dans le traitement d'un sujet souffrant d'une affection oculaire, associée à des anomalies/modifications pathologiques liées à la protéine bêta-amyloïde dans les tissus du système visuel et choisie parmi (i) le glaucome, (ii) la névrite optique, (iii) l'amyloïdose oculaire **caractérisée par** des vaisseaux conjonctivaux anormaux, une kératoconjonctivite sèche, des anomalies papillaires, des opacités du corps vitré et/ou un glaucome secondaire, et (iv) une dystrophie grillagée, selon la revendication 5 ou 6, par réduction de la charge ou de la quantité de plaques dans la couchede cellules ganglionnaires de la rétine.

**8.** Anticorps humanisé ou partie fonctionnelle de celui-ci pour utilisation dans le traitement d'un sujet souffrant d'une affection oculaire, associée à des anomalies/modifications pathologiques liées à la protéine bêta-amyloïde dans les tissus du système visuel et choisie parmi (i) le glaucome, (ii) la névrite optique, (iii) l'amyloïdose oculaire **caractérisée par** des vaisseaux conjonctivaux anormaux, une kératoconjonctivite sèche, des anomalies papillaires, des opacités du corps vitré et/ou un glaucome secondaire, et (iv) une dystrophie grillagée, selon la revendication 5 ou 6, par réduction de la quantité totale de protéine β-amyloïde soluble dans la couche de cellules ganglionnaires de la rétine.

**9.** Anticorps humanisé ou partie fonctionnelle de celui-ci pour utilisation dans le traitement d'un sujet souffrant d'une affection oculaire, associée à des anomalies/modifications pathologiques liées à la protéine bêta-amyloïde dans les tissus du système visuel et choisie parmi (i) le glaucome, (ii) la névrite optique, (iii) l'amyloïdose oculaire **caractérisée par** des vaisseaux conjonctivaux anormaux, une kératoconjonctivite sèche, des anomalies papillaires, des opacités du corps vitré et/ou un glaucome secondaire, et (iv) une dystrophie grillagée, selon la revendication 5 ou 6, par prétention, traitement ou atténuation des effets du glaucome, de la névrite optique, de l'amyloïdose oculaire et/ou de la dystrophie grillagée.

**10.** Anticorps humanisé ou partie fonctionnelle de celui-ci pour utilisation dans le traitement d'un sujet souffrant d'une affection oculaire, associée à des anomalies/modifications pathologiques liées à la protéine bêta-amyloïde dans les tissus du système visuel et choisie parmi (i) le glaucome, (ii) la névrite optique, (iii) l'amyloïdose oculaire **caractérisée par** des vaisseaux conjonctivaux anormaux, une kératoconjonctivite sèche, des anomalies papillaires, des opacités du corps vitré et/ou un glaucome secondaire, et (iv) une dystrophie grillagée, selon la revendication 5 ou 6, par maintien ou réduction de la tension oculaire au niveau des yeux d'un animal souffrant d'un glaucome, d'une névrite optique, d'une amyloïdose oculaire et/ou d'une dystrophie grillagée.

**11.** Anticorps humanisé ou partie fonctionnelle de celui-ci pour utilisation dans le traitement d'un sujet souffrant d'une affection oculaire, associée à des anomalies/modifications pathologiques liées à la protéine bêta-amyloïde dans les tissus du système visuel et choisie parmi (i) le glaucome, (ii) la névrite optique, (iii) l'amyloïdose oculaire **caractérisée par** des vaisseaux conjonctivaux anormaux, une kératoconjonctivite sèche, des anomalies papillaires, des opacités du corps vitré et/ou un glaucome secondaire, et (iv) une dystrophie grillagée, selon l'une quelconque des revendications 5 à 10, l'anticorps humanisé ou la partie fonctionnelle de celui-ci comprenant la région variable de la chaîne légère de SEQ ID NO : 12 et la région variable de la chaîne lourde de SEQ ID NO : 15.

12. Anticorps humanisé ou partie fonctionnelle de celui-ci pour utilisation selon l'une quelconque des revendications 5 à 11 dans le traitement du glaucome.

13. Anticorps humanisé ou partie fonctionnelle de celui-ci pour utilisation dans le traitement du glaucome selon l'une quelconque des revendications 5 à 11, le glaucome étant choisi dans le groupe constitué du glaucome chronique à angle ouvert (GCAO), du glaucome aigu à angle fermé (GAAF), du glaucome à mécanisme mixte ou combiné, du glaucome à pression normale, du glaucome congénital, du glaucome secondaire et du glaucome exfoliatif.

14. Composition pharmaceutique pour utilisation dans le traitement d'un sujet souffrant d'une affection oculaire, associée à des anomalies/modifications pathologiques liées à la protéine bêta-amyloïde dans les tissus du système visuel et choisie parmi (i) le glaucome, (ii) la névrite optique, (iii) l'amyloïdose oculaire **caractérisée par** des vaisseaux conjonctivaux anormaux, une kératoconjonctivite sèche, des anomalies papillaires, des opacités du corps vitré et/ou un glaucome secondaire, et (iv) une dystrophie grillagée, selon l'une quelconque des revendications 1 à 4, dans laquelle l'anticorps humanisé ou la partie fonctionnelle de celui-ci comprend la région variable de la chaîne légère de SEQ ID NO : 12 et la région variable de la chaîne lourde de SEQ ID NO : 15.

15. Anticorps humanisé ou partie fonctionnelle de celui-ci, ou composition pharmaceutique pour utilisation dans le traitement d'un sujet souffrant d'une affection oculaire, associée à des anomalies/modifications pathologiques liées à la protéine bêta-amyloïde dans les tissus du système visuel et choisie parmi (i) le glaucome, (ii) la névrite optique, (iii) l'amyloïdose oculaire **caractérisée par** des vaisseaux conjonctivaux anormaux, une kératoconjonctivite sèche, des anomalies papillaires, des opacités du corps vitré et/ou un glaucome secondaire, et (iv) une dystrophie grillagée, selon l'une quelconque des revendications 1 à 14, le sujet étant un mammifère.

16. Anticorps humanisé ou partie fonctionnelle de celui-ci, ou composition pharmaceutique pour utilisation dans le traitement d'un sujet souffrant d'une affection oculaire, associée à des anomalies/modifications pathologiques liées à la protéine bêta-amyloïde dans les tissus du système visuel et choisie parmi (i) le glaucome, (ii) la névrite optique, (iii) l'amyloïdose oculaire **caractérisée par** des vaisseaux conjonctivaux anormaux, une kératoconjonctivite sèche, des anomalies papillaires, des opacités du corps vitré et/ou un glaucome secondaire, et (iv) une dystrophie grillagée, selon la revendication 15, le mammifère étant un être humain.

17. Utilisation d'un anticorps humanisé ou d'une partie fonctionnelle de celui-ci comme défini dans l'une quelconque des revendications 5 à 16, pour la préparation d'un médicament pour utilisation dans le traitement d'un sujet souffrant d'une affection oculaire, associée à des anomalies/modifications pathologiques liées à la protéine bêta-amyloïde dans les tissus du système visuel et choisie parmi (i) le glaucome, (ii) la névrite optique, (iii) l'amyloïdose oculaire **caractérisée par** des vaisseaux conjonctivaux anormaux, une kératoconjonctivite sèche, des anomalies papillaires, des opacités du corps vitré et/ou un glaucome secondaire, et (iv) une dystrophie grillagée, par

i) réduction de la charge de plaques dans la couche de cellules ganglionnaires de la rétine d'un sujet souffrant d'un glaucome, d'une névrite optique, d'une amyloïdose oculaire ou d'une dystrophie grillagée ;
ii) réduction de la quantité de plaques dans la couche de cellules ganglionnaires de la rétine d'un sujet souffrant d'un glaucome, d'une névrite optique, d'une amyloïdose oculaire ou d'une dystrophie grillagée ;
iii) réduction de la quantité totale de protéine β-amyloïde soluble dans la couche de cellules ganglionnaires de la rétine d'un sujet souffrant d'un glaucome, d'une névrite optique, d'une amyloïdose oculaire ou d'une dystrophie grillagée ;
iv) prévention, traitement ou atténuation des effets du glaucome, de la névrite optique, de l'amyloïdose oculaire ou de la dystrophie grillagée ; ou
v) maintien ou réduction de la tension oculaire dans les yeux d'un sujet souffrant d'un glaucome, d'une névrite optique, d'une amyloïdose oculaire ou d'une dystrophie grillagée,

dans laquelle l'amyloïdose oculaire est **caractérisée par** des vaisseaux conjonctivaux anormaux, une kératoconjonctivite sèche, des anomalies papillaires, des opacités du corps vitré et/ou un glaucome secondaire.

18. Utilisation selon la revendication 17, dans laquelle le sujet est un mammifère.

**19.** Utilisation selon la revendication 18, dans laquelle le sujet est un être humain.

**FIG 1**

**FIG 2**

HindIII
|
AAGCTTATGAATATGCAAATCCTCTGAATCTACATGGTAAATATAGGTTTGTCTATACCACAAACAGAAAAACATGAGATCACAGTTCTCTCTACAGTTA
----+----+----+----+----+----+----+----+----+----+----+----+----+----+----+----+----+----+----+----+ 10
TTCGAATACTTATACGTTTAGGAGACTTAGATGTACCATTTATATCCAAACAGATATGGTGTTTGTCTTTTTGTACTCTAGTGTCAAGAGAGATGTCAAT

NcoI
|
CTGAGCACACAGGACCTCACCATGGGATGGACCTGTATCATCCTCTTCTTGGTAGCAACAGCTACAGGTAAGGGGCTCACAGTAGCAGGCTTGAGGTCTG
----+----+----+----+----+----+----+----+----+----+----+----+----+----+----+----+----+----+----+----+ 20
GACTCGTGTGTCCTGGAGTGGTACCCTACCTCGACATAGTAGGAGAAGAACCATCGTTGTCGATGTCCATTCCCCGAGTGTCATCGTCCGAACTCCAGAC
             M   G   W   S   C   I   I   L   F   L   V   A   T   A   T
             └─────────────────────────── SIGNAL ───────────────────────────┘

GACATATATATGGGTGACAATGACATCCACTTTGCCTTTCTCTCCACAGGTGTCCACTCCGATGTTGTGATGACCCAAACTCCACTCTCCCTGCCTGTCA
----+----+----+----+----+----+----+----+----+----+----+----+----+----+----+----+----+----+----+----+ 30
CTGTATATATACCCACTGTTACTGTAGGTGAAACGGAAAGAGAGGTGTCCACAGGTGAGGCTACAACACTACTGGGTTTGAGGTGAGAGGGACGGACAGT
                                            G   V   H   S   D   V   V   M   T   Q   T   P   L   S   L   P   V
                                            └──── SIG ────┘ ──────────── C2 VK ──────────────

BglII                                                                                      KpnI  PstI
|                                                                                          |     |
GTCTTGGAGATCAAGCCTCCATCTCTTGCAGATCTAGTCAGAGCCTTGTATATAGTAATGGAGACACCTATTTACATTGGTACCTGCAGAAGCCAGGCCA
----+----+----+----+----+----+----+----+----+----+----+----+----+----+----+----+----+----+----+----+ 40
CAGAACCTCTAGTTCGGAGGTAGAGAACGTCTAGATCAGTCTCGGAACATATATCATTACCTCTGTGGATAAATGTAACCATGGACGTCTTCGGTCCGGT
S   L   G   D   Q   A   S   I   S   C   R   S   S   Q   S   L   V   Y   S   N   G   D   T   Y   L   H   W   Y   L   Q   K   P   G   Q
──────────────────────────────────────────────── C2 VK ────────────────────────────────────────────────

             ┌──────────────────────────────────────────────────────────────────────┐
             │                                                                         ╲
             │                                                                          ──►
             └──────────────────────────────── CDR1 ──────────────────────────────────┘

*FIG. 3-1*

```
GTCTCCAAAGCTCCTGATCTACAAAGTTTCCAACCGATTTTCTGGGGTCCCAGACAGGTTCAGTGGCAGTGGATCAGGGACAGATTTCACACTCAAGATC
----+----+----+----+----+----+----+----+----+----+----+----+----+----+----+----+----+----+----+----+  50
CAGAGGTTTCGAGGACTAGATGTTTCAAAGGTTGGCTAAAAGACCCCAGGGTCTGTCCAAGTCACCGTCACCTAGTCCCTGTCTAAAGTGTGAGTTCTAG
   S  P  K  L  L  I  Y  K  V  S  N  R  F  S  G  V  P  D  R  F  S  G  S  G  S  G  T  D  F  T  L  K  I
───────────────────────────────────────────────C2 VK───────────────────────────────────────────────
```
```
AGCAGAGTGGAGGCTGAGGATCTGGGAGTTTATTTCTGCTCTCAAAGTACACATGTTCCTTGGACGTTCGGCGGAGGCACCAAGCTGGAAATCAAACGTG
----+----+----+----+----+----+----+----+----+----+----+----+----+----+----+----+----+----+----+----+  60
TCGTCTCACCTCCGACTCCTAGACCCTCAAATAAAGACGAGAGTTTCATGTGTACAAGGAACCTGCAAGCCGCCTCCGTGGTTCGACCTTTAGTTTGCAC
   S  R  V  E  A  E  D  L  G  V  Y  F  C  S  Q  S  T  H  V  P  W  T  F  G  G  G  T  K  L  E  I  K
────────────────────────────────────────────────C2 VK───────────────────────────────────────────────
```
```
                               BamHI
                                 I
AGTAGAATTTAAACTTTGCTTCCTCAGTTGGATCC
----+----+----+----+----+----+----+  635
TCATCTTAAATTTGAAACGAAGGAGTCAACCTAGG
```

**FIG. 3-2**

HindIII
|

AAGCTTATGAATATGCAAATCCTCTGAATCTACATGGTAAATATAGGTTTGTCTATACCACAAACAGAAAAACATGAGATCACAGTTCTCTCTACAGTTA

----+----+----+----+----+----+----+----+----+----+----+----+----+----+----+----+----+----+----+----+  10

TTCGAATACTTATACGTTTAGGAGACTTAGATGTACCATTTATATCCAAACAGATATGGTGTTTGTCTTTTTGTACTCTAGTGTCAAGAGAGATGTCAAT

Ncol
|

CTGAGCACACAGGACCTCACCATGGGATGGAGCTGTATCATCCTCTTCTTGGTAGCAACAGCTACAGGTAAGGGGCTCACAGTAGCAGGCTTGAGGTCTG

----+----+----+----+----+----+----+----+----+----+----+----+----+----+----+----+----+----+----+----+  20

GACTCGTGTGTCCTGGAGTGGTACCCTACCTCGACATAGTAGGAGAAGAACCATCGTTGTCGATGTCCATTCCCCGAGTGTCATCGTCCGAACTCCAGAC

                 M  G  W  S  C  I  I  L  F  L  V  A  T  A  T
                 |_____ SIGNAL _____|

                                                           Pvull                    Bsgl
                                                           |                        |

GACATATATATGGGTGACAATGACATCCACTTTGCCTTTCTCTCCACAGGTGTCCACTCCGAGGTGCAGCTGGTCGAGTCTGGGGGAGGCTTAGTGCAGC

----+----+----+----+----+----+----+----+----+----+----+----+----+----+----+----+----+----+----+----+  30

CTGTATATATACCCACTGTTACTGTAGGTGAAACGGAAAGAGAGGTGTCCACAGGTGAGGCTCCACGTCGACCAGCTCAGACCCCCTCCGAATCACGTCG

                                                  G  V  H  S  E  V  Q  L  V  E  S  G  G  G  L  V  Q
                                                  |___ SIG ___|_____ C2VHAF _____

                    Bsgl                                          Bsgl
                    |                                             |

CTGGAGGGTCCCTGAAACTCTCCTGTGCAGCCTCTGGATTCACTTTCAGTAGCTATGGCATGTCTTGGGTTCGCCAGACTCCAGACAAGAGGCTGGAATT

----+----+----+----+----+----+----+----+----+----+----+----+----+----+----+----+----+----+----+----+  40

GACCTCCCAGGGACTTTGAGAGGACACGTCGGAGACCTAAGTGAAAGTCATCGATACCGTACAGAACCCAAGCGGTCTGAGGTCTGTTCTCCGACCTTAA

 P  G  G  S  L  K  L  S  C  A  A  S  G  F  T  F  S  S  Y  G  M  S  W  V  R  Q  T  P  D  K  R  L  E  L
_____ C2VHAF _____
                    |_____ CDR1 _____|

*FIG. 4-1*

```
GGTCGCAAGCATCAATAGTAATGGTGGTAGCACCTATTATCCAGACAGTGTGAAGGGCCGATTCACCATCTCCAGAGACAATGCCAAGAACACCCTGTAC
----+----+----+----+----+----+----+----+----+----+----+----+----+----+----+----+----+----+----+----+  50
CCAGCGTTCGTAGTTATCATTACCACCATCGTGGATAATAGGTCTGTCACACTTCCCGGCTAAGTGGTAGAGGTCTCTGTTACGGTTCTTGTGGGACATG
  V   A   S   I   N   S   N   G   G   S   T   Y   Y   P   D   S   V   K   G   R   F   T   I   S   R   D   N   A   K   N   T   L   Y
```
──────────────────────────────────────────────C2VHAF──────────────────────────────────────────────

CDR2 (arrow)

```
CTGCAAATGAGCAGTCTGAAGTCTGAGGACACAGCCATGTATTACTGTGCAAGTGGTGACTACTGGGGCCAAGGCTCCACTCTCACAGTCTCCTCAGGTG
----+----+----+----+----+----+----+----+----+----+----+----+----+----+----+----+----+----+----+----+  60
GACGTTTACTCGTCAGACTTCAGACTCCTGTGTCGGTACATAATGACACGTTCACCACTGATGACCCCGGTTCCGAGGTGAGAGTGTCAGAGGAGTCCAC
  L   Q   M   S   S   L   K   S   E   D   T   A   M   Y   Y   C   A   S   G   D   Y   W   G   Q   G   S   T   L   T   V   S   S
```
──────────────────────────────────────────────C2VHAF──────────────────────────────────────────────

CDR3 (arrow)

```
AGTCCTTACAACCTCTCTCTTCTATTCAGCTTAAATAGATTTTACTGCATTTGTTGGGGGGGAAATGTGTGTATCTGAATTTCAGGTCATGAAGGACTAG
----+----+----+----+----+----+----+----+----+----+----+----+----+----+----+----+----+----+----+----+  70
TCAGGAATGTTGGAGAGAGAAGATAAGTCGAATTTATCTAAAATGACGTAAACAACCCCCCCTTTACACACATAGACTTAAAGTCCAGTACTTCCTGATC
```

```
                      Xmal
                       |    Smal
                       |     |
GGACACCTTGGGAGTCAGAAAGGGTCATTGGGAGCCCGGGCTGATGCAGACAGACATCCTCAGCTCCCAGACTTCATGGCCAGAGATTTATAGGATCC
----+----+----+----+----+----+----+----+----+----+----+----+----+----+----+----+----+----+----+---  798
CCTGTGGAACCCTCAGTCTTTCCCAGTAACCCTCGGGCCCGACTACGTCTGTCTGTAGGAGTCGAGGGTCTGAAGTACCGGTCTCTAAATATCCTAGG
```
                                                                                        BamHI
                                                                                         |

## FIG. 4-2

```
                        10                  20                  30
C2VHAF    E  V  Q  L  V  E  S  G  G  G  L  V  Q  P  G  G  S  L  K  L  S  C  A  A  S  G  F  T  F  S
AF120466  E  V [K] L  V  E  S  G  G  G  L  V [K] P  G  G  S  L  K  L  S  C  A  A  S  G  F  T  F  S

                        40                  50                  60
C2VHAF    S  Y  G  M  S  W  V  R  Q  T  P  D  K  R  L  E  L  V  A  S  I  N  S  N  G  G  S  T  Y  Y
AF120466  S  Y  G  M  S  W  V  R  Q  T  P  D  K  R  L  E [W] V  A [T] I [S] S [G] G [S  Y] T  Y  Y

                        70                  80                  90
C2VHAF    P  D  S  V  K  G  R  F  T  I  S  R  D  N  A  K  N  T  L  Y  L  Q  M  S  S  L  K  S  E  D
AF120466  P  D  S  V  K  G  R  F  T  I  S  R  D  N  A  K  N  T  L  Y  L  Q  M  S  S  L  K  S  E  D

                        100                 110
C2VHAF    T  A  M  Y  Y  C  A  S  G  D  Y  W  G  Q  G  S  T  L  T  V  S  S
AF120466  T  A  M  Y  Y  C  A [R  R]
```

# FIG 5

A405

NEGATIVE CONTROL

CHIMERIC ch VHAF/CHVK

CHIMERIC AT/HuIgG4

CHIMERIC B HuIgG4

HuVHv2/HuVKv1

HuVHv2/HuVKv2

HuVHv2/HuVKv3

HuVHv2/HuVKv4

o  HuVHv4/HuVKv1

HuVHv4/HuVKv2

HuVHv4/HuVKv3

HuVHv4/HuVKv4

*FIG. 6*

*FIG. 7*

C. Antibody hC2 — A. Chimeric antibody

Control patient — Temporal neocortex region I, 40 x — CA4 (Cornu ammonis region 4), 40 x

AD patient — Temporal neocortex region I, 40 x — Temporal neocortex region I, 40 x

preAD patient — Temporal neocortex region I, 40 x — Temporal neocortex region I, 40 x

FIG. 8

FIG. 9A

| RESONANCE | PBS | | | MOUSE C2 | | |
|---|---|---|---|---|---|---|
| | δ ISO (ppm) | FWHH (Hz) | % INTEGRAL INTENSITY | δ ISO (ppm) | FWHH (Hz) | % INTEGRAL INTENSITY |
| Val Cβ -SHEET | 32.60 | 479 | 81.7 | 33.09 | 366 | 53.5 |
| Val Cβ -RANDOM | 30.27 | 200 | 18.3 | 30.27 | 340 | 46.5 |

FIG. 9B

EP 2 205 632 B1

FIG. 10

SOLUBLE POLYMERIC AMYLOID BETA
PREPARATION

PELLET PREPARATION

SUPERNATANT PREPARATION

*FIG. 11*

```
                              10                    20                    30
C2VK       D V V M T Q T P L S L P V S L G D Q A S I S C R S S Q S L V
C2HuVK1    D I V M T Q S P L S L P V T P G E P A S I S C R S S Q S L V
C2HuVK2    D I V M T Q S P L S L P V T P G E P A S I S C R S S Q S L V
C2HuVK3    D I V M T Q S P L S L P V T P G E P A S I S C R S S Q S L V
C2HuVK4    D I V M T Q S P L S L P V T P G E P A S I S C R S S Q S L V
dpk15      D I V M T Q S P L S L P V T P G E P A S I S C R S S Q S L L
JK1        - - - - - - - - - - - - - - - - - - - - - - - - - - - - -


                              40                    50                    60
C2VK       Y S N G D T Y L H W Y L Q K P G Q S P K L L I Y K V S N R F
C2HuVK1    Y S N G D T Y L H W Y L Q K P G Q S P Q L L I Y K V S N R F
C2HuVK2    Y S N G D T Y L H W Y L Q K P G Q S P Q L L I Y K V S N R F
C2HuVK3    Y S N G D T Y L H W Y L Q K P G Q S P K L L I Y K V S N R F
C2HuVK4    Y S N G D T Y L H W Y L Q K P G Q S P K L L I Y K V S N R F
dpk15      H S N G Y N Y L D W Y L Q K P G Q S P Q L L I Y L G S N R A
JK1        - - - - - - - - - - - - - - - - - - - - - - - - - - - - -


                              70                    80                    90
C2VK       S G V P D R F S G S G S G T D F T L K I S R V E A E D L G V
C2HuVK1    S G V P D R F S G S G S G T D F T L K I S R V E A E D V G V
C2HuVK2    S G V P D R F S G S G S G T D F T L K I S R V E A E D V G V
C2HuVK3    S G V P D R F S G S G S G T D F T L K I S R V E A E D V G V
C2HuVK4    S G V P D R F S G S G S G T D F T L K I S R V E A E D V G V
dpk15      S G V P D R F S G S G S G T D F T L K I S R V E A E D V G V
JK1        - - - - - - - - - - - - - - - - - - - - - - - - - - - - -


                              100                   110
C2VK       Y F C S Q S T H V P W T F G G G T K L E I K
C2HuVK1    Y Y C S Q S T H V P W T F G Q G T K V E I K
C2HuVK2    Y F C S Q S T H V P W T F G Q G T K V E I K
C2HuVK3    Y Y C S Q S T H V P W T F G Q G T K V E I K
C2HuVK4    Y F C S Q S T H V P W T F G Q G T K V E I K
dpk15      Y Y C M Q - - - - - - - - - - A - L Q T P
JK1        - - - - - - - - - - W T F G Q G T K V E I K
```

# FIG 12.

```
                         10                    20                    30
           ┌─────────────┼─────────────────────┼─────────────────────┼──┐
C2VHAF      E V Q L V E S G G G L V Q P G G S L K L S C A A S │G F T F S
C2HuVHAF1   E V Q L V E S G G G L V Q P G G S L │R│ L S C A A S │G F T F S
C2HuVHAF2   E V Q L V E S G G G L V Q P G G S L │R│ L S C A A S │G F T F S
C2HuVHAF3   E V Q L V E S G G G L V Q P G G S L │R│ L S C A A S │G F T F S
C2HuVHAF4   E V Q L V E S G G G L V Q P G G S L'R│ L S C A A S │G F T F S
DP-54       E V Q L V E S G G G L V Q P G G S L'R│ L S C A A S │G F T F S
HUJH6      │- - - - - - - - - - - - - - - - - - - - - - - - - │- - - - -│

                         40                    50                    60
                     ┌──────────────────────────┐
C2VHAF      S Y G M S │W V R Q T P D K R L E L V A│S I N S N G G S T Y Y
C2HuVHAF1   S Y G M S │W V R Q │A│ P│G│K│G│L E │W│V A│S I N S N G G S T Y Y
C2HuVHAF2   S Y G M S │W V R Q │A│ P│G│K│G│L E L V A│S I N S N G G S T Y Y
C2HuVHAF3   S Y G M S │W V R Q │A│ P│G│K│G│L E L V A│S I N S N G G S T Y Y
C2HuVHAF4   S Y G M S │W V R Q │A│ P│G│K│G│L E L V A│S I N S N G G S T Y Y
DP-54       S Y│W│M S │W V R Q │A│ P│G│K│G│L E │W│V A│N│I│K Q D│G│S E K│Y Y
HUJH6      │- -'- - - │- - - - - - - - - - - - - - - - - - - - - - - -│Y Y

                         70                    80                    90
C2VHAF      P D S V K G│R F T I S R D N A K N T L Y L Q M S S L K S E D
C2HuVHAF1   P D S V K G│R F T I S R D N A K N│S│L Y L Q M│N│S L│R A│E D
C2HuVHAF2   P D S V K G│R F T I S R D N A K N│S│L Y L Q M│N│S L│R A│E D
C2HuVHAF3   P D S V K G│R F T I S R D N A K N│S│L Y L Q M│N│S L│R A│E D
C2HuVHAF4   P D S V K G│R F T I S R D N A K N│S│L Y L Q M│N│S L│R A│E D
DP-54      │V│D S V K G│R F T I S R D N A K N│S│L Y L Q M│N│S L│R A'E D
HUJH6      │- - - - - - - - - - - - - - - - - - - - - - - - - - - - -│

                         100                   110
C2VHAF      T A M Y Y C A S│G D Y│W G Q G S T L T V S S
C2HuVHAF1   T A│V│Y Y C A│R│G D Y│W G Q G│T│T│V│T V S S
C2HuVHAF2   T A│V│Y Y C A S│G D Y│W G Q G│T│T│V│T V S S
C2HuVHAF3   T A│V│Y Y C A│R│G D Y│W G Q G│T│T│V│T V S S
C2HuVHAF4   T A│V│Y Y C A S│G D Y│W G Q G│T│T│V│T V S S
DP-54       T A│V│Y Y C A│R│
HUJH6      │- - -│Y Y│Y G M│-│D│V│W G Q G│T│T│V│T V S S
```

# FIG 13.

HindIII
|
AAGCTTATGAATATGCAAATCCTCTGAATCTACATGGTAAATATAGGTTTGTCTATACCACAAACAGAAAAACATGAGATCACAGTTCTCTCTACAGTTA
----+----+----+----+----+----+----+----+----+----+----+----+----+----+----+----+----+----+----+----+ 10
TTCGAATACTTATACGTTTAGGAGACTTAGATGTACCATTTATATCCAAACAGATATGGTGTTTGTCTTTTTGTACTCTAGTGTCAAGAGAGATGTCAAT

                    NcoI
                    |
CTGAGCACACAGGACCTCACCATGGGATGGAGCTGTATCATCCTCTTCTTGGTAGCAACAGCTACAGGTAAGGGGCTCACAGTAGCAGGCTTGAGGTCTG
----+----+----+----+----+----+----+----+----+----+----+----+----+----+----+----+----+----+----+----+ 20
GACTCGTGTGTCCTGGAGTGGTACCCTACCTCGACATAGTAGGAGAAGAACCATCGTTGTCGATGTCCATTCCCCGAGTGTCATCGTCCGAACTCCAGAC
                    M  G  W  S  C  I  I  L  F  L  V  A  T  A  T
                    └────────────────── SIGNAL ──────────────────┘

GACATATATATGGGTGACAATGACATCCACTTTGCCTTTCTCTCCACAGGTGTCCACTCCGATATTGTGATGACCCAATCTCCACTCTCCCTGCCTGTCA
----+----+----+----+----+----+----+----+----+----+----+----+----+----+----+----+----+----+----+----+ 30
CTGTATATATACCCACTGTTACTGTAGGTGAAACGGAAAGAGAGGTGTCCACAGGTGAGGCTATAACACTACTGGGTTAGAGGTGAGAGGGACGGACAGT
                              G  V  H  S  D  I  V  M  T  Q  S  P  L  S  L  P  V
                              └── SIG ──┴────────────── C2HuVKv1──────────────

            BglII                                                  KpnI  PstI
            |                                                      |     |
CTCCTGGTGAGCCTGCCTCCATCTCTTGCAGATCTAGTCAGAGCCTTGTATATAGTAATGGAGACACCTATTTACATTGGTACCTGCAGAAGCCAGGCCA
----+----+----+----+----+----+----+----+----+----+----+----+----+----+----+----+----+----+----+----+ 40
GAGGACCACTCGGACGGAGGTAGAGAACGTCTAGATCAGTCTCGGAACATATATCATTACCTCTGTGGATAAATGTAACCATGGACGTCTTCGGTCCGGT
 T  P  G  E  P  A  S  Q  S  C  R  S  S  Q  S  L  V  Y  S  N  G  D  T  Y  L  H  W  Y  L  Q  K  P  G  Q
────────────────────────────────────── C2HuVKv1 ──────────────────────────────────────
            └──────────────────────────── CDR1 ────────────────────────────────┘

*FIG. 14-1*

```
GTCTCCACAGCTCCTGATCTACAAAGTTTCCAACCGATTTTCTGGGGTCCCAGACAGGTTCAGTGGCAGTGGATCAGGGACAGATTTCACACTCAAGATC
----+----+----+----+----+----+----+----+----+----+----+----+----+----+----+----+----+----+----+----+ 50
CAGAGGTGTCGAGGACTAGATGTTTCAAAGGTTGGCTAAAAGACCCCAGGGTCTGTCCAAGTCACCGTCACCTAGTCCCTGTCTAAAGTGTGAGTTCTAG
   S   P   Q   L   L   I   Y   K   V   S   N   R   F   S   G   V   P   D   R   F   S   G   S   G   T   D   F   T   L   K   I
                                                              ———————— C2HuVKv1 ————————
```

CDR2

```
AGCAGAGTGGAGGCTGAGGATGTGGGAGTTTATTACTGCTCTCAAAGTACACATGTTCCTTGGACGTTCGGCCAAGGCACCAAGGTGGAAATCAAACGTG
----+----+----+----+----+----+----+----+----+----+----+----+----+----+----+----+----+----+----+----+ 60
TCGTCTCACCTCCGACTCCTACACCCTCAAATAATGACGAGAGTTTCATGTGTACAAGGAACCTGCAAGCCGGTTCCGTGGTTCCACCTTTAGTTTGCAC
   S   R   V   E   A   E   D   V   G   V   Y   Y   C   S   Q   S   T   H   V   P   W   T   F   G   Q   G   T   K   V   E   I   K   I
                                                              ———————— C2HuVKv1 ————————
```

CDR3

```
                              BamHI
                                I
AGTAGAATTTAAACTTTGCTTCCTCAGTTGGATCC
----+----+----+----+----+----+----+ 635
TCATCTTAAATTTGAAACGAAGGAGTCAACCTAGG
```

*FIG. 14-2*

EP 2 205 632 B1

EP 2 205 632 B1

```
BamHI                                                    BgIII
|                                                        |
GGATCCTGGCAGAGTCTCACAGATGCTTCTGAGACAACATTTGCTTTCAAAAAATGAACCACACACATCCTAAAGATCTCAGCCACTTCCCATGTTTCAT
----+----+----+----+----+----+----+----+----+----+----+----+----+----+----+----+----+----+----+----+ 100
CCTAGGACCGTCTCAGAGTGTCTACGAAGACTCTGTTGTAAACGAAAGTTTTTTACTTGGTGTGTGTAGGATTTCTAGAGTCGGTGAAGGGTACAAAGTA

TTTATGTTACAGCAAACATCACAACAATCATTCCTACAGATCACCACTGCATGTGATCAATAAAATAGTTTTTGCAACAATGGTACTTATGATAATCATC
----+----+----+----+----+----+----+----+----+----+----+----+----+----+----+----+----+----+----+----+ 200
AAATACAATGTCGTTTGTAGTGTTGTTAGTAAGGATGTCTAGTGGTGACGTACACTAGTTATTTTATCAAAAACGTTGTTACCATGAATACTATTAGTAG

TTTTATTGTTTACAAATACTGCTTTACAATAGTTATTCGGTTGCACTGTTCATATTAGATTTCCAATTAGCTCACTTAGGAACATAAGTCCCTCGAACAG
----+----+----+----+----+----+----+----+----+----+----+----+----+----+----+----+----+----+----+----+ 300
AAAATAACAAATGTTTATGACGAAATGTTATCAATAAGCCAACGTGACAAGTATAATCTAAAGGTTAATCGAGTGAATCCTTGTATTCAGGGAGCTTGTC

CTCAGTCATCTTTTTCATTCCTGTTTCTATCCCCTACATCTCTTTCCTTTGCAGACGACTATCTCCTACACTGAAACAGGAAAGCTAGCTTTTTTTTTTC
----+----+----+----+----+----+----+----+----+----+----+----+----+----+----+----+----+----+----+----+ 400
GAGTCAGTAGAAAAAGTAAGGACAAAGATAGGGGATGTAGAGAAAGGAAACGTCTGCTGATAGAGGATGTGACTTTGTCCTTTCGATCGAAAAAAAAAAG

AGTGCTATTTAATTATTTCAATATCCTCTCATCAAATGTATTTAAATAACAAAAGCTCAACCAAAAAGAAAGAAATATGTAATTCTTTCAGAGTAAAAAT
----+----+----+----+----+----+----+----+----+----+----+----+----+----+----+----+----+----+----+----+ 500
TCACGATAAATTAATAAAGTTATAGGAGAGTAGTTTACATAAATTTATTGTTTTCGAGTTGGTTTTTCTTTCTTTATACATTAAGAAAGTCTCATTTTTA

CACACCCATGACCTGGCCACTGAGGGCTTGATCAATTCACTTTGAATTTGGCATTAAATACCATTAAGGTATATTAACTGATTTTAAAATAAGATATATT
----+----+----+----+----+----+----+----+----+----+----+----+----+----+----+----+----+----+----+----+ 600
GTGTGGGTACTCGACCGGTGACTCCCGAACTAGTTAAGTGAAACTTAAACCGTAATTTATGGTAATTCCATATAATTGACTAAAATTTTATTCTATATAA

CGTGACCATGTTTTTAACTTTCAAAAATGTAGCTGCCAGTGTGTGATTTTATTTCAGTTGTACAAAATATCTAAACCTATAGCAATGTGATTAATAAAAA
----+----+----+----+----+----+----+----+----+----+----+----+----+----+----+----+----+----+----+----+ 700
GCACTGGTACAAAAATTGAAAGTTTTTACATCGACGGTCACACACTAAAATAAAGTCAACATGTTTTATAGATTTGGATATCGTTACACTAATTATTTTT
```

<div align="center">

## FIG. 15-1

</div>

EP 2 205 632 B1

```
CTTAAACATATTTTCCAGTACCTTAATTCTGTGATAGGAAAATTTTAATCTGAGTATTTTAATTTCATAATCTCTAAAATAGTTTAATGATTTGTCATTG
----+----+----+----+----+----+----+----+----+----+----+----+----+----+----+----+----+----+----+----+ 800
GAATTTGTATAAAAGGTCATGGAATTAAGACACTATCCTTTTAAAATTAGACTCATAAAATTAAAGTATTAGAGATTTTATCAAATTACTAAACAGTAAC
                        PvuII
                        |
TGTTGCTGTCGTTTACCCCAGCTGATCTCAAAAGTGATATTTAAGGAGATTATTTTGGTCTGCAACAACTTGATAGGACTATTTTAGGGCCTTTTTAAAG
----+----+----+----+----+----+----+----+----+----+----+----+----+----+----+----+----+----+----+----+ 900
ACAACGACAGCAAATGGGGTCGACTAGAGTTTTCACTATAAATTCCTCTAATAAAACCAGACGTTGTTGAACTATCCTGATAAAATCCCGGAAAAATTTC

CTCTATTAAAACTAACTTACAACGATTCAAAACTGTTTTAAACTATTTCAAAATGATTTTAGAGCCTTTTGAAAACTCTTTTAAACACTTTTTAAACTCT
----+----+----+----+----+----+----+----+----+----+----+----+----+----+----+----+----+----+----+----+ 1000
GAGATAATTTTGATTGAATGTTGCTAAGTTTTGACAAAATTTGATAAAGTTTTACTAAAATCTCGGAAAACTTTTGAGAAAATTTGTGAAAAATTTGAGA
                                                                                     EcoRI
                                                                                     |
ATTAAAACTAATAAGATAACTTGAAATAATTTTCATGTCAAATACATTAACTGTTTAATGTTTAAATGCCAGATGAAAAATGTAAAGCTATCAAGAATTC
----+----+----+----+----+----+----+----+----+----+----+----+----+----+----+----+----+----+----+----+ 1100
TAATTTTGATTATTCTATTGAACTTTATTAAAAGTACAGTTTATGTAATTGACAAATTACAAATTTACGGTCTACTTTTTACATTTCGATAGTTCTTAAG
                                                                          NcoI
                                                                          |
ACCCAGATAGGAGTATCTTCATAGCATGTTTTTCCCTGCTTATTTTCCAGTGATCACATTATTTTGCTACCATGGTTATTTTATACAATTATCTGAAAAA
----+----+----+----+----+----+----+----+----+----+----+----+----+----+----+----+----+----+----+----+ 1200
TGGGTCTATCCTCATAGAAGTATCGTACAAAAAGGGACGAATAAAAGGTCACTAGTGTAATAAAACGATGGTACCAATAAAATATGTTAATAGACTTTTT

AATTAGTTATGAAGATTAAAAGAGAAGAAAATATTAAACATAAGAGATTCAGTCTTTCATGTTGAACTGCTTGGTTAACAGTGAAGTTAGTTTTAAAAAA
----+----+----+----+----+----+----+----+----+----+----+----+----+----+----+----+----+----+----+----+ 1300
TTAATCAATACTTCTAATTTTCTCTTCTTTTATAATTTGTATTCTCTAAGTCAGAAAGTACAACTTGACGAACCAATTGTCACTTCAATCAAAATTTTT
                        PvuII
                        |
AAAAAAAACTATTTCTGTTATCAGCTGACTTCTCCCTATCTGTTGACTTCTCCCAGCAAAAGATTCTTATTTTACATTTTAACTACTGCTCTCCCACCCA
----+----+----+----+----+----+----+----+----+----+----+----+----+----+----+----+----+----+----+----+ 1400
TTTTTTTTGATAAAGACAATAGTCGACTGAAGAGGGATAGACAACTGAAGAGGGTCGTTTTCTAAGAATAAAATGTAAAATTGATGACGAGAGGGTGGGT
```

*FIG. 15-2*

```
ACGGGTGGAATCCCCCAGAGGGGGATTTCCAAGAGGCCACCTGGCAGTTGCTGAGGGTCAGAAGTGAAGCTAGCCACTTCCTCTTAGGCAGGTGGCCAAG
----+----+----+----+----+----+----+----+----+----+----+----+----+----+----+----+----+----+----+----+  150
TGCCCACCTTAGGGGGTCTCCCCCTAAAGGTTCTCCGGTGGACCGTCAACGACTCCCAGTCTTCACTTCGATCGGTGAAGGAGAATCCGTCCACCGGTTC


ATTACAGTTGACCTCTCCTGGTATGGCTGAAAATTGCTGCATATGGTTACAGGCCTTGAGGCCTTTGGGAGGGCTTAGAGAGTTGCTGGAACAGTCAGAA
----+----+----+----+----+----+----+----+----+----+----+----+----+----+----+----+----+----+----+----+  160
TAATGTCAACTGGAGAGGACCATACCGACTTTTAACGACGTATACCAATGTCCGGAACTCCGGAAACCCTCCCGAATCTCTCAACGACCTTGTCAGTCTT


                                            PstI                                         XmaI
                                            |                                            | SmaI
                                            |                                            | |
GGTGGAGGGGCTGACACCACCCAGGCGCAGAGGCAGGGCTCAGGGCCTGCTCTGCAGGGAGGTTTTAGCCCAGCCCAGCCAAAGTAACCCCCGGGAGCCT
----+----+----+----+----+----+----+----+----+----+----+----+----+----+----+----+----+----+----+----+  170
CCACCTCCCCGACTGTGGTGGGTCCGCGTCTCCGTCCCGAGTCCCGGACGAGACGTCCCTCCAAAATCGGGTCGGGTCGGTTTCATTGGGGGCCCTCGGA


                                                                          EcoRI
                                                                          |
GTTATCCCAGCACAGTCCTGGAAGAGGCACAGGGGAAATAAAAGCGGACGGAGGCTTTCCTTGACTCAGCCGCTGCCTGGTCTTCTTCAGACCTGTTCTG
----+----+----+----+----+----+----+----+----+----+----+----+----+----+----+----+----+----+----+----+  180
CAATAGGGTCGTGTCAGGACCTTCTCCGTGTCCCCTTTATTTTCGCCTGCCTCCGAAAGGAACTGAGTCGGCGACGGACCAGAAGAAGTCTGGACAAGAC
```

## FIG. 15-3

EP 2 205 632 B1

SphI

AATTCTAAACTCTGAGGGGGTCGGATGACGTGGCCATTCTTTGCCTAAAGCATTGAGTTTACTGCAAGGTCAGAAAAGCATGCAAAGCCCTCAGAATGGC
----+----+----+----+----+----+----+----+----+----+----+----+----+----+----+----+----+----+----+----+  190
TTAAGATTTGAGACTCCCCCAGCCTACTGCACCGGTAAGAAACGGATTTCGTAACTCAAATGACGTTCCAGTCTTTTCGTACGTTTCGGGAGTCTTACCG

SacI

TGCAAAGAGCTCCAACAAAACAATTTAGAACTTTATTAAGGAATAGGGGGAAGCTAGGAAGAAACTCAAAACATCAAGATTTTAAATACGCTTCTTGGTC
----+----+----+----+----+----+----+----+----+----+----+----+----+----+----+----+----+----+----+----+  200
ACGTTTCTCGAGGTTGTTTTGTTAAATCTTGAAATAATTCCTTATCCCCCTTCGATCCTTCTTTGAGTTTTGTAGTTCTAAAATTTATGCGAAGAACCAG

SphI

TCCTTGCTATAATTATCTGGGATAAGCATGCTGTTTTCTGTCTGTCCCTAACATGCCCTGTGATTATCCGCAAACAACACACCCAAGGGCAGAACTTTGT
----+----+----+----+----+----+----+----+----+----+----+----+----+----+----+----+----+----+----+----+  210
AGGAACGATATTAATAGACCCTATTCGTACGACAAAAGACAGACAGGGATTGTACGGGACACTAATAGGCGTTTGTTGTGTGGGTTCCCGTCTTGAAACA

BsgI

TACTTAAACACCATCCTGTTTGCTTCTTTCCTCAGGAACTGTGGCTGCACCATCTGTCTTCATCTTCCCGCCATCTGATGAGCAGTTGAAATCTGGAACT
----+----+----+----+----+----+----+----+----+----+----+----+----+----+----+----+----+----+----+----+  220
ATGAATTTGTGGTAGGACAAACGAAGAAAGGAGTCCTTGACACCGACGTGGTAGACAGAAGTAGAAGGGCGGTAGACTACTCGTCAACTTTAGACCTTGA

| SPLICE | T V A A P S V F I P P P S D E Q L K S G T |

HuCK

R

FIG. 15-4

FIG. 15-5

CCCTCCCATCCTTTGGCCTCTGACCCTTTTTCCACAGGGGACCTACCCCTATTGCGGTCCTCCAGCTCATCTTTCACCTCACCCCCCTCCTCCTCCTTGG
----+----+----+----+----+----+----+----+----+----+----+----+----+----+----+----+----+----+----+----+ 260
GGGAGGGTAGGAAACCGGAGACTGGGAAAAAGGTGTCCCCTGGATGGGGATAACGCCAGGAGGTCGAGTAGAAAGTGGAGTGGGGGGAGGAGGAGGAACC

CTTTAATTATGCTAATGTTGGAGGAGAATGAATAAATAAAGTGAATCTTTGCACCTGTGGTTTCTCTCTTTCCTCATTTAATAATTATTATCTGTTGTTT
----+----+----+----+----+----+----+----+----+----+----+----+----+----+----+----+----+----+----+----+ 270
GAAATTAATACGATTACAACCTCCTCTTACTTATTTATTTCACTTAGAAACGTGGACACCAAAGAGAGAAAGGAGTAAATTATTAATAATAGACAACAAA

TACCAACTACTCAATTTCTCTTATAAGGGACTAAATATGTAGTCATCCTAAGGCGCATAACCATTTATAAAAATCATCCTTCATTCTATTTTACCCTATC
----+----+----+----+----+----+----+----+----+----+----+----+----+----+----+----+----+----+----+----+ 280
ATGGTTGATGAGTTAAAGAGAATATTCCCTGATTTATACATCAGTAGGATTCCGCGTATTGGTAAATATTTTTAGTAGGAAGTAAGATAAAATGGGATAG

                                                                        NcoI
                                                                        I
ATCCTCTGCAATACAGTCCTCCCTCAAACCCACAAGCCTTCTGTCCTCACAGTCCCCTGGGCCATGGTAGGAGAGACTTGCTTCCTTGTTTTCCCCTCCT
----+----+----+----+----+----+----+----+----+----+----+----+----+----+----+----+----+----+----+----+ 290
TAGGAGACGTTATGTCAGGAGGGAGTTTGGGTGTTCGGAAGACAGGAGTGTCAGGGGACCCGGTACCATCCTCTCTGAACGAAGGAACAAAAGGGGAGGA

CAGCAAGCCCTCATAGTCCTTTTTAAGGGTGACAGGTCTTACAGTCATATATCCTTTGATTCAATTCCCTGAGAATCAACCAAAGCAAATTTTTCAAAAG
----+----+----+----+----+----+----+----+----+----+----+----+----+----+----+----+----+----+----+----+ 300
GTCGTTCGGGAGTATCAGGAAAAATTCCCACTGTCCAGAATGTCAGTATATAGGAAACTAAGTTAAGGGACTCTTAGTTGGTTTCGTTTAAAAAGTTTTC

AAGAAACCTGCTATAAAGAGAATCATTCATTGCAACATGATATAAAATAACAACACAATAAAAGCAATTAAATAAACAAACAATAGGGAAATGTTTAAGT
----+----+----+----+----+----+----+----+----+----+----+----+----+----+----+----+----+----+----+----+ 310
TTCTTTGGACGATATTTCTCTTAGTAAGTAACGTTGTACTATATTTTATTGTTGTGTTATTTTCGTTAATTTATTTGTTTGTTATCCCTTTACAAATTCA

TCATCATGGTACTTAGACTTAATGGAATGTCATGCCTTATTTACATTTTTAAACAGGTACTGAGGGACTCCTGTCTGCCAAGGGCCGTATTGAGTACTTT
----+----+----+----+----+----+----+----+----+----+----+----+----+----+----+----+----+----+----+----+ 320
AGTAGTACCATGAATCTGAATTACCTTACAGTACGGAATAAATGTAAAAATTTGTCCATGACTCCCTGAGGACAGACGGTTCCCGGCATAACTCATGAAA

CCACAACCTAATTTAATCCACACTATACTGTGAGATTAAAAACATTCATTAAAATGTTGCAAAGGTTCTATAAAGCTGAGAGACAAATATATTCTATAAC
----+----+----+----+----+----+----+----+----+----+----+----+----+----+----+----+----+----+----+----+ 330
GGTGTTGGATTAAATTAGGTGTGATATGACACTCTAATTTTTGTAAGTAATTTTACAACGTTTCCAAGATATTTCGACTCTCTGTTTATATAAGATATTG

*FIG. 15-6*

EP 2 205 632 B1

Xbal
|
TCAGCAATCCCACTTCTAGATGACTGAGTGTCCCCACCCACCAAAAAACTATGCAAGAATGTTCAAAGCAGCTTTATTTACAAAAGCCAAAAATTGGAAA
----+----+----+----+----+----+----+----+----+----+----+----+----+----+----+----+----+----+----+----+ 340
AGTCGTTAGGGTGAAGATCTACTGACTCACAGGGGTGGGTGGTTTTTTGATACGTTCTTACAAGTTTCGTCGAAATAAATGTTTTCGGTTTTTAACCTTT

TAGCCCGATTGTCCAACAATAGAATGAGTTATTAAACTGTGGTATGTTTATACATTAGAATACCCAATGAGGAGAATTAACAAGCTACAACTATACCTAC
----+----+----+----+----+----+----+----+----+----+----+----+----+----+----+----+----+----+----+----+ 350
ATCGGGCTAACAGGTTGTTATCTTACTCAATAATTTGACACCATACAAATATGTAATCTTATGGGTTACTCCTCTTAATTGTTCGATGTTGATATGGATG

TCACACAGATGAATCTCATAAAAATAATGTTACATAAGAGAAACTCAATGCAAAAGATATGTTCTGTATGTTTTCATCCATATAAAGTTCAAAACCAGGT
----+----+----+----+----+----+----+----+----+----+----+----+----+----+----+----+----+----+----+----+ 360
AGTGTGTCTACTTAGAGTATTTTTATTACAATGTATTCTCTTTGAGTTACGTTTTCTATACAAGACATACAAAAGTAGGTATATTTCAAGTTTTGGTCCA

AAAAATAAAGTTAGAAATTTGGATGGAAATTACTCTTAGCTGGGGGTGGGCGAGTTAGTGCCTGGGAGAAGACAAGAAGGGGCTTCTGGGGTCTTGGTAA
----+----+----+----+----+----+----+----+----+----+----+----+----+----+----+----+----+----+----+----+ 370
TTTTTATTTCAATCTTTAAACCTACCTTTAATGAGAATCGACCCCCACCCGCTCAATCACGGACCCTCTTCTGTTCTTCCCCGAAGACCCCAGAACCATT

Bsgl
|
TGTTCTGTTCCTCGTGTGGGGGTTGTGCAGTTATGATCTGTGCACTGTTCTGTATACACATTATGCTTCAAAATAACTTCACATAAAGAACATCTTATACC
----+----+----+----+----+----+----+----+----+----+----+----+----+----+----+----+----+----+----+----+ 380
ACAAGACAAGGAGCACACCCCAACACGTCAATACTAGACACGTGACAAGACATATGTGTAATACGAAGTTTTATTGAAGTGTATTTCTTGTAGAATATGG

Pvull
|
CAGTTAATAGATAGAAGAGGAATAAGTAATAGGTCAAGACCATGCAGCTGGTAAGTGGGGGGGCCTGGGATCAAATAGCTACCTGCCTAATCCTGCCCTC
----+----+----+----+----+----+----+----+----+----+----+----+----+----+----+----+----+----+----+----+ 390
GTCAATTATCTATCTTCTCCTTATTCATTATCCAGTTCTGGTACGTCGACCATTCACCCCCCCGGACCCTAGTTTATCGATGGACGGATTAGGACGGGAG

*FIG. 15-7*

```
TTGAGCCCTGAATGAGTCTGCCTTCCAGGGCTCAAGGTGCTCAACAAAACAACAGGCCTGCTATTTTCCTGGCATCTGTGCCCTGTTTGGCTAGCTAGGA
----+----+----+----+----+----+----+----+----+----+----+----+----+----+----+----+----+----+----+----+ 400
AACTCGGGACTTACTCAGACGGAAGGTCCCGAGTTCCACGAGTTGTTTTGTTGTCCGGACGATAAAAGGACCGTAGACACGGGACAAACCGATCGATCCT

GCACACATACATAGAAATTAAATGAAACAGACCTTCAGCAAGGGGACAGAGGACAGAATTAACCTTGCCCAGACACTGGAAACCCATGTATGAACACTCA
----+----+----+----+----+----+----+----+----+----+----+----+----+----+----+----+----+----+----+----+ 410
CGTGTGTATGTATCTTTAATTTACTTTGTCTGGAAGTCGTTCCCCTGTCTCCTGTCTTAATTGGAACGGGTCTGTGACCTTTGGGTACATACTTGTGAGT

CATGTTTGGGAAGGGGGAAGGGCACATGTAAATGAGGACTCTTCCTCATTCTATGGGGCACTCTGGCCCTGCCCCTCTCAGCTACTCATCCATCCAACAC
----+----+----+----+----+----+----+----+----+----+----+----+----+----+----+----+----+----+----+----+ 420
GTACAAACCCTTCCCCCTTCCCGTGTACATTTACTCCTGAGAAGGAGTAAGATACCCCGTGAGACCGGGACGGGGAGAGTCGATGAGTAGGTAGGTTGTG

                                       XmnI
                                         I
ACCTTTCTAAGTACCTCTCTCTGCCTACACTCTGAAGGGGTTCAGGAGTAACTAACACAGCATCCCTTCCCTCAAATGACTGACCATCCCTTTGTCCTGC
----+----+----+----+----+----+----+----+----+----+----+----+----+----+----+----+----+----+----+----+ 430
TGGAAAGATTCATGGAGAGAGACGGATGTGAGACTTCCCCAAGTCCTCATTGATTGTGTCGTAGGGAAGGGAGTTTACTGACTGGTAGGGAAACAGGACG

TTTGTTTTTCTTTCCAGTCAGTACTGGGAAAGTGGGGAAGGACAGTCATGGAAAAACTACATAAGGAAGCACCTTGCCCTTCTGCCTCTTGAGAATGTTG
----+----+----+----+----+----+----+----+----+----+----+----+----+----+----+----+----+----+----+----+ 440
AAACAAAAGAAAGGTCAGTCATGACCCTTTCACCCCTTCCTGTCAGTACCTTTTTGATGTATTCCTTCGTGGAACGGGAAGACGGAGAACTCTTACAAC

ATGAGTATCAAATCTTTCAAACTTTGGAGGTTTGAGTAGGGGTGAGACTCAGTAATGTCCCTTCCAATGACATGAACTTGCTCACTCATCCCTGGGGGCC
----+----+----+----+----+----+----+----+----+----+----+----+----+----+----+----+----+----+----+----+ 450
TACTCATAGTTTAGAAAGTTTGAAACCTCCAAACTCATCCCCACTCTGAGTCATTACAGGGAAGGTTACTGTACTTGAACGAGTGAGTAGGGACCCCCGG

                                       EcoRI
                                         I
AAATTGAACAATCAAAGGCAGGCATAATCCAGTTATGAATTCAAACCTTCTTCTCAGAAGATAACACTCTGAAGGGAAACCCACCCATAACCTAAGCAAG
----+----+----+----+----+----+----+----+----+----+----+----+----+----+----+----+----+----+----+----+ 460
TTTAACTTGTTAGTTTCCGTCCGTATTAGGTCAATACTTAAGTTTGGAAGAAGAGTCTTCTATTGTGAGACTTCCCTTTGGGTGGGTATTGGATTCGTTC

   PstI
     I
TGAAGACAGGTGCTGCAGGTGGAATTGTGTCCTTCAAAAAGGTATGCTCAACTCCTTGCTCTTGGTACTCATAAATGGGTCACATAAATGTGACTTTATT
----+----+----+----+----+----+----+----+----+----+----+----+----+----+----+----+----+----+----+----+ 470
ACTTCTGTCCACGACGTCCACCTTAACACAGGAAGTTTTTCCATACGAGTTGAGGAACGAGAACCATGAGTATTTACCCAGTGTATTTACACTGAAATAA
```

## FIG. 15-8

```
TGGAAATAGGGTCTTTGCAGAGGTAATCAAGTCAAAATTAGGTCATACTGAAATGTTTGTGAGGATGCGGTGAAAATGGATCATTCATATATTGCTGGTG
----+----+----+----+----+----+----+----+----+----+----+----+----+----+----+----+----+----+----+----+480
ACCTTTATCCCAGAAACGTCTCCATTAGTTCAGTTTTAATCCAGTATGACTTTACAAACACTCCTACGCCACTTTTACCTAGTAAGTATATAACGACCAC
```

**Xbal**
```
GGAATATAAAAGGGTATAGCTACTCTAGAAAATAGTTGTCAGTTTCTTGAAAAACTAAACAAAAGACACCTACCATATGACCCAGGAATTGTACTCCTTG
----+----+----+----+----+----+----+----+----+----+----+----+----+----+----+----+----+----+----+----+490
CCTTATATTTTCCCATATCGATGAGATCTTTTATCAACAGTCAAAGAACTTTTTGATTTGTTTTCTGTGGATGGTATACTGGGTCCTTAACATGAGGAAC
```

```
GGAATTTACCCCCAGGAAATAAAAACTTATGTCCACACAGAACCCATACATGATTGTTCACAGCAGCTTTATTTGTTGTAGCCAAAGCTAGAAAGAGCCA
----+----+----+----+----+----+----+----+----+----+----+----+----+----+----+----+----+----+----+----+500
CCTTAAATGGGGGTCCTTTATTTTTGAATACAGGTGTGTCTTGGGTATGTACTAACAAGTGTCGTCGAAATAAACAACATCGGTTTCGATCTTTCTCGGT
```

```
ACCCATCCCTCAATAGGCAACTAGCCTAACAAATTGTAATATATCCATGCCATAGAATGCTATGAGGCAATAAAAAGGAACGAAGTGTTCATACAGAGAA
----+----+----+----+----+----+----+----+----+----+----+----+----+----+----+----+----+----+----+----+510
TGGGTAGGGAGTTATCCGTTGATCGGATTGTTTAACATTATATAGGTACGGTATCTTACGATACTCCGTTATTTTTCCTTGCTTCACAAGTATGTCTCTT
```

```
CTGGAGTGATTCTGAAGGACTTTCTACTGAGTGAAAAAAGCCAATCTGAAAGGGTCACATACCATGTGATTCCTTTTATGTAACATTGTTGAAGTGACAA
----+----+----+----+----+----+----+----+----+----+----+----+----+----+----+----+----+----+----+----+520
GACCTCACTAAGACTTCCTGAAAGATGACTCACTTTTTTCGGTTAGACTTTCCCAGTGTATGGTACACTAAGGAAAATACATTGTAACAACTTCACTGTT
```

## FIG. 15-9

Xmnl
 ‖
AATTATAGGGATAGAGAACAGATTCTGGTTGCCAGGGGTTAGGGTGGTGGAGAAAGAAGAGTAGGCGAAACTATAAAGGGAGATCTTTGTGATCATGGGA
----+----+----+----+----+----+----+----+----+----+----+----+----+----+----+----+----+----+----+----+ 530
TTAATATCCCTATCTCTTGTCTAAGACCAACGGTCCCCAATCCCACCACCTCTTTCTTCTCATCCGCTTTGATATTTCCCTCTAGAAACACTAGTACCCT

                                        Xbal
                                         ‖
TAAATCTGTATCTTGATTGCAGTGGTAGTTGCAGGCATCTAGACATGTGATAAAATGACATAGAACTGTACACACTTATTTTATCAATGTCAAATTCTTG
----+----+----+----+----+----+----+----+----+----+----+----+----+----+----+----+----+----+----+----+ 540
ATTTAGACATAGAACTAACGTCACCATCAACGTCCGTAGATCTGTACACTATTTTACTGTATCTTGACATGTGTGAATAAAATAGTTACAGTTTAAGAAC

                                                               Bsgl
                                                                ‖
GTTTTAATATCGTACTGTAATTACGTAAGAAGTAACCAACAGGAGAAACTGGGTGCAGGACACATCAGACCTCTGTGCTTTATATCCTGTCTTTGCTACT
----+----+----+----+----+----+----+----+----+----+----+----+----+----+----+----+----+----+----+----+ 550
CAAAATTATAGCATGACATTAATGCATTCTTCATTGGTTGTCCTCTTTGACCCACGTCCTGTGTAGTCTGGAGACACGAAATATAGGACAGAAACGATGA

TTCTGTGAATCTATAATTATTTCCAAATAATTTTTTTAAACTTTTTTTTTATGCTGGATCG
----+----+----+----+----+----+----+----+----+----+----+----+- 5561
AAGACACTTAGATATTAATAAAGGTTTATTAAAAAAATTTGAAAAAAAAATACGACCTAGC

## FIG. 15-10

BamHI XbaI

```
GGATCCTCTAGATTGAGCTTTCTGGGGCAGGCCAGGCCTGACCTTGGCTGGGGGCAGGGAGGGGGCTAAGGTGACGCAGGTGGCGCCAGCCAGGTGCACA
----+----+----+----+----+----+----+----+----+----+----+----+----+----+----+----+----+----+----+----+  100
CCTAGGAGATCTAACTCGAAAGACCCCGTCCGGTCCGGACTGGAACCGACCCCCGTCCCTCCCCCGATTCCACTGCGTCCACCGCGGTCGGTCCACGTGT
```
                                                                                      Apat
                                                                                        I

```
CCCAATGCCCATGAGCCCAGACACTGGACCCTGCATGGACCATCGCGGATAGACAAGAACCGAGGGGCCTCTGCGCCCTGGGCCCAGCTCTGTCCCACAC
----+----+----+----+----+----+----+----+----+----+----+----+----+----+----+----+----+----+----+----+  200
GGGTTACGGGTACTCGGGTCTGTGACCTGGGACGTACCTGGTAGCGCCTATCTGTTCTTGGCTCCCCGGAGACGCGGGACCCGGGTCGAGACAGGGTGTG
```
                                                    Apzl
                                                     I

```
CGCGGTCACATGGCACCACCTCTCTTGCAGCTTCCACCAAGGGCCCATCCGTCTTCCCCCTGGCGCCCTGCTCCAGATCGACCTCCGAGAGCACAGCCGC
----+----+----+----+----+----+----+----+----+----+----+----+----+----+----+----+----+----+----+----+  300
GCGCCAGTGTACCGTGGTGGAGAGAACGTCGAAGGTGGTTCCCGGGTAGGCAGAAGGGGGACCGCGGGACGAGGTCTAGCTGGAGGCTCTCGTGTCGGCG
       A   S   T   K   G   P   S   V   F   P   L   A   P   C   S   R   S   T   S   E   S   T   A   A
       └────────────────────────────────────────CH1─────────────────────────────────────────────────
```
                Agal
                 I

```
CCTGGGCTGCCTGGTCAAGGACTACTTCCCCGAACCGGTGACGGTGTCGTGGAACTCAGGCGCCCTGACCAGCGGCGTGCACACCTTCCCGGCTGTCCTA
----+----+----+----+----+----+----+----+----+----+----+----+----+----+----+----+----+----+----+----+  400
GGACCCGACGGACCAGTTCCTGATGAACGGGCTTGGCCACTGCCACAGCACCTTGAGTCCGCGGGACTGGTCGCCGCACGTGTGGAAGGGCCGACAGGAT
   L   G   C   L   V   K   D   V   F   P   E   P   V   T   V   S   W   N   S   G   A   L   T   S   G   V   H   T   F   P   A   V   L
───────────────────────────────────────────────CH1───────────────────────────────────────────────────
```
                    BsEll
                     I

```
CAGTCCTCAGGACTCTACTCCCTCAGCAGCGTGGTGACCGTGCCCTCCAGCAGCTTGGGCACGAAGACCTACACCTGCAACGTAGATCACAAGCCCAGCA
----+----+----+----+----+----+----+----+----+----+----+----+----+----+----+----+----+----+----+----+  500
GTCAGGAGTCCTGAGATGAGGGAGTCGTCGCACCACTGGCACGGGAGGTCGTCGAACCCGTGCTTCTGGATGTGGACGTTGCATCTAGTGTTCGGGTCGT
   Q   S   S   G   L   Y   S   L   S   S   V   V   T   V   P   S   S   S   L   G   T   K   T   Y   T   C   N   V   D   H   K   P   S
───────────────────────────────────────────────CH1───────────────────────────────────────────────────
```

## FIG. 16-1

```
ACACCAAGGTGGACAAGAGAGTTGGTGAGAGGCCAGCACAGGGAGGGAGGGTGTCTGCTGGAAGCCAGGCTCAGCCCTCCTGCCTGGACGCACCCCGGCT
----+----+----+----+----+----+----+----+----+----+----+----+----+----+----+----+----+----+----+----+
TGTGGTTCCACCTGTTCTCTCAACCACTCTCCGGTCGTGTCCCTCCCTCCCACAGACGACCTTCGGTCCGAGTCGGGAGGACGGACCTGCGTGGGGCCGA
N   T   K   V   D   K   R   V
———————————CH1———————————⌐
```

```
                                        SphI
                                          |
GTGCAGCCCCAGCCCAGGGCAGCAAGGCATGCCCCATCTGTCTCCTCACCCGGAGGCCTCTGACCACCCCACTCATGCTCAGGGAGAGGGTCTTCTGGAT
----+----+----+----+----+----+----+----+----+----+----+----+----+----+----+----+----+----+----+----+
CACGTCGGGGTCGGGTCCCGTCGTTCCGTACGGGGTAGACAGAGGAGTGGGCCTCCGGAGACTGGTGGGGTGAGTACGAGTCCCTCTCCCAGAAGACCTA
```

```
TTTTCCACCAGGCTCCGGGCAGCCACAGGCTGGATGCCCCTACCCCAGGCCCTGCGCATACAGGGGCAGGTGCTGCGCTCAGACCTGCCAAGAGCCATAT
----+----+----+----+----+----+----+----+----+----+----+----+----+----+----+----+----+----+----+----+
AAAAGGTGGTCCGAGGCCCGTCGGTCTCCGACCTACGGGGATGGGGTCCGGGACGCGTATGTCCCCGTCCACGACGCGAGTCTGGACGGTTCTCGGTATA
```

```
                                                                                        ClaI
                                                                                          |
CCGGGAGGACCCTGCCCCTGACCTAAGCCCACCCCAAAGGCCAAACTCTCCACTCCCTCAGCTCAGACACCTTCTCTCCTCCCAGATCGATCTGAGTAAC
----+----+----+----+----+----+----+----+----+----+----+----+----+----+----+----+----+----+----+----+
GGCCCTCCTGGGACGGGGACTGGATTCGGGTGGGGTTTCCGGTTTGAGAGGTGAGGGAGTCGAGTCTGTGGAAGAGAGGAGGGTCTAGCTAGACTCATTG
```

```
        PstI
          |
TCCCAATCTTCTCTCTGCAGAGTCCAAATATGGTCCCCCGTGTCCCCCATGCCCAGGTAAGCCAACCCAGGCCTCGCCCTCCAGCTCAAGGCGGGACAGG
----+----+----+----+----+----+----+----+----+----+----+----+----+----+----+----+----+----+----+----+
AGGGTTAGAAGAGAGACGTCTCAGGTTTATACCAGGGGGCACAGGGGGTACGGGTCCATTCGGTTGGGTCCGGAGCGGGAGGTCGAGTTCCGCCCTGTCC
        ⌐E   S   K   Y   G   P   P   C   P   P   C   P⌐
        L———————————————HINGE———————————————L
```

*FIG. 16-2*

```
TGCCCTAGAGTAGCCTGCATCCAGGGACAGGCCCCAGCCGGGTGCTGACGCATCCACCTCCATCTCTTCCTCAGCACCTGAGTTCCTGGGGGGACCATCA
----+----+----+----+----+----+----+----+----+----+----+----+----+----+----+----+----+----+----+----+ 110
ACGGGATCTCATCGGACGTAGGTCCCTGTCCGGGGTCGGCCCACGACTGCGTAGGTGGAGGTAGAGAAGGAGTCGTGGACTCAAGGACCCCCCTGGTAGT
                                                        A  P  E  F  L  G  G  P  S
                                                        ⌐――――――――――――CH2――――――――――


GTCTTCCTGTTCCCCCCAAAACCCAAGGACACTCTCATGATCTCCCGGACCCCTGAGGTCACGTGCGTGGTGGTGGACGTGAGCCAGGAAGACCCCGAGG
----+----+----+----+----+----+----+----+----+----+----+----+----+----+----+----+----+----+----+----+ 120
CAGAAGGACAAGGGGGGGTTTTGGGTTCCTGTGAGAGTACTAGAGGGCCTGGGGACTCCAGTGCACGCACCACCACCTGCACTCGGTCCTTCTCGGGCTCC
 V  F  L  P  P  P  K  P  K  D  T  L  M  I  S  R  T  P  E  V  T  C  V  V  V  D  V  S  Q  E  D  P  E
――――――――――――――――――――――――――――――――――――――――――CH2――――――――――――――――――――――――――――――――――――――――――――――――――――――――


TCCAGTTCAACTGGTACGTGGATGGCGTGGAGGTGCATAATGCCAAGACAAAGCCGCGGGAGGAGCAGTTCAACAGCACGTACCGTGTGGTCAGCGTCCT
----+----+----+----+----+----+----+----+----+----+----+----+----+----+----+----+----+----+----+----+ 130
AGGTCAAGTTGACCATGCACCTACCGCACCTCCACGTATTACGGTTCTGTTTCGGCGCCCTCCTCGTCAAGTTGTCGTGCATGGCACACCAGTCGCAGGA
 V  Q  F  N  W  Y  V  D  G  V  E  V  H  N  A  K  T  K  P  R  E  E  Q  F  N  S  T  Y  R  V  V  S  V  L
――――――――――――――――――――――――――――――――――――――――――CH2――――――――――――――――――――――――――――――――――――――――――――――――――――――――


CACCGTCCTGCACCAGGACTGGCTGAACGGCAAGGAGTACAAGTGCAAGGTCTCCAACAAAGGCCTCCCGTCCTCCATCGAGAAAACCATCTCCAAAGCC
----+----+----+----+----+----+----+----+----+----+----+----+----+----+----+----+----+----+----+----+ 140
GTGGCAGGACGTGGTCCTGACCGACTTGCCGTTCCTCATGTTCACGTTCCAGAGGTTGTTTCCGGAGGGCAGGAGGTAGCTCTTTTGGTAGAGGTTTCGG
  T  V  L  H  Q  D  W  L  N  G  K  E  Y  K  C  K  V  S  N  K  G  L  P  S  S  I  E  K  T  I  S  K  A
――――――――――――――――――――――――――――――――――――――――――CH2――――――――――――――――――――――――――――――――――――――――――――――――――――――――


AAAGGTGGGACCCACGGGGGTGCGAGGGCCACATGGACAGAGGTCAGCTCGGCCCACCCTCTGCCCTGGGAGTGACCGCTGTGCCAACCTCTGTCCCTACA
----+----+----+----+----+----+----+----+----+----+----+----+----+----+----+----+----+----+----+----+ 150
TTTCCACCCTGGGTGCCCCACGCTCCCGGTGTACCTGTCTCCAGTCGAGCCGGGTGGGAGACGGGACCCTCACTGGCGACACGGTTGGAGACAGGGATGT
 K
⌐CH⌐


GGGCAGCCCCGAGAGCCACAGGTGTACACCCTGCCCCCCATCCCAGGAGGAGATGACCAAGAACCAGGTCAGCCTGACCTGCCTGGTCAAAGGCTTCTACC
----+----+----+----+----+----+----+----+----+----+----+----+----+----+----+----+----+----+----+----+ 160
CCCGTCGGGGCTCTCGGTGTCCACATGTGGGACGGGGGTAGGGTCCTCCTCTACTGGTTCTTGGTCCAGTCGGACTGGACGCACCAGTTTCCGAAGATGG
 G  Q  P  R  E  P  Q  V  Y  T  L  P  P  S  Q  E  E  M  T  K  N  Q  V  S  L  T  C  L  V  K  G  F  Y
⌐――――――――――――――――――――――――――――――――――――――――――CH3――――――――――――――――――――――――――――――――――――――――――――――――――――――――
```

## FIG. 16-3

EP 2 205 632 B1

EP 2 205 632 B1

```
CCAGCGACATCGCCGTGGAGTGGGAGAGCAATGGGCAGCCGGAGAACAACTACAAGACCACGCCTCCCGTCCTCGATTCCGACGGCTCCTTCTTCCTCTA
----+----+----+----+----+----+----+----+----+----+----+----+----+----+----+----+----+----+----+----+   170
GGTCGCTGTAGCGGCACCTCACCCTCTCGTTACCCGTCGGCCTCTTGTTGATGTTCTGGTGCGGAGGGCAGGAGCTAAGGCTGCCGAGGAAGAAGGAGAT
 P  S  D  I  A  V  E  W  E  S  N  G  Q  P  E  N  N  Y  K  T  T  P  P  V  L  D  S  D  G  S  F  F  L  Y
                                              ————————————————————CH3————————————————————————————————————
```

                                              ·Xmnl
                                               |

```
CAGCAGGCTAACCGTGGACAAGAGCAGGTGGCAGGAGGGGAATGTCTTCTCATGCTCCGTGATGCATGAGGCTCTGCACAACCACTACACACAGAAGAGC
----+----+----+----+----+----+----+----+----+----+----+----+----+----+----+----+----+----+----+----+   180
GTCGTCCGATTGGCACCTGTTCTCGTCCACCGTCCTCCCCTTACAGAAGAGTACGAGGCACTACGTACTCCGAGACGTGTTGGTGATGTGTGTCTTCTCG
  S  R  L  T  V  D  K  S  R  W  Q  E  G  N  V  F  S  C  S  V  M  H  E  A  L  H  N  H  Y  T  Q  K  S
                                              ————————————————————CH3————————————————————————————————————
```

                                       Xmal  Smal
                                       | |   | |

```
CTCTCCCTGTCTCTGGGTAAATGAGTGCCAGGGCCGGCAAGCCCCCGCTCCCCGGGCTCTCGGGGTCGCGCGAGGATGCTTGGCACGTACCCCGTCTACA
----+----+----+----+----+----+----+----+----+----+----+----+----+----+----+----+----+----+----+----+   190
GAGAGGGACAGAGACCCATTTACTCACGGTCCCGGCCGTTCGGGGGCGAGGGGCCCGAGAGCCCCAGCCCGCTCCTACGAACCGTGCATGGGGCAGATGT
 L  S  L  S  L  G  K
 ———————————CH3—————————┘
```

                                            Apal
                                            |

```
TACTTCCCAGGCACCCAGCATGGAAATAAAGCACCCACCACTGCCCTGGGCCCCTGTGAGACTGTGATGGTTCTTTCCACGGGTCAGGCCGAGTCTGAGG
----+----+----+----+----+----+----+----+----+----+----+----+----+----+----+----+----+----+----+----+   200
ATGAAGGGTCCGTGGGTCGTACCTTTATTTCGTGGGTGGTGACGGGACCCGGGGACACTCTGACACTACCAAGAAAGGTGCCCAGTCCGGCTCAGACTCC
```

```
CCTGAGTGACATGAGGGAGGCAGATCC
----+----+----+----+----+--   2027
GGACTCACTGTACTCCCTCCGTCTAGG
```

⇨

## FIG. 16-4

EPITOPE MAPPING hC2_NC_20060505

FIG. 17A

EPITOPE MAPPING hC2_NC_20060519

FIG. 17B

EPITOPE MAPPING hC2_NC_20060925-26

FIG. 17C

EP 2 205 632 B1

**Fig 18**

**FIG 19**

FIG 20

133

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 60960617 B
- WO 20070688412 A
- WO 2006039327 A
- US 6737056 B1
- WO 2008011348 A
- WO 2008060364 A
- WO 2008156622 A
- WO 2004058258 A
- US 5091513 A
- US 5132405 A
- US 4956778 A
- US 5843708 A
- US 6180370 B
- US 5693762 A
- US 5585089 A
- US 5530101 A
- US 5565332 A
- US 5639641 A
- US 6632976 B
- US 5500362 A
- US 5821337 A
- EP 05027092 A
- US 6737056 B, Presta
- EP 1071700 A
- US 2005272128 A
- US 20020038086 A
- US 5112596 A
- US 5268164 A
- US 5506206 A
- US 5686416 A
- US 20030083299 A
- US 20020025313 A
- US 20040204354 A
- US 20040131692 A
- US 20020065259 A
- US 20030162695 A
- US 20050124533 A
- US 20050089473 A
- US 20030073713 A
- US 20030129186 A
- US 5004697 A
- WO 9613590 A, Maertens
- WO 9629605 A
- US 0896670156 B

**Non-patent literature cited in the description**

- *Experimental Eye Research,* 2004, vol. 78, 243-256
- **GLENNER ; WONG.** *Biochem Biophys Res Comm,* 1984, vol. 129, 885-890
- Fundamental Immunology. Raven Press, 1993
- **KHAW, B. A. et al.** *J. Nucl. Med.,* 1982, vol. 23, 1011-1019
- **ROUSSEAUX et al.** Methods Enzymology. Academic Press, 1986, vol. 121, 663-69
- **HUSTON et al.** *Proc. Nat. Acad. Sci. USA,* 1988, vol. 85, 5879-5883
- **E. KABAT et al.** Sequences of Proteins of Immunological Interest. U.S. Department of Health and Human Services
- **JOHNSON, G ; WU, TT.** Kabat Database and its applications: future directions. *Nucleic Acids Research,* 2001, vol. 29, 205-206, http://immuno.bme.nwa.edu
- **CHOTHIA ; LESK.** *J. Mol. Biol.,* 1987, vol. 196, 901
- **CHOTHIA et al.** *Nature,* 1989, vol. 342, 877
- **TRAMONTANO et al.** *J. Mol. Biol.,* 1990, vol. 215, 175
- **MACALLUM et al.** Antibody-antigen interactions: contact analysis and binding site topography. *J Mol Biol.,* 11 October 1996, vol. 262 (5), 732-45
- **RIECHMANN et al.** *Nature,* 1988, vol. 332, 323-327
- **RADER et al.** *Proc. Natl. Acad. Sci. USA,* 1998, vol. 95, 8910-8915
- **QUEEN et al.** *Proc. Natl Acad Sci USA,* 1989, vol. 86, 10029-10032
- **HODGSON et al.** *Bio/Technology,* 1991, vol. 9, 421
- **HEITER et al.** *Cell,* 1980, vol. 22, 197-207
- **ELLMAN et al.** *Meth. Enzym.,* 1991, vol. 202, 301-336
- **NOREN et al.** *Science,* 1989, vol. 244, 182
- **RAVETCH ; KINET.** *Annu. Rev. Immunol,* 1991, vol. 9, 457-92
- **CLYNES et al.** *PNAS (USA),* 1998, vol. 95, 652-656

- **GAZZANO-SANTORO et al.** *J. Immunol. Methods,* 1996, vol. 202, 163
- **SMITH ; WATERMAN.** *Advances in Applied Mathematics,* 1981, vol. 2, 482-489
- **W.R. PEARSON.** *Methods in Enzymology,* 1990, vol. 183, 63-98
- **SONGSIVILAI ; LACHMANN.** *Clin. Exp. Immunol.,* 1990, vol. 79, 315-321
- **KOSTELNY et al.** *J. Immunol.,* 1992, vol. 148, 1547-1553
- **FOOTE ; WINTER.** *J. Mol. Biol.,* 1992, vol. 224, 487-499
- **BERZOFSKY.** *Science,* 1985, vol. 229, 932-940
- **BOWIE et al.** *Science,* 1990, vol. 247, 1306-1310
- **WASSERMAN et al.** *J. Immunol.,* 1961, vol. 87, 290-295
- **LEVINE et al.** *Meth. Enzymol.,* 1967, vol. 11, 928-936
- **LEWIS et al.** *Biochem.,* 1983, vol. 22, 948-954
- **KABAT et al.** Sequence of Proteins of Immunological Interest. The United States Department of Health and Human Services, The United States Government Printing Office, 1991
- **CHOTHIA et al.** *Nature,* 1989, vol. 342, 877-883
- **CHOTHIA et al.** *J. Mol. Biol.,* 1985, vol. 186, 651-663
- **CARTER et al.** *Nucleic Acids Res.,* 1985, vol. 13, 4431-4443
- **KUNKEL et al.** *Proc. Natl. Acad. Sci. USA,* 1987, vol. 82, 488
- **HIGUCHI.** PCR Protocols. Academic Press, 1990, 177-183
- **VALLETTE et al.** *Nuc. Acids Res.,* 1989, vol. 17, 723-733
- **WELLS et al.** *Gene,* 1985, vol. 34, 315-323
- **FERRARA et al.** *J Biol Chem,* 2006, vol. 281 (8), 5032-5036
- **FERRARA et al.** *Biotechnology and Bioengineering,* 2006, vol. 93 (5), 851-861
- **PAPANASTASSIOU et al.** *Gene Therapy,* 2002, vol. 9, 398-406
- **GILL et al.** *Nature Med.,* 2003, vol. 9, 589-595
- **NEUWELT, E. A.** Implication of the Blood-Brain Barrier and its Manipulation. Plenum Press, 1989, vol. 1 & 2
- **HARLOW ; LANE.** Antibodies: A Laboratory Manual. Cold Spring Harbor Laboratory, 1988, 555-612
- **KENNEDY, J. H. et al.** *Clin. Chim. Acta,* 1976, vol. 70, 1-31
- **SCHURS, A. H. W. M. et al.** *Clin. Chim Acta,* 1977, vol. 81, 1-40
- **BARGHORN S ; NIMMRICH V ; STRIEBINGER A ; KRANTZ C ; KELLER P ; JANSON B ; BAHR M ; SCHMIDT M ; BITNER RS ; HARLAN J.** Globular amyloid beta-peptide oligomer - a homogenous and stable neuropathological protein in Alzheimer's disease. *J Neurochem,* 2005, vol. 95, 834-847
- **BLOND ; GOLDBERG.** *PNAS,* 01 March 1987, vol. 84 (5), 1147-1151
- **COX JPL ; TOMLINSON IM ; WINTER G.** A directory of human germ-line Vκ segments reveals a strong bias in their usage. *Eur. J. Immunol.,* 1994, vol. 24, 827-836
- **KABAT EA ; WU TT ; PERRY HM ; GOTTESMAN KS ; FOELLER C.** Sequences of proteins of Immunological Interest. US Department of Health and Human Services, 1991
- **KLEIN WL.** Abeta toxicity in Alzheimer's disease: globular soluble polymeric amyloid beta (ADDLs) as new vaccine and drug targets. *Neurochem Int,* 2002, vol. 41 (5), 345-352
- **LANGDON SD ; INAIOKI M ; KELSOE G ; TEDDER TF.** Germline sequences of V(H)7183 gene family members in C57BL/6 mice demonstrate natural selection of particular sequences during recent evolution. *Immunogenetics,* 2000, vol. 51, 241-245
- **MULLIGAN RC ; BERG P.** Expression of a bacterial gene in mammalian cells. *Science,* 1980, vol. 209, 1422-1427
- **RIECHMANN L ; CLARK M ; WALDMANN H ; WINTER G.** Reshaping human antibodies for therapy. *Nature,* 1988, vol. 332, 323-327
- **SCHABLE KF ; THIEBE R ; BENSCH A ; BRENSING-KUEPPERS J ; HEIM V ; KIRSCHBAUM T ; LAMM R ; OHNRICH M ; POURRAJABI S ; ROSCHENTHALER F.** Characteristics of the immunoglobulin V kappa genes, pseudogenes, relics and orphons in the mouse genome. *Eur. J. Immunol.,* 1999, vol. 29, 2082-2086
- **TOMLINSON IM ; WALTER G ; MARKS JD ; LLEWELYN MB ; WINTER G.** The repertoire of human germline VH sequences reveals about 50 groups of VH segments with different hypervariable loops. *J. Mol. Biol.,* 1992, vol. 227, 776-798